(19) Europäisches Patentamt
European Patent Office
Office européen des brevets

(11) EP 4 163 278 A1

(12) **EUROPEAN PATENT APPLICATION**
published in accordance with Art. 153(4) EPC

(43) Date of publication:
**12.04.2023 Bulletin 2023/15**

(21) Application number: **21811893.3**

(22) Date of filing: **28.05.2021**

(51) International Patent Classification (IPC):
***C07D 403/00*** *(2006.01)* ***C07D 403/14*** *(2006.01)*
***C07D 405/14*** *(2006.01)* ***C07D 417/14*** *(2006.01)*
***C07D 401/14*** *(2006.01)* ***C07D 413/00*** *(2006.01)*
***A61K 31/506*** *(2006.01)* ***A61P 35/00*** *(2006.01)*

(52) Cooperative Patent Classification (CPC):
**A61K 31/506; A61P 35/00; C07D 401/14; C07D 403/00; C07D 403/14; C07D 405/14; C07D 413/00; C07D 417/14;** Y02E 10/549

(86) International application number:
**PCT/CN2021/096942**

(87) International publication number:
**WO 2021/239133 (02.12.2021 Gazette 2021/48)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.05.2020 CN 202010471712**
**04.01.2021 CN 202110003579**

(71) Applicant: **Nanjing Chia Tai Tianqing Pharmaceutical Co., Ltd.**
**Jiangsu 210046 (CN)**

(72) Inventors:
- **MA, Changyou**
  **NANJING, Jiangsu 210046 (CN)**
- **ZHANG, Linlin**
  **NANJING, Jiangsu 210046 (CN)**
- **LI, Dongdong**
  **NANJING, Jiangsu 210046 (CN)**
- **WU, Youzhi**
  **NANJING, Jiangsu 210046 (CN)**
- **FENG, Haiwei**
  **NANJING, Jiangsu 210046 (CN)**
- **ZHOU, Qiuhua**
  **NANJING, Jiangsu 210046 (CN)**
- **PEI, Junjie**
  **NANJING, Jiangsu 210046 (CN)**
- **WU, Jian**
  **NANJING, Jiangsu 210046 (CN)**
- **XU, Dan**
  **NANJING, Jiangsu 210046 (CN)**
- **ZHU, Chunxia**
  **NANJING, Jiangsu 210046 (CN)**
- **TIAN, Zhoushan**
  **NANJING, Jiangsu 210046 (CN)**

(74) Representative: **Elzaburu S.L.P.**
**Edificio Torre de Cristal**
**Paseo de la Castellana 259 C, planta 28**
**28046 Madrid (ES)**

# (54) PYRIMIDINE COMPOUND AS AXL INHIBITOR

(57) Disclosed in the present invention is a pyrimidine compound as an AXL inhibitor. The structure of the pyrimidine compound is as shown in general formula I, and the definition of each substituent is as described in the description. The present invention further provides a preparation method for the pyrimidine compound. The pyrimidine compound of the present invention has significant AXL inhibitory activity, and can be used as an AXL inhibitor.

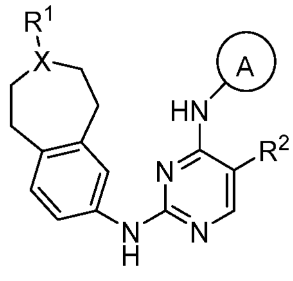

I

## Description

The present application claims the priority to Chinese invention patent application No. 202010471712.7 filed with China National Intellectual Property Administration on May 29, 2020, and Chinese invention patent application No. 202110003579.7 filed with China National Intellectual Property Administration on January 5, 2021, the content of which are incorporated herein by reference in their entireties.

## TECHNICAL FIELD

The present invention belongs to the field of medicine, and particularly relates to a pyrimidine compound, which is an AXL kinase inhibitor. The present invention also relates to use of the compound to treat diseases related to an AXL activity.

## BACKGROUND

Receptor tyrosine kinase (RTK) is a multi-domain transmembrane protein that can be used as a sensor for an extracellular ligand. Ligand and receptor binding induces receptor dimerization and activation of its intracellular kinase domain, which in turn leads to the recruitment, phosphorylation and activation of a plurality of downstream signaling cascade reaction (Robinson, DR, et al., Oncogene, 19:5548-5557, 2000). So far, 58 RTKs, which have been identified from the human genome, can regulate a variety of cellular processes, including cell survival, growth, differentiation, proliferation, adhesion and movement (Segaliny, A.I., et al., J. Bone Oncol, 4:1-12, 2015).

AXL (also known as UFO, ARK and Tyro7) belongs to a TAM family of receptor tyrosine kinases, which also includes Mer and Tyro3. Among them, AXL and Tyro3 have the most similar gene structures, while AXL and Mer have the most similar tyrosine kinase domain amino acid sequences. Like other receptor tyrosine kinases (RTKs), the structure of the TAM family includes an extracellular domain, a transmembrane domain, and a conserved intracellular kinase domain. The extracellular domain of AXL has a unique structure in which immunoglobulin and type III fibronectin repeat units are juxtaposed, and which is reminiscent of the structure of neutrophil adhesion molecules. Members of the TAM family have a common ligand - Growth arrest specific protein 6 (Gas6), which can bind to all TAM receptor tyrosine kinases. After binding to Gas6, AXL will cause receptor dimerization and AXL autophosphorylation, thereby activating a plurality of downstream signal transduction pathways, and participating in a plurality of processes of tumorigenesis (Linger, RM, et al., Ther. Targets, 14 (10), 1073-1090, 2010; Rescigno, J. et al., Oncogene, 6(10), 1909-1913, 1991).

AXL is widely expressed in normal human tissues, such as monocytes, macrophages, platelets, endothelial cells, cerebellum, heart, skeletal muscles, liver, and kidney. AXL has the highest expression in myocardium and skeletal muscles, has relatively high expression in bone marrow CD34+ cells and stromal cells and has a very low expression in normal lymphoid tissues (Wu YM, Robinson DR, Kung HJ, Cancer Res, 64(20), 7311-7320, 2004; hung BI, et al., DNA Cell Biol, 22(8), 533-540, 2003). In the study of many cancer cells, it is found that AXL genes are overexpressed or ectopically expressed in hematopoietic cells, interstitial cells and endothelial cells. In various types of leukemia and most solid tumors, the overexpression of AXL kinase is particularly prominent. By inhibiting the AXL receptor tyrosine kinases, pro-survival signals of tumor cells can be reduced, the invasion ability of tumors can be blocked, and the sensitivity of targeted drug therapy and chemotherapy can be increased. Therefore, finding an effective AXL inhibitor is an important direction of current tumor-targeted drug research and development.

## SUMMARY OF THE INVENTION

In an aspect, the present invention provides a pyrimidine compound of Formula I, or a pharmaceutically acceptable salt thereof,

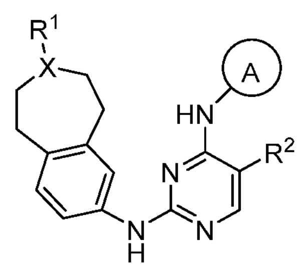

I

wherein X is CH or N;

$R^1$ is a 5-12 membered saturated heterocyclic ring or a 5-8 membered saturated carbocyclic ring, which is optionally substituted by one or more of $C_{1-6}$ alkyl, $C_{1-6}$ alkoxyl, halogen, cyano, deuterium or hydroxyl, and $R^1$ is not

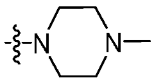 , 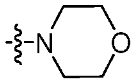 and 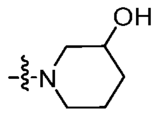 ;

$R^2$ is halogen;

ring A is selected from phenyl, 5-6 membered heteroaryl or 9-12 membered benzoheterocyclyl, wherein phenyl and 5-6 membered heteroaryl are optionally substituted by one or more $R^3$, and 9-12 membered benzoheterocyclyl is optionally substituted by

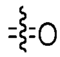

or one or more $R^3$;

$R^3$ is selected from: deuterium, halogen, $C_{1-6}$ alkyl, $C_{1-6}$ alkoxyl, $C_{3-10}$ cycloalkyloxyl,

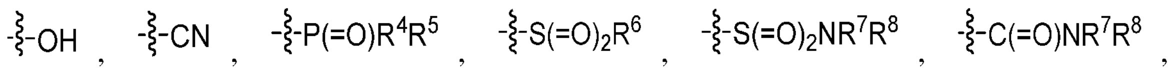

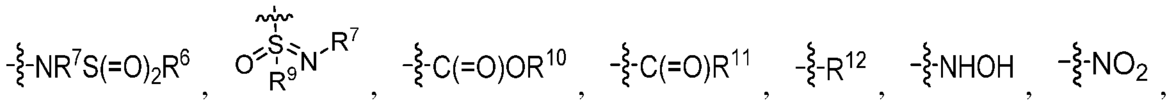

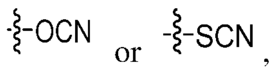 ,

wherein the $C_{1-6}$ alkyl or $C_{1-6}$ alkoxyl is optionally substituted by hydroxyl, halogen, cyano, $C_{1-3}$ alkoxyl or 4-7 membered heterocycloalkyl;

in one embodiment, $R^3$ is selected from: deuterium, halogen, $C_{1-6}$ alkyl, $C_{1-6}$ alkoxyl,

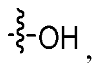 ,

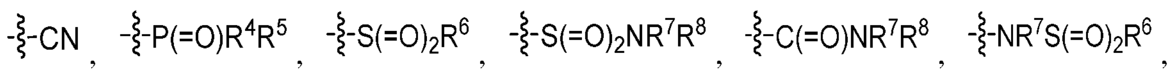

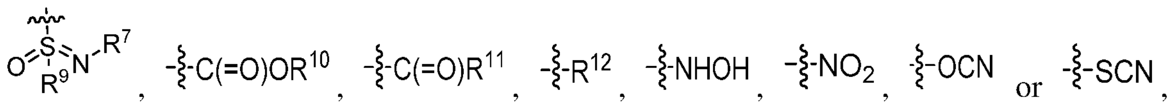

wherein the $C_{1-6}$ alkyl or $C_{1-6}$ alkoxyl is optionally substituted by hydroxyl, halogen, cyano, $C_{1-3}$ alkoxyl or 4-7 membered heterocycloalkyl;

$R^4$ and $R^5$ are independently selected from $C_{1-6}$ alkyl, hydroxyl, $C_{2-6}$ alkenyl, $C_{2-6}$ alkynyl, $C_{1-6}$ alkoxyl or $C_{3-10}$ cycloalkyl, wherein the $C_{1-6}$ alkyl is optionally substituted by deuterium, hydroxyl, halogen, cyano or $C_{1-3}$ alkoxyl; or $R^4$ and $R^5$ can form a 3-6 membered phosphorus-containing saturated monocyclic ring together with adjacent P atom;

$R^6$ is selected from $C_{1-6}$ alkyl, $C_{2-6}$ alkenyl, $C_{2-6}$ alkynyl, $C_{1-6}$ alkoxyl, $C_{3-10}$ cycloalkyl, 4-7 membered heterocycloalkyl or 5-7 membered heteroaryl;

$R^7$ and $R^8$ are independently selected from hydrogen, $C_{1-6}$ alkyl, $C_{1-6}$ alkoxyl, $C_{3-10}$ cycloalkyl, 4-7 membered heterocycloalkyl or 5-7 membered heteroaryl, wherein the $C_{1-6}$ alkyl is optionally substituted by hydroxyl, halogen, cyano or $C_{1-3}$ alkoxyl; or $R^7$, $R^8$ and their adjacent N atom together form a 3-6 membered nitrogen-containing saturated monocyclic ring;

$R^9$ and $R^{10}$ are independently selected from $C_{1-6}$ alkyl, $C_{2-6}$ alkenyl, $C_{2-6}$ alkynyl or $C_{3-10}$ cycloalkyl;

$R^{11}$ is selected from $C_{1-6}$ alkyl, $C_{2-6}$ alkenyl, $C_{2-6}$ alkynyl, $C_{3-10}$ cycloalkyl, 4-7 membered heterocycloalkyl or 5-7 membered heteroaryl; and

$R^{12}$ is selected from $C_{3-10}$ cycloalkyl, 4-7 membered heterocycloalkyl or 5-7 membered heteroaryl, which is optionally substituted by one or more hydroxyl, halogen, cyano, $C_{1-6}$ alkyl or 3 -7 membered heterocycloalkyl.

In some embodiments, X is CH.

In some embodiments, X is N.

In some embodiments, $R^1$ is a 5-12 membered saturated heterocyclic ring or a 5-7 membered saturated carbocyclic ring, which is optionally substituted by one or more $C_{1-6}$ alkyl, $C_{1-6}$ alkoxyl, halogen, cyano, deuterium or hydroxyl, and $R^1$ is not

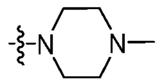,
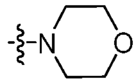

and

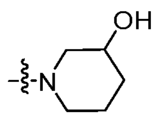.

In some embodiments, $R^1$ is a 5-8 membered saturated heterocyclic ring or a 5-8 membered saturated carbocyclic ring, which is optionally substituted by one or more $C_{1-6}$ alkyl, $C_{1-6}$ alkoxyl, halogen, cyano, deuterium or hydroxyl, and $R^1$ is not

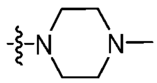,
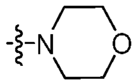

and

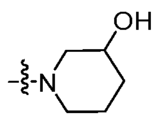.

In some embodiments, $R^1$ is a 5-8 membered saturated heterocyclic ring or a 5-8 membered saturated carbocyclic ring, which is optionally substituted by one or more methyl, methoxyl, F, Cl, cyano, deuterium or hydroxyl, and $R^1$ is not

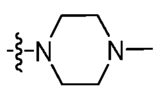,
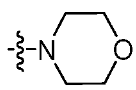

and

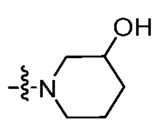.

In some embodiments, $R^1$ is a 5-8 membered saturated heterocyclic ring or a 5-8 membered saturated carbocyclic ring, which is optionally substituted by one or more methyl, methoxyl, Cl, cyano, deuterium or hydroxyl, and $R^1$ is not

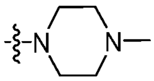, 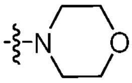 and 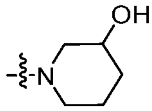.

In some embodiments, $R^1$ is a 5-8 membered saturated heterocyclic ring or a 5-8 membered saturated carbocyclic ring, which is optionally substituted by one or more methoxyl, F, cyano or hydroxyl, and $R^1$ is not

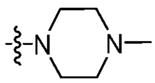, 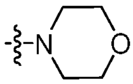 and 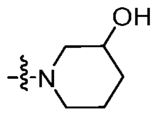.

In some embodiments, $R^1$ is a 5-8 membered saturated heterocyclic ring or a 5-8 membered saturated carbocyclic ring, which is optionally substituted by one or more F, cyano or deuterium, and $R^1$ is not

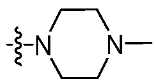, 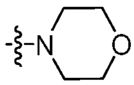 and 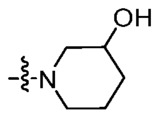.

In some embodiments, $R^1$ is a 5-8 membered saturated heterocyclic ring or a 5-8 membered saturated carbocyclic ring, which is optionally substituted by one or more F or cyano, and $R^1$ is not

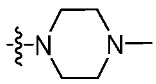, 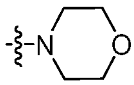 and 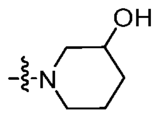.

In some embodiments, $R^1$ is a 5-8 membered saturated heterocyclic ring or a 5-8 membered saturated carbocyclic ring, which is optionally substituted by one or more deuterium, and $R^1$ is not

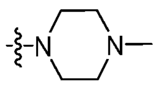, 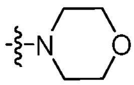 and 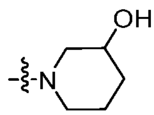.

In some embodiments, $R^1$ is a 5-8 membered saturated heterocyclic ring or a 5-8 membered saturated carbocyclic ring, which is optionally substituted by one or more methoxyl, and $R^1$ is not

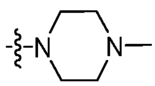, 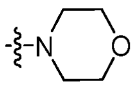 and 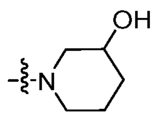.

In some embodiments, $R^1$ is a 5-8 membered saturated heterocyclic ring or a 5-8 membered saturated carbocyclic ring, which is optionally substituted by one or more hydroxyl, and $R^1$ is not

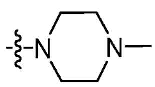, 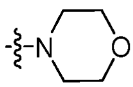 and 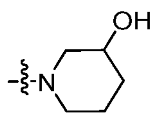.

In some embodiments, $R^1$ is a 5-8 membered saturated heterocyclic ring or a 5-8 membered saturated carbocyclic ring, which is optionally substituted by two F, and $R^1$ is not

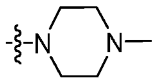, 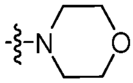 and 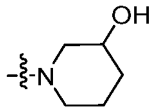.

In some embodiments, $R^1$ is a 5-8 membered saturated heterocyclic ring or a 5-8 membered saturated carbocyclic ring, which is optionally substituted by a cyano, and $R^1$ is not

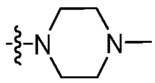, 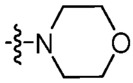 and 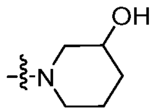.

In some embodiments, $R^1$ is a 5-8 membered saturated heterocyclic ring or a 5-8 membered saturated carbocyclic ring, which is optionally substituted by a methoxyl, and $R^1$ is not

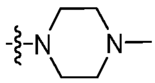, 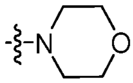 and 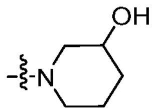.

In some embodiments, $R^1$ is a 5-8 membered saturated heterocyclic ring or a 5-8 membered saturated carbocyclic ring, which is optionally substituted by a hydroxyl, and $R^1$ is not

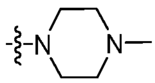, 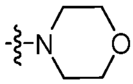 and 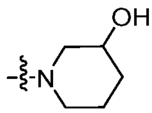.

In some embodiments, $R^1$ is a 5-8 membered saturated heterocyclic ring or a 5-8 membered saturated carbocyclic ring, which is optionally substituted by a F, and $R^1$ is not

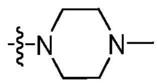,

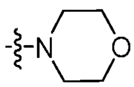 and 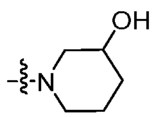.

In some embodiments, $R^1$ is an unsubstituted 5-8 membered saturated heterocyclic ring or an unsubstituted 5-8 membered saturated carbocyclic ring, and $R^1$ is not

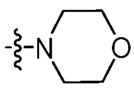.

In some embodiments, $R^1$ is an unsubstituted 5-8 membered saturated heterocyclic ring, and $R^1$ is not

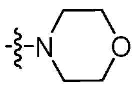.

In some embodiments, $R^1$ is a 5-7 membered saturated heterocyclic ring or a 5-7 membered saturated carbocyclic ring, which is optionally substituted by one or more $C_{1-6}$ alkyl, $C_{1-6}$ alkoxyl, halogen, cyano, deuterium or hydroxyl, and $R^1$ is not

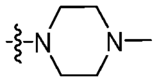, 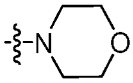

and

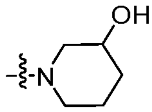.

In some embodiments, $R^1$ is a 5-7 membered saturated heterocyclic ring or a 5-7 membered saturated carbocyclic ring, which is optionally substituted by one or more methyl, methoxyl, F, Cl, cyano, deuterium or hydroxyl, and $R^1$ is not

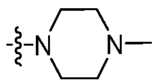, 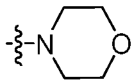

and

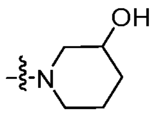.

In some embodiments, $R^1$ is a 5-7 membered saturated heterocyclic ring or a 5-7 membered saturated carbocyclic ring, which is optionally substituted by one or more methyl, methoxyl, Cl, cyano, deuterium or hydroxyl, and $R^1$ is not

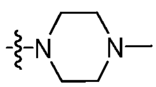, 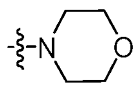 and 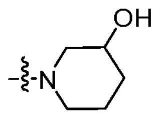.

In some embodiments, $R^1$ is a 5-7 membered saturated heterocyclic ring or a 5-7 membered saturated carbocyclic ring, which is optionally substituted by one or more methoxyl, F, cyano or hydroxyl, and $R^1$ is not

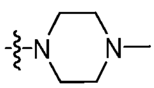, 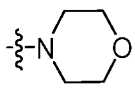 and 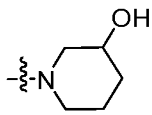.

In some embodiments, $R^1$ is a 5-7 membered saturated heterocyclic ring or a 5-7 membered saturated carbocyclic ring, which is optionally substituted by one or more F, cyano or deuterium, and $R^1$ is not

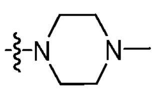, 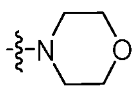 and 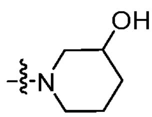.

In some embodiments, $R^1$ is a 5-7 membered saturated heterocyclic ring or a 5-7 membered saturated carbocyclic ring, which is optionally substituted by one or more F or cyano, and $R^1$ is not

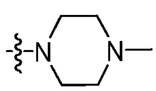, 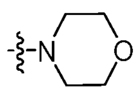 and 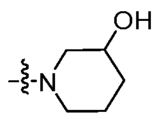.

In some embodiments, $R^1$ is a 5-7 membered saturated heterocyclic ring or a 5-7 membered saturated carbocyclic ring, which is optionally substituted by one or more deuterium, and $R^1$ is not

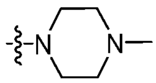, 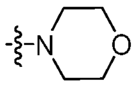 and 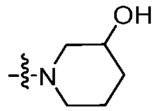.

In some embodiments, $R^1$ is a 5-7 membered saturated heterocyclic ring or a 5-7 membered saturated carbocyclic ring, which is optionally substituted by one or more methoxyl, and $R^1$ is not

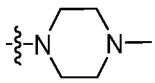, 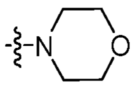 and 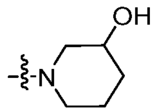.

In some embodiments, $R^1$ is a 5-7 membered saturated heterocyclic ring or a 5-7 membered saturated carbocyclic ring, which is optionally substituted by one or more hydroxyl, and $R^1$ is not

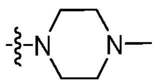, 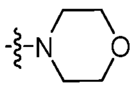 and 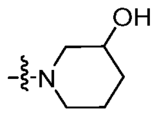.

In some embodiments, $R^1$ is a 5-7 membered saturated heterocyclic ring or a 5-7 membered saturated carbocyclic ring, which is optionally substituted by two F, and $R^1$ is not

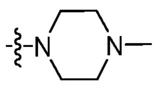, 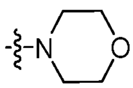 and 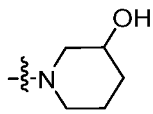.

In some embodiments, $R^1$ is a 5-7 membered saturated heterocyclic ring or a 5-7 membered saturated carbocyclic ring, which is optionally substituted by a cyano, and $R^1$ is not

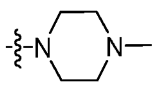, 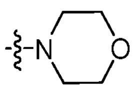 and 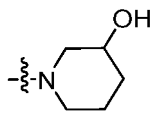.

In some embodiments, $R^1$ is a 5-7 membered saturated heterocyclic ring or a 5-7 membered saturated carbocyclic ring, which is optionally substituted by a methoxyl, and $R^1$ is not

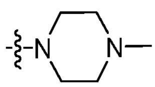, 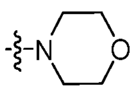 and 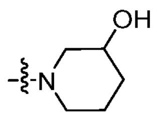.

In some embodiments, $R^1$ is a 5-7 membered saturated heterocyclic ring or a 5-7 membered saturated carbocyclic ring, which is optionally substituted by a hydroxyl, and $R^1$ is not

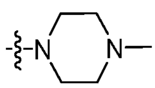, 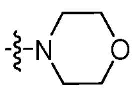 and 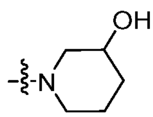.

In some embodiments, $R^1$ is a 5-7 membered saturated heterocyclic ring or a 5-7 membered saturated carbocyclic ring, which is optionally substituted by a F, and $R^1$ is not

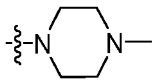,

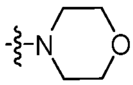 and 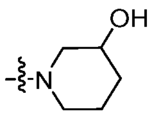.

In some embodiments, $R^1$ is an unsubstituted 5-7 membered saturated heterocyclic ring or an unsubstituted 5-7 membered saturated carbocyclic ring, and $R^1$ is not

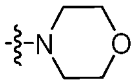.

In some embodiments, $R^1$ is an unsubstituted 5-7 membered saturated heterocyclic ring, and $R^1$ is not

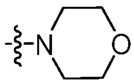.

In some typical embodiments, $R^1$ is

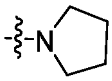, 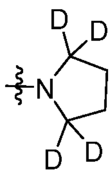, 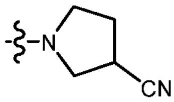, 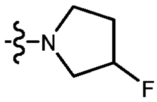,

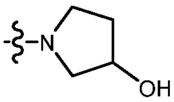, 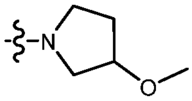, 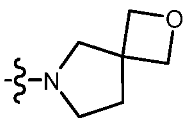, 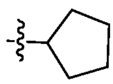, 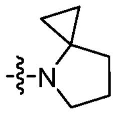, 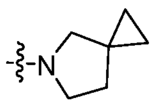,

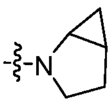, 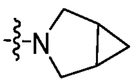, 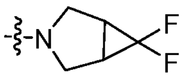, 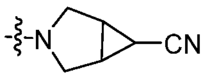, 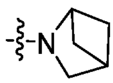, 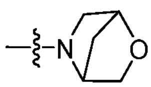,

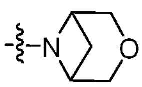, 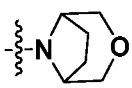, 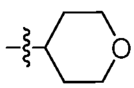, 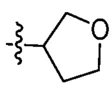 or 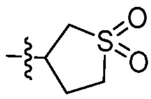.

In some typical embodiments, $R^1$ is

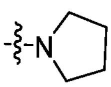, 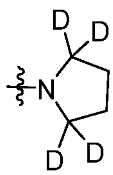, 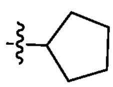, 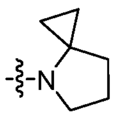, 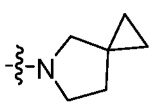,

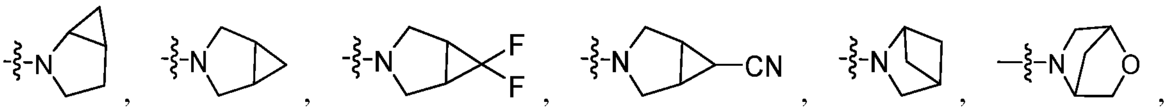
, , , , , ,

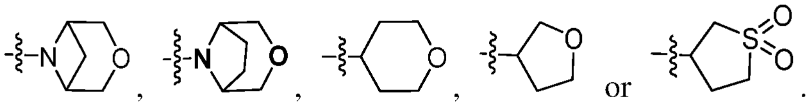
, , , or .

In some typical embodiments, $R^1$ is

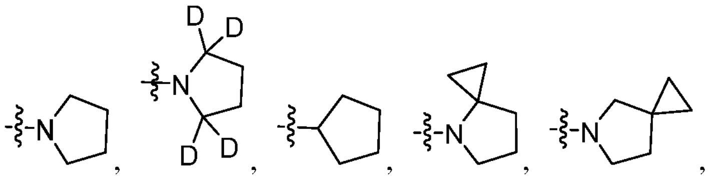
, , , , ,

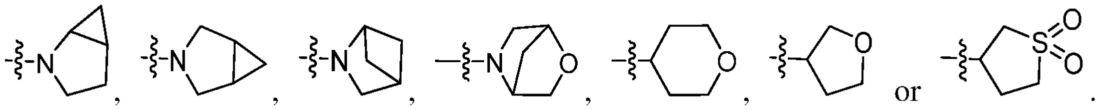
, , , , , or .

In some typical embodiments, $R^1$ is

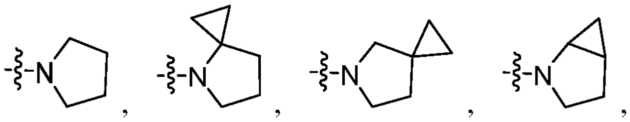
, , , ,

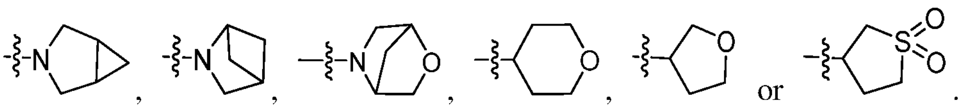
, , , , or .

In some typical embodiments, $R^1$ is

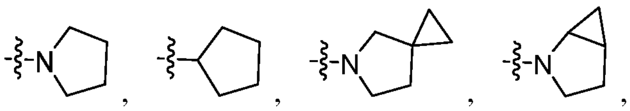
, , , ,

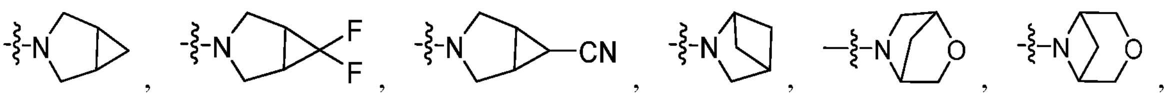
, , , , , ,

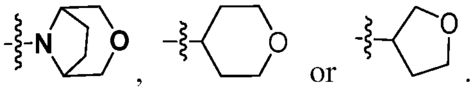
, or .

In some typical embodiments, $R^1$ is

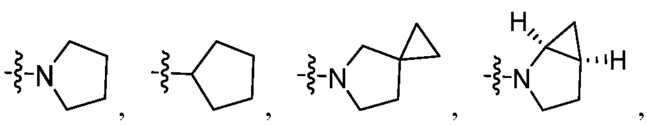
, , , ,

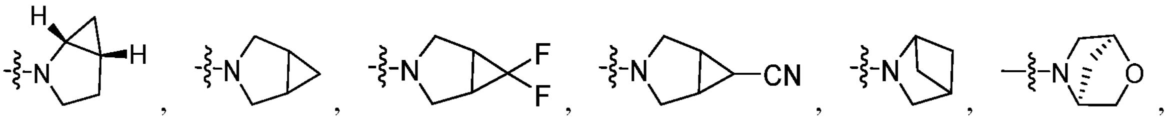

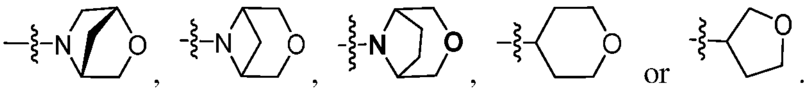 or .

In some typical embodiments, $R^1$ is

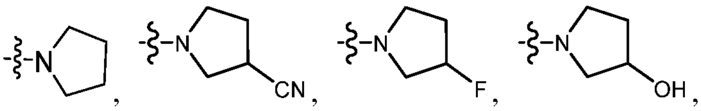

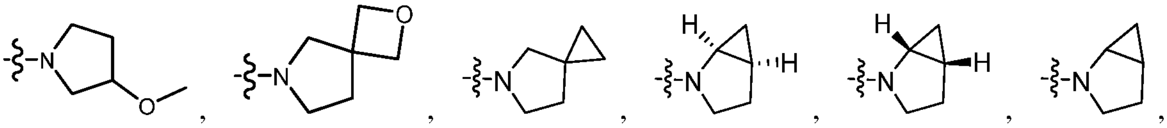

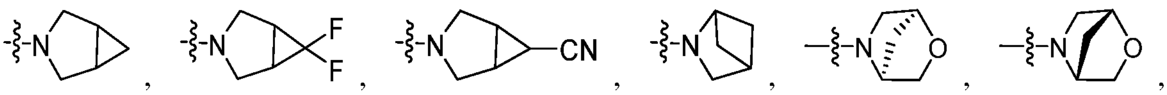

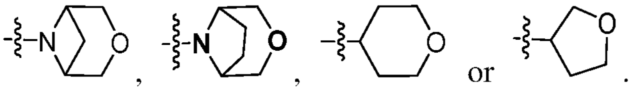 or .

In some typical embodiments, $R^1$ is

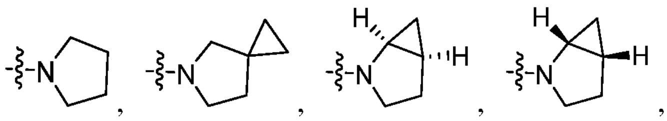

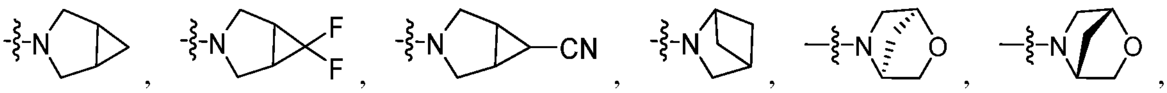

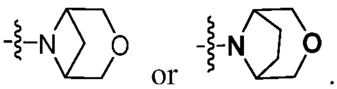 or .

In some typical embodiments, $R^1$ is

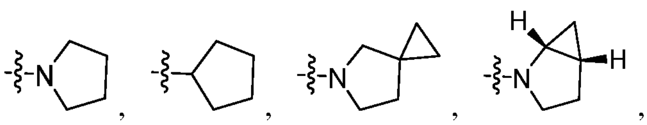

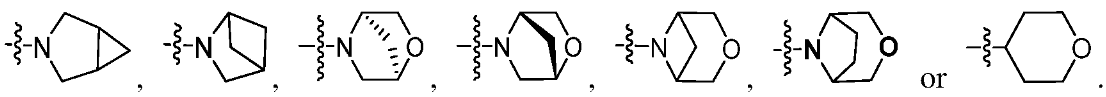 or .

In some typical embodiments, $R^1$ is

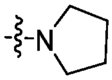, 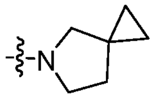, 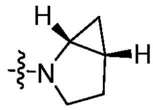, 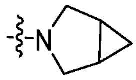,

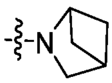, 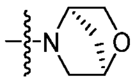, 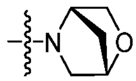, 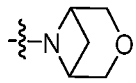 or 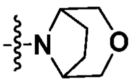.

In some typical embodiments, $R^1$ is

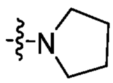, 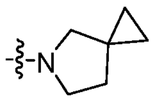, 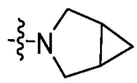, 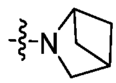,

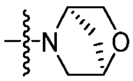, 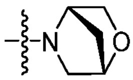, 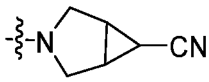, 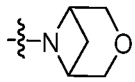 or 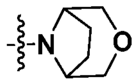.

In some typical embodiments, $R^1$ is

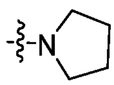, 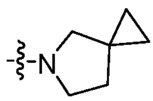, 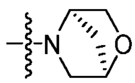, 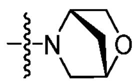,

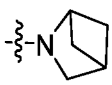, 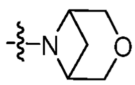 or 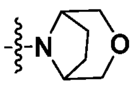.

In some typical embodiments, $R^1$ is

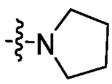, 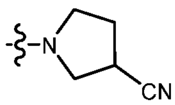, 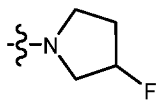, 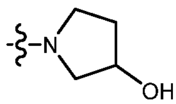,

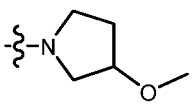, 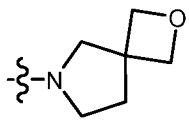 or 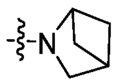.

In some more typical embodiments, $R^1$ is

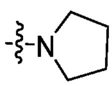, 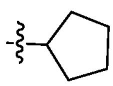, 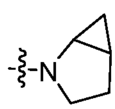, 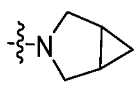,

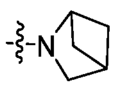 or 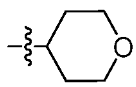.

In some more typical embodiments, $R^1$ is

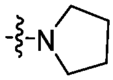, 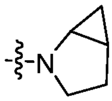, 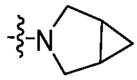 or 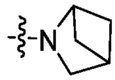.

In some more typical embodiments, $R^1$ is

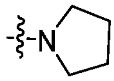.

In some embodiments, $R^2$ is F, Cl or Br.
In some typical embodiments, $R^2$ is Cl.
In some embodiments, ring A is phenyl, furyl, thienyl, pyrrolyl, pyrazolyl, imidazolyl, thiazolyl, oxazolyl, 1,2,3-triazolyl, 1,2,4-triazolyl, pyridyl, pyrimidinyl, pyridazinyl, pyrazinyl, benzo five-membered heterocyclyl or benzo six-membered heterocyclyl groups, wherein the groups are optionally substituted by one or more $R^3$.
In some embodiments, ring A is phenyl, furyl, thienyl, pyrrolyl, pyrazolyl, imidazolyl, thiazolyl, oxazolyl, 1,2,3-triazolyl, 1,2,4-triazolyl, pyridyl, pyrimidinyl, pyridazinyl, pyrazinyl,

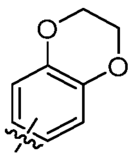, 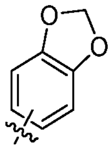, 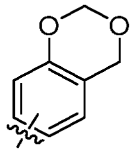 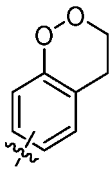 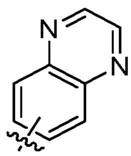, 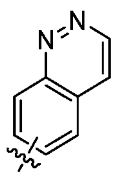, 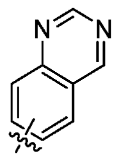,

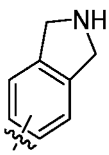, 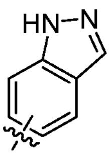, 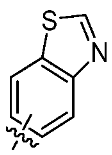 or 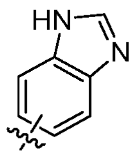

groups, wherein the groups are optionally substituted by one or more $R^3$.
In some typical embodiments, ring A is phenyl, furyl, thiazolyl, pyridyl,

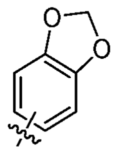,

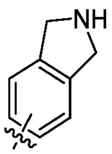 or 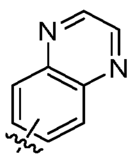

groups, wherein the groups are optionally substituted by one or more $R^3$.
In some typical embodiments, ring A is phenyl, pyridyl,

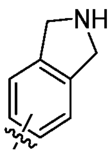 or 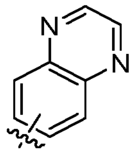

groups, wherein the groups are optionally substituted by one or more $R^3$.

In some typical embodiments, ring A is phenyl or pyridyl groups, wherein the groups are optionally substituted by one or more $R^3$.

In some more typical embodiments, ring A is a phenyl group, wherein the group is optionally substituted by one or more $R^3$.

In some embodiments, $R^3$ is selected from: deuterium, halogen, $C_{1-6}$ alkyl, $C_{1-6}$ alkoxyl, $C_{3-10}$ cycloalkyloxyl,

$-OH$, $-CN$, $-P(=O)R^4R^5$, $-S(=O)_2R^6$, $-S(=O)_2NR^7R^8$,

$-C(=O)NR^7R^8$, $-NR^7S(=O)_2R^6$, 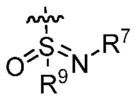, $-C(=O)OR^{10}$, $-C(=O)R^{11}$, $-R^{12}$,

$-NHOH$, $-NO_2$, $-OCN$ or $-SCN$,

wherein the $C_{1-6}$ alkyl or $C_{1-6}$ alkoxyl is optionally substituted by hydroxyl, halogen, cyano, $C_{1-3}$ alkoxyl or 4-7 membered heterocycloalkyl. In some embodiments, $R^3$ is selected from: deuterium, halogen, $C_{1-6}$ alkyl, $C_{1-6}$ alkoxyl,

$-OH$, $-CN$, $-P(=O)R^4R^5$, $-S(=O)_2R^6$, $-S(=O)_2NR^7R^8$, $-C(=O)NR^7R^8$,

$-NR^7S(=O)_2R^6$, 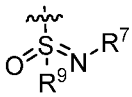, $-C(=O)OR^{10}$ or $-R^{12}$,

wherein the $C_{1-6}$ alkyl or $C_{1-6}$ alkoxyl is optionally substituted by hydroxyl, halogen, cyano, $C_{1-3}$ alkoxyl or 4-7 membered heterocycloalkyl.

$R^3$ is selected from: deuterium, halogen, $C_{1-6}$ alkyl, $C_{1-6}$ alkoxyl, $C_{3-10}$ cycloalkyloxyl,

$-OH$, $-CN$, $-P(=O)R^4R^5$, $-S(=O)_2R^6$, $-S(=O)_2NR^7R^8$, $-C(=O)NR^7R^8$,

$-NR^7S(=O)_2R^6$, 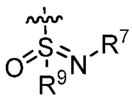, $-C(=O)OR^{10}$ or $-R^{12}$,

wherein the $C_{1-6}$ alkyl or $C_{1-6}$ alkoxyl is optionally substituted by hydroxyl, halogen, cyano, $C_{1-3}$ alkoxyl or 4-7 membered heterocycloalkyl.

In some typical embodiments, $R^3$ is selected from: deuterium, halogen, $C_{1-6}$ alkyl, $C_{1-6}$ alkoxyl,

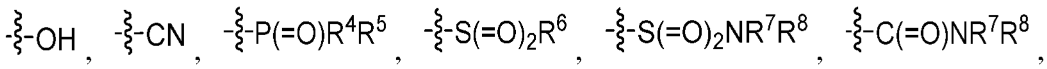

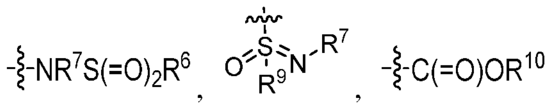 or 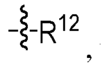

wherein the $C_{1-6}$ alkyl or $C_{1-6}$ alkoxyl is optionally substituted by hydroxyl, F, Cl, Br, cyano, $C_{1-3}$ alkoxyl or 4-7 membered heterocycloalkyl.

In some more typical embodiments, $R^3$ is selected from: deuterium, F, Cl, Br, $C_{1-6}$ alkyl, $C_{1-6}$ alkoxyl,

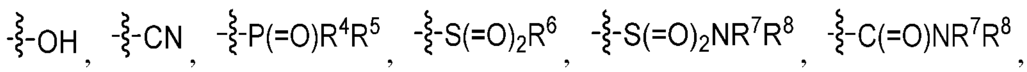

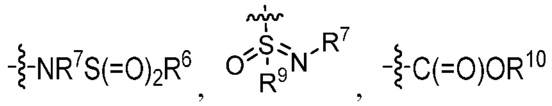 or 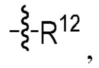

wherein the $C_{1-6}$ alkyl or $C_{1-6}$ alkoxyl is optionally substituted by methoxyl, hydroxyl, F, cyano or

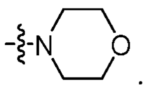

In some more typical embodiments, $R^3$ is selected from: deuterium, F, Cl, Br, $C_{1-6}$ alkyl, $C_{1-6}$ alkoxyl,

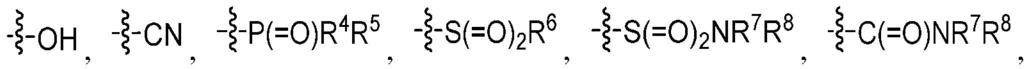

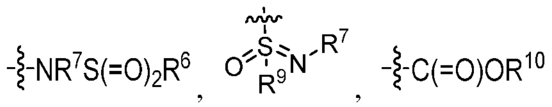 or 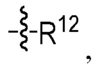

wherein the $C_{1-6}$ alkyl or $C_{1-6}$ alkoxyl is optionally substituted by hydroxyl, F, cyano or

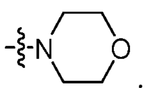

In some more typical embodiments, $R^3$ is selected from: F, Cl, $C_{1-6}$ alkyl, $C_{1-6}$ alkoxyl,

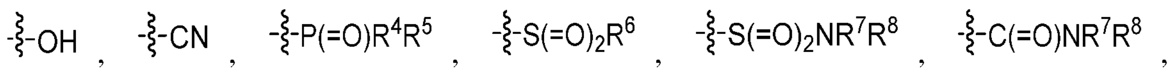

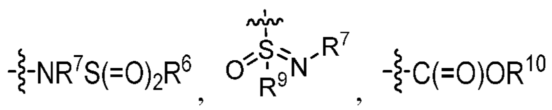 or 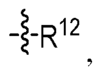

wherein the $C_{1-6}$ alkyl or $C_{1-6}$ alkoxyl is optionally substituted by methoxyl, hydroxyl, F, cyano or

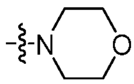.

In some more typical embodiments, $R^3$ is selected from: F, Cl, $C_{1-6}$ alkyl, $C_{1-6}$ alkoxyl,

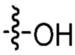, 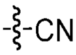, 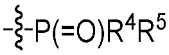, 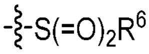, 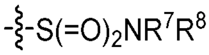, 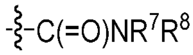,

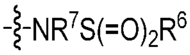, 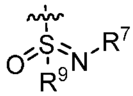, 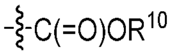 or 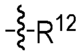,

wherein the $C_{1-6}$ alkyl or $C_{1-6}$ alkoxyl is optionally substituted by hydroxyl, F, cyano or

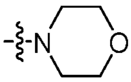.

In some more typical embodiments, $R^3$ is selected from: deuterium, F, Cl, Br, methyl, trifluoromethyl,

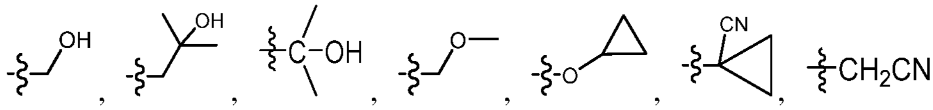,

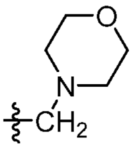, 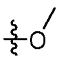, 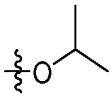, 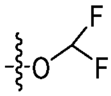, 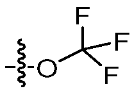, 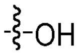, 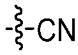, 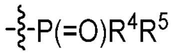,

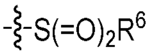, 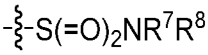, 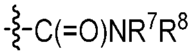, 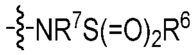, 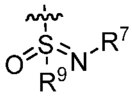,

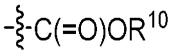 or 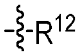.

In some more typical embodiments, $R^3$ is selected from: deuterium, F, Cl, Br, methyl, trifluoromethyl,

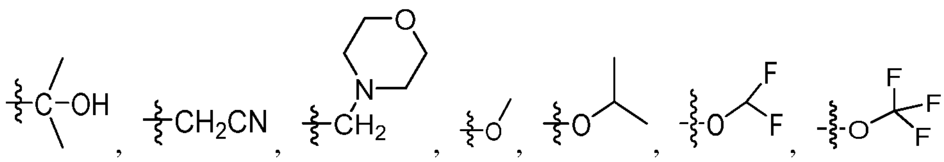,

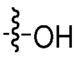, 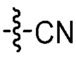, 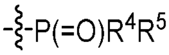, 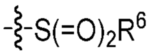, 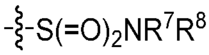, 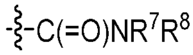,

$-NR^7S(=O)_2R^6$, 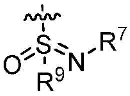, $-C(=O)OR^{10}$ or $-R^{12}$.

In some more typical embodiments, $R^3$ is selected from: F, Cl, methyl, trifluoromethyl,

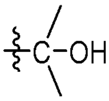, $-CH_2CN$, 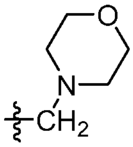, 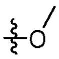, 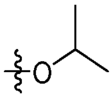, $-OH$, $-CN$, $-P(=O)R^4R^5$,

$-S(=O)_2R^6$, $-S(=O)_2NR^7R^8$, $-C(=O)NR^7R^8$, $-NR^7S(=O)_2R^6$, 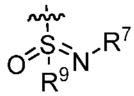,

$-C(=O)OR^{10}$ or $-R^{12}$.

In some more typical embodiments, $R^3$ is selected from: deuterium, F, Cl, Br, methyl, trifluoromethyl,

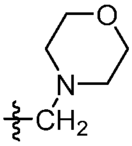, 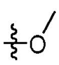, 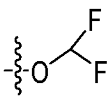, 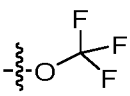, $-OH$, $-CN$, $-P(=O)R^4R^5$,

$-S(=O)_2R^6$, $-S(=O)_2NR^7R^8$, $-C(=O)NR^7R^8$, $-NR^7S(=O)_2R^6$, 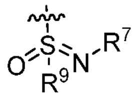,

$-C(=O)OR^{10}$ or $-R^{12}$.

In some more typical embodiments, $R^3$ is selected from: F, Cl, methyl, trifluoromethyl,

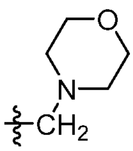, 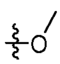, $-OH$, $-CN$, $-P(=O)R^4R^5$, $-S(=O)_2R^6$, $-S(=O)_2NR^7R^8$,

$-C(=O)NR^7R^8$, $-NR^7S(=O)_2R^6$, 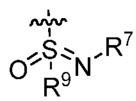, $-C(=O)OR^{10}$ or $-R^{12}$.

In some more typical embodiments, $R^3$ is selected from: F, Cl, methyl, trifluoromethyl,

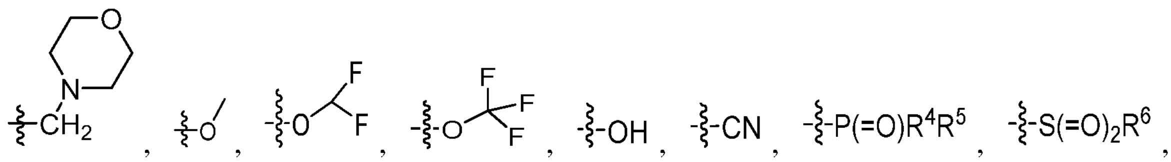

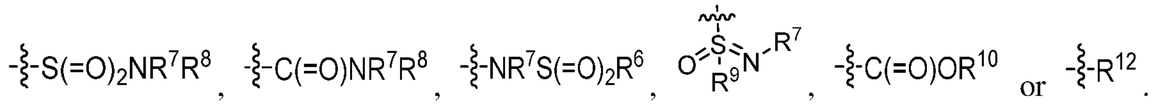

In some more typical embodiments, $R^3$ is selected from: F, Cl, methyl, trifluoromethyl,

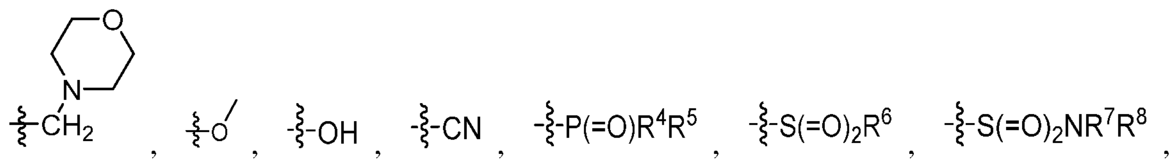

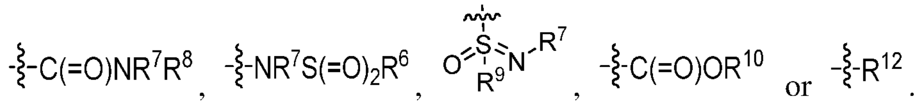

In some more typical embodiments, $R^3$ is selected from: F, Cl, methyl, trifluoromethyl,

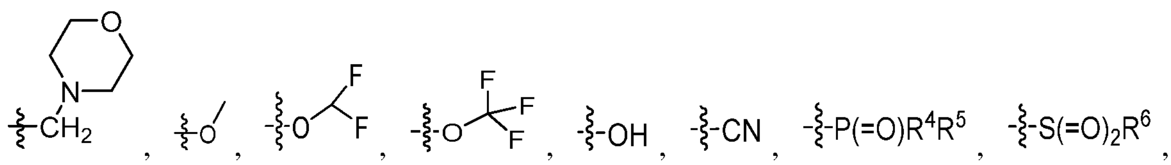

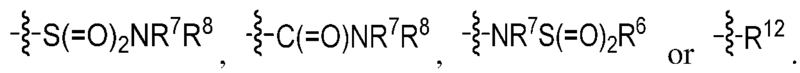

In some more typical embodiments, $R^3$ is selected from: F, Cl, methyl, trifluoromethyl,

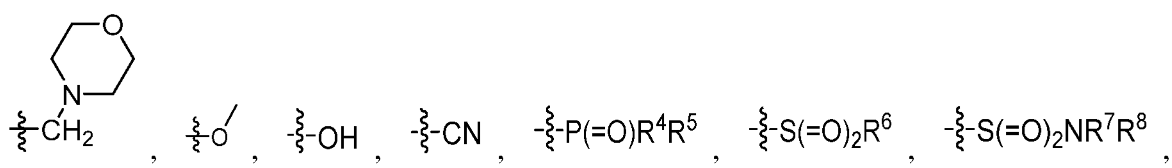

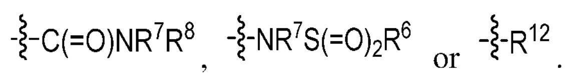

In some typical embodiments, $R^3$ is F, methyl,

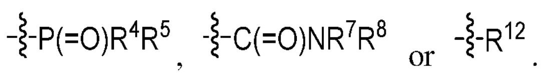

In some typical embodiments, $R^3$ is F, Cl, methyl, $C_{1-6}$ alkyl, $C_{1-6}$ alkoxyl, $C_{3-10}$ cycloalkyloxyl,

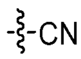, 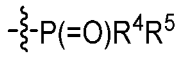 or 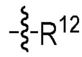.

In some typical embodiments, $R^3$ is F, Cl, cyano, methyl, $C_{1-3}$ alkoxyl, $C_{3-6}$ cycloalkyloxyl,

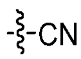, 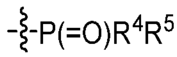 or 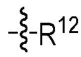.

In some embodiments, $R^4$ and $R^5$ are independently selected from $C_{1-6}$ alkyl or $C_{3-10}$ cycloalkyl, wherein the $C_{1-6}$ alkyl is optionally substituted by deuterium, hydroxyl, halogen, cyano or $C_{1-3}$ alkoxyl; or $R^4$ and $R^5$ can form a 3-6 membered phosphorus-containing saturated monocyclic ring together with adjacent P atom.

In some more typical embodiments, $R^4$ and $R^5$ are independently selected from $C_{1-6}$ alkyl, wherein the $C_{1-6}$ alkyl is optionally substituted by deuterium, hydroxyl, halogen, cyano or $C_{1-3}$ alkoxyl.

In some typical embodiments, $R^4$ and $R^5$ are independently selected from $C_{1-6}$ alkyl, wherein the $C_{1-6}$ alkyl is optionally substituted by deuterium.

In some more typical embodiments, $R^4$ and $R^5$ are independently selected from methyl, ethyl, n-propyl, isopropyl, $CD_3$, $CH_2D$, $CHD_2$, $CH_2CD_3$, $CD_2CD_3$, $CH_2CH_2CD_3$, $CH(CD_3)_2$ or $CD(CD_3)_2$.

In some more typical embodiments, $R^4$ and $R^5$ are independently selected from methyl or $CD_3$.

In some more typical embodiments, $R^4$ and $R^5$ are both methyl.

In some more typical embodiments, $R^4$ and $R^5$ are both $CD_3$.

In some embodiments, $R^6$ is $C_{1-6}$ alkyl, $C_{3-10}$ cycloalkyl, or 4-7 membered heterocycloalkyl.

In some embodiments, $R^6$ is $C_{1-6}$ alkyl or $C_{3-10}$ cycloalkyl.

In some typical embodiments, $R^6$ is methyl, ethyl, n-propyl, isopropyl, tert-butyl, cyclopropyl, cyclobutyl, cyclopentyl, or cyclohexyl.

In some more typical embodiments, $R^6$ is methyl, isopropyl or cyclopropyl.

In some embodiments, $R^7$ and $R^8$ are independently selected from hydrogen, $C_{1-6}$ alkyl, $C_{3-10}$ cycloalkyl or 4-7 membered heterocycloalkyl, wherein the $C_{1-6}$ alkyl is optionally substituted by hydroxyl, halogen, cyano or $C_{1-3}$ alkoxyl; or $R^7$ and $R^8$ can form a 3-6 membered nitrogen-containing saturated monocyclic ring together with their adjacent N atom.

In some typical embodiments, $R^7$ and $R^8$ are independently selected from hydrogen, $C_{1-6}$ alkyl, $C_{3-7}$ cycloalkyl or 5-membered heterocycloalkyl, wherein the $C_{1-6}$ alkyl is optionally substituted by hydroxyl, F, cyano or $C_{1-3}$ alkoxyl; or $R^7$ and $R^8$ can form

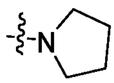,

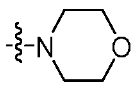 or 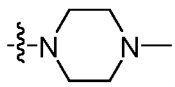

together with their adjacent N atom.

In some more typical embodiments, $R^7$ and $R^8$ are independently selected from hydrogen, methyl, ethyl, n-propyl, isopropyl, cyclopropyl, cyclobutyl, $CH_2CH_2OCH_3$, $CF_3$,

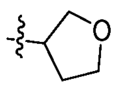

or

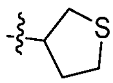;

or $R^7$ and $R^8$ can form

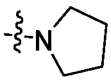, 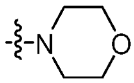 or 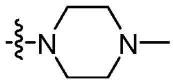

together with their adjacent N atom.

In some more typical embodiments, $R^7$ is hydrogen or methyl, and $R^8$ is selected from hydrogen, methyl, ethyl, n-propyl, isopropyl, cyclopropyl, cyclobutyl, $CH_2CH_2OCH_3$, $CF_3$,

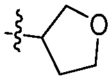 or 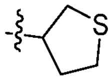;

or $R^7$ and $R^8$ can form

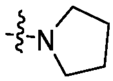

together with their adjacent N atom.

In some more typical embodiments, $R^7$ is hydrogen, and $R^8$ is selected from hydrogen, methyl, cyclopropyl, $CH_2CH_2OCH_3$, or

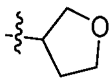;

or $R^7$ and $R^8$ can form

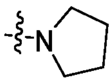

together with their adjacent N atom.

In some most typical embodiments, $R^7$ is hydrogen, and $R^8$ is selected from hydrogen, methyl, cyclopropyl, or

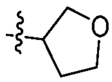;

or $R^7$ and $R^8$ can form

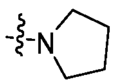

together with their adjacent N atom.

In some typical embodiments, $R^9$ and $R^{10}$ are independently selected from $C_{1-6}$ alkyl or $C_{3-10}$ cycloalkyl.

In some more typical embodiments, $R^9$ and $R^{10}$ are independently selected from $C_{1-6}$ alkyl.

In some more typical embodiments, $R^9$ and $R^{10}$ are independently selected from methyl, ethyl, n-propyl, isopropyl or tert-butyl.

In some more typical embodiments, $R^9$ and $R^{10}$ are both methyl.

In some embodiments, $R^{11}$ is 4-7 membered heterocycloalkyl.

In some more typical embodiments, $R^{11}$ is a 5-membered heterocycloalkyl.

In some embodiments, $R^{12}$ is a $C_{3-10}$ cycloalkyl or a 5-7 membered heteroaryl, which is optionally substituted by one or more $C_{1-6}$ alkyl.

In some embodiments, $R^{12}$ is a 5-7 membered heteroaryl, which is optionally substituted by one or more $C_{1-6}$

alkyl.

In some embodiments, $R^{12}$ is cyclopropyl or 5-membered heteroaryl, which is optionally substituted by one or more methyl.

In some embodiments, $R^{12}$ is 5-membered heteroaryl, which is optionally substituted by one or more methyl.

In some more typical embodiments, $R^{12}$ is cyclopropyl, pyrazolyl, imidazolyl, thiazolyl, oxazolyl, 1,2,3-triazolyl, 1,2,4-triazolyl or tetrazolyl, which is optionally substituted by one or more methyl.

In some more typical embodiments, $R^{12}$ is pyrazolyl, imidazolyl, thiazolyl, oxazolyl, 1,2,3-triazolyl or 1,2,4-triazolyl, which is optionally substituted by one or more methyl.

In some more typical embodiments, $R^{12}$ is cyclopropyl, imidazolyl, oxazolyl, 1,2,4-triazolyl or tetrazolyl, which is optionally substituted by one or more methyl.

In some more typical embodiments, $R^{12}$ is imidazolyl, oxazolyl or 1,2,4-triazolyl, which is optionally substituted by one or more methyl.

In some more typical embodiments, $R^{12}$ is selected from

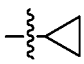, 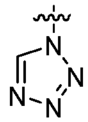, 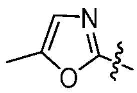,

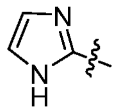, 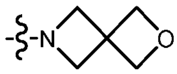 or 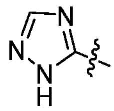.

In some more typical embodiments, $R^{12}$ is selected from

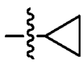, 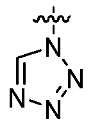, 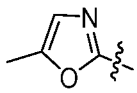,

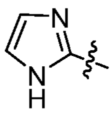 or 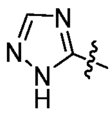.

In some more typical embodiments, $R^{12}$ is selected from

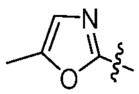, 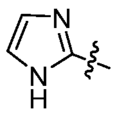 or 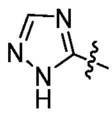.

In some more typical embodiments, $R^{12}$ is selected from

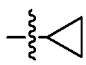, 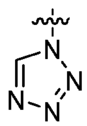, 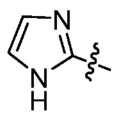

or

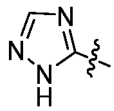.

In some more typical embodiments, $R^{12}$ is selected from

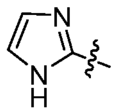 or 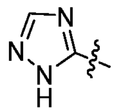.

In some more typical embodiments, $R^3$ is selected from: deuterium, F, Cl, Br, methyl, trifluoromethyl,

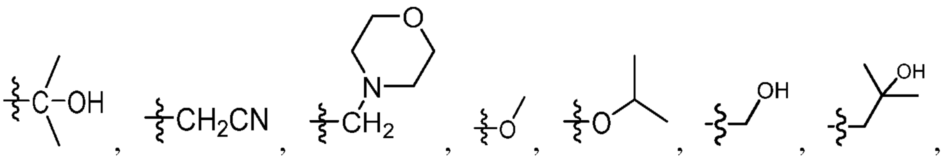, , , , , , ,

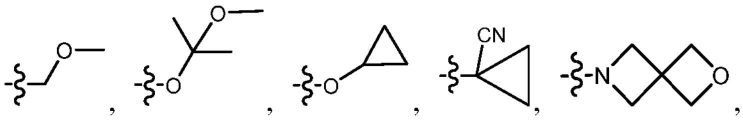, , , , ,

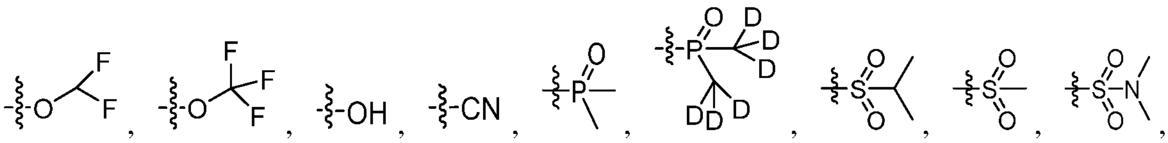, , , , , , , , ,

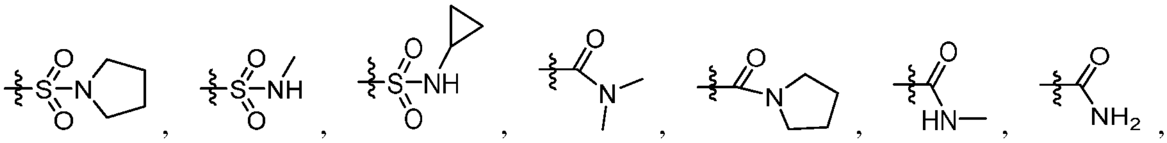, , , , , , ,

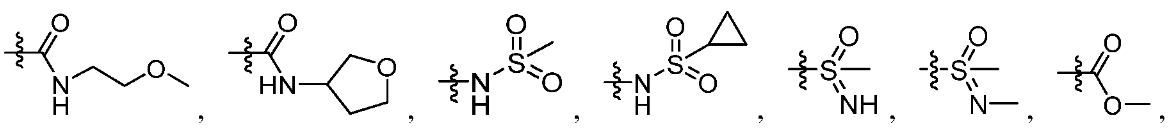, , , , , , ,

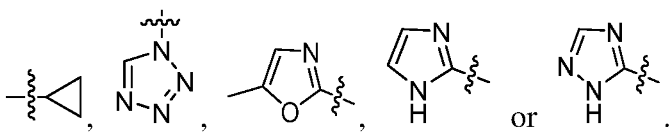, , , or .

In some more typical embodiments, $R^3$ is selected from: deuterium, F, Cl, Br, methyl, trifluoromethyl,

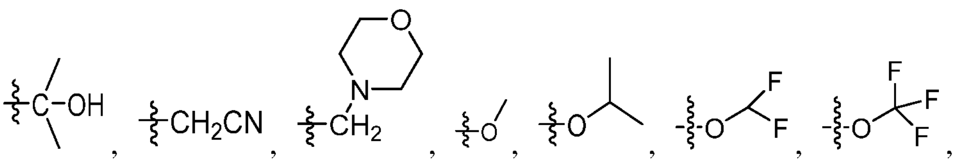, , , , , , ,

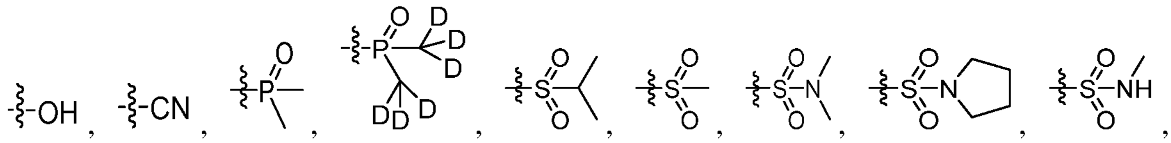
, , , , , , , , , ,

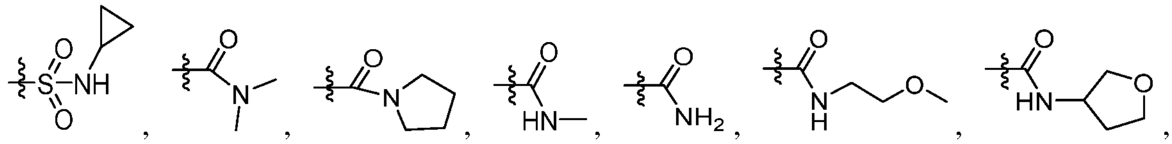
, , , , , ,

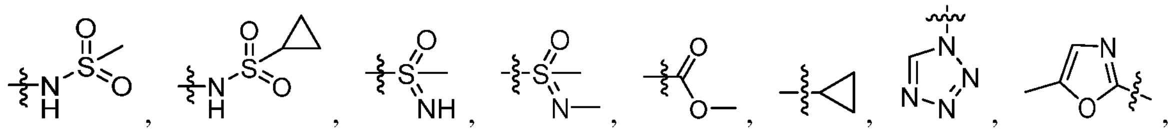
, , , , , , , ,

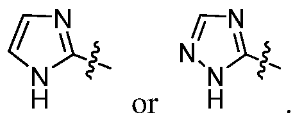
or .

In some more typical embodiments, $R^3$ is selected from: F, Cl, methyl, trifluoromethyl,

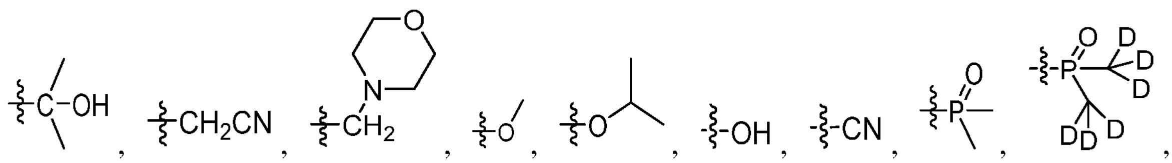
, , , , , , , , , ,

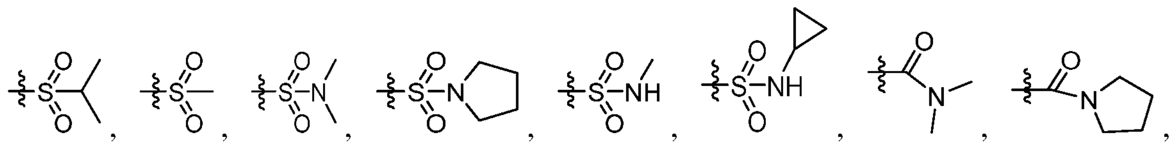
, , , , , , , ,

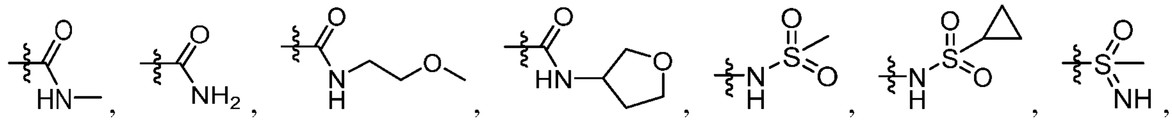
, , , , , , ,

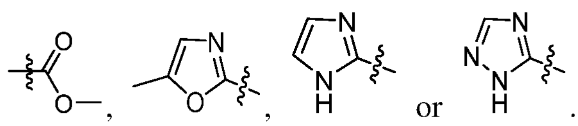
, , or .

In some more typical embodiments, $R^3$ is selected from: F, Cl, methyl, trifluoromethyl,

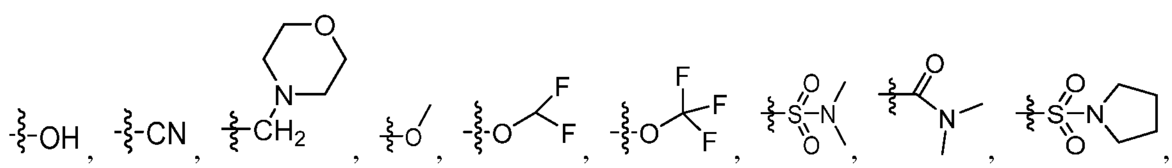
, , , , , , , , , ,

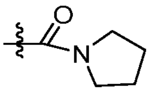, 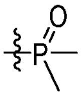, 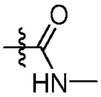, 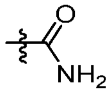, 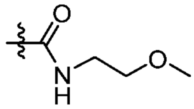, 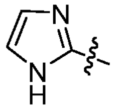, 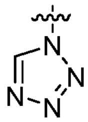, 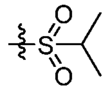,

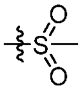, 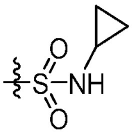, 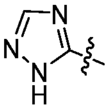, 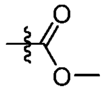, 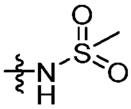, 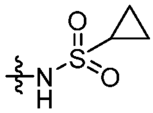 or 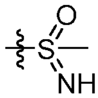.

In some more typical embodiments, $R^3$ is selected from: F, Cl, methyl, trifluoromethyl,

-OH, -CN, 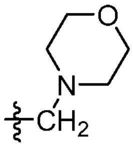, 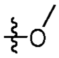, 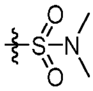, 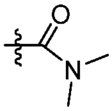, 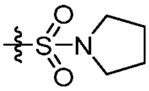, 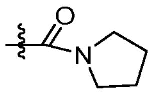, 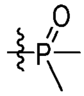,

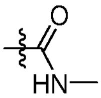, 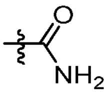, 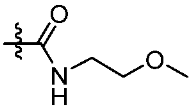, 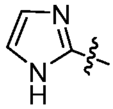, 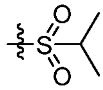, 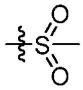, 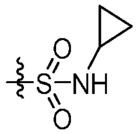, 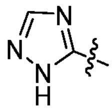,

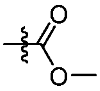, 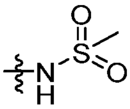, 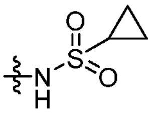 or 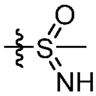.

In some more typical embodiments, $R^3$ is selected from: F, Cl, methyl,

-OH, -CN,

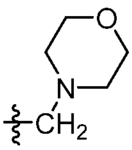, 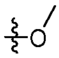, 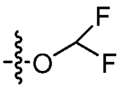, 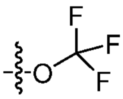, 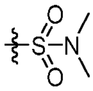, 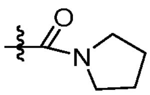, , ,

, , , , , , 

or

.

In some more typical embodiments, $R^3$ is selected from: F, Cl, methyl,

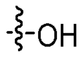, 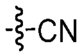,

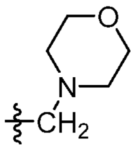, 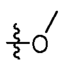, 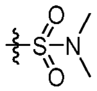, 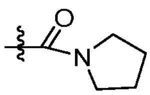, 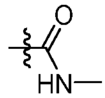, 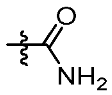, 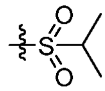, 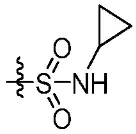,

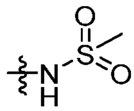, 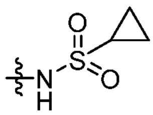, 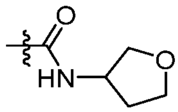, 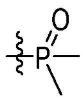, 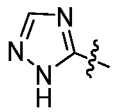 or 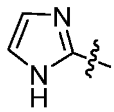.

In some more typical embodiments, $R^3$ is selected from: F, Cl, methyl,

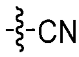, 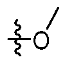,

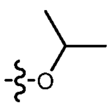, 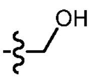, 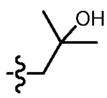, 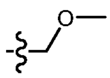, 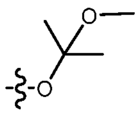, 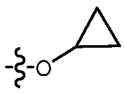, 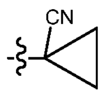, 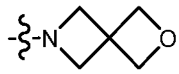

or

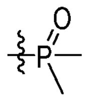.

In some more typical embodiments, $R^3$ is F, methyl,

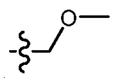, 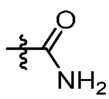, 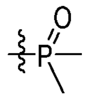, 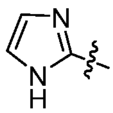

or

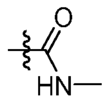.

In some more typical embodiments, $R^3$ is F, methyl,

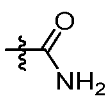, 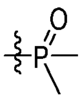, 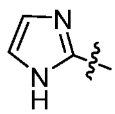 or 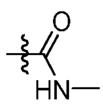.

In some more typical embodiments, $R^3$ is F, methyl,

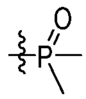 or 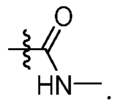.

In some more typical embodiments, $R^3$ is selected from F,

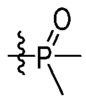 or 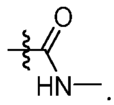.

In some more typical embodiments, $R^3$ is selected from

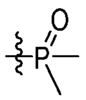 or 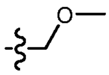.

In some embodiments, the above compound of Formula I has a structure shown in the following compound of Formula I-1, or a pharmaceutically acceptable salt thereof:

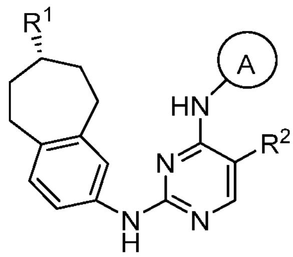

I-1

wherein the definitions of $R^1$, $R^2$ and ring A are as defined in the compound of Formula I.

In some embodiments, ring A is selected from phenyl, pyridyl,

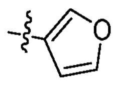, 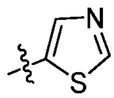,

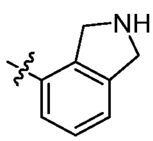, 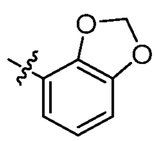 or 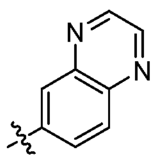

groups, wherein the groups are optionally substituted by one or two $R^3$; and the definition of $R^3$ is as defined in the compound of Formula I.

In some embodiments, ring A is selected from phenyl, pyridyl,

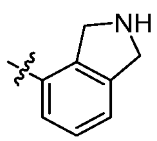

or

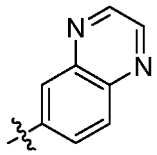

groups, wherein the groups are optionally substituted by one or two $R^3$; and the definition of $R^3$ is as defined in the compound of Formula I.

In some embodiments, the above compound of Formula I has a structure shown in the following compound of Formula I-2, or a pharmaceutically acceptable salt thereof:

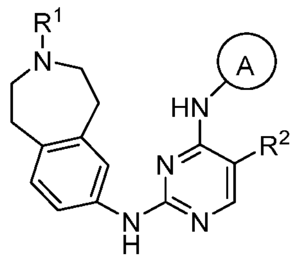

I-2

wherein the definitions of $R^1$, $R^2$ and ring A are as defined in the compound of Formula I.

In some more typical embodiments, $R^1$ is selected from

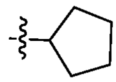, 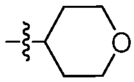, 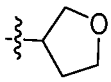

or

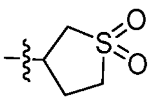.

In some typical embodiments, $R^1$ is selected from

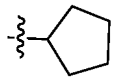 or 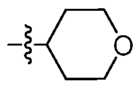.

In some embodiments, the above compound of Formula I has a structure shown in the following compound of Formula II, or a pharmaceutically acceptable salt thereof:

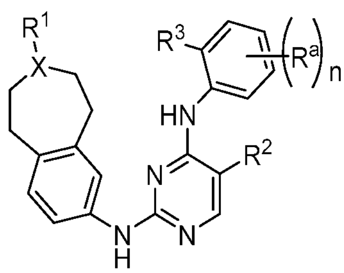

II

wherein the definitions of $R^1$, $R^2$ and $R^3$ are consistent with those defined in the compound of Formula I; and n is an integer from 0 to 4.

In some embodiments, $R^a$ is deuterium, halogen, $C_{1-6}$ alkyl, $C_{1-6}$ alkoxyl, $C_{3-10}$ cycloalkyloxyl,

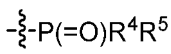, 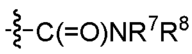, 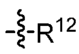, 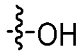 or 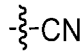,

wherein the $C_{1-6}$ alkyl or $C_{1-6}$ alkoxyl is optionally substituted by one or more deuterium, methoxyl, hydroxyl, halogen or cyano; and the definitions of $R^4$, $R^5$, $R^7$, $R^8$ and $R^{12}$ are consistent with those defined in the compound of Formula I.

In some embodiments, $R^a$ is deuterium, halogen, $C_{1-6}$ alkyl, $C_{1-6}$ alkoxyl,

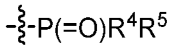,

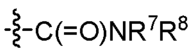, 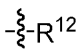, 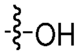 or 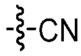,

wherein the $C_{1-6}$ alkyl or $C_{1-6}$ alkoxyl is optionally substituted by one or more deuterium, halogen or cyano; and the definitions of $R^4$, $R^5$, $R^7$, $R^8$ and $R^{12}$ are consistent with those defined in the compound of Formula I.

In some embodiments, $R^a$ is deuterium, halogen, $C_{1-6}$ alkyl, $C_{1-6}$ alkoxyl,

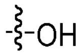 or 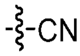,

wherein the $C_{1-6}$ alkyl or $C_{1-6}$ alkoxyl is optionally substituted by one or more deuterium, halogen or cyano.

In some embodiments, n is 0, 1, or 2.

Further, in some embodiments, n is 0 or 1.

In some more typical embodiments, n is 1.

In some embodiments, $R^a$ is deuterium, Cl, F, Br, cyano, hydroxyl,

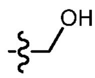, 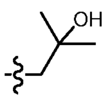,

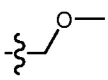, 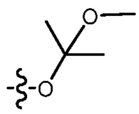, 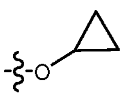, 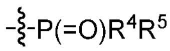, 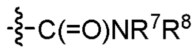, 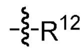,

or methyl or methoxyl optionally substituted by one or more halogen, wherein the definitions of $R^4$, $R^5$, $R^7$, $R^8$ and $R^{12}$ are consistent with those defined in the compound of Formula I.

In some embodiments, $R^a$ is deuterium, Cl, F, Br, cyano, hydroxyl,

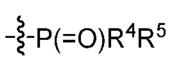,

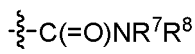, 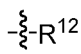,

or methyl or methoxyl optionally substituted by one or more halogen, wherein the definitions of $R^4$, $R^5$, $R^7$, $R^8$ and $R^{12}$ are consistent with those defined in the compound of Formula I.

In some embodiments, $R^a$ is deuterium, Cl, F, cyano, hydroxyl, or methyl or methoxyl optionally substituted by one or more halogen.

In some embodiments, $R^a$ is deuterium, Cl, F, Br, cyano, hydroxyl, $P(=O)R^4R^5$ ,

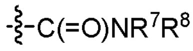 , 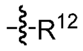 ,

or methyl or methoxyl optionally substituted by one or more F, wherein the definitions of $R^4$, $R^5$, $R^7$, $R^8$ and $R^{12}$ are consistent with those defined in the compound of Formula I.

In some embodiments, $R^a$ is deuterium, Cl, F, cyano, hydroxyl, or methyl or methoxyl optionally substituted by one or more F.

In some more typical embodiments, $R^a$ is deuterium, F, Cl, Br, methyl, trifluoromethyl,

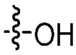 , 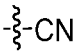 , 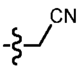 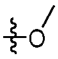 , 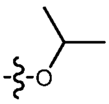 , 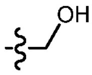 , 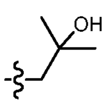 , 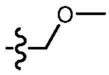 , 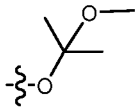 , 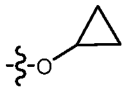 ,

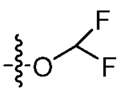 , 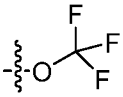 , 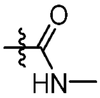 , 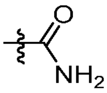 , 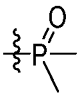 , 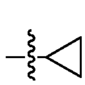 , 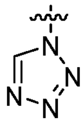 , 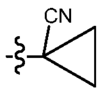 , 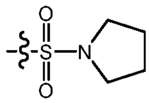

or

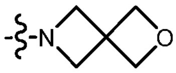 .

In some more typical embodiments, $R^a$ is selected from: deuterium, Cl, F, Br, cyano, hydroxyl, methyl, trifluoromethyl, methoxyl,

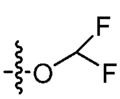 , 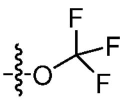 , 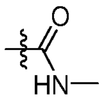 , 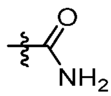 ,

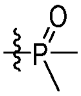 , 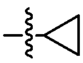 or 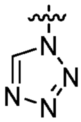 .

In some more typical embodiments, $R^a$ is Cl, F, cyano, hydroxyl, methyl, trifluoromethyl, methoxyl,

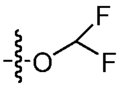 , 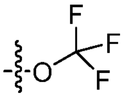 , 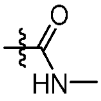 or 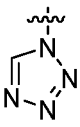 .

In some more typical embodiments, $R^a$ is Cl, F, cyano, hydroxyl, methyl, methoxyl,

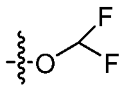, 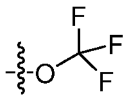, 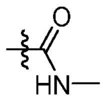 or 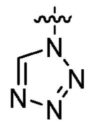.

In some more typical embodiments, $R^a$ is F, Cl, methyl,

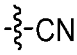, 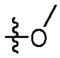, 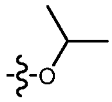, 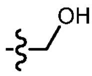,

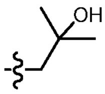, 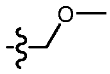, 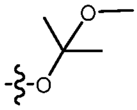, 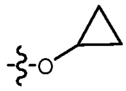, 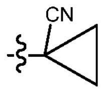 or 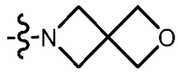.

In some more typical embodiments, $R^a$ is Cl, F, cyano, methyl, methoxyl or

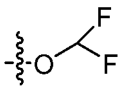.

In some more typical embodiments, $R^a$ is deuterium, Cl, F, cyano, hydroxyl, methyl, trifluoromethyl, difluoromethyl or methoxyl.

In some more typical embodiments, $R^a$ is Cl, F, cyano, hydroxyl, methyl, trifluoromethyl or methoxyl.

In some more typical embodiments, $R^a$ is Cl, F, cyano, hydroxyl, methoxyl or methyl.

In some more typical embodiments, $R^a$ is Cl, F, cyano, hydroxyl, methoxyl,

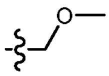

or methyl.

In some more typical embodiments, $R^a$ is

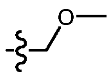

or methyl.

In some more typical embodiments, $R^a$ is methyl.

In some more typical embodiments, $R^a$ is

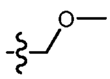.

In some embodiments, $R^3$ is

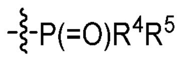, 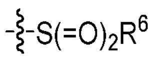, 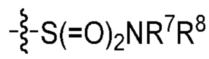,

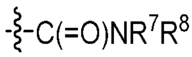 , 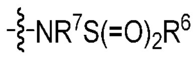 , 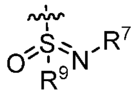 , 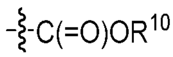 or 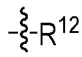 ,

wherein the definitions of $R^4$, $R^5$, $R^6$, $R^7$, $R^8$, $R^9$, $R^{10}$ and $R^{12}$ are as defined in the compound of Formula I.

In some embodiments, $R^3$ is

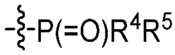 , 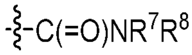 , 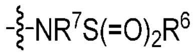 or 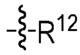 ,

wherein the definitions of $R^4$, $R^5$, $R^6$, $R^7$, $R^8$ and $R^{12}$ are as defined in the compound of Formula I.

In some embodiments, $R^3$ is

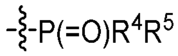 , 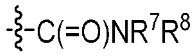 or 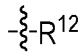 ,

wherein the definitions of $R^4$, $R^5$, $R^7$, $R^8$ and $R^{12}$ are as defined in the compound of Formula I.

In some embodiments, $R^3$ is

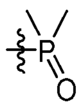 , 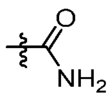 or 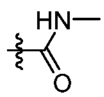 ,

which is optionally substituted by one or more deuterium.

In some embodiments, $R^3$ is

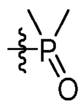 or 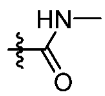 ,

which is optionally substituted by one or more deuterium.

In some embodiments, $R^3$ is

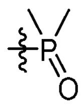 .

In some embodiments, $R^3$ is

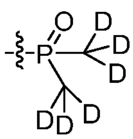 .

In some embodiments, the above compound of Formula II has a structure shown in the following compound of Formula II-1, or a pharmaceutically acceptable salt thereof:

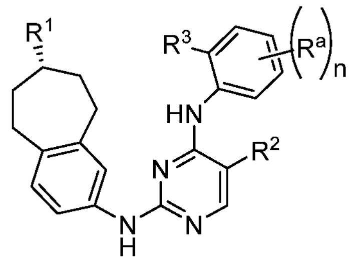

II-1

wherein the definitions of $R^1$, $R^2$, $R^3$, $R^a$ and n are as defined in the compound of Formula II.

In some embodiments, the above compound of Formula II has a structure shown in the following compound of Formula III, or a pharmaceutically acceptable salt thereof:

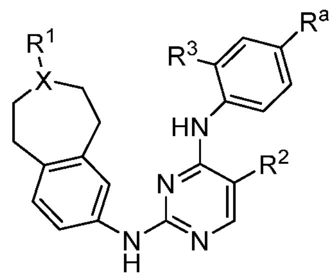

III

wherein the definitions of X, $R^1$, $R^2$, $R^3$ and $R^a$ are consistent with those defined in the compound of Formula II.

In some embodiments, the above compound of Formula II has a structure shown in the following compound of Formula III-1, or a pharmaceutically acceptable salt thereof:

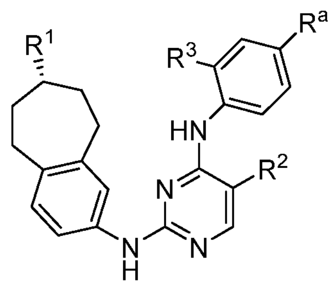

III-1

wherein the definitions of $R^1$, $R^2$, $R^3$ and $R^a$ are consistent with those defined in the compound of Formula II.

In some embodiments, the above compound of Formula I has a structure shown in the following compound of Formula IV, or a pharmaceutically acceptable salt thereof:

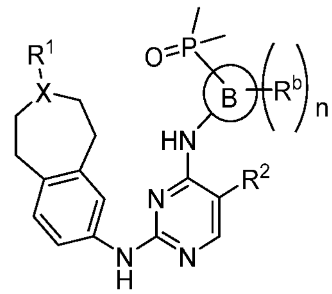

IV

wherein the definitions of X, $R^1$ and $R^2$ are consistent with those as defined in the compound of Formula I;
n is an integer from 0 to 4;
ring B is selected from phenyl, 5-6 membered heteroaryl, or 9-12 membered benzoheterocyclyl optionally substituted by

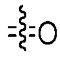;

and
$R^b$ is deuterium, halogen, $C_{1-6}$ alkyl, $C_{1-6}$ alkoxyl, $C_{3-10}$ cycloalkyloxyl,

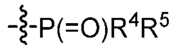,

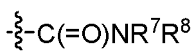, 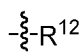, 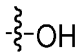 or 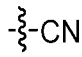,

wherein the $C_{1-6}$ alkyl or $C_{1-6}$ alkoxyl is optionally substituted by one or more methoxyl, hydroxyl, deuterium, halogen or cyano; and the definitions of $R^4$, $R^5$, $R^7$, $R^8$ and $R^{12}$ are consistent with those defined in the compound of Formula I.

In some embodiments, $R^b$ is deuterium, halogen, $C_{1-6}$ alkyl, $C_{1-6}$ alkoxyl,

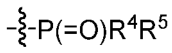,

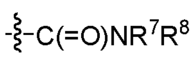, 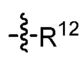, 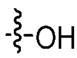 or 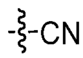,

wherein the $C_{1-6}$ alkyl or $C_{1-6}$ alkoxyl is optionally substituted by one or more deuterium, halogen or cyano; and the definitions of $R^4$, $R^5$, $R^7$, $R^8$ and $R^{12}$ are consistent with those defined in the compound of Formula I.

In some embodiments, the definitions of X, $R^1$ and $R^2$ are consistent with those defined in the compound of Formula I;

n is an integer from 0 to 4;
ring B is selected from phenyl, 5-6 membered heteroaryl or 9-12 membered benzoheterocyclyl optionally substituted by

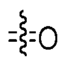;

$R^b$ is deuterium, halogen, $C_{1-6}$ alkyl, $C_{1-6}$ alkoxyl,

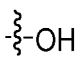 or 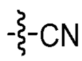,

wherein the $C_{1-6}$ alkyl or $C_{1-6}$ alkoxyl is optionally substituted by one or more deuterium, halogen or cyano.

In some embodiments, n is 0, 1, 2, or 3.
Further, in some embodiments, n is 0, 1 or 2.
Further, in some embodiments, n is 0 or 1.
In some more typical embodiments, n is 1.
In some embodiments, $R^b$ is deuterium, Cl, F, Br, cyano, hydroxyl,

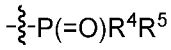,

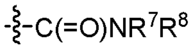, 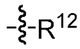,

or methyl or methoxyl optionally substituted by one or more halogen, wherein the definitions of $R^4$, $R^5$, $R^7$, $R^8$ and $R^{12}$ are consistent with those defined in the compound of Formula I.

In some embodiments, $R^b$ is deuterium, Cl, F, Br, cyano, hydroxyl,

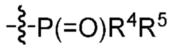,

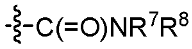, 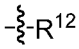,

or methyl or methoxyl optionally substituted by one or more F, wherein the definitions of $R^4$, $R^5$, $R^7$, $R^8$ and $R^{12}$ are consistent with those defined in the compound of Formula I.

In some more typical embodiments, $R^b$ is deuterium, Cl, F, Br, cyano, hydroxyl, methyl, trifluoromethyl,

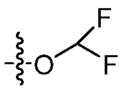, 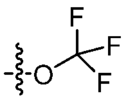, 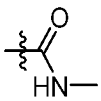, 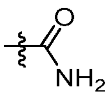, 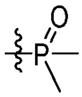, 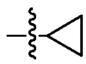 or 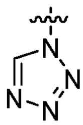.

In some more typical embodiments, $R^b$ is Cl, F, cyano, hydroxyl, methyl, trifluoromethyl,

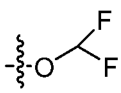, 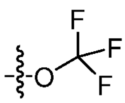, 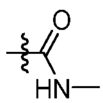 or 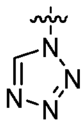.

In some more typical embodiments, $R^b$ is Cl, F, cyano, hydroxyl, methyl,

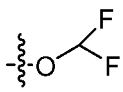,

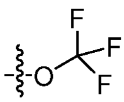, 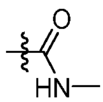 and 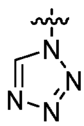.

In some more typical embodiments, $R^b$ is Cl, F, cyano, methyl, methoxyl or

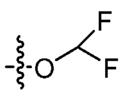.

In some embodiments, $R^b$ is deuterium, Cl, F, cyano, or methyl or methoxyl optionally substituted by one or

more halogen.

In some typical embodiments, $R^b$ is deuterium, Cl, F, cyano, or methyl or methoxyl optionally substituted by one or more F.

In some more typical embodiments, $R^b$ is deuterium, F, Cl, Br, methyl, trifluoromethyl,

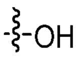, 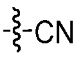, 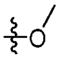, 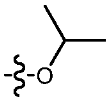, 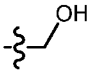, 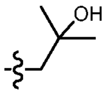, 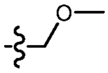, 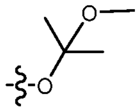, 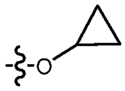,

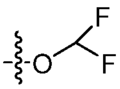, 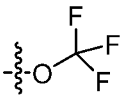, 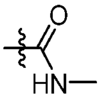, 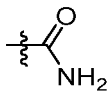, 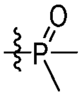, 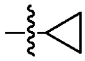, 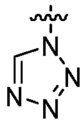, 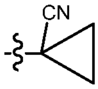

or

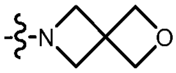.

In some more typical embodiments, $R^b$ is F, Cl, methyl,

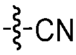, 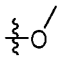, 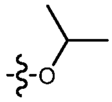, 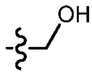,

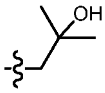, 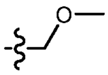, 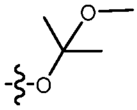, 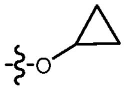, 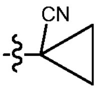 or 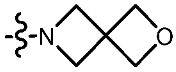.

In some more typical embodiments, $R^b$ is F, Cl, methyl,

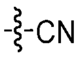, 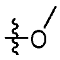, 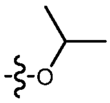, 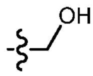,

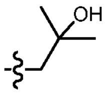, 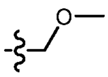, 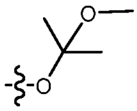, 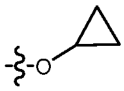, 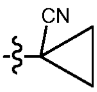 or 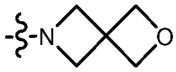.

In some more typical embodiments, $R^b$ is deuterium, Cl, F, cyano, methyl, trifluoromethyl, difluoromethyl or methoxyl.

In some more typical embodiments, $R^b$ is Cl, F, cyano, methyl, or methoxyl.

In some more typical embodiments, $R^b$ is Cl, F, cyano,

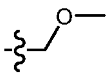,

or methyl.

In some more typical embodiments, $R^b$ is Cl, F, cyano, or methyl.

In some more typical embodiments, $R^b$ is methyl.

In some more typical embodiments, $R^b$ is

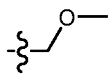.

In some embodiments, ring B is phenyl, furyl, thienyl, pyrrolyl, pyrazolyl, imidazolyl, thiazolyl, oxazolyl, 1,2,3-triazolyl, 1,2,4-triazolyl, pyridyl, pyrimidinyl, pyridazinyl, pyrazinyl, benzo five-membered heterocyclyl or benzo six-membered heterocyclyl.

In some embodiments, ring B is phenyl, furyl, thienyl, pyrrolyl, pyrazolyl, imidazolyl, thiazolyl, oxazolyl, 1,2,3-triazolyl, 1,2,4-triazolyl, pyridyl, pyrimidinyl, pyridazinyl, pyrazinyl,

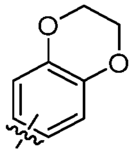, 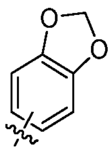, 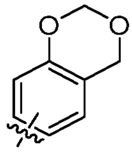 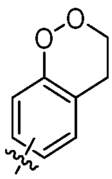 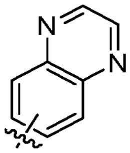, 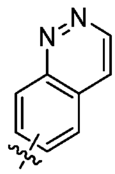, 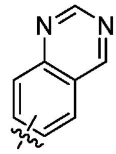,

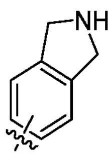, 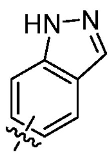, 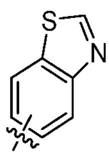 or 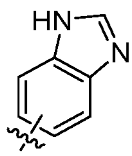.

In some typical embodiments, ring B is phenyl, furyl, thiazolyl, pyridyl,

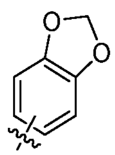,

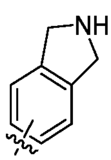 or 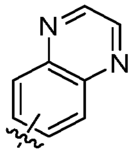.

In some typical embodiments, ring B is phenyl, pyridyl,

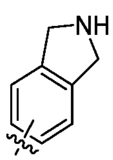 or 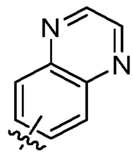.

In some typical embodiments, ring B is phenyl, pyridyl, or

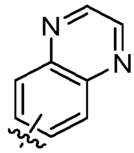.

In some typical embodiments, ring B is phenyl, pyridyl, or

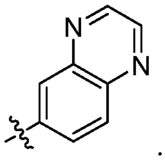.

In some more typical embodiments, ring B is phenyl, or pyridyl.
In some more typical embodiments, ring B is phenyl.
In some embodiments,

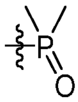 and 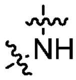

are positionally adjacent at ring B.
In some typical embodiments, $R^1$ is

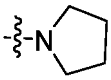, 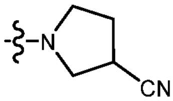, 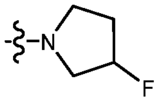, 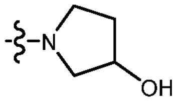,

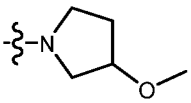, 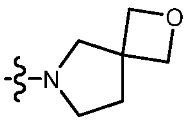, 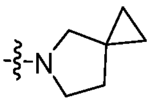, 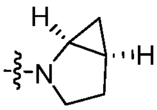, 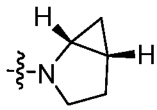, 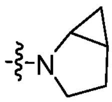,

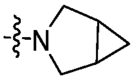, 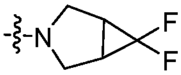, 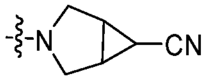, 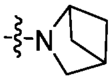, 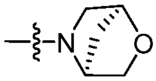, 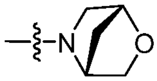,

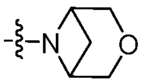, 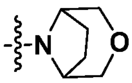, 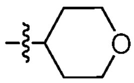 or 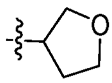.

In some typical embodiments, $R^1$ is

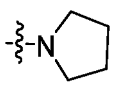, 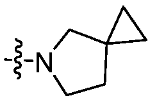, 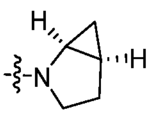, 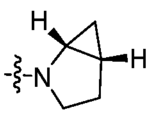,

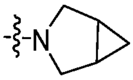, 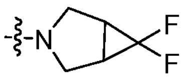, 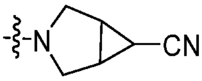, 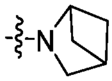, 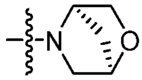, 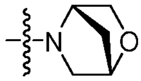,

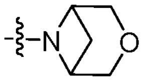 or 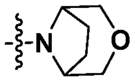.

In some typical embodiments, $R^1$ is

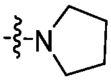, 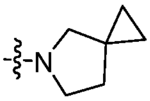, 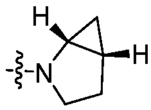, 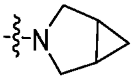,

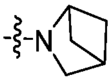, 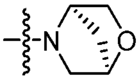, 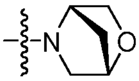, 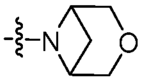 or 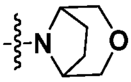.

In some typical embodiments, $R^1$ is

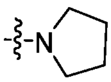, 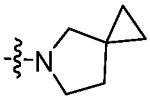, 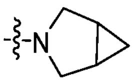, 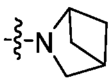,

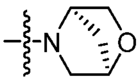, 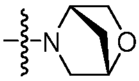, 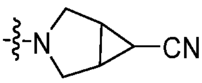, 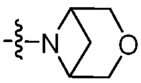 or 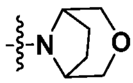.

In some typical embodiments, $R^1$ is

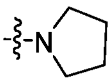, 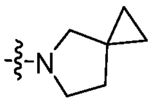, 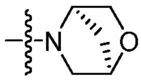, 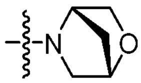,

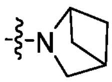, 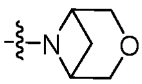 or 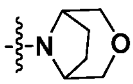.

In some embodiments, $R^1$ is selected from

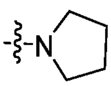, 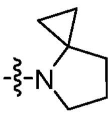, 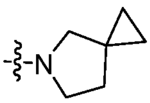, 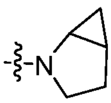,

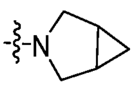, 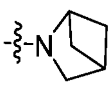, 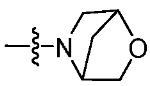, 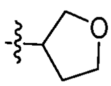 or 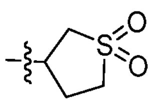.

In some typical embodiments, $R^1$ is

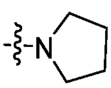, 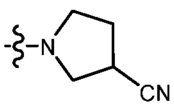, 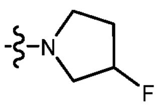, 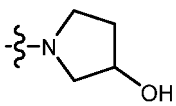,

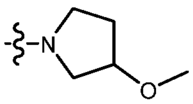, 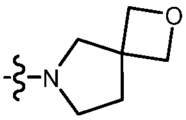 or 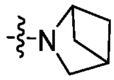.

In some more typical embodiments, $R^1$ is

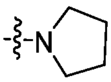, 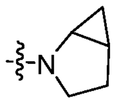, 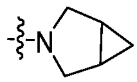 or 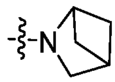.

In some more typical embodiments, $R^1$ is

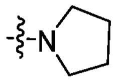.

In some embodiments, the above compound of Formula IV has a structure shown in the following compound of Formula IV-1, or a pharmaceutically acceptable salt thereof:

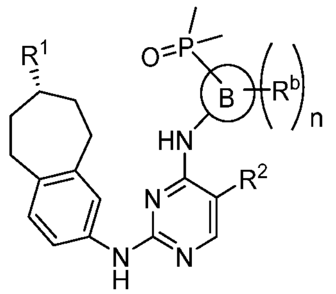

IV-1

wherein the definitions of $R^1$, $R^2$, $R^b$, n and ring B are consistent with those defined in the compound of Formula IV.

In some embodiments, the above compound of Formula IV has a structure shown in the following compound of Formula V, or a pharmaceutically acceptable salt thereof:

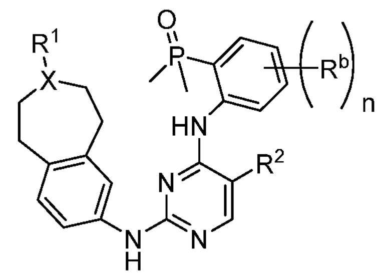

V

wherein the definitions of X, $R^1$, $R^2$, $R^b$ and n are consistent with those defined in the compound of Formula IV.

In some embodiments, the above compound of Formula IV has a structure shown in the following compound of Formula VI, or a pharmaceutically acceptable salt thereof:

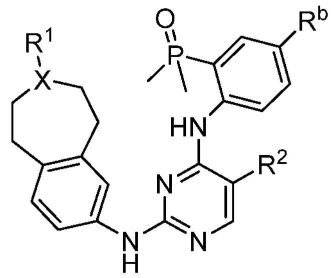

VI

wherein the definitions of X, $R^1$, $R^2$ and $R^b$ are consistent with those defined in the compound of Formula IV.

In some typical embodiments, $R^1$ is

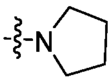, 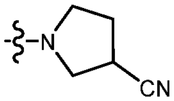, 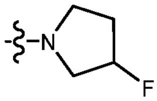, 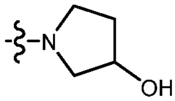,

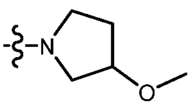, 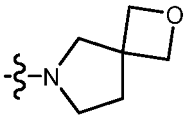, 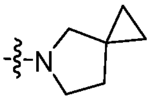, 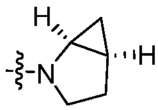, 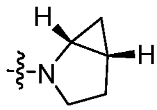, 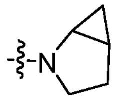,

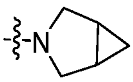, 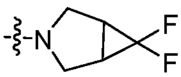, 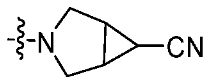, 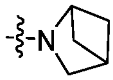, 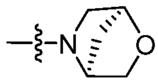, 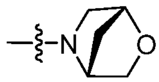,

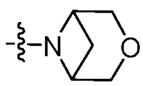, 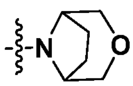, 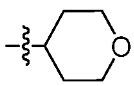 or 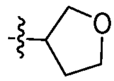.

In some typical embodiments, $R^1$ is

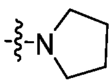, 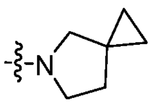, 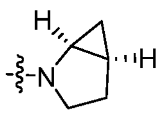, 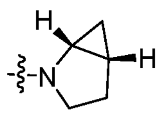,

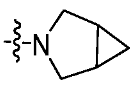, 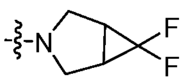, 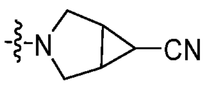, 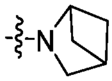, 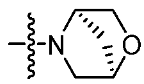, 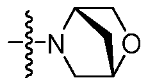,

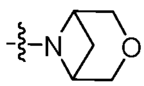 or 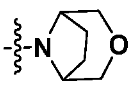.

In some typical embodiments, $R^1$ is

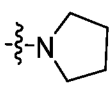, 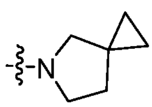, 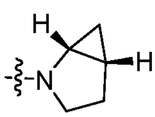, 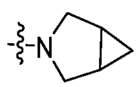,

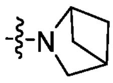, 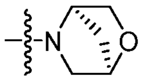, 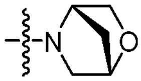, 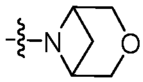 or 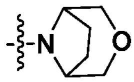.

In some typical embodiments, $R^1$ is

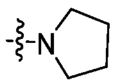, 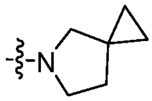, 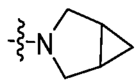, 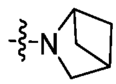,

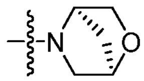, 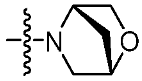, 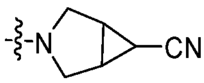, 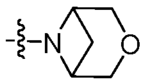 or 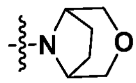.

In some typical embodiments, $R^1$ is

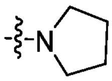, 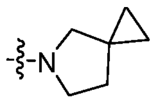, 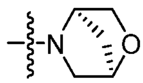, 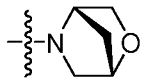,

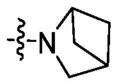, 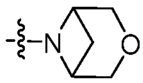 or 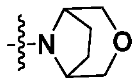.

In some typical embodiments, $R^1$ is

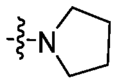, 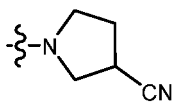, 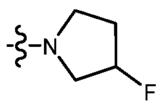, 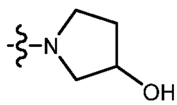,

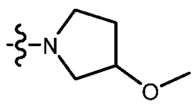, 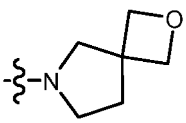 or 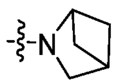.

In some more typical embodiments, $R^1$ is

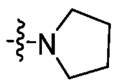.

In some more typical embodiments, $R^b$ is F, Cl, methyl,

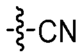, 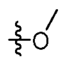, 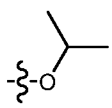, 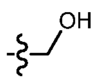,

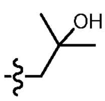, 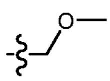, 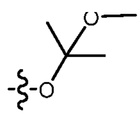, 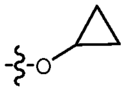, 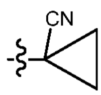 or 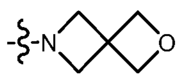.

In some more typical embodiments, $R^b$ is deuterium, Cl, F, Br, cyano, hydroxyl, methyl, trifluoromethyl,

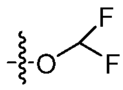, 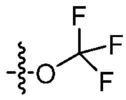, 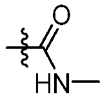, 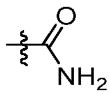, 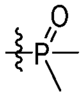, 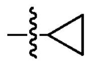 or 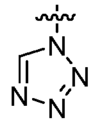.

In some more typical embodiments, $R^b$ is Cl, F, cyano, hydroxyl, methyl, trifluoromethyl,

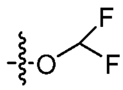, 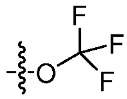, 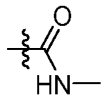 or 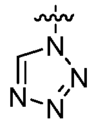.

In some more typical embodiments, $R^b$ is Cl, F, cyano, hydroxyl, methyl,

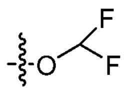,

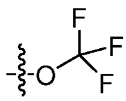, 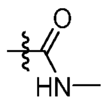 or 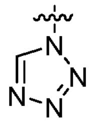.

In some more typical embodiments, $R^b$ is Cl, F, cyano, methyl, methoxyl,

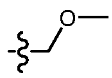

or

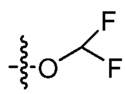.

In some more typical embodiments, $R^b$ is Cl, F, cyano, methyl, methoxyl or

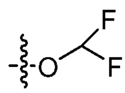.

In some more typical embodiments, $R^b$ is methyl.

In some more typical embodiments, $R^b$ is

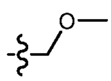.

In some specific embodiments, the present invention provides the following compounds, or pharmaceutically acceptable salts thereof:

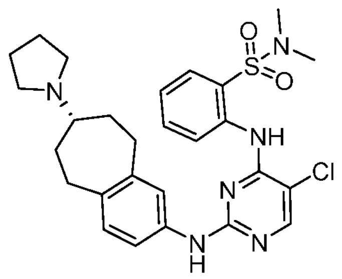
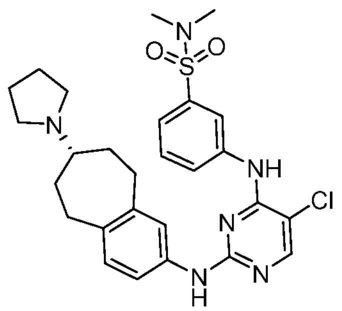
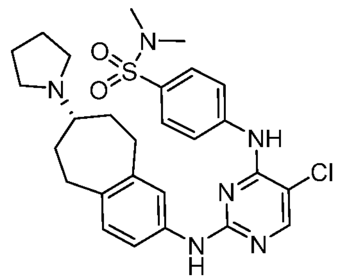

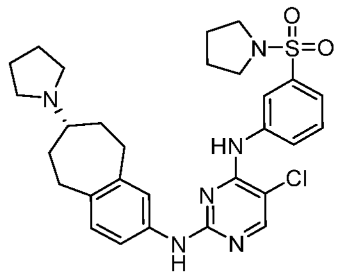
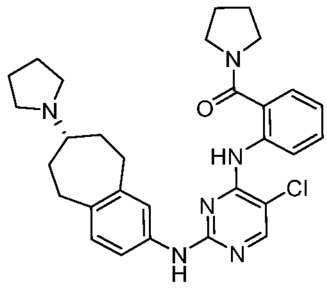
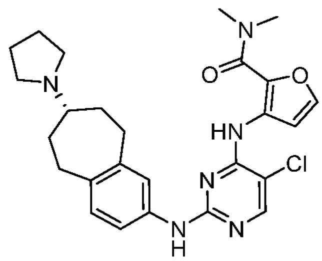

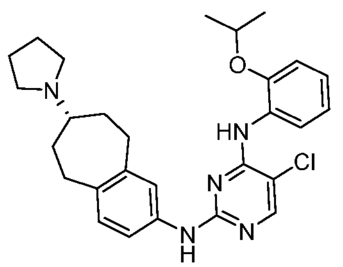
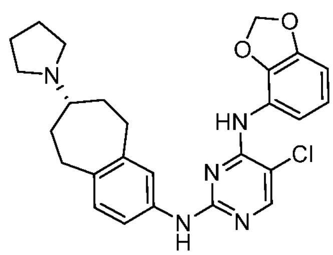
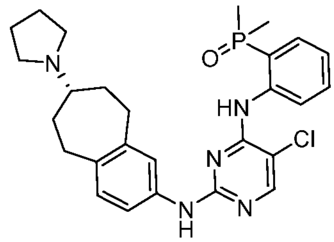

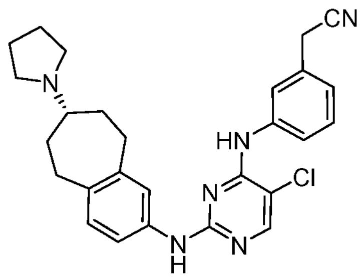
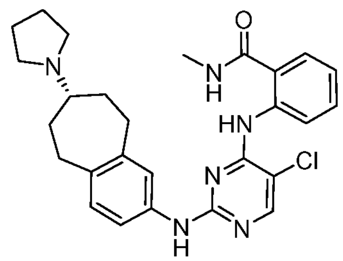
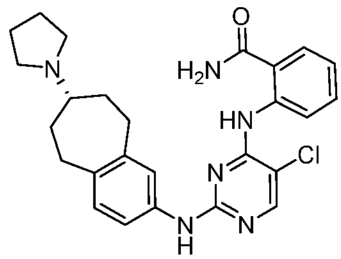

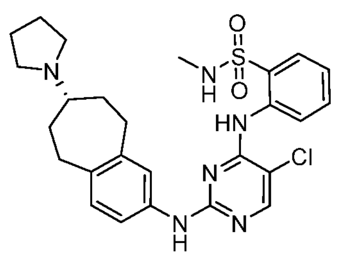
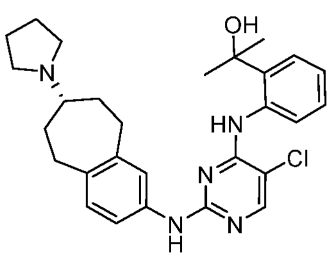
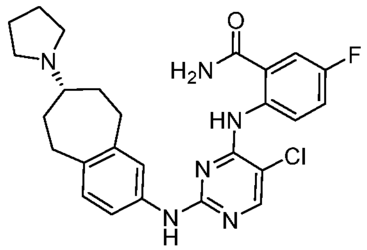

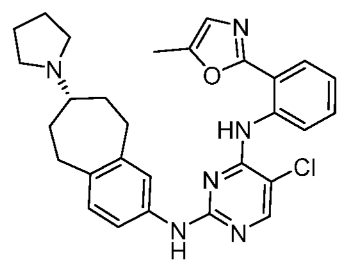
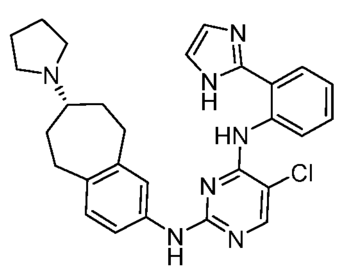
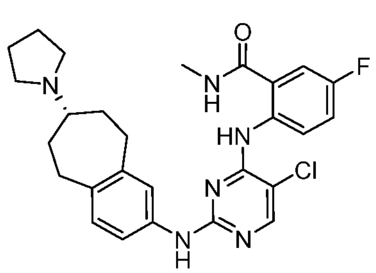
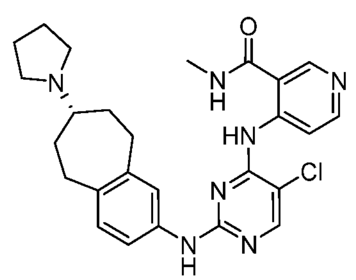
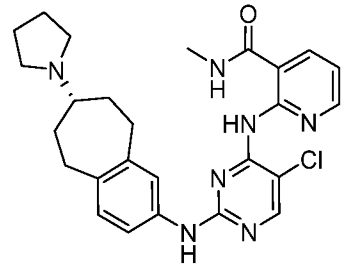
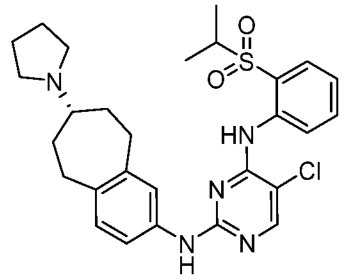
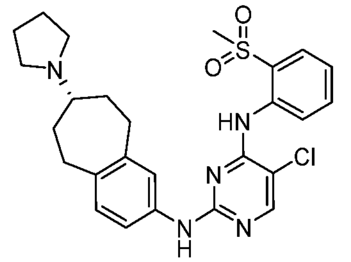
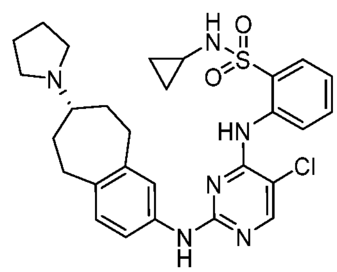
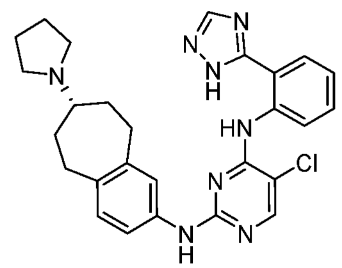
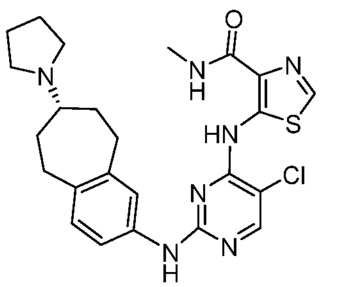
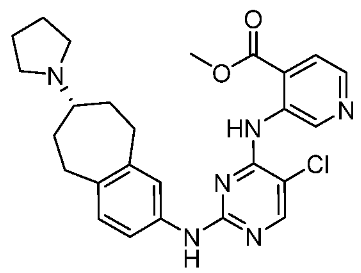
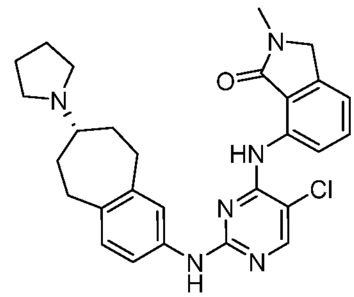
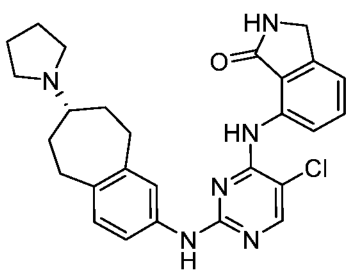
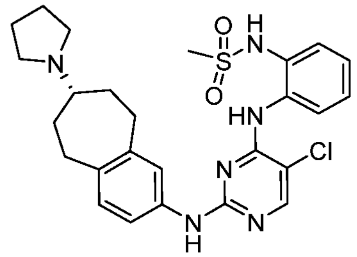
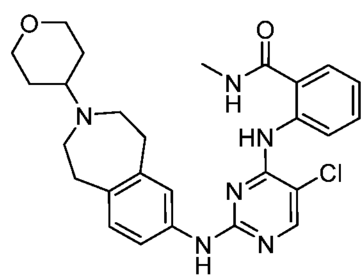

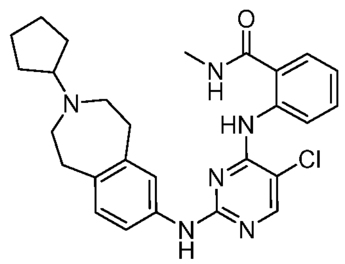

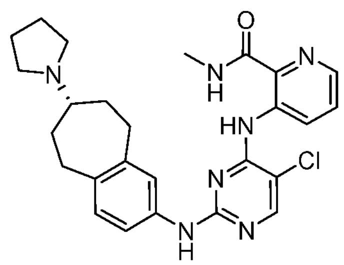

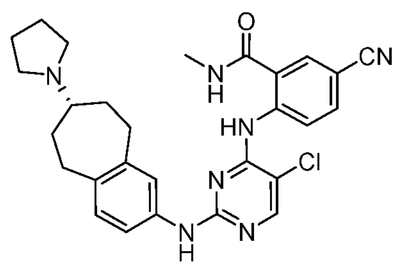

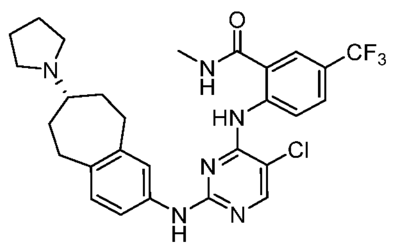

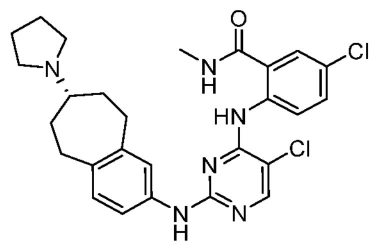

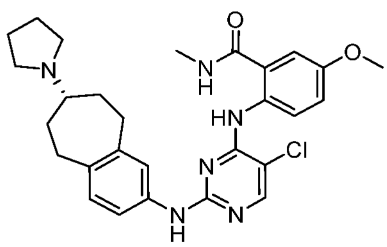

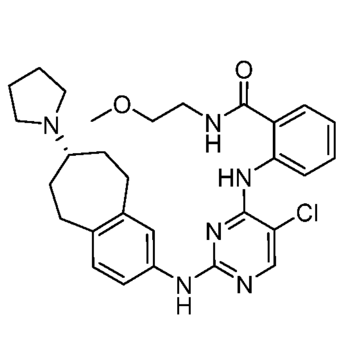

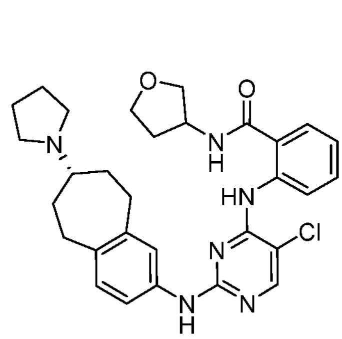

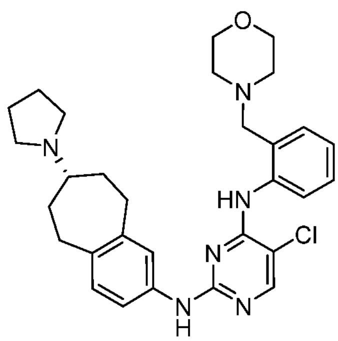

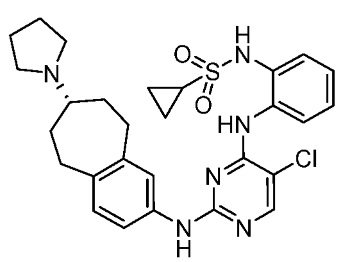

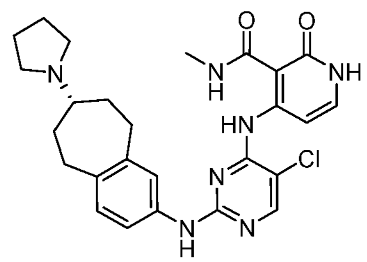

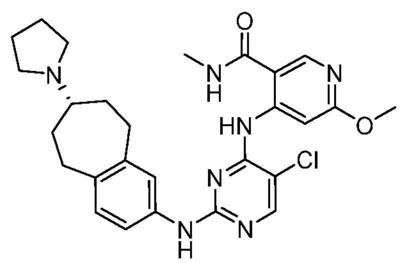

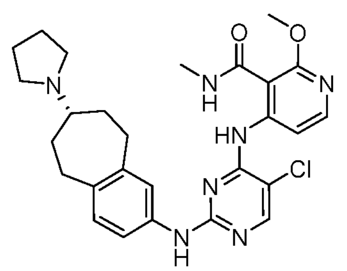

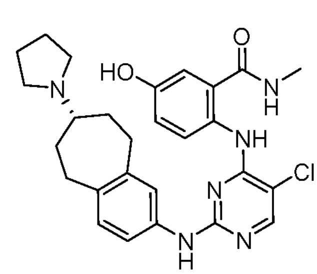

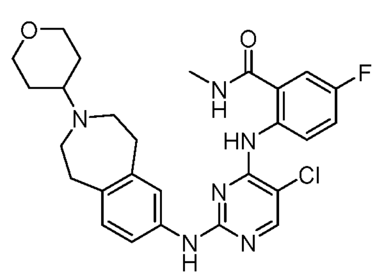

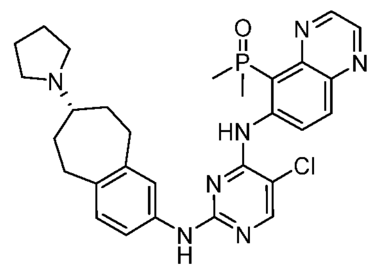
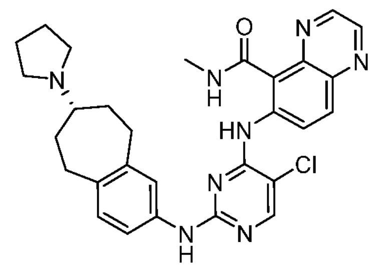
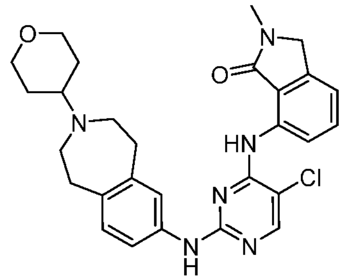
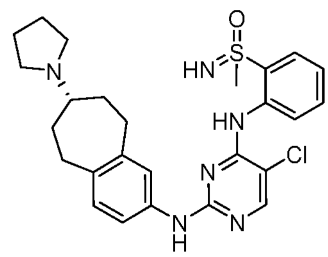
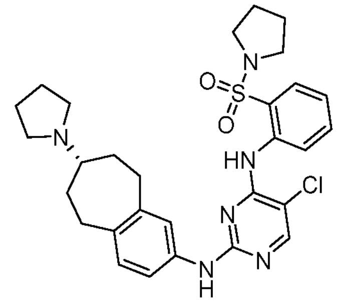
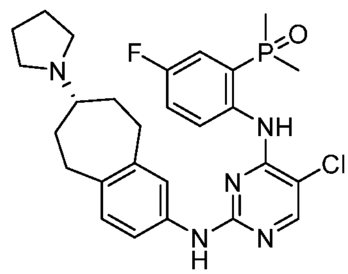
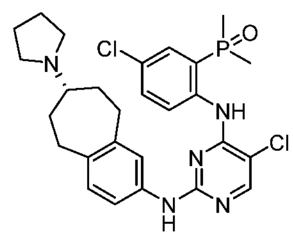
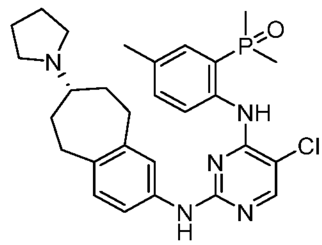
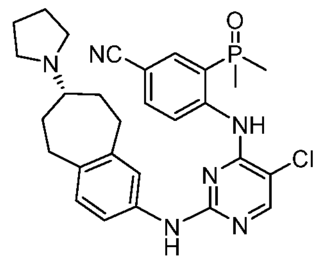
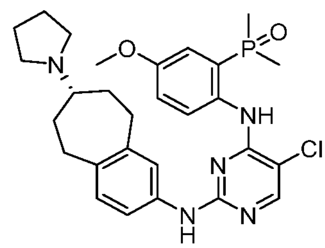
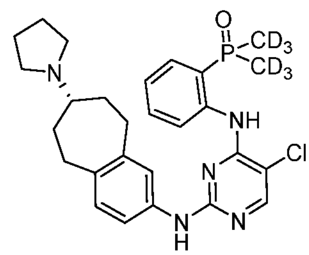
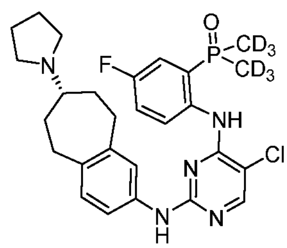
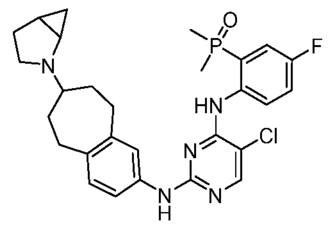
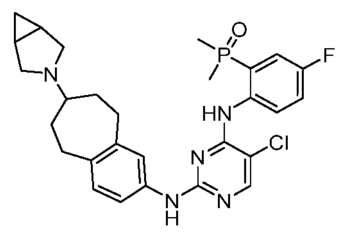
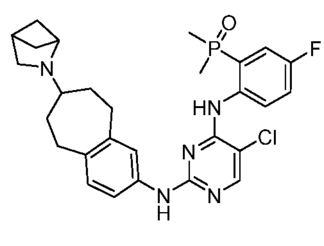
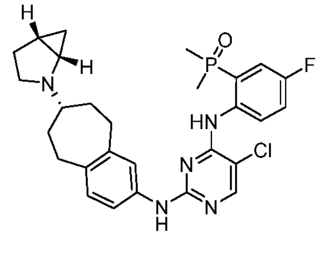
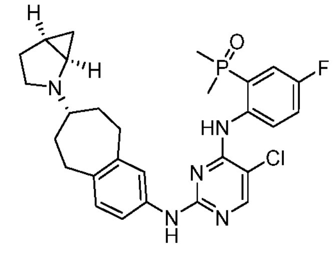
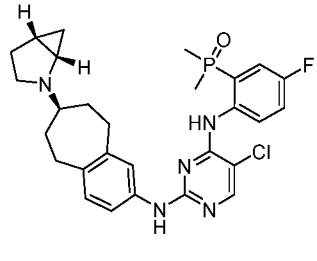

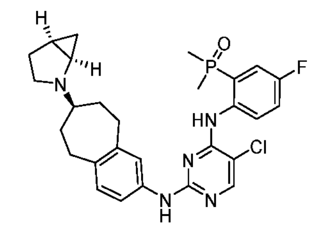

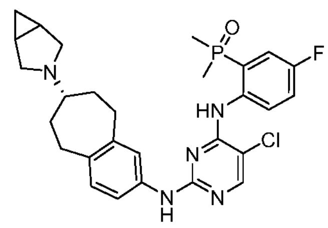

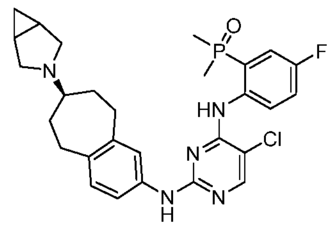

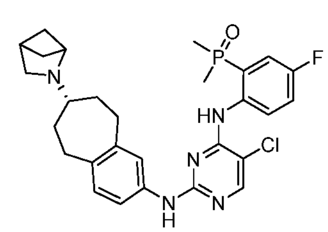

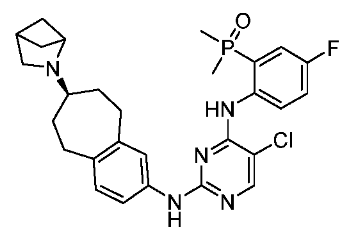

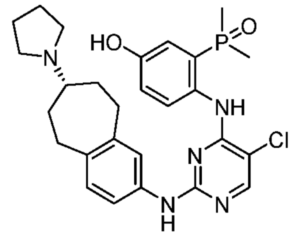

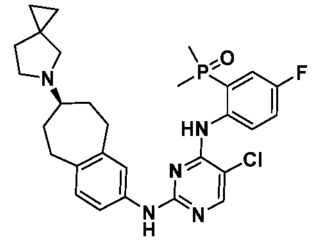

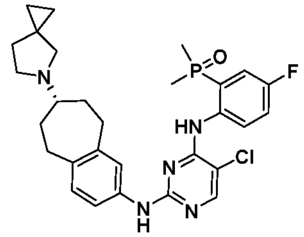

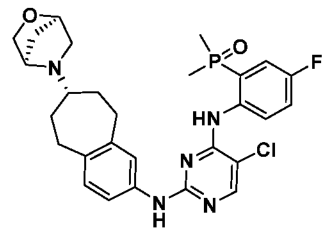

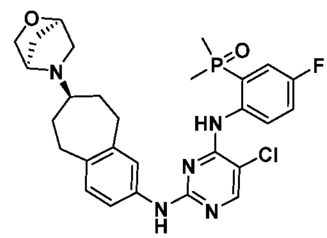

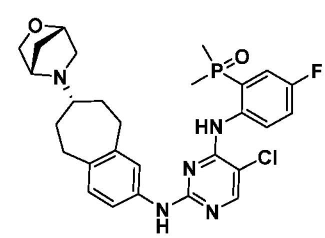

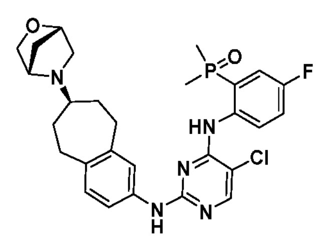

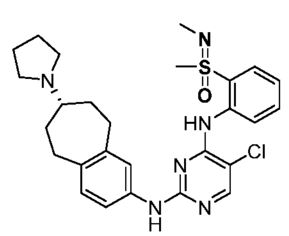

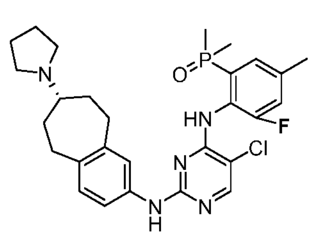

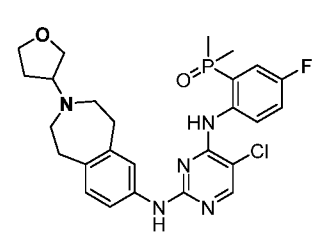

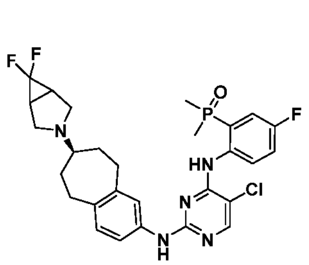

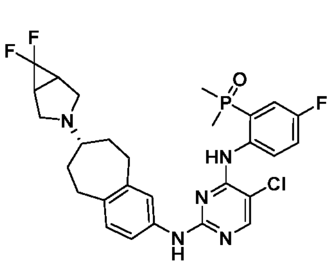

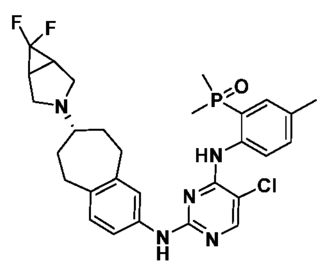

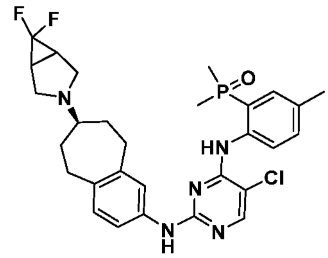
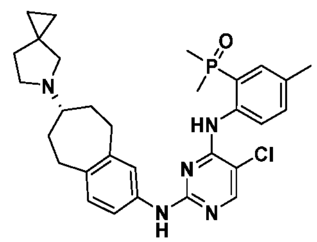
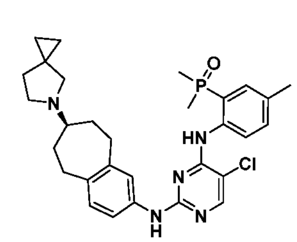

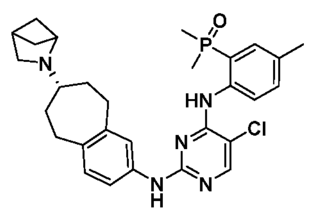
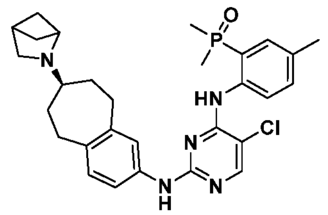
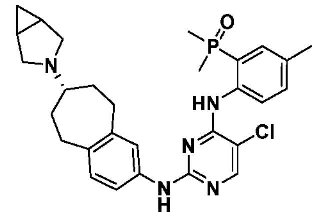

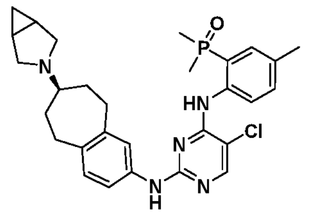
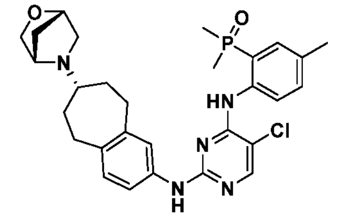
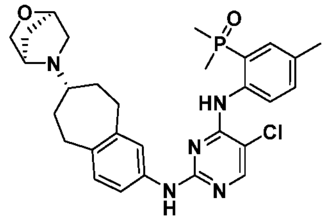

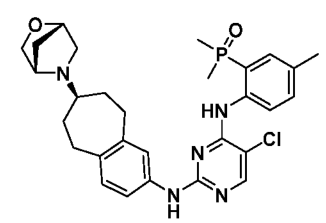
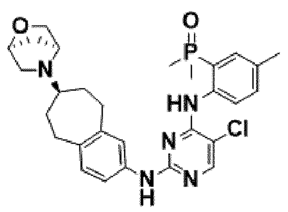
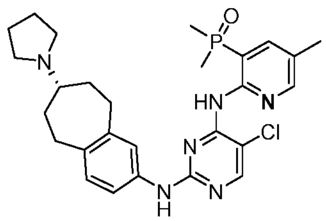

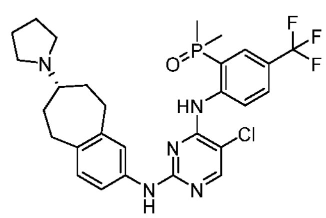
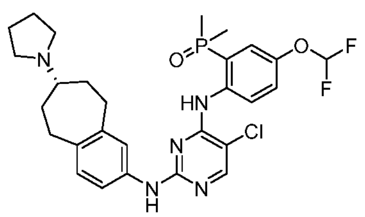
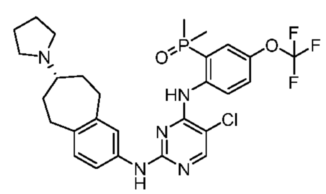

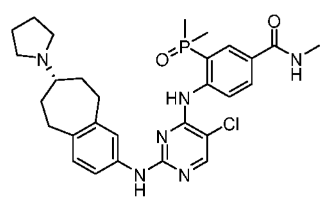
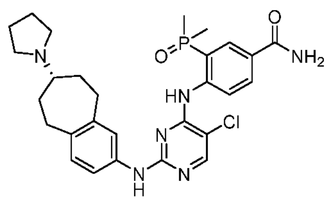
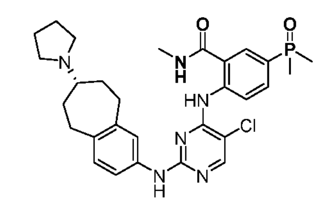

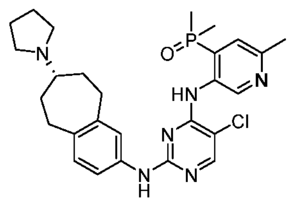
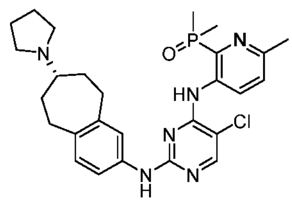
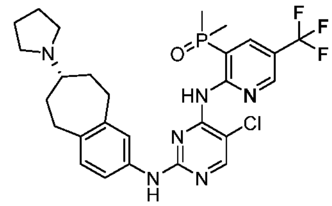

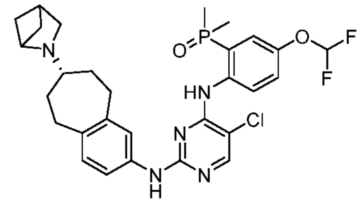
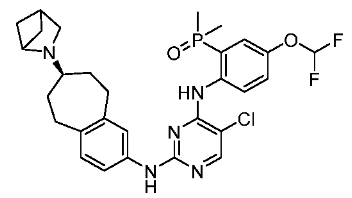
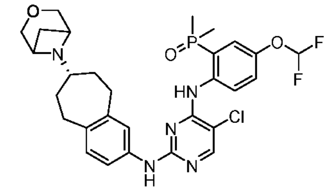

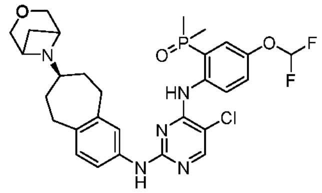
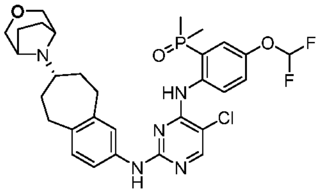
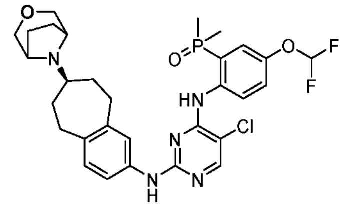

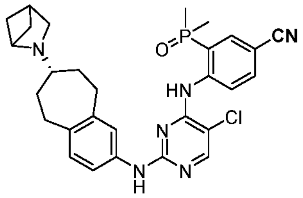
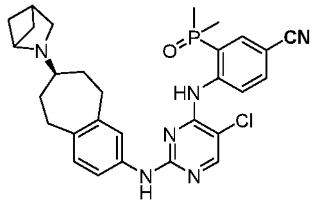
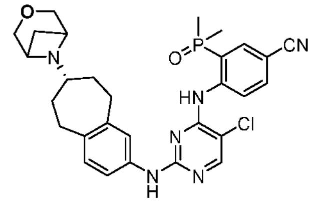

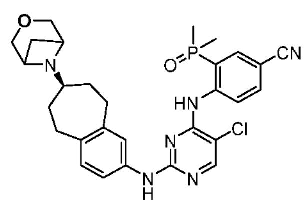
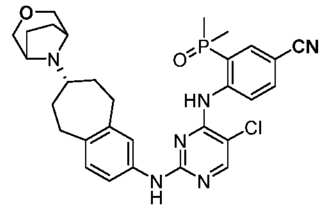
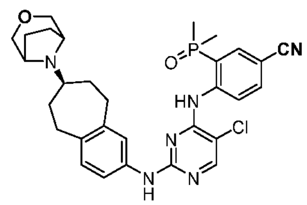

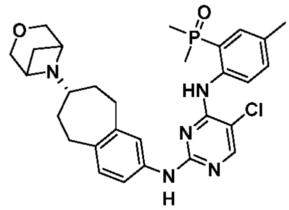
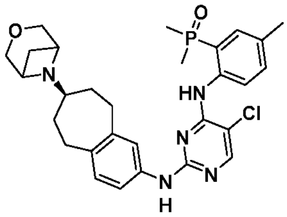
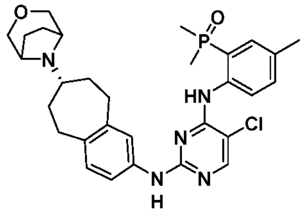
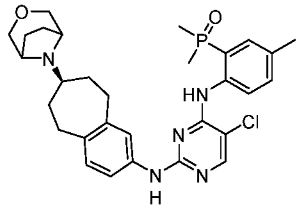

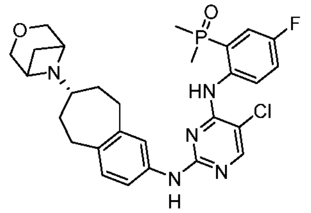
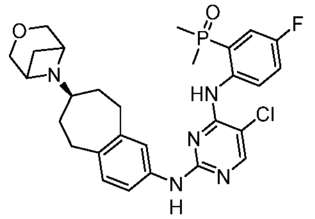
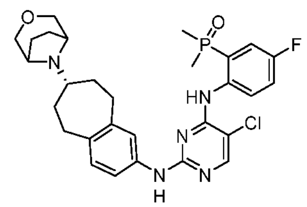

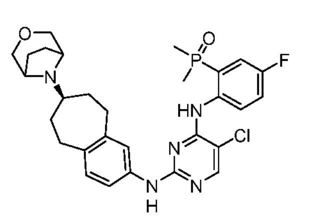
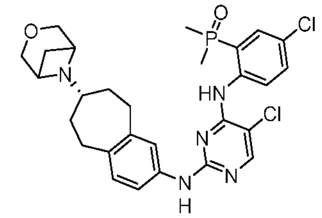
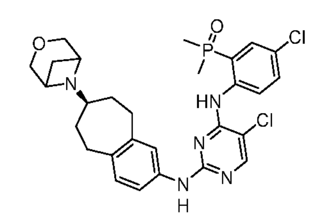

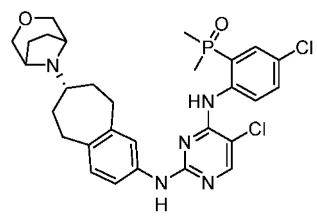
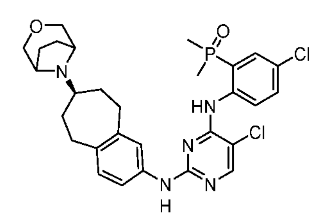
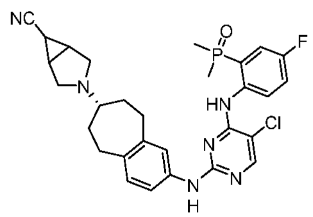

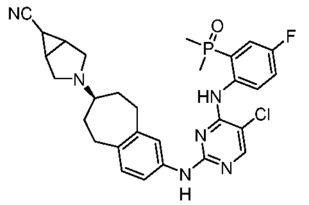
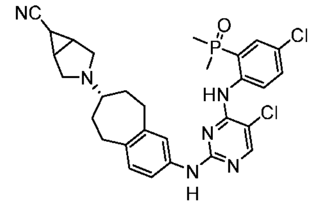
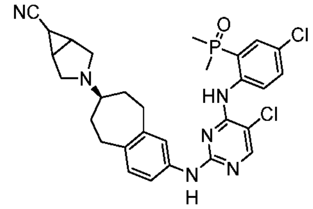

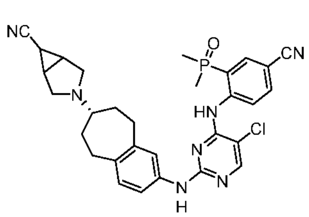
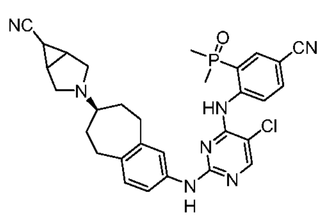
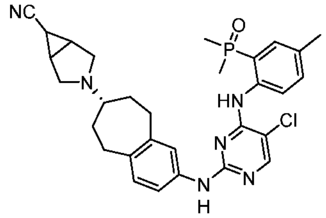

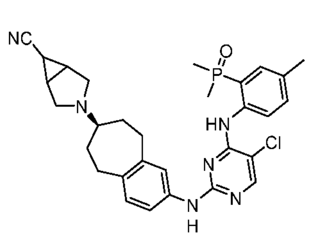
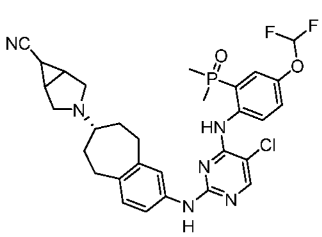
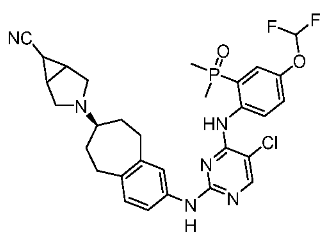

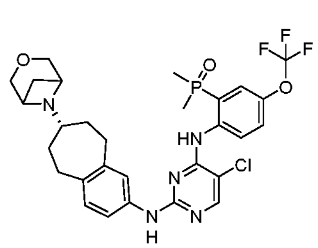
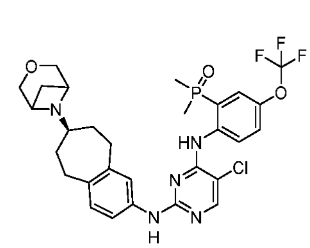
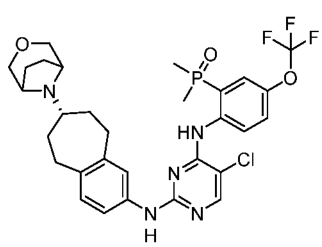

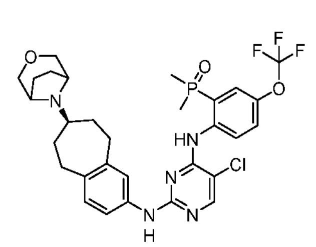
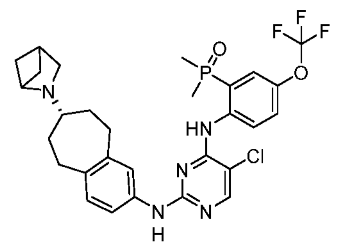
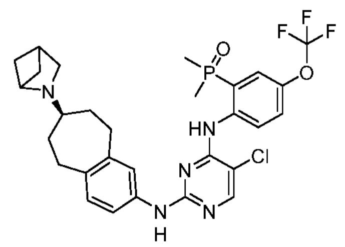
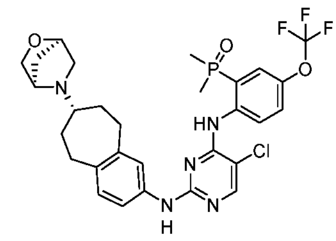
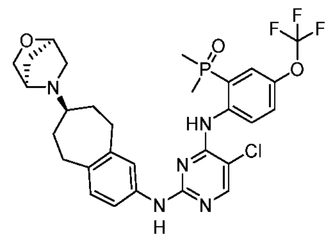
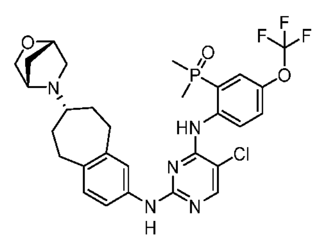
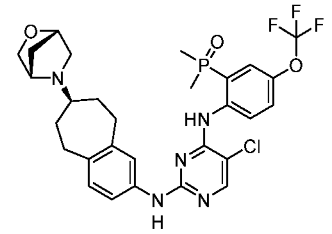
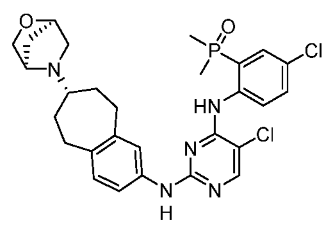
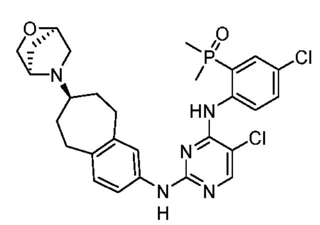
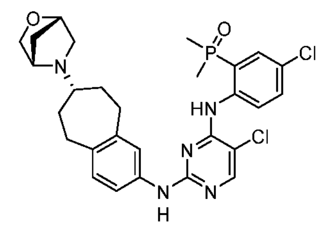
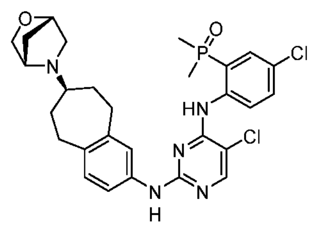
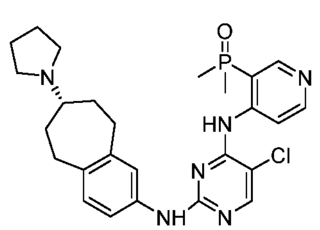
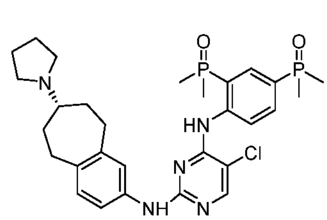
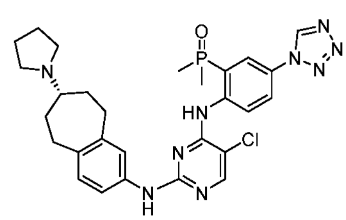
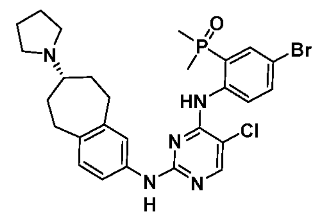
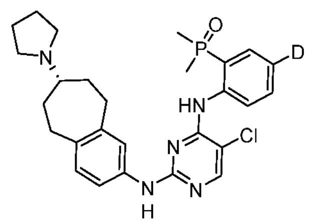
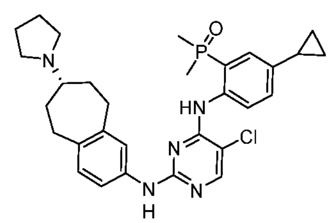
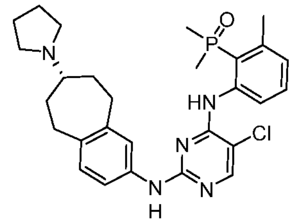

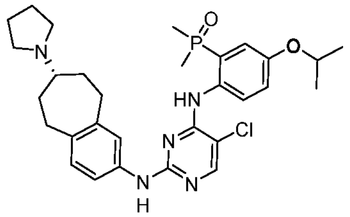
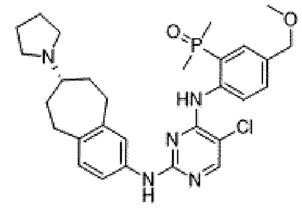
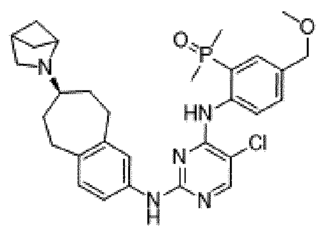

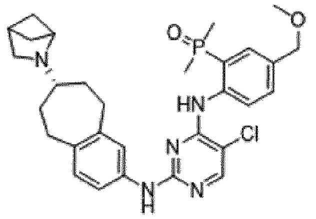
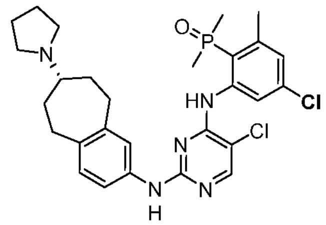
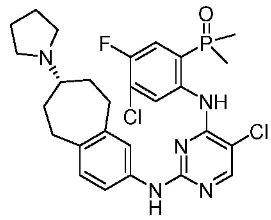

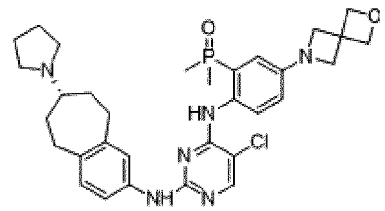
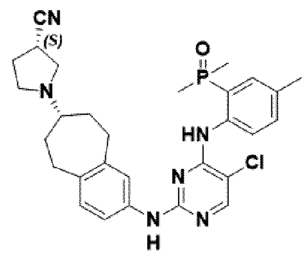
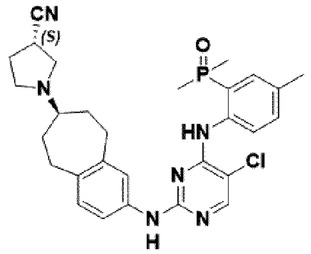

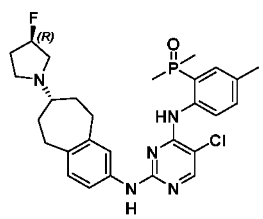
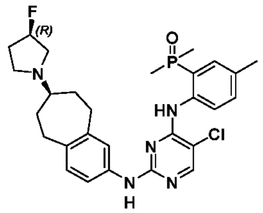
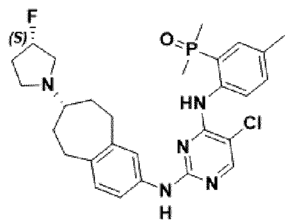

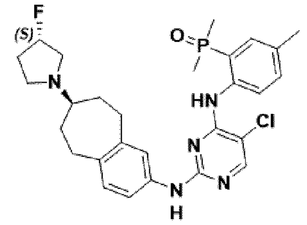
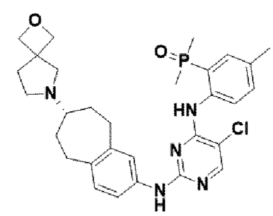
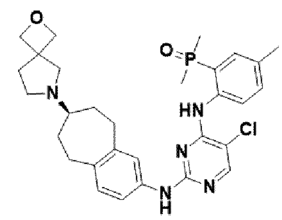

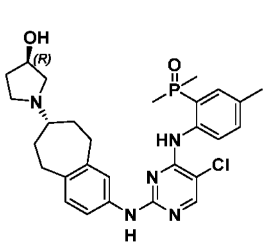
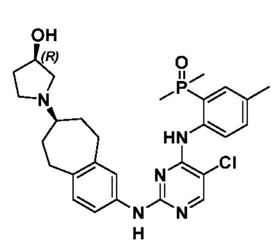
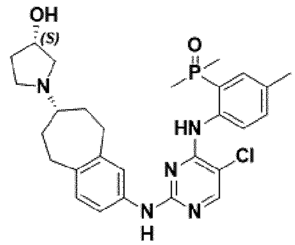

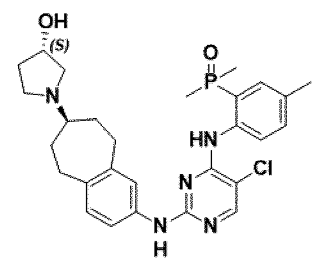
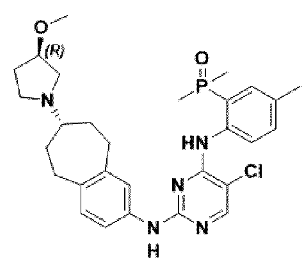
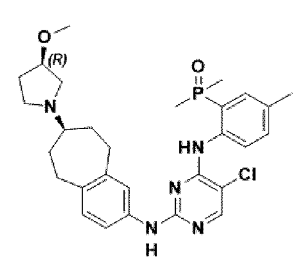

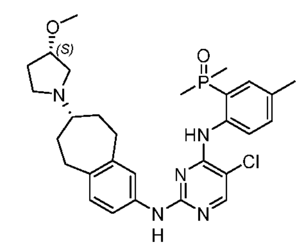
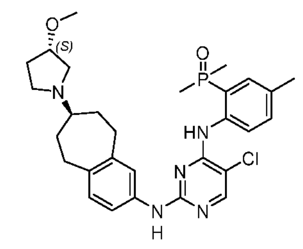
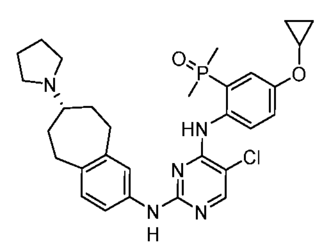

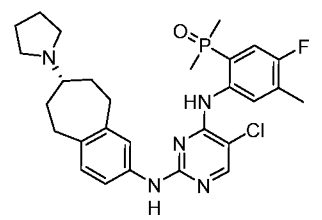
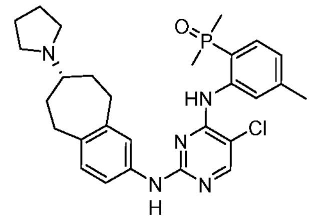
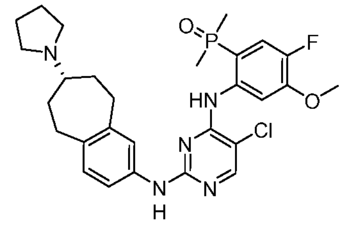

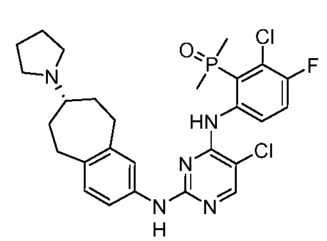
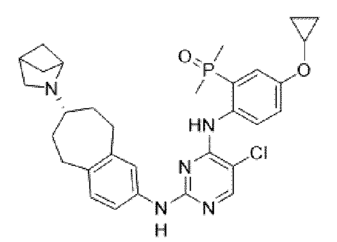
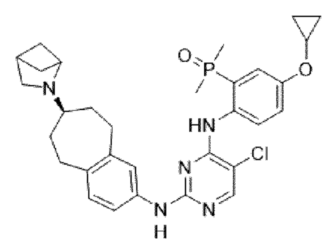

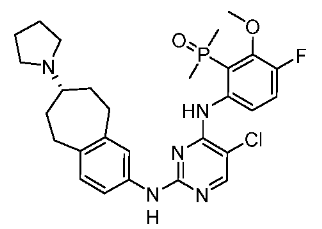
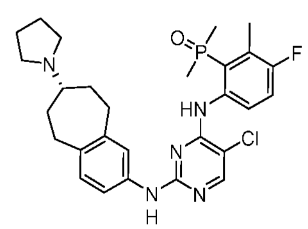
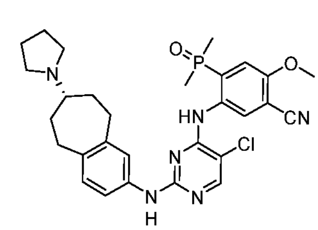

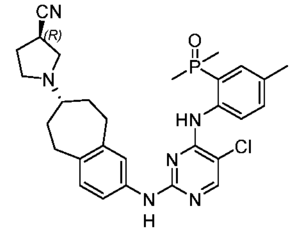
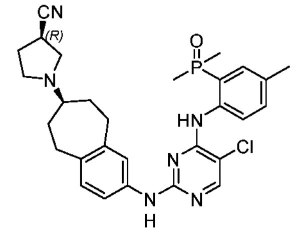
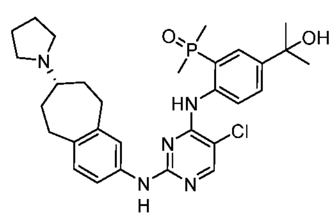

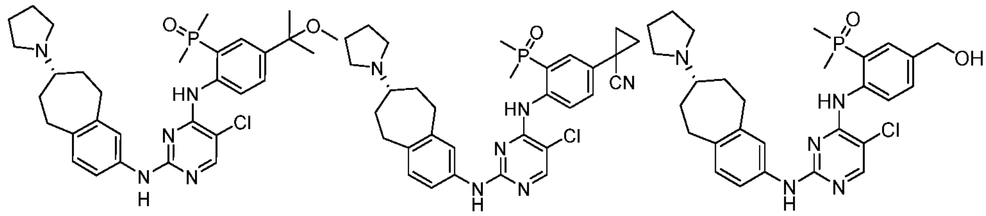

.

Further, in some specific embodiments, an isomer of the compound

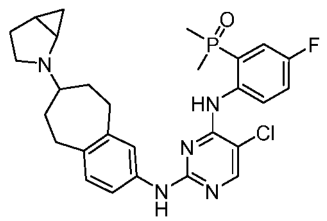

is chirally resolved by chiral liquid chromatography, wherein the chiral HPLC retention time is 1.96 min, and specific chromatographic conditions of resolution are as follows:

chromatographic column: Chiralpak IA, 2×25 cm, with a filler particle size of 5 μm;
mobile phase A: n-hexane (10 mM ammonia-methanol); mobile phase B: ethanol;
flow rate: 18 ml/min;
gradient: 50%B within 16 min, isogradient;
detection wavelength: 220/254 nm dual-wavelength detection; and
column temperature: 25°C.

Further, in some specific embodiments, an isomer of the compound

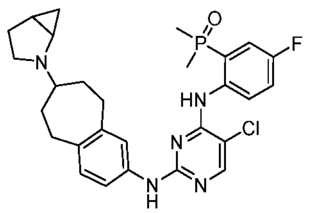

is chirally resolved by chiral liquid chromatography, wherein the chiral HPLC retention time is 3.58 min, and specific chromatographic conditions of resolution are as follows:

chromatographic column: Chiralpak IA, 2×25 cm, with a filler particle size of 5 μm;
mobile phase A: n-hexane (10 mM ammonia-methanol); mobile phase B: ethanol;
flow rate: 18 ml/min;
gradient: 50%B within 16 min, isogradient;
detection wavelength: 220/254 nm dual-wavelength detection; and
column temperature: 25°C.

Further, in some specific embodiments, an isomer of the compound

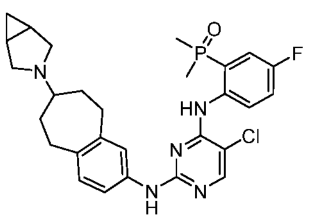

is chirally resolved by chiral liquid chromatography, wherein the chiral HPLC retention time is 8.10 min, and specific chromatographic conditions are as follows:

chromatographic column: CHIRALPAK IF, 2×25 cm, with a filler particle size of 5 μm;
mobile phase A: n-hexane (0.1% diethanolamine); mobile phase B: ethanol;
flow rate: 20 ml/min;
gradient: 20%B within 21 min, isogradient;
detection wavelength: 220/254 nm dual-wavelength detection; and
column temperature: 25°C.

Further, in some specific embodiments, an isomer of the compound

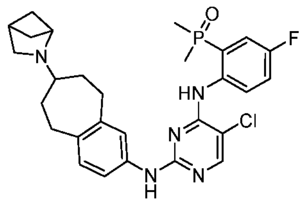

is chirally resolved by chiral liquid chromatography, wherein the chiral HPLC retention time is 1.67 min, and specific chromatographic conditions are as follows:

chromatographic column: CHIRALPAK IG, 2 cm×25 cm, with a filler particle size of 5 μm;
mobile phase A: methyl tert-butyl ether (10 mM ammonia-methanol); mobile phase B: ethanol;
flow rate: 20ml/min;
gradient: 20%B within 10 min, isogradient;
detection wavelength: 220/254 nm dual-wavelength detection; and
column temperature: 25°C.

Further, in some specific embodiments, an isomer of the compound

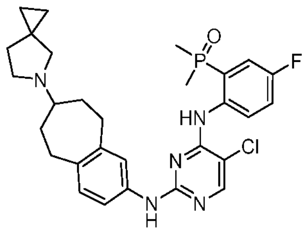

is chirally resolved by chiral liquid chromatography, wherein the chiral HPLC retention time is 9.8min, and specific chromatographic conditions are as follows:

chromatographic column: CHIRALPAK IC, 2 cm×25 cm, with a filler particle size of 5 μm;
mobile phase A: n-hexane (10 mM ammonia-methanol); mobile phase B: ethanol;
flow rate: 20ml/min;
gradient: 50%B within 12min, isogradient;
detection wavelength: 220/254 nm; and
column temperature: 25°C.

Further, in some specific embodiments, an isomer of the compound

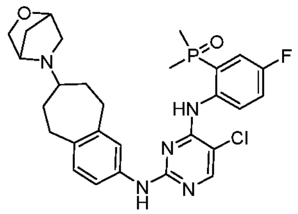

is chirally resolved by chiral liquid chromatography, wherein the chiral HPLC retention time is 13.5 min, and specific chromatographic conditions are as follows:

chromatographic column: CHIRALPAK IC, 2 cm×25 cm, with a filler particle size of 5 μm;
mobile phase A: methyl tert-butyl ether (10 mM ammonia-methanol); mobile phase B: methanol;
flow rate: 20ml/min;
gradient: 10%B within 27 min, isogradient;
detection wavelength: 220/254 nm; and
column temperature: 25°C.

Further, in some specific embodiments, an isomer of the compound

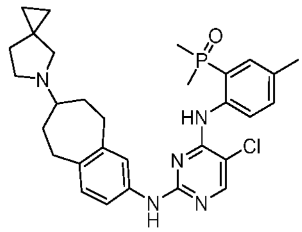

is chirally resolved by chiral liquid chromatography, wherein the chiral HPLC retention time is 10.8min, and specific chromatographic conditions are as follows:

chromatographic column: CHIRALPAK IF, 2 cm×25 cm, with a filler particle size of 5 μm;
mobile phase A: methyl tert-butyl ether (10 mM ammonia-methanol); mobile phase B: ethanol;
flow rate: 15ml/min;
gradient: 30%B within 24 min, isogradient;
detection wavelength: 220/254 nm; and
column temperature: 25°C.

Further, in some specific embodiments, an isomer of the compound

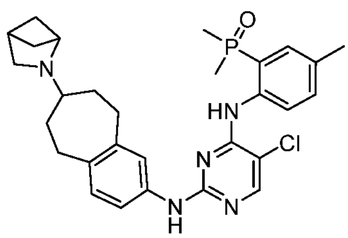

is chirally resolved by chiral liquid chromatography, wherein the chiral HPLC retention time is 6.5 min, and specific chromatographic conditions are as follows:

chromatographic column: CHIRALPAK IG, 2 cm×25 cm, with a filler particle size of 5 μm;
mobile phase A: methyl tert-butyl ether (10 nM ammonia-methanol solution); mobile phase B: ethanol;
flow rate: 20 ml/min;
gradient: 30%B within 11min, isogradient;
detection wavelength: 220/254 nm; and

column temperature: 25°C.

Further, in some specific embodiments, an isomer of the compound

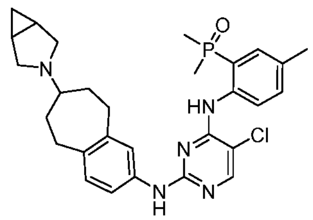

is chirally resolved by chiral liquid chromatography, wherein the chiral HPLC retention time is 12.4 min, and specific chromatographic conditions are as follows:

chromatographic column: CHIRALPAK IE, 2 cm×25 cm, with a filler particle size of 5 μm;
mobile phase A: methyl tert-butyl ether (0.1% diethylamine); mobile phase B: ethanol;
flow rate: 18 ml/min;
gradient: 20%B within 17min, isogradient;
detection wavelength: 220/254 nm; and
column temperature: 25°C.

Further, in some specific embodiments, an isomer of the compound

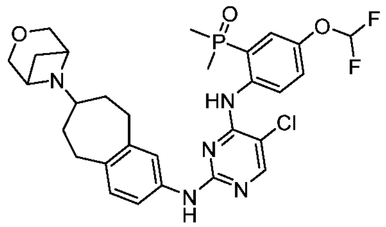

is chirally resolved by chiral liquid chromatography, wherein the chiral HPLC retention time is 12.1 min, and specific chromatographic conditions are as follows:

chromatographic column: CHIRALPAK IG, 2 cm×25 cm, with a filler particle size of 5 μm;
mobile phase A: n-hexane (0.1% diethylamine); mobile phase B: ethanol;
flow rate: 20ml/min;
gradient: 50%B within 18min, isogradient;
detection wavelength: 220/254 nm; and
column temperature: 25°C.

Further, in some specific embodiments, an isomer of the compound

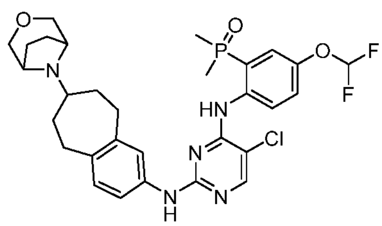

is chirally resolved by chiral liquid chromatography, wherein the chiral HPLC retention time is 16.0min, and specific chromatographic conditions are as follows:

chromatographic column: CHIRALPAK IF, 2 cm×25 cm, with a filler particle size of 5 μm;

mobile phase A: n-hexane (0.1% diethylamine); mobile phase B: ethanol;
flow rate: 20ml/min;
gradient: 30%B within 23min, isogradient;
detection wavelength: 220/254 nm; and
column temperature: 25°C.

Further, in some specific embodiments, an isomer of the compound

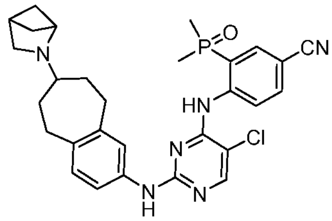

is chirally resolved by chiral liquid chromatography, wherein the chiral HPLC retention time is 18.4min, and specific chromatographic conditions are as follows:

chromatographic column: CHIRALPAK IG, 3 cm×25 cm, with a filler particle size of 5 μm;
mobile phase A: n-hexane (0.1% diethylamine); mobile phase B: ethanol;
flow rate: 20ml/min;
gradient: 50%B within 21min, isogradient;
detection wavelength: 220/254 nm; and
column temperature: 25°C.

Further, in some specific embodiments, an isomer of the compound

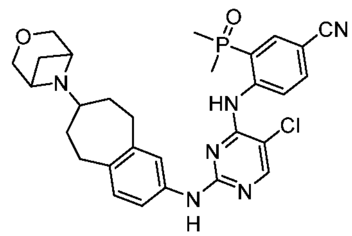

is chirally resolved by chiral liquid chromatography, wherein the chiral HPLC retention time is 16.4min, and specific chromatographic conditions are as follows:

chromatographic column: CHIRALPAK IG, 2 cm×25 cm, with a filler particle size of 5 μm;
mobile phase A: n-hexane (0.1% diethylamine); mobile phase B: ethanol;
flow rate: 20ml/min;
gradient: 30%B within 21min, isogradient;
detection wavelength: 220/254 nm; and
column temperature: 25°C.

Further, in some specific embodiments, an isomer of the compound

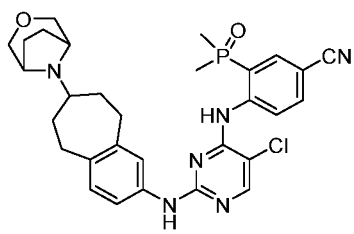

is chirally resolved by chiral liquid chromatography, wherein the chiral HPLC retention time is 12.7min, and specific chromatographic conditions are as follows:

chromatographic column: CHIRALPAK IA, 2 cm×25 cm, with a filler particle size of 5 μm;
mobile phase A: n-hexane (0.1% diethylamine); mobile phase B: ethanol;
flow rate: 20ml/min;
gradient: 50%B within 16 min, isogradient;
detection wavelength: 220/254 nm; and
column temperature: 25°C.

Further, in some specific embodiments, an isomer of the compound

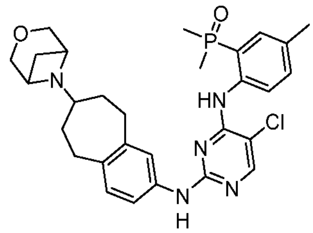

is chirally resolved by chiral liquid chromatography, wherein the chiral HPLC retention time is 13.7min, and specific chromatographic conditions are as follows:

chromatographic column: CHIRALPAK IF, 5 cm×25 cm, with a filler particle size of 5 μm;
mobile phase A: methyl tert-butyl ether (0.1% diethylamine); mobile phase B: ethanol;
flow rate: 15ml/min;
gradient: 25%B within 15min, isogradient;
detection wavelength: 220/254 nm; and
column temperature: 25°C.

Further, in some specific embodiments, an isomer of the compound

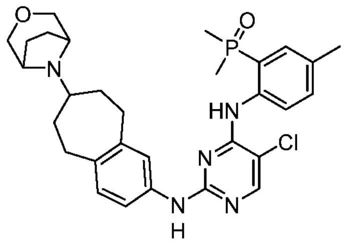

is chirally resolved by chiral liquid chromatography, wherein the chiral HPLC retention time is 13.7min, and specific chromatographic conditions are as follows:

chromatographic column: CHIRALPAK IA, 2 cm×25 cm, with a filler particle size of 5 μm;
mobile phase A: n-hexane (0.1% diethylamine); mobile phase B: ethanol;
flow rate: 20ml/min;
gradient: 30%B within 20min, isogradient;
detection wavelength: 220/254 nm; and
column temperature: 25°C.

Further, in some specific embodiments, an isomer of the compound

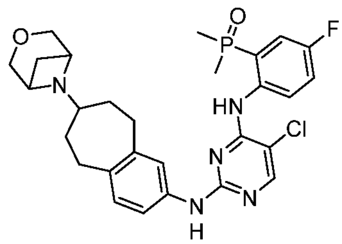

is chirally resolved by chiral liquid chromatography, wherein the chiral HPLC retention time is 17.9min, and specific chromatographic conditions are as follows:

chromatographic column: CHIRALPAK IA, 5 cm×25 cm, with a filler particle size of 5 μm;
mobile phase A: n-hexane (0.1% diethylamine); mobile phase B: ethanol;
flow rate: 20ml/min;
gradient: 20%B within 29min, isogradient;
detection wavelength: 220/254 nm; and
column temperature: 25°C.

Further, in some specific embodiments, an isomer of the compound

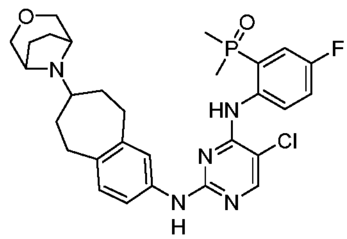

is chirally resolved by chiral liquid chromatography, wherein the chiral HPLC retention time is 3.65min, and specific chromatographic conditions are as follows:

chromatographic column: CHIRALPAK IF, 2 cm×25 cm, with a filler particle size of 5 μm;
mobile phase A: n-hexane (0.1% diethylamine); mobile phase B: ethanol;
flow rate: 15ml/min;
gradient: 30%B within 20min, isogradient;
detection wavelength: 220/254 nm; and
column temperature: 25°C.

Further, in some specific embodiments, an isomer of the compound

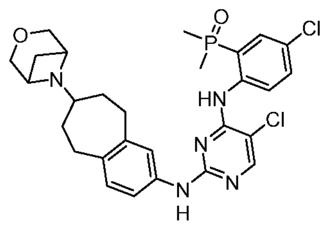

is chirally resolved by chiral liquid chromatography, wherein the chiral HPLC retention time is 23.7min, and specific chromatographic conditions are as follows:

chromatographic column: CHIRALPAK IE, 3 cm×25 cm, with a filler particle size of 5 μm;
mobile phase A: n-hexane (0.1% diethylamine); mobile phase B: ethanol;
flow rate: 15ml/min;
gradient: 50%B within 32min, isogradient;

detection wavelength: 220/254 nm; and
column temperature: 25°C.

Further, in some specific embodiments, an isomer of the compound

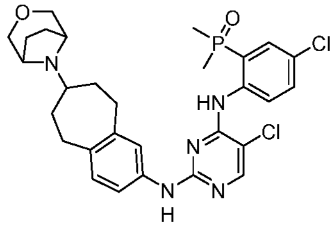

is chirally resolved by chiral liquid chromatography, wherein the chiral HPLC retention time is 1.85min, and specific chromatographic conditions are as follows:

chromatographic column: CHIRALPAK IA, 2 cm×25 cm, with a filler particle size of 5 μm;
mobile phase A: n-hexane (0.1% diethylamine); mobile phase B: ethanol;
flow rate: 15ml/min;
gradient: 50%B within 14min, isogradient;
detection wavelength: 254 nm; and
column temperature: 25°C.

In some specific embodiments, an isomer of the compound

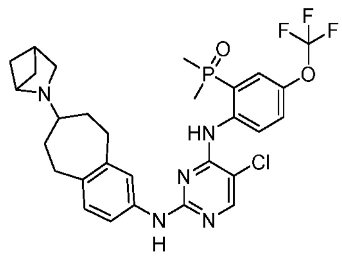

is chirally resolved by chiral liquid chromatography, wherein the chiral HPLC retention time is 15.7 min, and specific chromatographic conditions are as follows:

chromatographic column: CHIRALPAK IG, 3 cm×25 cm, with a filler particle size of 5 μm;
mobile phase A: n-hexane (0.1% diethylamine); mobile phase B: ethanol;
flow rate: 20ml/min;
gradient: 20%B within 20min, isogradient;
detection wavelength: 220/254 nm; and
column temperature: 25°C.

In some specific embodiments, an isomer of the compound

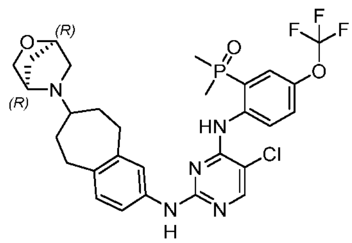

is chirally resolved by chiral liquid chromatography, wherein the chiral HPLC retention time is 13.5min, and specific

chromatographic conditions are as follows:

chromatographic column: CHIRALPAK IG, 2 cm×25 cm, with a filler particle size of 5 μm;
mobile phase A: methyl tert-butyl ether (0.1% diethylamine); mobile phase B: ethanol;
flow rate: 20ml/min;
gradient: 10%B within 21min, isogradient;
detection wavelength: 220/254 nm; and
column temperature: 25°C.

In some specific embodiments, an isomer of the compound

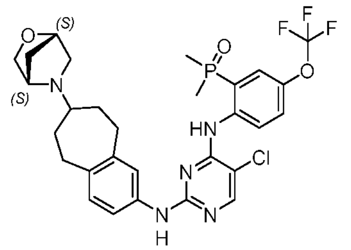

is chirally resolved by chiral liquid chromatography, wherein the chiral HPLC retention time is 11.4min, and specific chromatographic conditions are as follows:

chromatographic column: CHIRALPAK ID, 2 cm×25 cm, with a filler particle size of 5 μm;
mobile phase A: methyl tert-butyl ether (0.1% diethylamine); mobile phase B: ethanol;
flow rate: 20ml/min;
gradient: 10%B within 24min, isogradient;
detection wavelength: 220/254 nm; and
column temperature: 25°C.

Further, in some specific embodiments, an isomer of the compound

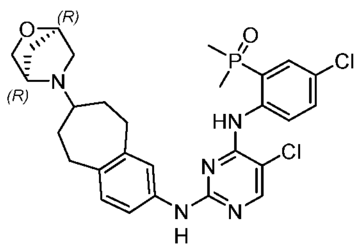

is chirally resolved by chiral liquid chromatography, wherein the chiral HPLC retention time is 11.7min, and specific chromatographic conditions are as follows:

chromatographic column: CHIRALPAK IF, 2 cm×25 cm, with a filler particle size of 5 μm;
mobile phase A: methyl tert-butyl ether (0.1% diethylamine); mobile phase B: ethanol;
flow rate: 15ml/min;
gradient: 30%B within 18min, isogradient;
detection wavelength: 220/254 nm; and
column temperature: 25°C.

In some specific embodiments, an isomer of the compound

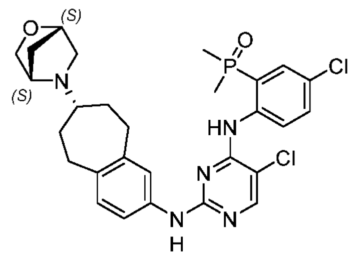

is chirally resolved by chiral liquid chromatography, wherein the chiral HPLC retention time is 11.7 min, and specific chromatographic conditions are as follows:

chromatographic column: CHIRALPAK IF, 2 cm×25 cm, with a filler particle size of 5 μm;
mobile phase A: methyl tert-butyl ether (0.1% diethylamine); mobile phase B: ethanol;
flow rate: 15ml/min;
gradient: 30%B within 18min, isogradient;
detection wavelength: 220/ 254 nm; and
column temperature: 25°C.

Further, in some specific embodiments, an isomer of the compound

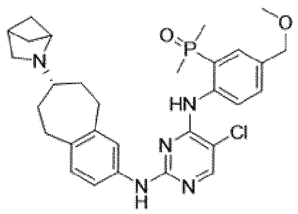

is chirally resolved by chiral liquid chromatography, wherein the chiral HPLC retention time is 8.6min, and specific chromatographic conditions are as follows:

chromatographic column: CHIRALPAK ID, 2 cm×25cm, with a filler particle size of 5 μm;
mobile phase A: methyl tert-butyl ether (0.1% diethylamine); mobile phase B: ethanol;
flow rate: 20ml/min;
gradient: 25%B within 13 min, isogradient;
detection wavelength: 220/254 nm; and
column temperature: 25°C.

Further, in some specific embodiments, an isomer of the compound

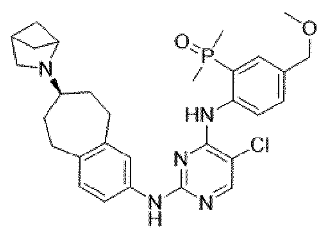

is chirally resolved by chiral liquid chromatography, wherein the chiral HPLC retention time is 10.8min, and specific chromatographic conditions are as follows:

chromatographic column: CHIRALPAK ID, 2 cm×25cm, with a filler particle size of 5 μm;
mobile phase A: methyl tert-butyl ether (0.1% diethylamine); mobile phase B: ethanol;
flow rate: 20ml/min;
gradient: 25%B within 13 min, isogradient;

detection wavelength: 220/254 nm; and
column temperature: 25°C.

Further, in some specific embodiments, an isomer of the compound

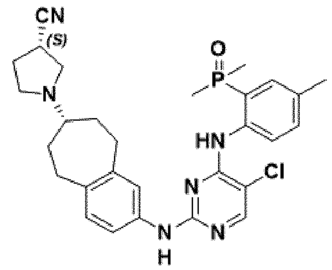

is chirally resolved by chiral liquid chromatography, wherein the chiral HPLC retention time is 13.4min, and specific chromatographic conditions are as follows:

chromatographic column: CHIRALPAK IF, 5 cm×25 cm, with a filler particle size of 5 μm;
mobile phase A: methyl tert-butyl ether (0.1% diethylamine); mobile phase B: ethanol; flow rate: 15 ml/min;
gradient: 20%B within 17.5 min, isogradient;
detection wavelength: 220/254 nm; and column temperature: 25°C.

Further, in some specific embodiments, an isomer of the compound

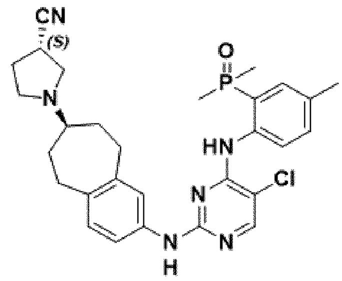

is chirally resolved by chiral liquid chromatography, wherein the chiral HPLC retention time is 16.2min, and specific chromatographic conditions are as follows:

chromatographic column: CHIRALPAK IF, 5 cm×25 cm, with a filler particle size of 5 μm;
mobile phase A: methyl tert-butyl ether (0.1% diethylamine); mobile phase B: ethanol; flow rate: 15 ml/min;
gradient: 20%B within 17.5 min, isogradient;
detection wavelength: 220/254 nm; and
column temperature: 25°C.

Further, in some specific embodiments, an isomer of the compound

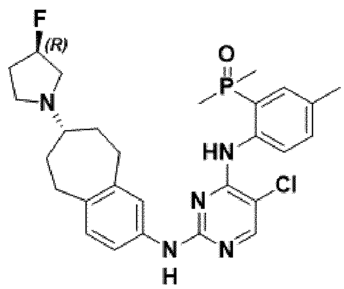

is chirally resolved by chiral liquid chromatography, wherein the chiral HPLC retention time is 13.4min, and specific chromatographic conditions are as follows:

chromatographic column: CHIRALPAK IF, 5 cm×25 cm, with a filler particle size of 5 μm;
mobile phase A: methyl tert-butyl ether (0.1% diethylamine); mobile phase B: ethanol;
flow rate: 15ml/min;
gradient: 20%B within 17.5 min, isogradient;
detection wavelength: 220/254 nm; and
column temperature: 25°C.

Further, in some specific embodiments, an isomer of the compound

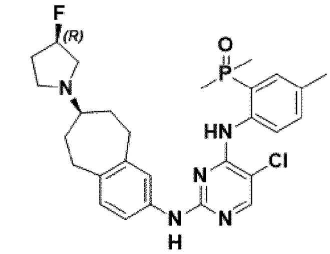

is chirally resolved by chiral liquid chromatography, wherein the chiral HPLC retention time is 16.2min, and specific chromatographic conditions are as follows:

chromatographic column: CHIRALPAK IF, 5 cm×25 cm, with a filler particle size of 5 μm;
mobile phase A: methyl tert-butyl ether (0.1% diethylamine); mobile phase B: ethanol;
flow rate: 15ml/min;
gradient: 20%B within 17.5 min, isogradient;
detection wavelength: 220/254 nm; and
column temperature: 25°C.

Further, in some specific embodiments, an isomer of the compound

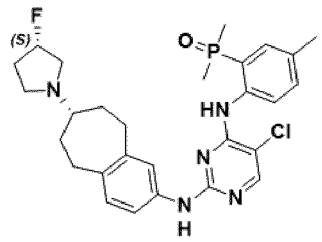

is chirally resolved by chiral liquid chromatography, wherein the chiral HPLC retention time is 5.9min, and specific chromatographic conditions are as follows:

chromatographic column: CHIRALPAK IE, 2 cm×25 cm, with a filler particle size of 5 μm;
mobile phase A: methyl tert-butyl ether (0.1% diethylamine); mobile phase B: ethanol;
flow rate: 19ml/min;
gradient: 35%B within 11 min, isogradient;
detection wavelength: 220/254 nm; and
column temperature: 25°C.

Further, in some specific embodiments, an isomer of the compound

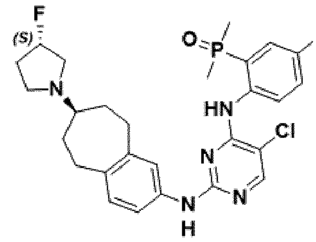

is chirally resolved by chiral liquid chromatography, wherein the chiral HPLC retention time is 9.0min, and specific chromatographic conditions are as follows:

chromatographic column: CHIRALPAK IE, 2 cm×25 cm, with a filler particle size of 5 μm;
mobile phase A: methyl tert-butyl ether (0.1% diethylamine); mobile phase B: ethanol;
flow rate: 19ml/min;
gradient: 35%B within 11 min, isogradient;
detection wavelength: 220/254 nm; and
column temperature: 25°C.

Further, in some specific embodiments, an isomer of the compound

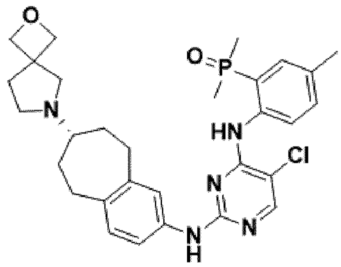

is chirally resolved by chiral liquid chromatography, wherein the chiral HPLC retention time is 9.1min, and specific chromatographic conditions are as follows:

chromatographic column: CHIRALPAK IE, 2 cm×25 cm, with a filler particle size of 5 μm;
mobile phase A: methyl tert-butyl ether (0.1% diethylamine); mobile phase B: ethanol;
flow rate: 19ml/min;
gradient: 35%B within 12 min, isogradient;
detection wavelength: 220/254 nm; and
column temperature: 25°C.

Further, in some specific embodiments, an isomer of the compound

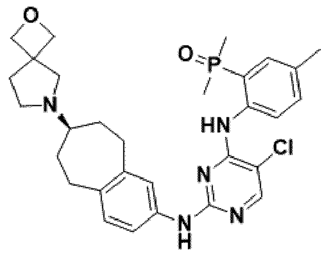

is chirally resolved by chiral liquid chromatography, wherein the chiral HPLC retention time is 10.6min, and specific chromatographic conditions are as follows:

chromatographic column: CHIRALPAK IE, 2 cm×25 cm, with a filler particle size of 5 μm;
mobile phase A: methyl tert-butyl ether (0.1% diethylamine); mobile phase B: ethanol;

flow rate: 19ml/min;
gradient: 35%B within 12 min, isogradient;
detection wavelength: 220/254 nm; and
column temperature: 25°C.

Further, in some specific embodiments, an isomer of the

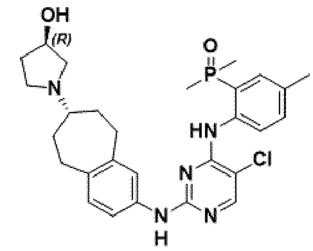

is chirally resolved by chiral liquid chromatography, wherein the chiral HPLC retention time is 7.9min, and specific chromatographic resolution conditions are as follows:

chromatographic column: CHIRALPAK IE, 2 cm×25 cm, with a filler particle size of 5 μm;
mobile phase A: methyl tert-butyl ether (0.1% diethylamine); mobile phase B: ethanol;
flow rate: 20ml/min;
gradient: 25%B within 12 min, isogradient;
detection wavelength: 220/254 nm; and
column temperature: 25°C.

Further, in some specific embodiments, an isomer of the

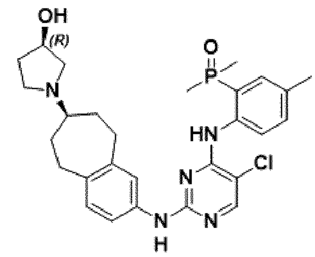

is chirally resolved by chiral liquid chromatography, wherein the chiral HPLC retention time is 10.0min, and specific chromatographic resolution conditions are as follows:

chromatographic column: CHIRALPAK IE, 2 cm×25 cm, with a filler particle size of 5 μm;
mobile phase A: methyl tert-butyl ether (0.1% diethylamine); mobile phase B: ethanol;
flow rate: 20ml/min;
gradient: 25%B within 12 min, isogradient;
detection wavelength: 220/254 nm; and
column temperature: 25°C.

Further, in some specific embodiments, an isomer of the

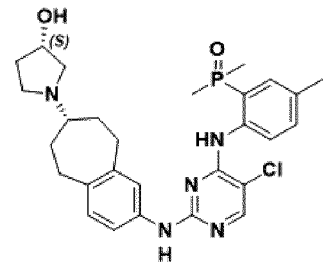

is chirally resolved by chiral liquid chromatography, wherein the chiral HPLC retention time is 13.2min, and specific chromatographic resolution conditions are as follows:

chromatographic column: CHIRALPAK IE, 2 cm×25 cm, with a filler particle size of 5 μm;
mobile phase A: methyl tert-butyl ether (0.1% diethylamine); mobile phase B: ethanol;
flow rate: 15ml/min;
gradient: 30%B within 20 min, isogradient;
detection wavelength: 220/254 nm; and
column temperature: 25°C.

Further, in some specific embodiments, an isomer of the

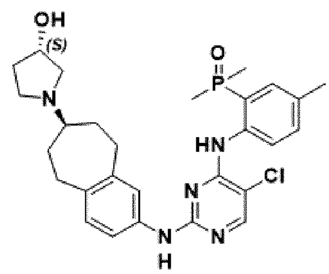

is chirally resolved by chiral liquid chromatography, wherein the chiral HPLC retention time is 17.9min, and specific chromatographic resolution conditions are as follows:

chromatographic column: CHIRALPAK IE, 2 cm×25 cm, with a filler particle size of 5 μm;
mobile phase A: methyl tert-butyl ether (0.1% diethylamine); mobile phase B: ethanol;
flow rate: 15ml/min;
gradient: 30%B within 20 min, isogradient;
detection wavelength: 220/254 nm; and
column temperature: 25°C.

Further, in some specific embodiments, an isomer of the

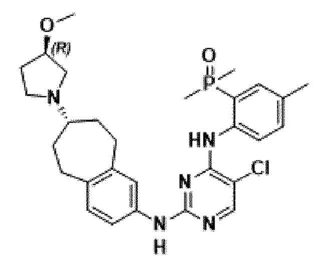

is chirally resolved by chiral liquid chromatography, wherein the chiral HPLC retention time is 6.7min, and specific chromatographic resolution conditions are as follows:

chromatographic column: CHIRALPAK IE, 2 cm×25 cm, with a filler particle size of 5 μm;

mobile phase A: methyl tert-butyl ether (0.1% diethylamine); mobile phase B: ethanol;
flow rate: 20ml/min;
gradient: 30%B within 20 min, isogradient;
detection wavelength: 220/254 nm; and
column temperature: 25°C.

Further, in some specific embodiments, an isomer of the

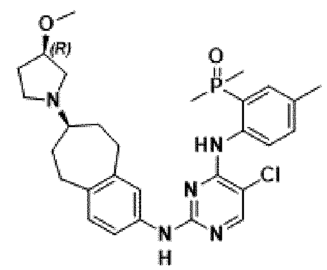

is chirally resolved by chiral liquid chromatography, wherein the chiral HPLC retention time is 8.45min, and specific chromatographic resolution conditions are as follows:

chromatographic column: CHIRALPAK IE, 2 cm×25 cm, with a filler particle size of 5 μm;
mobile phase A: methyl tert-butyl ether (0.1% diethylamine); mobile phase B: ethanol;
flow rate: 20ml/min;
gradient: 30%B within 20 min, isogradient;
detection wavelength: 220/254 nm; and
column temperature: 25°C.

Further, in some specific embodiments, an isomer of the

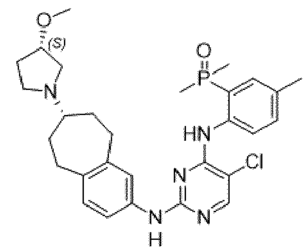

is chirally resolved by chiral liquid chromatography, wherein the chiral HPLC retention time is 13.2min, and specific chromatographic resolution conditions are as follows:

chromatographic column: CHIRALPAK IF, 2 cm×25 cm, with a filler particle size of 5 μm;
mobile phase A: methyl tert-butyl ether (0.1% diethylamine); mobile phase B: ethanol;
flow rate: 15ml/min;
gradient: 30%B within 20 min, isogradient;
detection wavelength: 220/254 nm; and
column temperature: 25°C.

Further, in some specific embodiments, an isomer of the

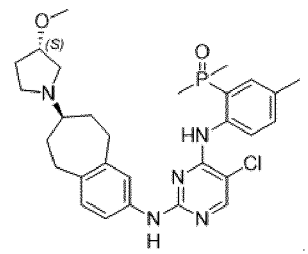

is chirally resolved by chiral liquid chromatography, wherein the chiral HPLC retention time is 17.9min, and specific chromatographic resolution conditions are as follows:

chromatographic column: CHIRALPAK IF, 2 cm×25 cm, with a filler particle size of 5 μm;
mobile phase A: methyl tert-butyl ether (0.1% diethylamine); mobile phase B: ethanol;
flow rate: 15ml/min;
gradient: 30%B within 20 min, isogradient;
detection wavelength: 220/254 nm; and
column temperature: 25°C.

Further, in some specific embodiments, an isomer of the

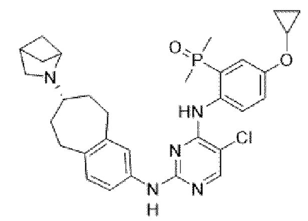

is chirally resolved by chiral liquid chromatography, wherein the chiral HPLC retention time is 7.5min, and specific chromatographic resolution conditions are as follows:

chromatographic column: CHIRALPAK IG, 2 cm×25 cm, with a filler particle size of 5 μm;
mobile phase A: methyl tert-butyl ether (0.1% diethylamine); mobile phase B: ethanol;
flow rate: 20ml/min;
gradient: 25%B within 9 min, isogradient;
detection wavelength: 220/254 nm; and
column temperature: 25°C.

Further, in some specific embodiments, an isomer of the

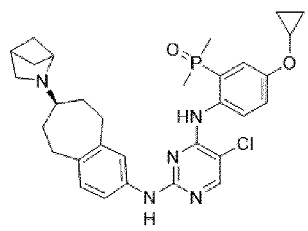

is chirally resolved by chiral liquid chromatography, wherein the chiral HPLC retention time is 11.2min, and specific chromatographic resolution conditions are as follows:

chromatographic column: CHIRALPAK IG, 2 cm×25 cm, with a filler particle size of 5 μm;
mobile phase A: methyl tert-butyl ether (0.1% diethylamine); mobile phase B: ethanol;
flow rate: 20ml/min;
gradient: 25%B within 9 min, isogradient;

detection wavelength: 220/254 nm; and
column temperature: 25°C.

In some specific embodiments, an isomer of the compound

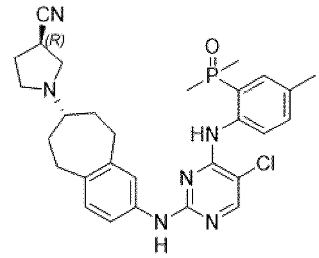

is chirally resolved by chiral liquid chromatography, wherein the chiral HPLC retention time is 6.7 min, and specific chromatographic conditions are as follows:

chromatographic column: CHIRALPAK IG, 2 cm×25 cm, with a filler particle size of 5 μm;
mobile phase A: methyl tert-butyl ether (0.1% diethylamine); mobile phase B: ethanol;
flow rate: 19ml/min;
gradient: 30%B within 9 min, isogradient;
detection wavelength: 220/254 nm; and
column temperature: 25°C.

In some specific embodiments, an isomer of

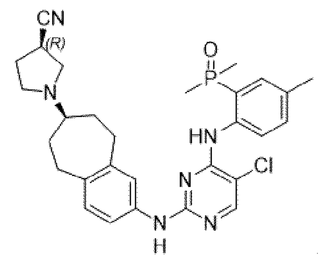

is chirally resolved by chiral liquid chromatography, wherein the chiral HPLC retention time is 7.8min, and specific chromatographic resolution conditions are as follows:

chromatographic column: CHIRALPAK IG, 2 cm×25 cm, with a filler particle size of 5 μm;
mobile phase A: methyl tert-butyl ether (0.1% diethylamine); mobile phase B: ethanol;
flow rate: 19ml/min;
gradient: 30%B within 9 min, isogradient;
detection wavelength: 220/254 nm; and
column temperature: 25°C.

In another aspect, the present invention further provides a pharmaceutical composition, including a therapeutically effective amount of compound of Formula I, I-1, I-2, II, II-1, III, III-1, IV, IV-1, V, or VI or a pharmaceutically acceptable salt thereof.

In some embodiments, the present invention further provides a pharmaceutical composition, including a therapeutically effective amount of compound of Formula I, I-1, I-2, II, II-1, III, III-1, IV, IV-1, V, or VI or a pharmaceutically acceptable salt thereof, and one or more pharmaceutically acceptable carriers.

The pharmaceutical composition of the present invention can be administered by any suitable way or method, for example, oral or parenteral (e.g., intravenous) administration. The therapeutically effective amount of the compound of Formula I, I-1, I-2, II, II-1, III, III-1, IV, IV-1, V, or VI ranges from about 0.001 mg to 50 mg/Kg body weight/day, preferably from 0.01 mg to 50 mg/Kg body weight/day.

For oral administration, the pharmaceutical composition of the present invention is generally provided in a form

of tablets, capsules or solutions. The tablets may contain the compound of the present invention or a pharmaceutically acceptable salt thereof, and a pharmaceutically acceptable carrier. The carrier includes, but is not limited to, a diluent, a disintegrant, a binder, a lubricant, a colorant or a preservative. The capsules include hard capsules and soft capsules.

For parenteral administration, the pharmaceutical composition of the present invention can be administered by intravenous injection, intramuscular injection or subcutaneous injection. The pharmaceutical composition is usually provided as a sterile aqueous solution or suspension or lyophilized powder, and adjusted to suitable pH and isotonicity.

In another aspect, the present invention further provides uses of a compound of Formula I, I-1, I-2, II, II-1, III, III-1, IV, IV-1, V, or VI in the preparation of a drug for preventing and/or treating diseases or disease conditions mediated by AXL protein kinase.

In another aspect, the present invention further provides a method for preventing and/or treating diseases or disease conditions mediated by AXL protein kinase, the method including: administering a compound of Formula I, I-1, I-2, II, II-1, III, III-1, IV, IV-1, V, or VI or the pharmaceutical composition of the present invention to an individual in need.

In another aspect, the present invention further provides a compound of Formula I, I-1, I-2, II, II-1, III, III-1, IV, IV-1, V, or VI or the pharmaceutical composition of the present invention for preventing and/or treating diseases or disease conditions mediated by AXL protein kinase.

Examples of the diseases or disease conditions mediated by AXL protein kinase include, but are not limited to, autoimmune diseases.

In another aspect, the present invention provides a method for preparing a compound of Formula I, I-1, I-2, II, II-1, III, III-1, IV, IV-1, V, or VI, which includes, but is not limited to, the following synthesis scheme:

synthesis scheme 1:

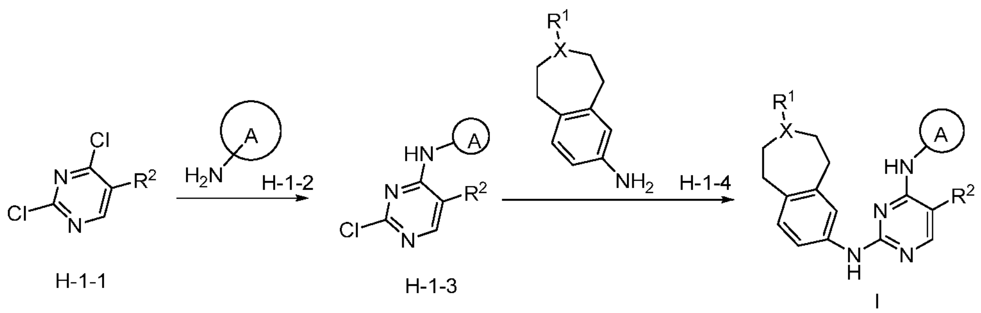


wherein the definitions of $R^1$, $R^2$, X and ring A are consistent with those defined in Formula I of general formulas.

A compound of Formula H-1-3 is prepared from a compound of Formula H-1-1 and a compound of Formula H-1-2 in the presence of a solvent (e.g., N,N-dimethyl formamide or tetrahydrofuran) and an alkaline (e.g., sodium hydride or lithium hexamethyldisilazide), and a compound of Formula I is prepared from the compound of Formula H-1-3 and a compound of Formula H-1-4 in the presence of a solvent (e.g., n-butanol) and an acid (trifluoroacetic acid or p-toluenesulfonic acid).

In another aspect, the present invention provides a method for preparing a compound of Formula I-1, II-1, III-1 or IV-1, which includes, but is not limited to, the following synthesis scheme:

synthesis scheme 2:

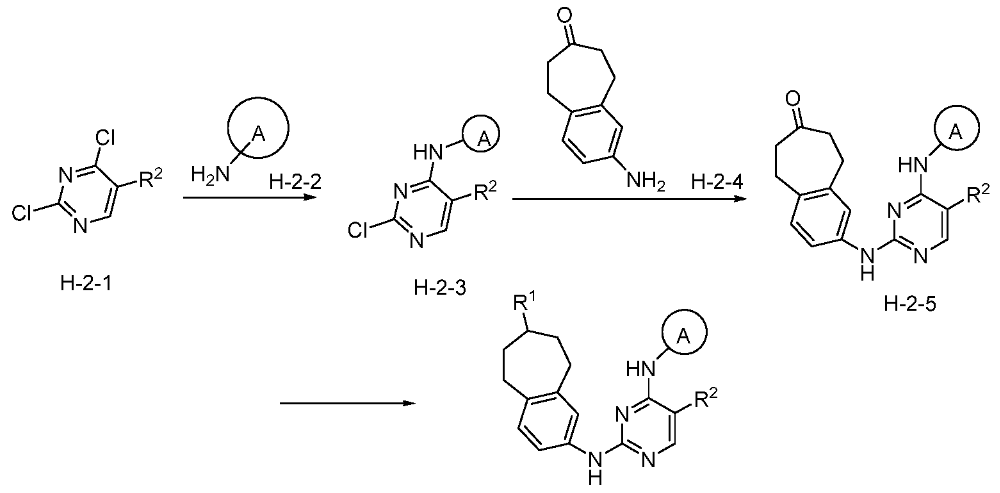
H-2-1 H-2-2 H-2-3 H-2-4 H-2-5 H-2-6

wherein the definitions of $R^2$ and ring A are consistent with those defined in Formula 1-1 of general formulas, and the definition of $R^1$ is consistent with that defined in Formula I.

A compound of Formula H-2-3 is prepared from a compound of Formula H-2-1 and a compound of Formula H-2-2 under the conditions of a solvent (e.g., N,N-dimethyl formamide or tetrahydrofuran) and an alkaline (e.g., sodium hydride or lithium hexamethyldisilazide); a compound of Formula H-2-5 is prepared by the reaction of the compound of Formula H-2-3 and a compound of Formula H-2-4 in the presence of a solvent (e.g., N,N-dimethyl formamide) and an acid (e.g., hydrochloric acid); a compound of Formula H-2-6 is obtained by reacting the compound of Formula H-2-5 with $R^1H$ or an acid addition salt thereof (e.g., hydrochloride, specific example of acid addition salt of $R^1H$ can be exemplified by 2-azabicyclo[3.1.0] hexane hydrochloride) in the presence of a solvent (e.g., dichloromethane) and a reducing agent (e.g., sodium cyanoborohydride); and optionally, an optically pure target product is prepared by chiral resolution.

Further, the present invention provides the following compounds as intermediates for the synthesis of the compound of Formula I:

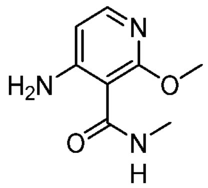
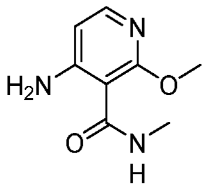
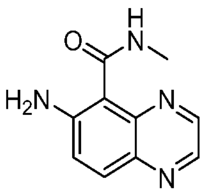
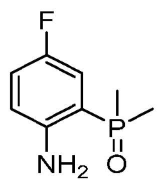
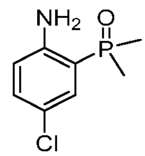
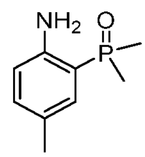

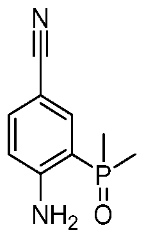
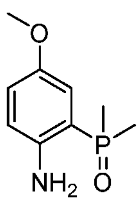
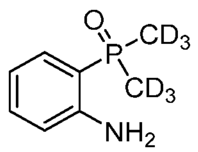
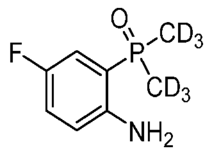
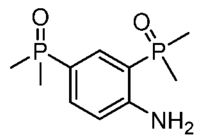
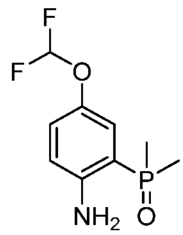

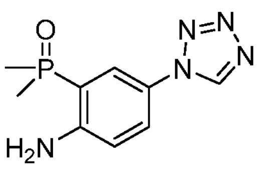
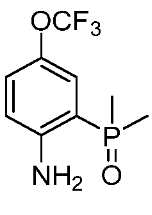
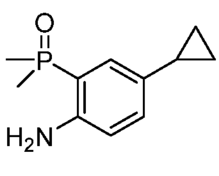
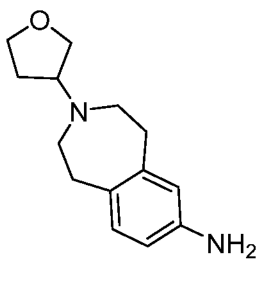
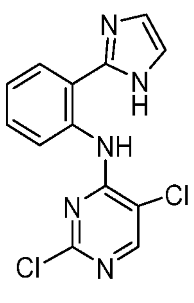

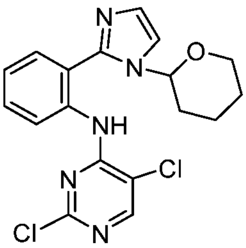
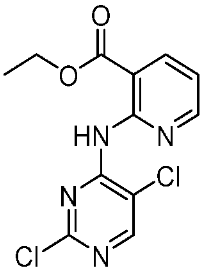
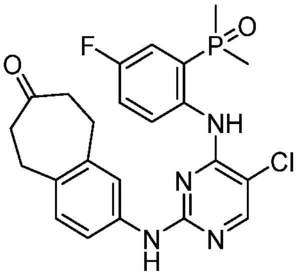
.

Further, the present invention provides the following compounds as intermediates for the synthesis of the compound of Formula I:

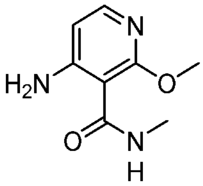
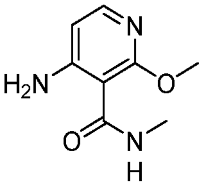
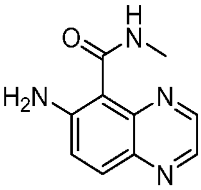
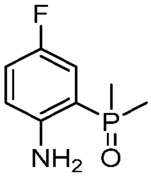
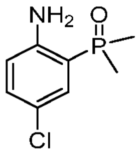
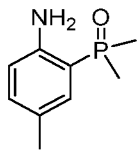

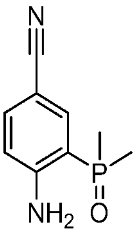
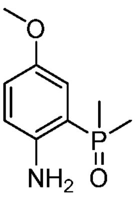
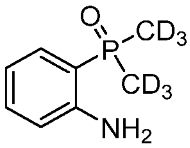
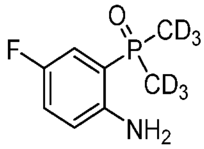
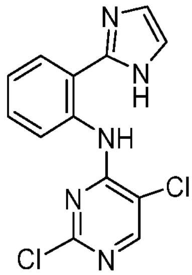

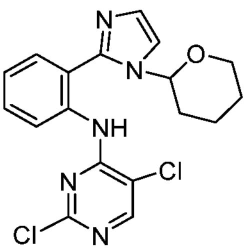
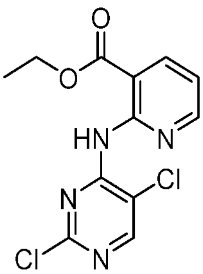
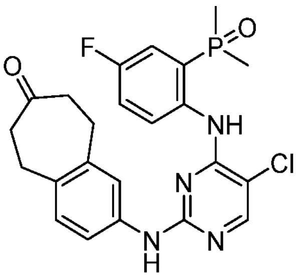
.

**Related definitions**

Unless otherwise specified, the following terms used in the description and claims have the following meanings: The "compound" of the present invention may be asymmetric, for example, has one or more chiral centers. Unless otherwise specified, the "compound" of the present invention refers to any one stereoisomer or a mixture of two or more stereoisomers. Stereoisomers include, but are not limited to, enantiomers and diastereomers. A compound containing asymmetric carbon atoms of the present invention can be isolated in the form of an optically pure or a mixture of two or more stereoisomers. The optically pure form may be resolved from a mixture of two or more stereoisomers, or synthesized by using a chiral raw material or chiral reagent.

The compound of the present invention also includes a tautomeric form. The tautomeric form is derived from the exchange of a single bond with an adjacent double bond accompanied by the migration of a proton. For example,

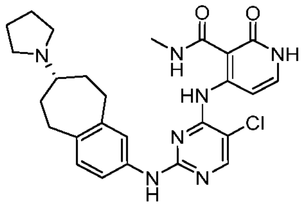

can be transformed into

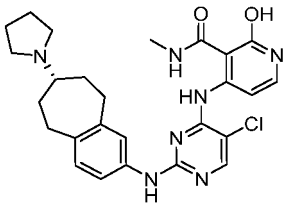

under certain conditions.

The term "optional" or "optionally" means that an event or situation described later may or may not occur, this description including the occurrence of the event or situation and the non-occurrence of the event or situation.

A numerical range herein refers to each integer in a given range. For example, "$C_{1-6}$" means that this group can have one carbon atom, two carbon atoms, three carbon atoms, four carbon atoms, five carbon atoms, or six carbon atoms.

The term "membered" refers to a number of skeletal atoms that make up a ring. For example, "5-7 membered" means that the number of skeletal atoms that make up a ring is 5, 6 or 7. Thus, for example, pyridine is a 6-membered ring, while thiophene is a 5-membered ring. The term "substituted" means that any one or more hydrogen atoms on a specific atom or group are substituted by a substituent, as long as the valence of the specific atom or group is normal and the substituted compound is stable. When the substituent is

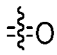,

it means that two hydrogen atoms are substituted. Unless otherwise specified, the type and number of substituents may be arbitrary on the basis that they can be chemically realized.

When any variable (e.g., $R^3$) occurs more than once in the composition or structure of a compound, its definition in each case is independent. So, for example, if a group is substituted by one or more $R^3$, there are independent options for $R^3$ in each case. In addition, combinations of substituents and/or variants thereof are only permitted if such combinations result in stable compounds.

The term "alkyl" refers to a saturated aliphatic hydrocarbon group, including linear or branched saturated hydrocarbyl, the hydrocarbyl having the indicated number of carbon atoms. For example, the term "$C_{1-6}$ alkyl" includes $C_1$ alkyl, $C_2$ alkyl, $C_3$ alkyl, $C_4$ alkyl, $C_5$ alkyl, C6 alkyl; and examples include, but are not limited to, methyl, ethyl, n-propyl , isopropyl, n-butyl, isobutyl, tert-butyl, n-pentyl, 2-pentyl, 3-pentyl, n-hexyl, 2-hexyl, 3-hexyl, etc. $C_{1-6}$ alkyl may be divalent, such as methylene or ethylidene.

The term "alkoxyl" refers to a group having an alkyl-O- structure, alkyl including linear or branched saturated monovalent hydrocarbyl. For example, "C1-C3 alkoxyl" includes methoxyl, ethoxyl, n-propoxyl, and isopropoxyl.

The term "$C_{2-6}$ alkenyl" is used to represent a linear or branched hydrocarbon group containing at least one carbon-carbon double bond and consisting of 2 to 6 carbon atoms, wherein the carbon-carbon double bond may be located in any position of this group. Examples include, but are not limited to, vinyl, propenyl, butenyl, pentenyl, hexenyl, and the like.

The term "$C_{2-6}$ alkynyl " is used to represent a linear or branched hydrocarbon group containing at least one carbon-carbon triple bond and consisting of 2 to 6 carbon atoms, wherein the carbon-carbon triple bond may be located in any position of this group. Examples include, but are not limited to, ethynyl, propynyl, butynyl, and the like.

The term "heterocycloalkyl" refers to a saturated monocyclic ring system having carbon atoms and 1 to 2 heteroatoms as ring atoms, wherein the heteroatoms are independently selected from nitrogen, sulfur, or oxygen atom. In a heterocycloalkyl group containing one or more nitrogen atoms, the connection point may be a carbon or nitrogen atom, as long as the valence of the atom allows. Examples include, but are not limited to,

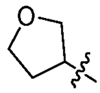

and

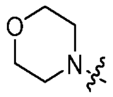.

The term "saturated carbocyclic ring" refers to saturated cycloalkanes.

The term "5-12 membered saturated heterocyclic ring" refers to a 5-12 membered saturated non-aromatic system having carbon atoms and 1, 2 or 3 heteroatoms or heteroatom groups as ring atoms, wherein the heteroatoms or heteroatom groups are independently selected from nitrogen, sulfur, oxygen, sulfoxide, sulfone,

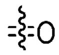, 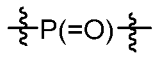 or 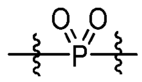.

In a heterocyclic ring group containing one or more nitrogen atoms, the connection point may be a carbon or nitrogen atom, as long as the valence of the atom allows. The heterocyclic ring may be a monocyclic or polycyclic ring system, such as a bicyclic ring, wherein two or more rings exist in a form of a fused ring, a bridged ring or a spiro ring, where at least one ring contains 1, 2 or 3 ring heteroatoms or heteroatom groups. Examples include, but are not limited to,

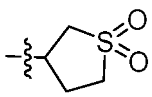, 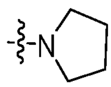, 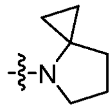, 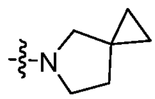, 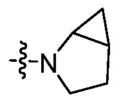, 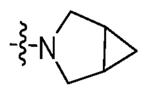,

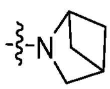, 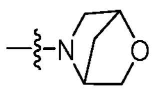, 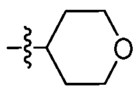, 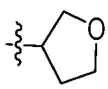 and 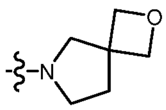.

The term "5-8 membered saturated heterocyclic ring" refers to a 5-8 membered saturated non-aromatic system having carbon atoms and 1, 2 or 3 heteroatoms or heteroatom groups as ring atoms, and other definitions are consistent with those of 5-12 membered saturated heterocyclic rings.

The term "5-7 membered saturated heterocyclic ring" refers to a 5-7 membered saturated non-aromatic system having carbon atoms and 1, 2 or 3 heteroatoms or heteroatom groups as ring atoms, and other definitions are consistent with those of the 5-12 membered saturated heterocyclic ring.

The term "5-7 membered heteroaryl" refers to a 5-, 6- or 7-membered monovalent aryl which containing at least one heteroatoms independently selected from nitrogen, oxygen, and sulfur. Examples include, but are not limited to, pyridyl, pyrimidinyl, thienyl and imidazolyl.

The term "5-6 membered heteroaryl" refers to a 5- or 6-membered monovalent aryl which containing at least one heteroatoms independently selected from nitrogen, oxygen, and sulfur. Examples include, but are not limited to, pyridyl, pyrimidinyl, thienyl and imidazolyl. The term "9-12 membered benzoheterocyclyl" refers to a bicyclic ring system with 9-12 ring atoms, one of which is a benzene ring, and the other is saturated, partially unsaturated or unsaturated 5-6 membered heterocyclyl containing one to two nitrogen, oxygen, and sulfur heteroatoms, both of which share a pair of adjacent ring atoms. Examples include, but are not limited to,

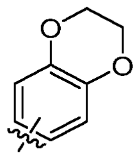, 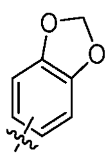, 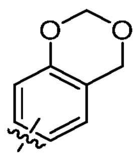 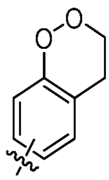 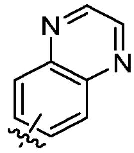, 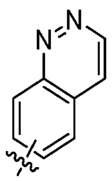

and

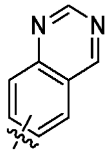
.

The term "cycloalkyl" refers to a monocyclic saturated hydrocarbon system without heteroatoms or double bonds. Examples of the term "3-10 membered cycloalkyl" include, but are not limited to, cyclopropyl, cyclobutyl, cyclopentyl, and cyclohexyl.

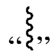

in the term

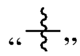

refers to a junction of chemical bonds. If

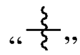

appears in a bicyclic ring and the connection position is uncertain, it means that the connection position is only limited to any atom on the single ring where "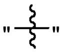" is located, as long as the valence allows.

For example,

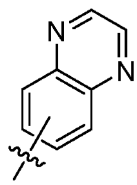

means that the connection position is only located on any carbon atom on the benzene ring in the bicyclic ring, and the valence-bond requirements must be met.

The term "halogen" refers to fluorine, chlorine, bromine and iodine.

The substituents $R^3$, $R^a$, and $R^b$ may be bonded to any atom on the ring, as long as the valence allows. Combinations of substituents and/or variants thereof are only permitted if such combinations result in stable compounds. Those skilled in the art can understand that for any group containing one or more $R^3$ substituents, any substitution or substitution pattern that is impossible to exist in space and/or cannot be synthesized will not be introduced. The term "deuterium substitution" means that one or more C-H bonds in a compound or group are substituted by C-D bonds. The deuterium substitution may be mono-, di-, poly, or full-substitution. The "deuteration" method adopts conventional methods in the art. For example, commercial deuterated raw materials can be used, or deuterium can be introduced into the compounds according to the methods disclosed in the prior art.

The term "effective amount" or "therapeutically effective amount" refers to a sufficient amount of a drug or medicament that is non-toxic but can achieve a desired effect.

The term "pharmaceutically acceptable carrier" refers to those carriers that have no obvious stimulating effect on the body and do not impair the biological activity and performance of the active compound. These carriers include, but are not limited to, any diluents, disintegrants, adhesives, glidants, and wetting agents that are approved by the State Food and Drug Administration and can be used in humans or animals.

The term "pharmaceutically acceptable salt" refers to a salt that retains the biological efficacy of a free acid and alkaline of a specific compound without biological adverse effects. For example, the salt includes acid (including organic acid and inorganic acid) addition salts or alkali (including organic alkali and inorganic alkali) addition salts.

Unless otherwise specified, the abbreviations in the present invention have the following meanings:

M: mol/L
mM: mmol/L
nM: nmol/L
$^1$H NMR: Proton Nuclear Magnetic Resonance Spectroscopy
MS(ESI+): mass spectrum
DMSO-$d_6$: deuterated dimethyl sulfoxide
$CDCl_3$: deuterated chloroform
DTT: dithiothreitol
SEB: Supplemented Enzymatic Buffer
IMDM: Iscove's Modified Dulbecco's Medium
Room temperature: 25°C.

DETAIL OF THE DESCRIPTION

The preparation methods of the compounds of the present invention are described in more detail below, but these specific preparation methods do not constitute any limitation on the scope of the present invention. In addition, reaction conditions such as reactants, solvents, alkali, amounts of compounds used, reaction temperature, reaction time, etc. are not limited to the following examples.

The compounds of the present invention can also be conveniently prepared by combining various synthetic methods described in this description or known in the art, and such combinations can be easily performed by a person skilled in the art.

**Part I Preparation**

**Preparation Example 1** 7-amino-2-methylisoindolin-1-one

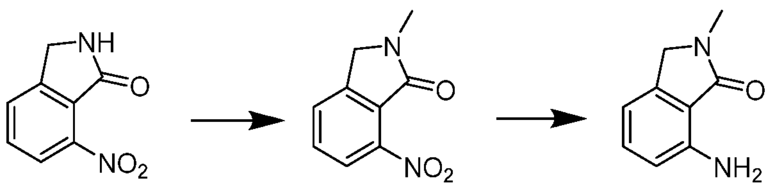

a) Preparation of 2-methyl-7-nitroisoindolin-1-one

7-nitroisoindolin-1-one (450 mg) is dissolved in N,N-dimethyl formamide (15 mL), added with potassium carbonate (690 mg) and methyl iodide (430 mg), and then stirred and stands overnight at room temperature; the reaction solution is poured into water, extracted with ethyl acetate and then purified by column chromatography (dichloromethane/methanol=20:1) to obtain the title compound (300 mg). MS(ESI+): 193.02(M+H).

b) Preparation of 7-amino-2-methylisoindolin-1-onein

2-methyl-7-nitroisoindolin-1-one (300 mg) is dissolved in methanol (30 mL), added with palladium charcoal (30 mg) and hydrazine hydrate (438 mg), and then stirred at room temperature for 6 h. The reaction solution is filtered with diatomite, concentrated to dryness and then purified by column chromatography (dichloromethane/methanol=20:1) to obtain the title compound (245 mg). MS(ESI+): 162.98(M+H).

**Preparation Example 2** N-(2-aminophenyl) methanesulfonamide

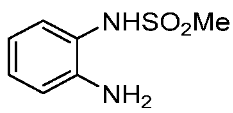

O-phenylenediamine (500 mg), triethylamine (1403 mg) and 10 mL of anhydrous dichloromethane are added to a reaction flask. After all of them are dissolved, the mixture is added dropwise with a dichloromethane solution of methanesulfonyl chloride (1095 mg) at 0°C. After the addition is completed, the temperature is gradually raised to room temperature and the reaction is performed for 12 h. After the reaction is completed, the reaction solution is quenched with 1 ml of methanol. The pH of the system is adjusted to 8 with 1M hydrochloric acid. The system is then concentrated to dryness to obtain a crude product. The crude product is purified by column chromatography (dichloromethane/methanol=20: 1) to obtain 505 mg of the title compound.

MS(ESI+): 187.1(M+H).

**Preparation Example 3** 3-(tetrahydro-2H-pyran-4-yl)-2,3,4,5-tetrahydro-1H-benzo [d] azepin-7-amine

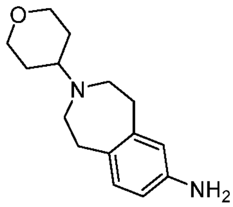

a) Synthesis of 7-nitro-3-(tetrahydro-2H-pyran-4-yl)-2,3,4,5-tetrahydro-1H-benzo[d]azepine

7-nitro-2,3,4,5-tetrahydro-1H-benzo[D]azepine (1920 mg), tetrahydropyran-4-one (1100 mg) and acetic acid (1110 mg) are dissolved in 30 ml of methanol, stirred for 1 h, and added with sodium triacetoxyborohydride (3180 mg) in batches to react overnight, and then filtered. The filtrate is concentrated to dryness, and purified by column chromatography (dichloromethane/methanol=20: 1) to obtain 1380 mg of the title compound. MS(ESI+): 277.2(M+H).

b) Synthesis of 3-(tetrahydro-2H-pyran-4-yl)-2,3,4,5-tetrahydro-1H-benzo[d]azepin-7-amine

7-nitro-3-(tetrahydro-2H-pyran-4-yl)-2,3,4,5-tetrahydro-1H-benzo[d]azepine (1380 mg) is dissolved in 50 ml of methanol, added with 10% palladium charcoal (150 mg) under the protection of nitrogen, added dropwise with 80% hydrazine hydrate (2 ml) and reacted to release gas. After 3 hours of reaction, the reactant is filtered. The mother liquor is concentrated to dryness under reduced pressure to obtain 1200 mg of the title compound. MS(ESI+): 247.2(M+H).

**Preparation Example 4** 2-amino-5-cyano-N-methylbenzamide

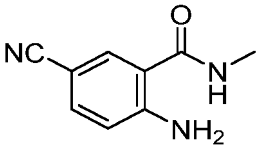

2-amino-5-cyano-N-methylbenzoic acid (500 mg), methylamine solution (6.2 mmol, 3.1 ml), 2-(7-azabenzotriazole)-N,N,N ',N'-tetramethylurea hexafluorophosphate (2.36 g) and N,N-diisopropylethylamine (1.2 g) are dissolved in N,N-dimethylformamide (15 mL) and stirred at room temperature for 4 h. The reaction solution is diluted with 80 mL of ethyl acetate and washed with a saturated sodium chloride aqueous solution (50 mL×3). An organic layer is taken and concentrated to dryness to obtain the title compound (550 mg). MS(ESI+): 176.1(M+H).

**Preparation Example 5** 2-amino-N-methyl-5-(trifluoromethyl)benzamide

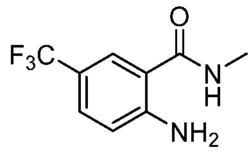

2-amino-5-(trifluoromethyl)benzoic acid (100 mg), methylamine (31 mg), N,N-dimethylformamide (5 ml), N,N-diisopropylethylamine (194 mg), and 2-(7-azabenzotriazole)-N,N,N',N'-tetramethylurea hexafluorophosphate (285 mg) are added at 0°C. After the addition, the mixture is transfered to room temperature and reacted for 2.0 h. After the reaction is completed, the reactant is added with 20 ml of water and then separated. The organic layers are extracted with ethyl acetate, merged, and dried with anhydrous sodium sulfate. A solvent is removed by rotary evaporation under reduced pressure. The reactant is purified by column chromatography (petroleum ether/ethyl acetate=3/1) to obtain 76 mg of the title compound. MS(ESI+): 219.1(M+H).

**Preparation Example 6** 2-amino-5-chloro-N-methylbenzamide

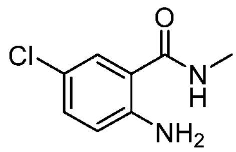

2-amino-5-chloro-benzoic acid (250 mg), 2-(7-azabenzotriazole)-N,N,N',N'-tetramethylurea hexafluorophosphate (665 mg) and N,N-diisopropylethylamine (377 mg) are sequentially added to a reaction flask, dissolved with 10 mL of anhydrous N,N-dimethylformamide, activated at room temperature for 10 min, then slowly added dropwise with a tetrahydrofuran solution of methylamine (1.75 mmol , 0.9 mL), and reacted for 1 h at room temperature after the addition is completed. After the reaction is completed, the reaction solution is added with 100 mL of ethyl acetate, and washed with a saturated sodium chloride solution for three times. Organic phases are merged, and concentrated to dryness under reduced pressure to obtain 495 mg of the crude title compound. MS(ESI+): 185.1(M+H).

**Preparation Example 7** N-(2-aminophenyl)cyclopropanesulfonamide

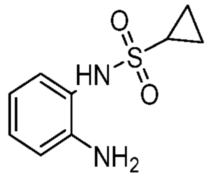

O-phenylenediamine (500 mg), triethylamine (1403 mg) and 10mL of anhydrous dichloromethane are added to a reaction flask. After all of them are dissolved, the mixture is added dropwise with cyclopropanesulfonyl chloride (715mg) at 0°C. After the addition is completed, the temperature is gradually raised to room temperature and the reaction is performed for 12 h. After the reaction is completed, the reaction solution is quenched with 1 ml of methanol. The pH of the system is adjusted to 8 with 1M hydrochloric acid and then concentrated to dryness to obtain a crude product. The crude product is purified by column chromatography (dichloromethane/methanol=20: 1) to obtain the title compound (1.094 g). MS(ESI+): 213.1(M+H).

**Preparation Example 8** 4-amino-2-methoxyl-N-methylnicotinamide

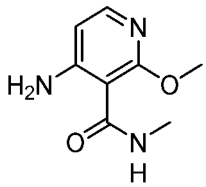

4-amino-2-methoxynicotinamide (1680 mg), 2-(7-azabenzotriazole)-N,N,N',N'-tetramethylurea hexafluorophosphate (4180 mg) and N,N-diisopropylethylamine (2580 mg) are dissolved in N,N-dimethylformamide (15 mL), added with 2M methylamine solution (5 mL) and stirred at room temperature for 1 h. After the reaction is completed, the reaction solution is poured into 30 mL of water, and extracted with ethyl acetate (15 mL×3 ). An organic layer is taken and concentrated to dryness, and purifed by column chromatography to obtain the title compound (900 mg). MS(ESI+): 182.1(M+H).

**Preparation Example 9** 4-amino-6-methoxyl-N-methylnicotinamide

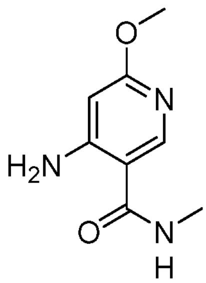

4-amino-6-methoxynicotinamide (1680 mg), 2-(7-azabenzotriazole)-N,N,N',N'-tetramethylurea hexafluorophosphate (4180 mg) and N,N-diisopropylethylamine (2580 mg) are dissolved in N,N-dimethylformamide (15 mL), added with 2M methylamine solution (5 mL, 10 mmol) and stirred at room temperature for 1 h. After the reaction is completed, the reaction solution is poured into 30 mL of water, and extracted with ethyl acetate (15 ml×3 ). An organic layer is taken and concentrated to dryness, and purified by column chromatography to obtain a yellow oily substance (560 mg). MS(ESI+): 182.1(M+H).

**Preparation Example 10** 6-amino-N-methylquinoxaline-5-carboxamide

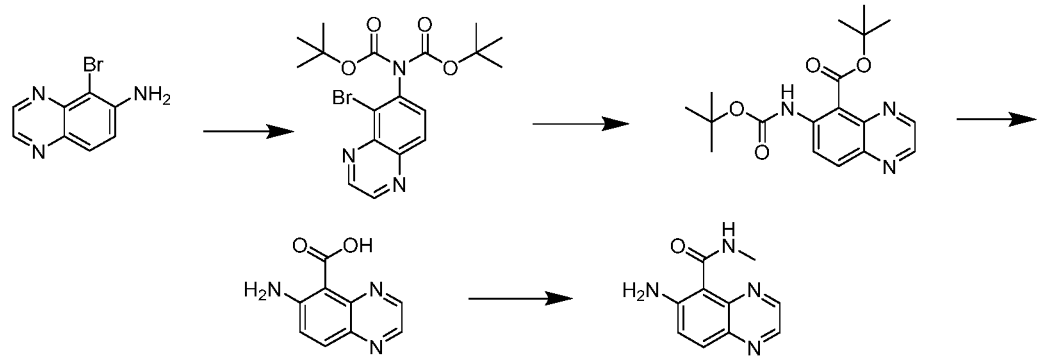

a) Preparation of N,N-bis(tert-butoxycarbonyl)-5-bromoquinoxaline-6-amine (2)

6-amino-5-bromoquinoxaline (1.0 g), 4-dimethylaminopyridine (0.05 g), and di-tert butyl carbonate (2.24 g) are dissolved in tetrahydrofuran (25 mL) and stirred at 40°C for 4 h. The reaction solution is poured into 50 mL of water and diluted, and then extracted with ethyl acetate (50 ml×3). An organic layer is taken and concentrated to dryness, and

purified by column chromatography (dichloromethane/methanol=40: 1) to obtain the title compound (1.5 g). MS(ESI+): 424.1(M+H).

b) Preparation of tert-butyl 6-((tert-butoxycarbonyl)amino)quinoxaline-5-carboxylate

N,N-bis(tert-butoxycarbonyl)-5-bromoquinoxaline-6-amine (500 mg) is dissolved in tetrahydrofuran (20 mL). After the temperature is cooled to -78°C, n-butyllithium (0.74 mL) is added under the protection of nitrogen, and the reaction ends after 30 min. The reaction solution is quenched by adding 5 mL of saturated ammonium chloride solution, and the tetrahydrofuran layer is taken and concentrated to dryness to obtain the title compound (320 mg). MS(ESI+): 346.2(M+H).

c) Preparation of 6-aminoquinoxaline-5-carboxylic acid

tert-butyl 6-((tert-butoxycarbonyl)amino)aminoquinoxaline-5-carboxylate (100 mg) is dissolved in dichloromethane (5 mL), added with trifluoroacetic acid (164 mg) and reacted at room temperature for 8 h. After the reaction is completed, the reactant is purified by column chromatography (dichloromethane/methanol=40: 1) to obtain the title compound (40 mg). MS(ESI+): 290.1(M+H).

d) Preparation of 6-amino-N-methylquinoxaline-5-carboxamide

6-aminoquinoxaline-5-carboxylic acid (40 mg), 2-(7-azabenzotriazole)-N,N,N',N'-tetramethylurea hexafluorophosphate (161 mg) and N,N-diisopropylethylamine (54 mg) are dissolved in N,N-dimethylformamide (5 mL), added with 2M methylamine solution (0.21 mL, 0.42 mmol) and then stirred at room temperature for 1 h. After the reaction is completed, the reaction solution is poured into 30 mL of water and extracted with ethyl acetate (15 mL×3). The organic layer is taken and concentrated to dryness, and purified by column chromatography (dichloromethane/methanol=20: 1) to obtain the title compound (28 mg). MS(ESI+): 203.1(M+H).

**Preparation Example 11** (2-amino-5-fluorophenyl)dimethylphosphine oxide

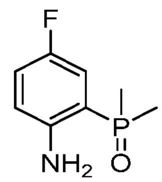

A compound 4-fluoro-2-iodoaniline (2.0 g), dimethyl phosphine oxide (0.79 g), potassium phosphate (2.14 g), tris(dibenzalacetone) dipalladium (0.153 g) and 4,5-bis(diphenylphosphine)-9,9-dimethylxanthene (0.097 g) are dissolved in DMF (15 mL), heated to 100°C under the protection of nitrogen and reacted for 16 h. The reaction solution is cooled and filtered. The filtrate is spin-dried, and added with 1M hydrochloric acid solution (30 mL) to adjust the pH to 1 to 2. Insoluble substances are removed by suction filtration. The filtrate is washed with dichloromethane (30mL×2). An aqueous phase is taken, added with a saturated sodium bicarbonate solution to adjust the pH to 8 to 9, and then extracted with dichloromethane (50 mL×3). The dichloromethane is spin-dried to obtain a crude product. The crude product is slurried with 30 mL of an ethyl acetate and petroleum ether (5:1) mixed solvent to obtain the title product (1.1 g). MS(ESI+): 188.08(M+H).

**Preparation Example 12** (2-amino-5-chlorophenyl) dimethyl phosphine oxide

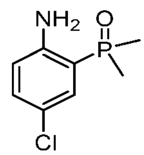

4-chloro-2-iodoaniline (1 g), dimethyl phosphine oxide (368 mg), potassium phosphate (996 mg), tris(dibenzalacetone) dipalladium (366 mg), 4,5-bis(diphenylphosphine)-9,9-dimethylxanthene (232 mg), and N,N-dimethylformamide/water (3:1, 10 ml) are sequentially added to a reaction flask. Nitrogen replacement is performed for 5 times. The reaction is performed in an oil bath at 100°C for 3 h. After the reaction is completed, the solvent is removed by rotary evaporation under reduced pressure, and purified by column chromatography (dichloromethane/methanol=20/1) to obtain 685 mg of the title product. MS(ESI+): 204.1(M+H).

**Preparation Example 13** (2-amino-5-methylphenyl) dimethyl phosphine oxide

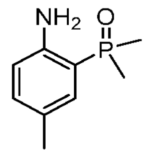

2-iodo-4-methylaniline (1 g), dimethyl phosphine oxide (505 mg), palladium acetate (97 mg), 4,5-bis(diphenylphosphine)-9,9-dimethylxanthene (249 mg), potassium phosphate (1.35 g), 15 mL of N,N-dimethylformamide and 3 mL of water are sequentially added to a reaction flask, dissolved, heated to 110°C under the protection of nitrogen and reacted for 3 h. After the reaction is completed, the temperature is cooled to room temperature. Insoluble inorganic salts and catalysts are removed by filtration. The filtrate is concentrated under reduced pressure and diluted with 30 mL of water. The pH is adjusted to 2 with 1M hydrochloric acid. Insoluble substances are removed by filtration. The filtrate is washed with dichloromethane and an aqueous layer is separated. The aqueous layer is adjusted to the pH of 9 with 1M sodium hydroxide solution and then extracted with dichloromethane. An organic phase is separated, dried with anhydrous sodium sulfate, filtered by suction, and concentrated to dryness under reduced pressure to obtain 1.6 g of the title product. MS(ESI+): 184.07(M+H).

**Preparation Example 14** (2-amino-5-cyanophenyl) dimethyl phosphine oxide

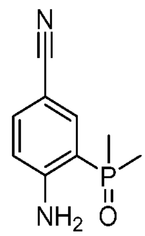

A compound 4-cyano-2-iodoaniline (0.98 g), dimethyl phosphine oxide (0.376 g), potassium phosphate (1.02 g), tris(dibenzalacetone) dipalladium (0.073 g) and 4,5-bis(diphenylphosphine)-9,9-dimethylxanthene (0.046 g) are dissolved in N,N-dimethylformamide (10 mL), heated to 100°C under the protection of nitrogen and reacted for 16 h. The reaction solution is cooled and filtered. The filtrate is concentrated to dryness, and added with 1M hydrochloric acid solution (30 mL) to adjust the pH to 1 to 2. Insoluble substances are removed by suction filtration. The filtrate is washed with dichloromethane (30 mL×2). A water phase is taken, added with a saturated sodium bicarbonate solution to adjust the pH to 8 to 9, then extracted with dichloromethane (50 mL×3), and concentrated to dryness to obtain the title product (0.7 g). MS(ESI+): 195.08(M+H).

**Preparation Example 15** (2-amino-5-methoxyphenyl) dimethyl phosphine oxide

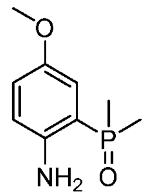

A compound 4-methoxyl-2-iodoaniline (1.0 g), dimethyl phosphine oxide (0.376 g), potassium phosphate (1.02 g), tris(dibenzalacetone) dipalladium (0.073 g) and 4,5-bis(diphenylphosphine)-9,9-dimethylxanthene (0.046 g) are dissolved in N,N-dimethylformamide (10 mL), heated to 100°C under the protection of nitrogen and reacted for 16 h. The reaction solution is cooled and filtered. The filtrate is concentrated to dryness, and added with 1M hydrochloric acid solution (30 mL) to adjust the pH to 1 to 2. Insoluble substances are removed by suction filtration. The filtrate is washed with dichloromethane (30 mL×2). A water phase is taken, added with a saturated sodium bicarbonate solution to adjust the pH to 8 to 9, then extracted with dichloromethane (50 mL×3), and concentrated to dryness to obtain the title product (0.7 g). MS(ESI+): 200.8(M+H).

**Preparation Example 16** (2-aminophenyl)bis(methyl-$d_3$) phosphine oxide

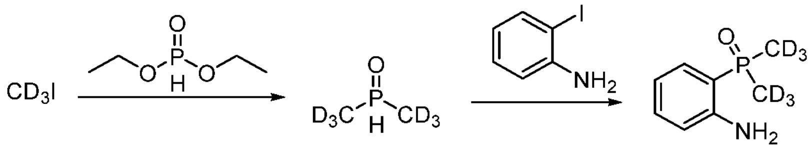

a) Preparation of bis(methyl-$d_3$) phosphine oxide

Magnesium chips (3.6 g) are added into a three-necked reaction flask. Nitrogen replacement is performed for three times. 30 ml of anhydrous ether is then added, cooled to -10°C and stirred. Iodomethane-$d_3$ (20 g) is diluted with 30 ml of ether and slowly dropwise added into the reaction flask. A reflux reaction is performed for 3 h after dropping. After the reflux reaction is completed, the temperature is cooled to 0°C. 20 ml of diethyl ether diluent of diethyl phosphite (6.35 g) is slowly dropwise added, and then quenched with a cold saturated potassium carbonate aqueous solution (19.2 g, 20 ml). Generated solid is removed by filtration. The filter cake is washed twice with ethanol, and the filtrate is concentrated at -0.8 Mpa and 50°C. The solid is removed by filtration again, and the filtrate is the title product (12 g).

b) Preparation of (2-aminophenyl)bis(methyl-$d_3$) phosphine oxide

2-iodoaniline (2.19 g), bis(methyl-$d_3$)phosphine oxide (1.68 g), potassium phosphate (3.17 g), palladium acetate (916 mg), 4,5-bisdiphenyl phosphine-9,9-dimethylxanthene (578 mg), 15 ml of N,N-dimethylformamide and 3 ml of water are sequentially added to a 100 ml three-necked flask. Nitrogen replacement is performed for 3 times. The reaction is then performed at 110°C for 3 h. After the reaction is completed, the reactant is spin-dried under reduced pressure, and purified by silica-gel column chromatography (dichloromethane/methanol=20: 1) to obtain 1.3 g of the title product. MS(ESI+): 176.1(M+H).

**Preparation Example 17** (2-amino-5-fluorophenyl)bis(methyl-$d_3$) phosphine oxide

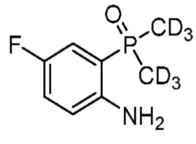

4-fluoro-2-iodoaniline (1.18 g), bis(methyl-$d_3$) phosphine oxide (1.68 g), potassium phosphate (2.12 g), palladium acetate (92 mg), 4,5-bisdiphenyl phosphine-9,9-dimethylxanthene (58 mg), 15 ml of N,N-dimethylformamide and

3 ml of water are sequentially added to a 100 ml three-necked flask. Nitrogen replacement is performed for 3 times. The reaction is then performed at 110°C for 3 h. After the reaction is completed, the solid is removed by filtration, the filtrate is concentrated to dryness under reduced pressure, and 15 ml of 2M hydrochloric acid is added to the residues to precipitate a yellow solid, and insoluble substances are removed by suction filtration. After an aqueous phase is washed twice with 15 ml of dichloromethane, the aqueous phase is added with a saturated potassium carbonate solution to adjust the pH to 10, and then extracted twice with 15 ml of dichloromethane. An organic phase is concentrated to dryness to obtain the title compound (1 g). MS(ESI+): 193.1(M+H).

**Preparation Example 18** (4-amino-1,3-phenylene)bis(dimethyl phosphine oxide)

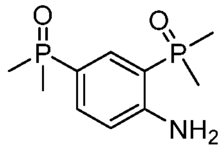

2,4-diiodoaniline (2 g), dimethyl phosphine oxide (1.6 g), potassium phosphate (2.12 g), palladium acetate (92 mg), 4,5-bisdiphenyl phosphine-9,9-dimethylxanthene (58 mg) and 15 ml of N,N-dimethylformamide are sequentially added to a 100 ml three-necked flask. Nitrogen replacement is performed for 3 times. The reaction is then performed at 110°C for 5 h. After the reaction is completed, the solid is removed by filtration, the filtrate is concentrated to dryness under reduced pressure, and 3 ml of 2M hydrochloric acid is added to the residues to precipitate a yellow solid, and insoluble substances are removed by suction filtration. After an aqueous phase is washed twice with 15 ml of dichloromethane, and the aqueous phase is added with a saturated potassium carbonate solution to adjust the pH to 7. The aqueous phase is concentrated to dryness, and purified by column chromatography (dichloromethane/methanol=20: 1, v/v) to obtain the title compound (1.4 g). MS(ESI+): 246.1(M+H).

**Preparation Example 19** (2-amino-5-difluoromethoxyphenyl) dimethyl phosphine oxide

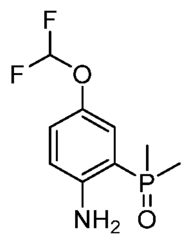

A compound 4-difluoromethoxyl-2-iodoaniline (1.0 g), dimethyl phosphine oxide (0.366 g), potassium phosphate (1.00 g), tris(dibenzalacetone) dipalladium (0.073 g) and 4,5-bis(diphenylphosphine)-9,9-dimethylxanthene (0.046 g) are dissolved in N,N-dimethylformamide (10 mL), heated to 100°C under the protection of nitrogen and reacted for 16 h. The reaction solution is cooled and filtered. The filtrate is concentrated to dryness, and added with 1M hydrochloric acid solution (30 mL) to adjust the pH to 1 to 2. Insoluble substances are removed by suction filtration. The filtrate is washed with dichloromethane (30 mL×2). A water phase is taken, added with a saturated sodium bicarbonate solution to adjust the pH to 8 to 9, then extracted with dichloromethane (50 mL×3), and concentrated to dryness to obtain the title product (0.7 g). MS(ESI+): 235.2(M+H).

**Preparation Example 20** (2-amino-5-(1H-tetrazol-1-yl)phenyl) dimethyl phosphine oxide

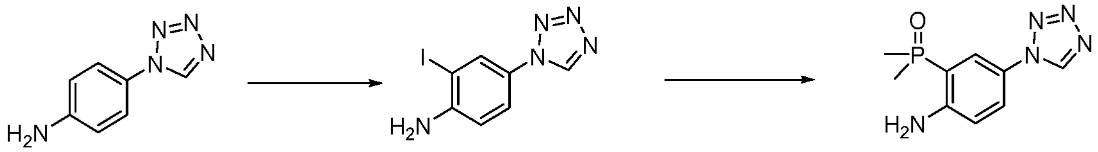

1) 2-iodo-4-(1H-tetrazol-1-yl)aniline

A compound 4-(1H-tetrazol-1-yl)aniline (0.97 g) is dissolved in glacial acetic acid (30 ml), stirred and cooled at 0°C, added with N-iodosuccinimide (1.57 g) in batches, and reacted for 15 min. Then, water (100 ml) and ethyl acetate (150 ml) are added to the reaction solution, added with a potassium carbonate solid to adjust the pH to 9, and separated to obtain an organic phase. The organic phase is then washed with water (100 ml). The organic phase is concentrated to dryness to obtain 1.8 g of the title product.

2) (2-amino-5-(1H-tetrazol-1-yl)phenyl) dimethyl phosphine oxide

A compound 2-iodo-4-(1H-tetrazol-1-yl)aniline (1.68 g), dimethyl phosphine oxide (0.685 g), potassium phosphate (2.48 g), tris(dibenzalacetone) dipalladium (0.267 g) and 4,5-bis(diphenylphosphine)-9,9-dimethylxanthene (0.338 g) are dissolved in N,N-dimethylformamide (30 mL), heated to 100°C under the protection of nitrogen, and reacted for 8 h. The reaction solution is cooled and filtered. The filtrate is concentrated to dryness, and added with 2M hydrochloric acid solution (30 mL) to adjust the pH to 2 to 3. Insoluble substances are removed by suction filtration. The filtrate is added with potassium carbonate to adjust the pH to 9, and then extracted with ethyl acetate (100 mL×3). The organic phase is concentrated to dryness to obtain the title compound (0.3 g). MS(ESI+): 238.2(M+H).

**Preparation Example 21** 3-(tetrahydrofuran-3-yl)-2,3,4,5-tetrahydro-1H-benzo[d]azepin-7-amine

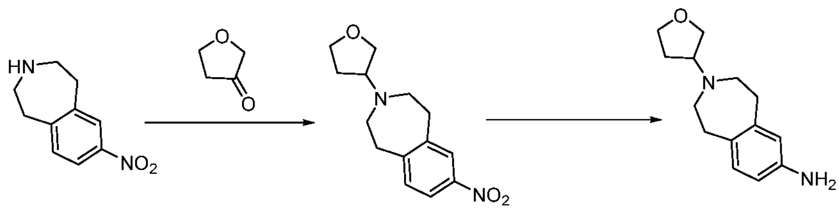

1) 7-nitro-3-(tetrahydrofuran-3-yl)-2,3,4,5-tetrahydro-1H-benzo[d]azepine

Dichloromethane (40 ml) is added to a 100 ml reaction flask, and added with 7-nitro-2,3,4,5-tetrahydro-1H-benzo[d]heterocyclic nitrogen (1.9 g), dihydrofuran-3(2H)-one (0.86 g) and acetic acid (1.1 g) under stirring at room temperature, and then stirred for 1 h. Then sodium triacetoxyborohyride (4.2 g) is added in batches, and stirred and stands overnight at room temperature. After the reaction is completed, a sodium hydroxide solution (100 ml, 1 M) is added for quenching. Then, liquid separation is performed. A dichloromethane phase is concentrated to dryness under reduced pressure, and the obtained residue is purified by silica-gel column chromatography (dichloromethane: methanol = 20: 1 v/v) to obtain 2.5 g of the title product.

2) 3-(tetrahydrofuran-3-yl)-2,3,4,5-tetrahydro-1H-benzo[d]azepin-7-amine

Methanol (40 ml) is added to a 100 ml reaction flask. Nitrogen replacement is performed for three times. Hydrazine hydrate (10 ml, 80% w/w) and palladium charcoal (0.25 g, 10% w/w) are sequentially added under stirring at room temperature, and stirred for 1 h. The mixture is then filtered. The mother liquor is concentrated to dryness under reduced pressure, and the obtained residue is purified by silica-gel column chromatography (dichloromethane: methanol = 20: 1 v/v) to obtain 1.5 g of the title product. MS(ESI+): 233.2(M+H).

**Preparation Example 22** (2-amino-5-trifluoromethoxyphenyl) dimethyl phosphine oxide

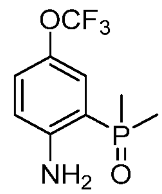

A compound 4-trifluoromethoxyl-2-iodoaniline (1.0 g), dimethyl phosphine oxide (0.366 g), potassium phosphate (1.00 g), tris(dibenzalacetone) dipalladium (0.073 g) and 4,5-bis(diphenylphosphine)-9,9-dimethylxanthene (0.046 g) are dissolved in N,N-dimethylformamide (10 mL), heated to 100°C under the protectionof nitrogen and reacted for 16 h. The reaction solution is cooled and filtered. The filtrate is concentrated to dryness, and added with 1M hydrochloric acid solution (30 mL) to adjust the pH to 1 to 2. Insoluble substances are removed by suction filtration. The filtrate is washed with dichloromethane (30 mL×2). A water phase is taken, added with a saturated sodium bicarbonate solution to adjust the pH to 8 to 9, then extracted with dichloromethane (50 mL×3), and concentrated to dryness to obtain the title product (0.7 g). MS(ESI+): 254.1(M+H).

**Preparation Example 23** (2-amino-5-cyclopropylphenyl) dimethyl phosphine oxide

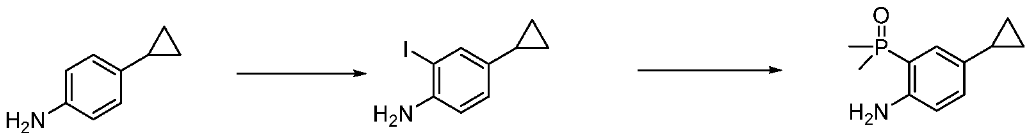

1) 2-iodo-4-cyclopropylaniline

A compound 4-cyclopropylaniline (1.0 g) is dissolved in glacial acetic acid (30 ml), stirred and cooled at 0°C, added with *N*-iodosuccinimide (1.57 g) in batches, and reacted for 15 min. Then, water (100 ml) and ethyl acetate (150 ml) are added to the reaction solution, added with a potassium carbonate solid to adjust the pH to 9, and separated to obtain an organic phase. The organic phase is then washed with water (100 ml). The organic phase is concentrated to dryness to obtain 1.2 g of the title product.

2) (2-amino-5-cyclopropylphenyl) dimethyl phosphine oxide

A compound 2-iodo-4-cyclopropylaniline (1.2 g), dimethyl phosphine oxide (0.685 g), potassium phosphate (2.48 g), tris(dibenzalacetone) dipalladium (0.267 g) and 4,5-bis(diphenylphosphine)-9,9-dimethylxanthene (0.338 g) are dissolved in *N,N*-dimethylformamide (30 mL), heated to 100°C under the protectionof nitrogen, and reacted for 8 h. The reaction solution is cooled and filtered. The filtrate is concentrated to dryness, and added with 2M hydrochloric acid solution (30 mL) to adjust the pH to 2 to 3. Insoluble substances are removed by suction filtration. The filtrate is added with potassium carbonate to adjust the pH to 9, and then extracted with ethyl acetate (100 mL×3). An organic phase is concentrated to dryness to obtain the title compound (0.3 g). MS(ESI+): 210.1(M+H).

**Preparation Example 24** (2-amino-5-isopropoxyphenyl) dimethyl phosphine oxide

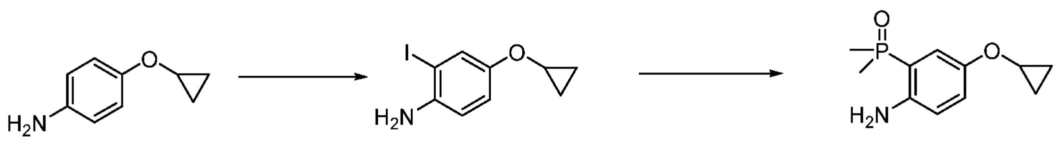

1) 2-iodo-4-isopropoxyaniline

A compound 4-isopropoxyaniline (1.0 g) is dissolved in glacial acetic acid (30 ml), stirred and cooled at 0°C,

added with *N*-iodosuccinimide (1.57 g) in batches, and reacted for 15 min. Then, water (100 ml) and ethyl acetate (150 ml) are added to the reaction solution, added with a potassium carbonate solid to adjust the pH to 9, and separated to obtain an organic phase. The organic phase is then washed with water (100 ml). The organic phase is concentrated to dryness to obtain 1.2g of the title product.

2) (2-amino-5-isopropoxyphenyl) dimethyl phosphine oxide

A compound 2-iodo-4-isopropoxyaniline (1.2 g), dimethyl phosphine oxide (0.685 g), potassium phosphate (2.48 g), tris(dibenzalacetone) dipalladium (0.267 g) and 4,5-bis(diphenylphosphine)-9,9-dimethylxanthene (0.338 g) are dissolved in *N,N*-dimethylformamide (30 mL), heated to 100°C under the protection of nitrogen, and reacted for 8 h. The reaction solution is cooled and filtered. The filtrate is concentrated to dryness, and added with 2M hydrochloric acid solution (30 mL) to adjust the pH to 2 to 3. Insoluble substances are removed by suction filtration. The filtrate is added with potassium carbonate to adjust the pH to 9, and then extracted with ethyl acetate (100 mL×3). The organic phase is concentrated to dryness to obtain the title compound (0.3 g). MS(ESI+): 226.1(M+H).

**Preparation Example 25 (2-amino-4-methoxyl-5-fluorophenyl) dimethyl phosphine oxide**

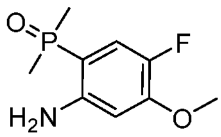

A compound 2-bromo-4-fluoro-5-methoxyaniline (2 g), dimethyl phosphine oxide (0. 85 g), potassium phosphate (2.48 g), palladium acetate (0.10 g) and 4,5-bis(diphenylphosphine)-9,9-dimethylxanthene (0.266 g) are dissolved in *N*,*N*-dimethylformamide (40 mL), heated to 120°C under the protection of nitrogen, and reacted for 48 h. The reaction solution is concentrated to dryness, and the residue is purified by silica-gel column chromatography (dichloromethane/methanol=40: 1 v/v) to obtain the title compound (0.5 g). MS(ESI+): 218.1(M+H).

**Preparation Example 26 (2-amino-5-fluoro-6-chlorophenyl) dimethyl phosphine oxide**

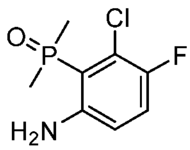

A compound 2-iodo-3-chloro-4-fluoroaniline (1.5 g), dimethyl phosphine oxide (0. 24 g), potassium phosphate (0.65 g), palladium acetate (0.06 g) and 4,5-bis(diphenylphosphine)-9,9-dimethylxanthene (0.09 g) are dissolved in *N*,*N*-dimethylformamide (30 mL), heated to 120°C under the protection nitrogen, and reacted for 2 h. The reaction solution is concentrated to dryness, and the residue is purified by silica-gel column chromatography (dichloromethane/methanol=10: 1 v/v) to obtain the title compound (0.35g). MS(ESI+): 222.1(M+H).

**Preparation Example 27 (2-amino-5-fluoro-6-chlorophenyl) dimethyl phosphine oxide**

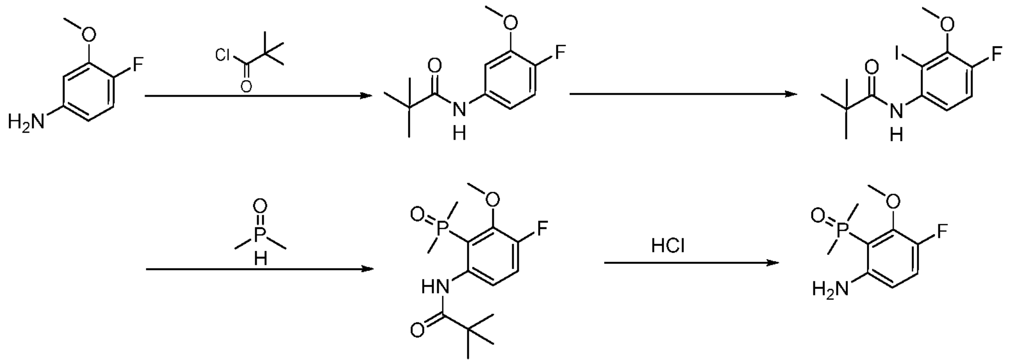


a) N-(4-fluoro-3-methoxyphenyl)-2,2-dimethylpropionamide

4-fluoro-3-methoxyaniline (5 g) and triethylamine (3.94 g) are added to dichloromethane (100 mL) in the atmosphere of nitrogen at room temperature, and then added dropwise with 2,2-dimethylpropionyl chloride (4.27 g) under stirring. The resulting mixture is stirred for 1 h. The reactant is quenched by adding water (100 mL) at room temperature and extracted with dichloromethane (3×100 mL). The merged organic layer is dried with anhydrous sodium sulfate. After filtration, the filtrate is concentrated under reduced pressure. The residue is purified by silica-gel column chromatography and eluted with petroleum ether: ethyl acetate = (1:1 v/v) to obtain the title product (7.7 g). MS(ESI+): 226.1(M+H).

b) N-(4-fluoro-2-iodo-3-methoxyphenyl)-2,2-dimethylpropionamide

N-(4-fluoro-3-methoxyphenyl)-2,2-dimethylpropionamide (1 g) is added to tetrahydrofuran (20 mL), and added dropwise with n-butyllithium (0.71 g) at 0°C in the atmosphere of nitrogen. The resulting mixture is stirred at 0°C in the atmosphere of nitrogen for 2 h. At - 78°C, iodine (1.41 g, dissolved in 10 mL of tetrahydrofuran) is added dropwise to the above mixture for 30 min. The resulting mixture is stirred for another 2 h at -78°C. The reaction is quenched by adding a saturated aqueous ammonium chloride solution (10 mL) at 0°C. The resulting mixture is extracted with ethyl acetate (3×100 mL). The merged organic layer is dried with anhydrous sodium sulfate. After filtration, the filtrate is concentrated under reduced pressure. The residue is purified by silica-gel column chromatography and eluted with petroleum ether: ethyl acetate = (1:1 v/v) to obtain the title product (1.3 g). MS(ESI+): 218.1(M+H).

c) N-[2-(dimethylphosphoryl)-4-fluoro-3-methoxyphenyl]-2,2-dimethylpropionamide

A compound N-(4-fluoro-2-iodo-3-methoxyphenyl)-2,2-dimethylpropionamide (0.55 g), dimethyl phosphine oxide (0. 15 g), potassium phosphate (1.0 g), palladium acetate (0.04 g) and 4,5-bis(diphenylphosphine)-9,9-dimethylxanthene (0.18 g) are dissolved in *N,N*-dimethylformamide (10 mL), heated to 120°C in the atmosphere of nitrogen, and reacted for 2 h. The reaction solution is concentrated to dryness, and the residue is purified by silica-gel column chromatography (dichloromethane/methanol=10: 1 v/v) to obtain the title compound (0.31g). MS(ESI+): 302.1(M+H).

d) (2-amino-5-fluoro-6-methoxyphenyl) dimethyl phosphine oxide

N-[2-(dimethylphosphoryl)-4-fluoro-3-methoxyphenyl]-2,2-dimethylpropionamide (290 mg) is added to hydrochloric acid (6M, 6 mL), and stirred at 100°C in the atmosphere of nitrogen and stands overnight. The mixture is neutralized to pH=8 with a saturated sodium carbonate aqueous solution. An aqueous layer is extracted with dichloromethane (3×5 mL). The merged organic layer is dried with anhydrous sodium sulfate. After filtration, the filtrate is concentrated under reduced pressure to obtain the title product (170 mg). MS(ESI+): 218.1(M+H).

**Example 1** (S)-2-((5-chloro-2-((7-(pyrrolidin-1-yl)-6,7,8,9-tetrahydro-5H-benzo[7]annulen-2-yl)amino)pyrimidin-4-yl)amino)-N,N-dimethyl benzenesulfonamide

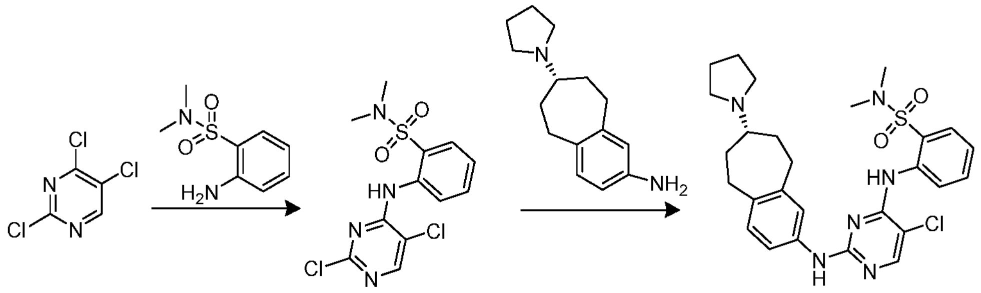

a) Preparation of 2-((2,5-dichloropyrimidin-4-yl)amino)-N,N-dimethyl benzenesulfonamide

10 ml of anhydrous N,N-dimethylformamide, 2-amino-N,N-dimethyl benzenesulfonamide (200 mg), and 2,4,5-trichloropyrimidine (183 mg) are sequentially added to a reaction flask, added with sodium hydride (120 mg, 60%) at 0°C and reacted at 0°C for 3 h. After the reaction is completed, 20 ml of water is added for quenching. The reaction solution is extracted with 20 ml of ethyl acetate. The organic phase is concentrated to dryness. The crude product is purified by column chromatography (petroleum ether/ethyl acetate = 20/1) to obtain the title compound (223 mg).

b) Preparation of (S)-2-((5-chloro-2-((7-(pyrrolidin-1-yl)-6,7,8,9-tetrahydro-5H-benzo[7]annulen-2-yl)amino)pyrimidin-4-yl)amino)-N,N-dimethyl benzenesulfonamide

N-butanol (10 ml), 2-((2,5-dichloropyrimidin-4-yl)amino)-N,N-dimethyl benzenesulfonamide (223 mg), 0.25 ml of trifluoroacetic acid, and (S)-7-(pyrrolidin-1-yl)-6,7,8,9-tetrahydro-5H-benzo[7]annulen-2-amine (115 mg) are sequentially added to a reaction flask, and reacted at 110°C for 5 h. After the reaction is completed, the reactant is concentrated to dryness. The residue is added with 20 ml of ethyl acetate, and then washed with 1M aqueous sodium hydroxide solution (10 ml) for three times. The organic phase is concentrated to dryness, and purified by column chromatography (dichloromethane/methanol=20/1) to obtain the title compound (180 mg).

$^1H$ NMR (400 MHz, $CDCl_3$) δ 9.43 (s, 1H), 8.54 (dd, J = 8.4, 1.1 Hz, 1H), 8.12 (s, 1H), 7.87 (dd, J = 8.0, 1.6 Hz, 1H), 7.56 (m, J = 8.7, 7.4, 1.6 Hz, 1H), 7.31 (d, J = 6.5 Hz, 2H), 7.24 (d, J = 8.2 Hz, 1H), 7.13 (s, 1H), 7.04 (d, J = 8.8 Hz, 1H), 3.36 - 3.15 (m, 5H), 2.87 (m, 1H), 2.75 (m, 9H), 2.38 (m, 2H), 2.09-1.92 (m, 4H), 1.57 (m, 2H). MS(ESI+): 541.2(M+H).

**Example 2** (S)-3-((5-chloro-2-((7-(pyrrolidin-1-yl)-6,7,8,9-tetrahydro-5H-benzo[7]annulen-2-yl)amino)pyrimidin-4-yl)amino)-N,N-dimethyl benzenesulfonamide

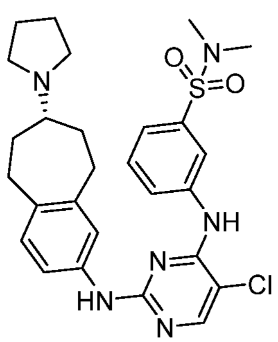

According to the preparation method of Example 1, 2-amino-N,N-dimethyl benzenesulfonamide in step a) is replaced with 3-amino-N,N-dimethyl benzenesulfonamide. $^1H$ NMR (400 MHz, $CDCl_3$) δ8.59 (s, 1H),8.10 (s, 1H), 8.02 (ddd, J = 5.5, 3.5, 2.3 Hz, 1H), 7.93-7.88 (m, 1H), 7.56-7.49 (m, 2H), 7.31 (s, 1H), 7.24 (dd, J = 8.0, 2.3 Hz, 1H), 7.19 (s, 1H), 7.02 (d, J = 8.0 Hz, 1H), 3.36-3.11 (m, 5H), 2.85 (m, 1H), 2.73 (m, 9H), 2.35 (m, 2H), 2.08-1.92 (m, 4H), 1.55 (m, 2H). MS(ESI+): 541.2(M+H).

**Example 3** (S)-4-((5-chloro-2-((7-(pyrrolidin-1-yl)-6,7,8,9-tetrahydro-5H-benzo[7]annulen-2-yl)amino)pyrimidin-4-yl)amino)-N,N-dimethyl benzenesulfonamide

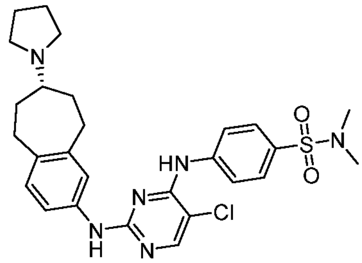

According to the preparation method of Example 1, 2-amino-N,N-dimethyl benzenesulfonamide in step a) is replaced with 4-amino-N,N-dimethyl benzenesulfonamide. $^1$H NMR (400 MHz, $CDCl_3$) δ8.11 (s, 1H), 7.87 - 7.79 (m, 2H), 7.73 (d, J = 8.6 Hz, 2H), 7.47 (d, J = 2.2 Hz, 1H), 7.41 (d, J = 12.3 Hz, 2H), 7.13 (dd, J = 8.1, 2.3 Hz, 1H), 7.04 (d, J = 8.1 Hz, 1H), 3.48-3.14 (m, 5H), 2.85 (m, 1H), 2.71 (m, 9H), 2.44 (m, 2H), 2.04 (m, 4H), 1.56 (m, 2H). MS(ESI+): 541.2(M+H).

**Example 4** (S)-5-chloro-$N^2$-(7-(pyrrolidin-1-yl)-6,7,8,9-tetrahydro-5H-benzo[7]annulen-2-yl)-$N^4$-(2-(pyrrolidin-1-ylsulfo)phenyl)pyrimidine-2,4-diamine

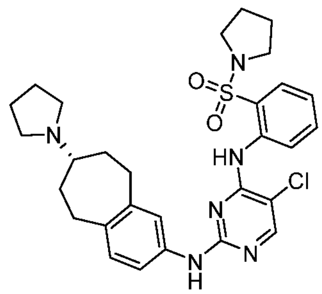

According to the preparation method of Example 1, 2-amino-N,N-dimethyl benzenesulfonamide in step a) is replaced with 1-(2-aminophenylsulfone)pyrrolidine.

$^1$H NMR (400 MHz, DMSO-$d_6$) δ 9.43 (s, 1H), 9.32 (s, 1H), 8.52 (s, 1H), 8.27 (s, 1H), 7.88 (dd, *J* = 8.0, 1.5 Hz, 1H), 7.75-7.61 (m, 1H), 7.36 (dd, *J* = 12.0, 4.7 Hz, 2H), 7.28 (d, *J* = 7.9 Hz, 1H), 6.99 (d, *J* = 8.2 Hz, 1H), 3.14 (t, *J* = 6.7 Hz, 5H), 2.89 (s, 6H), 2.73-2.59 (m, 2H), 2.05 (s, 2H), 1.79 (s, 4H), 1.73-1.57 (m, 4H), 1.45 (s, 2H). MS(ESI+): 567.2(M+H).

**Example 5** (S)-5-chloro-$N^2$-(7-(pyrrolidin-1-yl)-6,7,8,9-tetrahydro-5H-benzo[7]annulen-2-yl)-$N^4$-(3-(pyrrolidin-1-ylsulfo)phenyl)pyrimidine-2,4-diamine

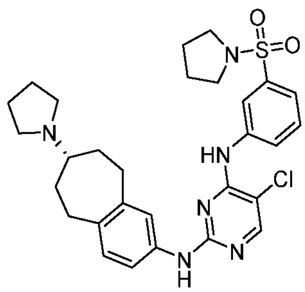

According to the preparation method of Example 1, 2-amino-N,N-dimethyl benzenesulfonamide in step a) is replaced with 3-(1-pyrrolylsulfonyl)aniline.

$^1H$ NMR (400 MHz, DMSO-$d_6$) δ 9.27 (s, 1H), 9.19 (s, 1H), 8.24 (t, *J* = 8.3 Hz, 1H), 8.19 (s, 1H), 7.89 (d, *J* = 1.7 Hz, 1H), 7.55 (ddd, *J* = 11.0, 7.8, 4.7 Hz, 2H), 7.38 (d, *J* = 1.8 Hz, 1H), 7.25 (d, *J* = 8.0 Hz, 1H), 6.94 (d, *J* = 8.2 Hz, 1H), 3.17 (t, *J* = 6.7 Hz, 5H), 3.04 - 2.76 (m, 6H), 2.65-2.54 (m, 2H), 2.06 (s, 2H), 1.80 (s, 4H), 1.69-1.58 (m, 4H), 1.42 (s, 2H). MS(ESI+): 567.2(M+H).

**Example 6** (S)-(2-((5-chloro-2-((7-(pyrrolidin-1-yl)-6,7,8,9-tetrahydro-5H-benzo[7]annulen-2-yl)amino)pyrimidin-4-yl)amino)phenyl)(pyrrolidin-1-yl)methanone

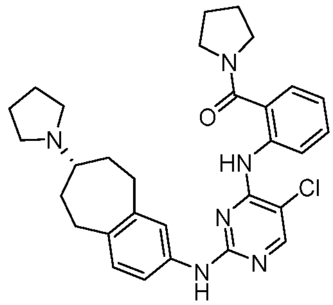

According to the preparation method of Example 1, 2-amino-N,N-dimethyl benzenesulfonamide in step a) is replaced with (2-aminophenyl)(pyrrolidin-1-yl)methanone. $^1H$ NMR (400 MHz, DMSO-$d_6$) δ 9.67 (s, 1H), 9.27 (s, 1H), 8.32 (d, *J* = 8.2 Hz, 1H), 8.17 (s, 1H), 7.56 (d, *J* = 7.6 Hz, 1H), 7.45 (dd, *J* = 8.2, 4.7 Hz, 2H), 7.30 (d, *J* = 8.0 Hz, 1H), 7.19 (t, *J* = 7.5 Hz, 1H), 6.97 (d, *J* = 8.2 Hz, 1H), 3.50-3.41 (m, 5H), 2.84 (s, 6H), 2.59 (d, *J* = 10.1 Hz, 2H), 2.03 (s, 2H), 1.81 (m, 8H), 1.45 (s, 2H). MS(ESI+): 531.3 (M+H).

**Example 7** (S)-3-((5-chloro-2-((7-(pyrrolidin-1-yl)-6,7,8,9-tetrahydro-5H-benzo[7]annulen-2-yl)amino)pyrimidin-4-yl)amino)-N,N-dimethylfuran-2-carboxamide

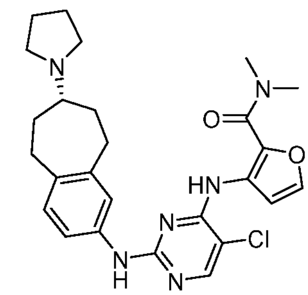

According to the preparation method of Example 1, 2-amino-N,N-dimethyl benzenesulfonamide in step a) is replaced with 3-amino-N,N-dimethylfuran-2-carboxamide. $^1H$ NMR (400 MHz, $CDCl_3$) δ10.67 (s, 1H), δ 8.04 (s, 1H), 7.55 (d, J = 2.0 Hz, 1H), 7.49-7.32 (m, 3H), 7.23 (dd, J = 8.0, 2.3 Hz, 1H), 7.07 (d, J = 8.1 Hz, 1H), 3.46-3.11 (m, 10H), 2.95-2.68 (m, 5H), 2.40 (m, 2H), 2.03 (m, 4H), 1.60 (m, 2H). MS(ESI+): 495.2(M+H).

**Example 8** (S)-5-chloro-4-(2-isopropoxyl)anilino-2-(7-(pyrrolidin-1-yl)-6,7,8,9-tetrahydro-5H-benzo[7]annulen-2-yl)pyrimidine

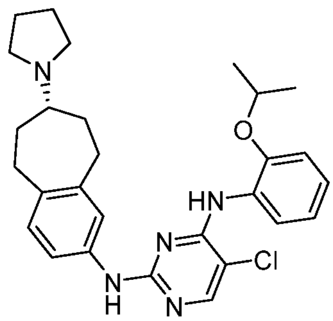

According to the preparation method of Example 1, 2-amino-N,N-dimethyl benzenesulfonamide in step a) is replaced with 2-(1-methylethoxyl)aniline.

$^1$H NMR (400 MHz, $CDCl_3$) $\delta$ 8.47 (d, J = 8.1 Hz, 1H), 8.05 (d, J = 3.6 Hz, 2H), 7.42 (s, 1H), 7.30 (s, 1H), 7.16 (s, 1H), 7.04 (dd, J = 17.9, 8.1 Hz, 2H), 6.93 (dd, J = 15.4, 7.8 Hz, 2H), 4.70 - 4.54 (m, 1H), 3.54-3.00 (m, 5H), 2.96-2.68 (m, 4H), 2.38 (m, 2H), 2.03 (m, 4H), 1.55 (m, 2H), 1.41 (m, 6H). MS(ESI+): 492.2(M+H).

**Example 9** (S)-4-(benzo[d][1,3]dioxo-4-yl)-5-chloro-2-(7-(pyrrolidin-1-yl)-6,7,8,9-tetrahydro-5H-benzo[7]annulen-2-yl)pyrimidine-2,4-diamine

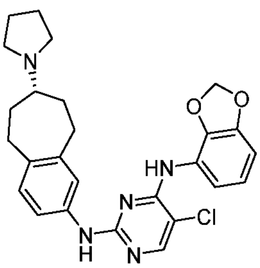

According to the preparation method of Example 1, 2-amino-N,N-dimethyl benzenesulfonamide in step a) is replaced with 4-amino-1,3-benzodioxole.

$^1$H NMR (400 MHz, $CDCl_3$) $\delta$ 8.04 (s, 1H), 7.51 (d, J = 8.3 Hz, 1H), 7.36 (d, J = 2.4 Hz, 1H), 7.29 (s, 1H), 7.25-7.19 (m, 1H), 7.00 (d, J = 7.8 Hz, 2H), 6.83 (t, J = 8.1 Hz, 1H), 6.71 (d, J = 7.7 Hz, 1H), 5.99-.93 (m, 2H), 3.56-3.05 (m, 5H), 2.85 (m, 1H), 2.71 (m, 3H), 2.37 (m, 2H), 2.03 (m, 4H), 1.60-1.47 (m, 2H). MS(ESI+): 478.2(M+H).

**Example 10** (S)-(2-((5-chloro-2-((7-(pyrrolidin-1-yl)-6,7,8,9-tetrahydro-5H-benzo[7]annulen-2-yl)amino)pyrimidin-4-yl)amino)phenyl)dimethyl phosphine oxide

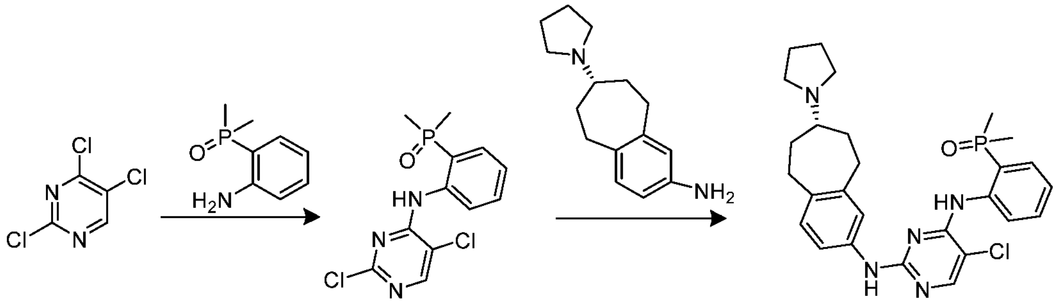

a) (2-((2,5-dichloropyrimidin-4-yl)amino)phenyl)dimethyl phosphine oxide

10 ml of anhydrous N,N-dimethylformamide, (2-aminophenyl) dimethyl phosphine oxide (169 mg), and 2,4,5-trichloropyrimidine (183 mg) are sequentially added to a reaction flask, added with sodium hydride (120 mg, 60%) at 0°C and reacted at 0°C for 3 h. After the reaction is completed, 20 ml of water is added for quenching. The reaction solution is extracted with 20 ml of ethyl acetate. The organic phase is concentrated to dryness. The crude product is purified by column chromatography (petroleum ether/ethyl acetate = 20/1) to obtain the title compound (200 mg).

b) (S)-(2-((5-chloro-2-((7-(pyrrolidin-1-yl)-6,7,8,9-tetrahydro-5H-benzo[7]annulen-2-yl)amino)pyrimidin-4-yl)amino)phenyl)dimethyl phosphine oxide

10 ml of N-butanol, (2-((2,5-dichloropyrimidin-4-yl)amino)phenyl)dimethyl phosphine oxide (158 mg), p-toluenesulfonic acid monohydrate (190 mg), (S)-7-(pyrrolidin-1-yl)-6,7,8,9-tetrahydro-5H-benzo[7]annulen-2-amine (115 mg) are sequentially added to a reaction flask, and reacted at 110°C for 5 h. After the reaction is completed, the reactant is concentrated to dryness. The residue is added with 20 ml of ethyl acetate, and then washed with 1M aqueous sodium hydroxide solution (10 ml) for three times. The organic phase is concentrated to dryness, and purified by column chromatography (dichloromethane/methanol=20/1) to obtain the title compound (115 mg).
$^1$H NMR (400 MHz, $CDCl_3$) $\delta$ 10.88 (s, 1H), 8.62-8.54 (m, 1H), 8.08 (s, 1H), 7.51-7.43 (m, 1H), 7.37 (d, J = 2.3 Hz, 1H), 7.31 (m, 2H), 7.22 (s, 1H), 7.15 (m, 1H), 7.04 (d, J = 8.1 Hz, 1H), 3.45-3.19 (m, 5H), 2.91- 2.68 (m, 4H), 2.37 (m, 2H), 2.02 (m, 4H), 1.84 (m, 6H), 1.56 (m, 2H). MS(ESI+): 510.2(M+H).

**Example 11** (S)-2-(3-((5-chloro-2-((7-(pyrrolidin-1-yl)-6,7,8,9-tetrahydro-5H-benzo[7]annulen-2-yl)amino)pyrimidin-4-yl)amino)phenyl)acetonitrile

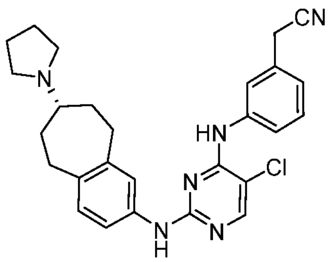

According to the preparation method of Example 1, 2-amino-N,N-dimethyl benzenesulfonamide in step a) is replaced with 3-aminobenzeneacetonitrile.
$^1$H NMR (400 MHz, DMSO-$d_6$) $\delta$ 9.17 (s, 1H), 8.91 (s, 1H), 8.28 (s, 1H), 8.16 (d, J = 17.6 Hz, 1H), 7.68 (t, J = 10.8 Hz, 1H), 7.60 (s, 1H), 7.37 (t, J = 7.9 Hz, 1H), 7.29 (d, J = 8.0 Hz, 1H), 7.12 (d, J = 7.8 Hz, 1H), 6.98-6.88 (m, 1H), 4.03 (m, 2H), 2.84 (s, 1H), 2.66 (s, 4H), 2.39 (m, 4H), 1.91 (s, 2H), 1.72 (s, 4H), 1.46 (s, 2H). MS(ESI+): 473.2(M+H).

**Example 12** (S)-2-((5-chloro-2-((7-(pyrrolidin-1-yl)-6,7,8,9-tetrahydro-5H-benzo[7]annulen-2-yl)amino)pyrimidin-4-yl)amino)-N-methylbenzamide

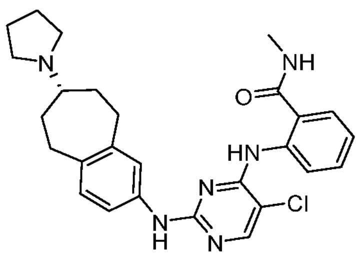

According to the preparation method of Example 1, 2-amino-N,N-dimethyl benzenesulfonamide in step a) is replaced with 2-amino-N-methylbenzamide.
$^1$H NMR (400 MHz, Chloroform-$d$) $\delta$ 11.02 (s, 1H), 8.74-8.48 (m, 1H), 8.08 (s, 1H), 7.51 (dd, J = 8.0, 1.5 Hz, 1H), 7.43 (ddd, J = 8.7, 7.3, 1.5 Hz, 1H), 7.36 (d, J = 2.3 Hz, 1H), 7.26-7.22 (m, 1H), 7.14-6.93 (m, 3H), 6.55 (m, 1H),

3.11 (m, 4H), 3.02 (m, 3H), 2.85 (m, 2H), 2.77-2.66 (m, 3H), 2.30 (m, 2H), 1.95 (m, 4H), 1.51 (m, 2H). MS(ESI+): 491.2(M+H).

**Example 13** (S)-2-((5-chloro-2-((7-(pyrrolidin-1-yl)-6,7,8,9-tetrahydro-5H-benzo[7]annulen-2-yl)amino)pyrimidin-4-yl)amino)benzamide

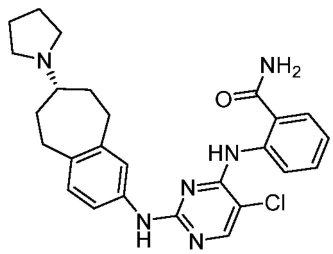

According to the preparation method of Example 1, 2-amino-N,N-dimethyl benzenesulfonamide in step a) is replaced with 2-amino-benzamide.

$^1$H NMR (400 MHz, $CDCl_3$) δ 11.18 (s, 1H), 8.67 (dd, J = 8.4, 1.1 Hz, 1H), 8.08 (s, 1H), 7.60 (dd, J = 7.9, 1.6 Hz, 1H), 7.47 (ddd, J = 8.7, 7.3, 1.6 Hz, 1H), 7.39 (d, J = 2.3 Hz, 1H), 7.28 (d, J = 2.4 Hz, 1H), 7.18-7.08 (m, 2H), 7.04 (d, J = 8.1 Hz, 1H), 3.48 - 3.12 (m, 5H), 2.93-2.65 (m, 5H), 2.38-2.02 (m, 7H), 1.56 (m, 2H). MS(ESI+): 477.2(M+H).

Example 14 (S)-2-((5-chloro-2-((7-(pyrrolidin-1-yl)-6,7,8,9-tetrahydro-5H-benzo[7]annulen-2-yl)amino)pyrimidin-4-yl)amino)-N-methyl benzenesulfonamide

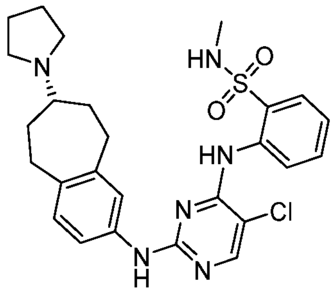

According to the preparation method of Example 1, 2-amino-N,N-dimethyl benzenesulfonamide in step a) is replaced with 2-amino-N-methyl benzenesulfonamide.

$^1$H NMR (400 MHz, $CDCl_3$) δ 8.06 (s, 1H), 7.53 (d, J = 2.3 Hz, 1H), 7.49 (dd, J = 8.2, 1.6 Hz, 1H), 7.24-7.11 (m, 3H), 7.03 (s, 1H), 6.59 (dd, J = 8.2, 1.1 Hz, 1H), 6.47 (ddd, J = 8.2, 7.1, 1.1 Hz, 1H), 5.02 (s, 1H), 3.57 (s, 3H), 3.35-3.04 (m, 5H), 2.95 (m, 2H), 2.77 (m, 2H), 2.37 (m, 2H), 2.01-1.93 (m, 4H), 1.62 (m, 2H). MS(ESI+): 527.2(M+H).

**Example 15** (S)-2-(2-((5-chloro-2-((7-(pyrrolidin-1-yl)-6,7,8,9-tetrahydro-5H-benzo[7]annulen-2-yl)amino)pyrimidin-4-yl)amino)phenyl)propan-2-ol

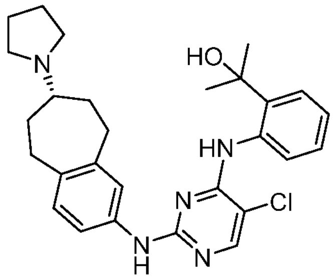

According to the preparation method of Example 1, 2-amino-N,N-dimethyl benzenesulfonamide in step a) is replaced with 2-(2-aminophenyl)propan-2-ol.

$^1H$ NMR (400 MHz, $CDCl_3$) δ 9.96 (s, 1H), 8.25 (dd, J = 8.1, 1.4 Hz, 1H), 8.02 (s, 1H), 7.31 (m, 4H), 7.08 (m, 2H), 7.00 (d, J = 7.8 Hz, 1H), 3.41-3.08 (m, 5H), 2.96-2.63 (m, 5H), 2.34 (m, 2H), 1.99 (m, 4H), 1.70 (s, 6H), 1.58-1.44 (m, 2H). MS(ESI+): 492.2(M+H).

**Example 16** (S)-2-((5-chloro-2-((7-(pyrrolidin-1-yl)-6,7,8,9-tetrahydro-5H-benzo[7]annulen-2-yl)amino)pyrimidin-4-yl)amino)-5-fluorobenzamide

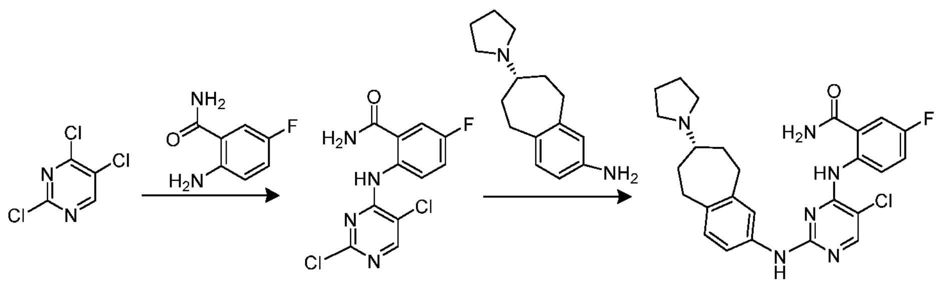

a) 2-((2,5-dichloropyrimidin-4-yl)amino)-5-fluoro-benzamide

2,4,5-trichloropyrimidine (1.33 mmol), 2-amino-5-fluoro-benzamide (1.1 mmol) and tetrahydrofuran (10 mL) are added into a 100 mL three-necked flask, stirred and cooled to -20°C, added dropwise with a tetrahydrofuran solution of lithium hexamethyldisilazide (1.65 mmol, 1 mol/L), heated naturally to room temperature and reacted for 8 h. After the reaction is completed, the resolution solution is quenched with a saturated ammonium chloride solution, and extracted with 30 mL of ethyl acetate and 30 mL of water. An organic phase is dried with anhydrous sodium sulfate and concentrated to dryness under reduced pressure to obtain the title compound (470 mg). MS(ESI+): 300.9(M+H).

b) (S)-2-((5-chloro-2-((7-(pyrrolidin-1-yl)-6,7,8,9-tetrahydro-5H-benzo[7]annulen-2-yl)amino)pyrimidin-4-yl)amino)-5-fluorobenzamide

2-((2,5-dichloropyrimidin-4-yl)amino)-5-fluoro-benzamide (0.9 mmol), (S)-7-(pyrrolidin-1-yl)-6,7,8,9-tetrahydro-5H-benzo[7]annulen-2-amine (1.08 mmol), tris(dibenzylideneacetone) dipalladium (0.09 mmol), 2-dicyclohexylphosphino-2'-(N,N-dimethylamine)-biphenyl (0.27 mmol), cesium carbonate (1.8 mmol), 2-methyltetrahydrofuran (10 mL) and water (5 mL) are added to a 50 mL three-necked flask. Nitrogen protection is performed. The reaction is then performed at 76°C for 24 h. The aqueous phase is extracted with 30 mL of ethyl acetate, and an organic layer is concentrated to dryness under reduced pressure, and purified with silica-gel column chromatography to obtain 28 mg of the title compound.

$^1H$ NMR (400 MHz, DMSO-$d_6$) δ 11.59 (s, 1H), 9.35 (s, 1H), 8.76 (s, 1H), 8.35 (s, 1H), 8.25 (s, 1H), 7.88 (s, 1H), 7.69 (dd, J = 9.7, 3.0 Hz, 1H), 7.43 (d, J = 1.9 Hz, 1H), 7.38-7.28 (m, 2H), 7.04 (d, J = 8.2 Hz, 1H), 2.96-2.71 (m, 7H), 2.68-2.55 (m, 2H), 2.08 (br, 2H), 1.78 (br, 4H), 1.47 (br, 2H). MS(ESI+): 495.2(M+H).

**Example 17** (S)-5-chloro-$N^4$-(2-(5-methyloxazol-2-yl)phenyl)-$N^2$-(7-(pyrrolidin-1-yl)-6,7,8,9-tetrahydro-5H-benzo[7]annulen-2-yl)pyrimidine-2,4-diamine

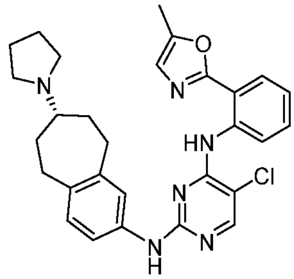

According to the preparation method of Example 1, 2-amino-N,N-dimethyl benzenesulfonamide in step a) is replaced with 2-(5-methyl-2-oxazolyl)aniline.

$^1$H NMR (400 MHz, DMSO-$d_6$) δ 11.50 (s, 1H), 9.37 (s, 1H), 8.95 (d, J = 7.3 Hz, 1H), 8.26 (s, 1H), 7.99 (dd, J = 7.9, 1.3 Hz, 1H), 7.48 - 7.39 (m, 2H), 7.35 (d, J = 8.0 Hz, 1H), 7.25-7.15 (m, 2H), 7.02 (d, J = 8.1 Hz, 1H), 2.96-2.80 (m, 2H), 2.69-2.54 (m, 7H), 2.43 (s, 3H), 1.92 (br, 2H), 1.71 (br, 4H), 1.52 (br, 2H). MS(ESI+): 515.2(M+H).

**Example 18** (S)-$N^4$-(2-(1H-imidazol-2-yl)phenyl)-5-chloro-$N^2$-(7-(pyrrolidin-1-yl)-6,7,8,9-tetrahydro-5H-benzo[7]annulen-2-yl)pyrimidine-2,4-diamine

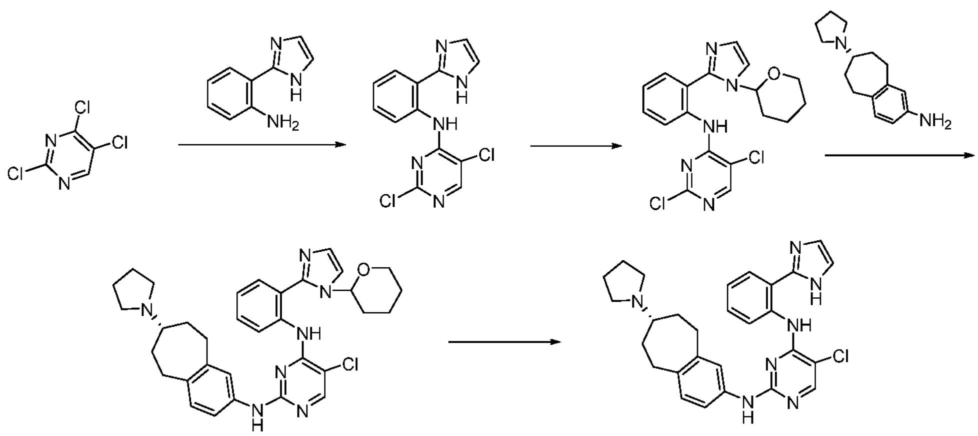

a) Preparation of N-(2(1H-imidazol-2-yl)phenyl)-2,5-dichloropyrimidin-4-amine

2-(1H-imidazol-2-yl)aniline (200 mg) and 2,4,5-trichloropyrimidine (200 mg), and N,N-diisopropylethylamine (390 mg) are sequentially added to a reaction flask, and reacted in an oil bath at 80°C for 5.0 h. After the reaction is completed, water (50 ml) is added. The aqueous layer is extracted with ethyl acetate (5 ml*3). Organic layers are merged, dried with anhydrous sodium sulfate, concentrated under reduced pressure to dryness, and purified by column chromatography (petroleum ether/ethyl acetate=5/1) to obtain the title compound (152 mg).

b) Preparation of 2,5-dichloro-N-(2-(1-(tetrahydro-2H-pyran-2-yl)-1H-imidazol-2-yl)phenyl)pyrimidin-4-amine

N-(2-(1H-imidazol-2-yl)phenyl)-2,5-dichloropyrimidin-4-amine (150 mg), dihydropyran (84 mg), p-toluenesulfonic acid (19 mg), and ethyl acetate (10 ml) are sequentially added to a reaction flask. The reaction is performed at 50°C for 3.5 h. After the reaction is completed, the organic layer is washed with saturated sodium chloride solution, dried with anhydrous sodium sulfate, and concentrated to dryness under reduced pressure. The crude product is purified by column chromatography (dichloromethane/methanol=20: 1) to obtain the title compound (120 mg).

c) Preparation of 5-chloro-$N^2$-((S)-7-(pyrrolidin-1-yl)-6,7,8,9-tetrahydro-5H-benzo[7]annulen-2-yl)-$N^4$-(2-(1-(tetrahydro-2H-pyran-2-yl)-1H-imidazol-2-yl)phenyl)pyrimidine-2,4-diamine

(2,5-dichloro-N-(2-(1-(tetrahydro-2H-pyran-2-yl)-1H-imidazol-2-yl)phenyl)pyrimidine-4 -amine) (120 mg), 5((S)-7-(pyrrolidin-1-yl)-6,7,8,9-tetrahydro-5H-benzo[7]annulen-2-amine) (185 mg), tris(dibenzylideneacetone) dipalladium(0)(48 mg), 2-dicyclohexylphosphino-2'-(N,N-dimethylamine)-biphenyl(20 mg), cesium carbonate (460 mg), and

N,N-dimethylformamide (9 ml) are sequentially added to a reaction flask. Nitrogen replacement is performed for five times. A microwave reaction is performed at 130°C for 1 h. After the reaction is completed, the solid is removed by suction filtration, and the filter cake is washed with dichloromethane. Organic layers are merged, dried with anhydrous sodium sulfate, and concentrated under reduced pressure to dryness. The crude product is purified by column chromatography (dichloromethane/methanol=20: 1) to obtain the title compound (95 mg).

d) Preparation of (S)-$N^4$-(2-(1H-imidazol-2-yl)phenyl)-5-chloro-$N^2$-(7-(pyrrolidin-1-yl)-6,7,8,9-tetrahydro-5H-benzo[7]annulen-2-yl)pyrimidine-2,4-diamine

5-chloro-$N^2$-((S)-7-(pyrrolidin-1-yl)-6,7,8,9-tetrahydro-5H-benzo[7]annulen-2-yl)-$N^4$-(2-(1-(tetrahydro-2H-pyran-2-yl)-1H-imidazol-2-yl)phenyl)pyrimidine-2,4-diamine (95 mg), camphorsulfonic acid (187 mg), and dichloromethane/methanol (5 ml: 5 ml) are sequentially added to a reaction flask, and stirred and stands overnight at room temperature. After the reaction is completed, water is added for extraction. The organic phase is dried with anhydrous sodium sulfate. The solid substance obtained by drying under reduced pressure. The solid substance is purified by silica-gel column chromatography to obtain the title compound (15 mg).

$^1$H NMR (400 MHz, $CDCl_3$) $\delta$ 12.17 (s, 1H), 8.75 - 8.58 (m, 2H), 8.07 (s, 1H), 7.58 (dd, *J* = 7.9, 1.3 Hz, 1H), 7.35 (d, *J* = 2.0 Hz, 1H), 7.32-7.28 (m, 1H), 7.25-7.14 (m, 3H), 7.11 (d, *J* = 7.7 Hz, 1H), 7.01-6.94 (m, 2H), 3.06 (s, 4H), 2.71 (m, 5H), 2.23 (m, 4H), 1.56-1.48 (m, 2H), 1.45 (s, 2H). MS(ESI+): 500.2(M+H).

**Example 19** (S)-2-((5-chloro-2-((7-(pyrrolidin-1-yl)-6,7,8,9-tetrahydro-5H-benzo[7]annulen- 2-yl)amino)pyrimidin-4-yl)amino)-5-fluoro-N-methylbenzamide

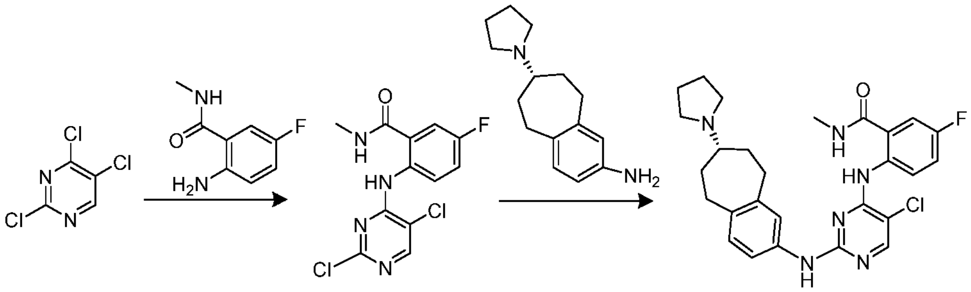

a) Preparation of 2-((2,5-dichloropyrimidin-4-yl)amino)-5-fluoro-N-methyl-benzamide

2,4,5-trichloropyrimidine (1.2 mmol), 2-amino-5-fluoro-N-methyl-benzamide (1.0 mmol) and tetrahydrofuran (10 mL) are added into a 100 mL three-necked flask, added dropwise with lithium hexamethyldisilazide (2.5 mmol) at -20°C, heated to room temperature after dropping, and stirred for 8 h. After the reaction of the raw materials is completed, the reaction is stopped. A saturated ammonium chloride solution is added for quenching. 30 mL of ethyl acetate and 30 mL of water are added to the reaction solution, stirred and extracted. The aqueous layer is extracted again with 30 mL of ethyl acetate. Organic phases are merged, washed sequentially with water and saturated sodium chloride solution, and dried with anhydrous sodium sulfate. After filtration, the filtrate is concentrated under reduced pressure to dryness to obtain the title compound (250 mg).

b) Preparation of (S)-2-((5-chloro-2-((7-(pyrrolidin-1-yl)-6,7,8,9-tetrahydro-5H-benzo[7]annulen-2-yl)amino)pyrimidin-4-yl)amino)-5-fluoro-N-methylbenzamide

2-((2,5-dichloropyrimidin-4-yl)amino)-5-fluoro-N-methyl-benzamide (0.38 mmol), (S)-7-(pyrrolidin-1-yl)-6,7,8,9-tetrahydro-5H-benzo[7]annulen-2-amine (0.38 mmol), tris(dibenzylideneacetone) dipalladium (0.04 mmol), 2-dicyclohexylphosphino-2'-(N,N-dimethylamine)-biphenyl (0.12 mmol), cesium carbonate (0.76 mmol), 2-methyltetrahydrofuran (10 mL) and water (5 mL) are added to a 50 mL three-necked flask. Nitrogen protection is performed. The reaction is then performed at 76°C for 24 h. The aqueous phase is extracted with 30 mL of ethyl acetate, and an organic layer is concentrated to dryness, and purified with silica-gel column chromatography to obtain the title compound (53 mg).

$^1$H NMR (400 MHz, DMSO-*d*6) $\delta$ 11.31 (s, 1H), 9.31 (s, 1H), 8.82 (d, J = 4.6 Hz, 1H), 8.75-8.66 (m, 1H), 8.20 (s, 1H), 7.62 (dd, J = 9.6, 3.0 Hz, 1H), 7.41 (d, J = 2.0 Hz, 1H), 7.36-7.26 (m, 2H), 7.01 (d, J = 8.2 Hz, 1H), 2.96-2.76 (m, 5H), 2.70-2.53 (m, 7H), 1.93 (m, 2H), 1.72 (br, 4H), 1.51 (m, 2H). MS(ESI+): 509.1(M+H).

**Example 20** (S)-4-((5-chloro-2-((7-(pyrrolidin-1-yl)-6,7,8,9-tetrahydro-5H-benzo[7]annulen- 2-yl)amino)pyrimidin-4-yl)amino)-N-methylnicotinamide

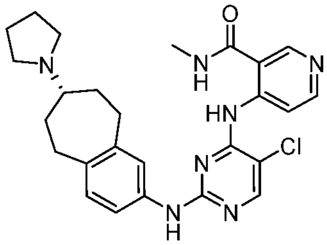

According to the preparation method of Example 1, 2-amino-N,N-dimethyl benzenesulfonamide in step a) is replaced with 4-amino-N-methylnicotinamide. MS(ESI+): 492.2(M+H).

**Example 21** (S)-2-((5-chloro-2-((7-(pyrrolidin-1-yl)-6,7,8,9-tetrahydro-5H-benzo[7]annulen-2-yl)amino)pyrimidin-4-yl)amino)-N-methylnicotinamide

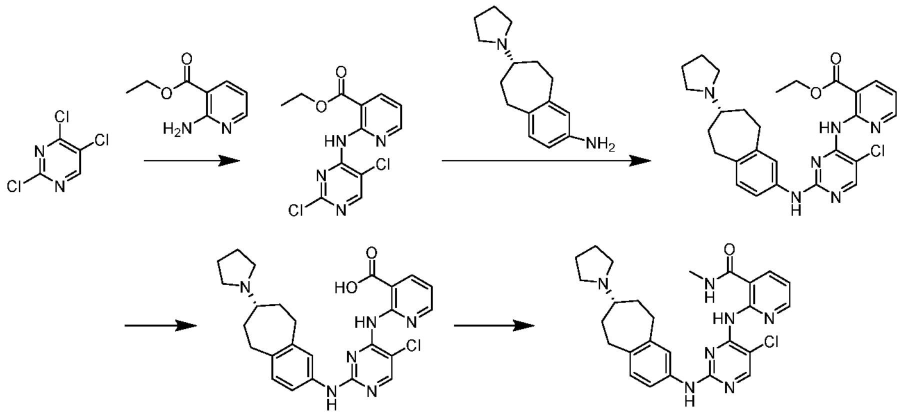

a) Preparation of ethyl 2-((2,5-dichloropyrimidin-4-yl)amino)nicotinate

Sodium hydride (130.83 mg) is added at 0°C to a N,N dimethylformamide solution (15 mL) in which ethyl 2-aminonicotinate (543.59 mg) is dissolved. The reaction mixture is stirred for 20 min. 2,3,5-trichloropyrimidine (500 mg) is added at 0°C. The reaction mixture is stirred at 0°C for 3 h. The reaction solution is poured into water (100 mL), and extracted with ethyl acetate (100 mL). The organic phase is washed with a saturated sodium chloride solution (100 mL*3), dried with anhydrous sodium sulfate, and concentrated to dryness under reduced pressure. The crude product is purified by silica-gel column chromatography (petroleum ether: ethyl acetate = 1: 1) to obtain the title compound (0.2 g).

b) Preparation of ethyl (S)-2-((5-chloro-2-((7-(pyrrolidin-1-yl)-6,7,8,9-tetrahydro-5H-benzo[7]annulen-2-yl)amino) pyrimidin-4-yl)amino)nicotinate

(S)-7-(pyrrolidin-1-yl)-6,7,8,9-tetrahydro-5H-benzo[7]annulen-2-amine (220.68 mg) and p-toluenesulfonic acid (219.97 mg) are added at 20°C to a dimethylsulfoxide solution (10 ml) in which ethyl 2-((2,5-dichloropyrimidine-4-yl)amino)nicotinate (200 mg) is dissolved.

The reaction mixture is stirred in microwaves at 150°C for 1 h. The reaction solution is poured into water (100 mL), and then extracted with dichloromethane (100 mL). The organic phase is washed with a saturated sodium chloride solution (100 mL*3), and spin-dried with anhydrous sodium sulfate. The product is purified by silica-gel plate chromatography (dichloromethane: methanol = 10: 1) to obtain the title compound (0.2 g).

c) Preparation of (S)-2-((5-chloro-2-((7-(pyrrolidin-1-yl)-6,7,8,9-tetrahydro-5H-benzo[7]annulen-2 -yl)amino)pyrimidin-4-yl)amino)nicotinic acid

Ethyl (S)-2-((5-chloro-2-((7-(pyrrolidin-1-yl)-6,7,8,9-tetrahydro-5H-benzo[7]annulen-2-yl)amino)pyrimidin-4-yl)amino)nicotinate (200 mg) is dissolved in methanol (10 mL) at 20°C, and added with sodium hydroxide (78.89 mg) and water (1 mL). The reaction mixture is stirred at 80°C for 3 h. The reaction solution is concentrated to dryness to obtain the title compound (0.15 g).

d) Preparation of (S)-2-((5-chloro-2-((7-(pyrrolidin-1-yl)-6,7,8,9-tetrahydro-5H-benzo[7]annulen-2-yl)amino)pyrimidin-4-yl)amino)-N-methyl nicotinamide

A 2M methylaminotetrahydrofuran solution (0.18 mL) and diisopropylethylamine (121.43 mg) are added at 20°C to N,N dimethylformamide (5 mL) in which (S)-2-((5-chloro-2-((7-(pyrrolidin-1-yl)-6,7,8,9-tetrahydro-5H-benzo[7]annulen-2-yl)amino)pyrimidin-4-yl)amino) nicotinic acid (150 mg) is dissolved. The reaction mixture is stirred for 2 min. 2-(7-benzotriazole oxide)-N,N,N',N'-tetramethylurea hexafluorophosphate (142.89 mg) is added. The reaction mixture is stirred at 20°C for 3 h. The reaction solution is poured into water (100 mL), and then extracted with dichloromethane (100 mL). The organic phase is washed with a saturated sodium chloride solution (100 mL*3), dried with anhydrous sodium sulfate, and concentrated to dryness. The product is purified by silica-gel plate chromatography (dichloromethane: methanol = 10: 1) to obtain the title compound (20 mg). $^1$H NMR (400 MHz, DMSO-$d_6$) δ11.37 (s, 1H),9.35 (s, 1H), 9.02 (s, 1H), 8.59 (s, 1H), 8.27 (s, 1H), 8.24 (s, 1H), 7.89 (s, 1H), 7.44 (s, 1H), 7.24 (s, 1H), 6.96-7.05 (m, 1H), 3.17 (d, J = 4.8 Hz, 1H), 2.70-2.98 (m, 9H), 1.97-2.16 (m, 2H), 1.77 (m, 4H), 1.41-1.56 (m, 2H), 1.24 (d, J = 6.0 Hz, 2H). MS(ESI+): 492.2(M+H).

**Example 22** (S)-5-chloro-N$^4$-(2-(isopropylsulfo)phenyl)-N$^2$-(7-(pyrrolidin-1-yl)-6,7,8,9-tetrahydro-5H-benzo[7]annulen-2-yl)pyrimidine-2,4-diamine

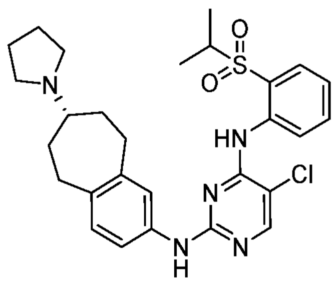

According to the preparation method of Example 1, 2-amino-N,N-dimethyl benzenesulfonamide in step a) is replaced with 2-(isopropylsulfonyl)aniline.

$^1$H NMR (400 MHz, $CDCl_3$) δ 9.59 (s, 1H), 8.62-8.54 (m, 1H), 8.14 (s, 1H), 7.92 (d, J = 6.9 Hz, 1H), 7.60 (t, J = 7.4 Hz, 1H), 7.28 (d, J = 9.1 Hz, 3H), 7.10-6.92 (m, 2H), 3.24 (dd, J = 13.4, 6.6 Hz, 1H), 3.07 (m, 4H), 2.96-2.88 (m, 1H), 2.89-2.67 (m, 4H), 1.97 (m, 4H), 1.56 (m, 4H), 1.30 (t, J = 11.9 Hz, 6H). MS(ESI+): 540.2(M+H).

**Example 23** (S)-5-chloro-N$^4$-(2-(methylsulfonyl)phenyl)-N$^2$-(7-(pyrrolidin-1-yl)-6,7,8,9-tetrahydro- 5H-benzo[7]annulen-2-yl)pyrimidine-2,4-diamine

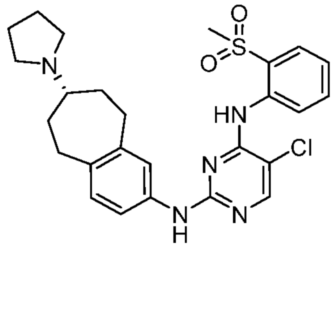

According to the preparation method of Example 1, 2-amino-N,N-dimethyl benzenesulfonamide in step a) is replaced with 2-(methylsulfonyl)aniline.

$^1H$ NMR (400 MHz, DMSO-$d_6$) $\delta$ 9.37 (m, 2H), 8.49 (d, J = 7.4 Hz, 1H), 8.24 (m, 1H), 7.94 (d, J = 7.9 Hz, 1H), 7.73 (t, J = 7.8 Hz, 1H), 7.48-7.35 (m, 2H), 7.30 (d, J = 7.9 Hz, 1H), 7.00 (d, J = 8.1 Hz, 1H), 3.27 (s, 3H), 3.10 (m, 4H), 2.62 (m, 3H), 2.50 (m, 2H), 2.17 (m, 2H), 1.85 (m, 4H), 1.41 (d, J = 8.1 Hz, 2H). MS(ESI+): 512.2(M+H).

**Example 24** (S)-2-((5-chloro-2-((7-(pyrrolidin-1-yl)-6,7,8,9-tetrahydro-5H-benzo[7]annulen-2-yl)amino)pyrimidin-4-yl)amino)-N-cyclopropyl benzenesulfonamide

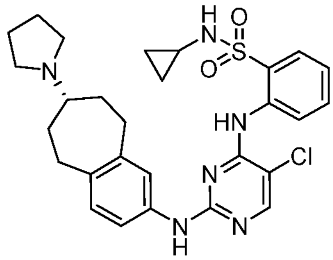

According to the preparation method of Example 1, 2-amino-N,N-dimethyl benzenesulfonamide in step a) is replaced with 2-amino-N-cyclopropyl benzenesulfonamide.

$^1H$ NMR (400 MHz, DMSO-$d_6$) $\delta$ 9.43 (s, 1H), 9.28 (s, 1H), 8.49 (d, J = 8.3 Hz, 1H), 8.27 (s, 2H), 7.87 (td, J = 8.3, 1.6 Hz, 1H), 7.65 (ddd, J = 8.6, 7.3, 1.7 Hz, 1H), 7.47 (d, J = 2.3 Hz, 1H), 7.40-7.26 (m, 2H), 7.01 (d, J = 8.1 Hz, 1H), 3.10 (s, 4H), 2.81 (dd, J = 14.4, 7.6 Hz, 1H), 2.63 (s, 3H), 2.14 (tt, J = 6.8, 3.5 Hz, 3H), 1.86 (d, J = 6.4 Hz, 4H), 1.42 (s, 2H), 0.54-0.26 (m, 5H). MS(ESI+): 553.2(M+H).

**Example 25** (S)-$N^4$-(2-(1H-1,2,4-triazol-5-yl)phenyl)-5-chloro-$N^2$-(7-(pyrrolidin-1-yl)-6,7,8,9-tetrahydro-5H-benzo[7]annulen-2-yl)pyrimidine-2,4-diamine

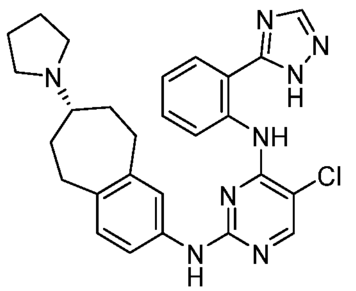

According to the preparation method of Example 1, 2-amino-N,N-dimethyl benzenesulfonamide in step a) is replaced with 2-(1H-1,2,4-thiazol-5-yl)aniline.

$^1H$ NMR (400 MHz, DMSO-$d_6$) $\delta$ 11.48 (s, 1H), 9.34 (s, 1H), 8.82 (d, J = 8.5 Hz, 1H), 8.69 (s, 1H), 8.22 (d, J = 3.2 Hz, 2H), 8.15 (dd, J = 7.9, 1.6 Hz, 1H), 7.46 (d, J = 2.2 Hz, 1H), 7.45-7.30 (m, 2H), 7.21 (t, J = 7.5 Hz, 1H), 7.03 (d, J = 8.1 Hz, 1H), 2.84 (s, 7H), 2.61 (t, J = 12.6 Hz, 2H), 2.05 (s, 2H), 1.77 (s, 4H), 1.49 (s, 2H). MS(ESI+): 501.2(M+H).

**Example 26** (S)-5-((5-chloro-2-((7-(pyrrolidin-1-yl)-6,7,8,9-tetrahydro-5H-benzo[7]annulen-2-yl)amino)pyrimidin-4-yl)amino)-N-methylthiazole-4-carboxamide

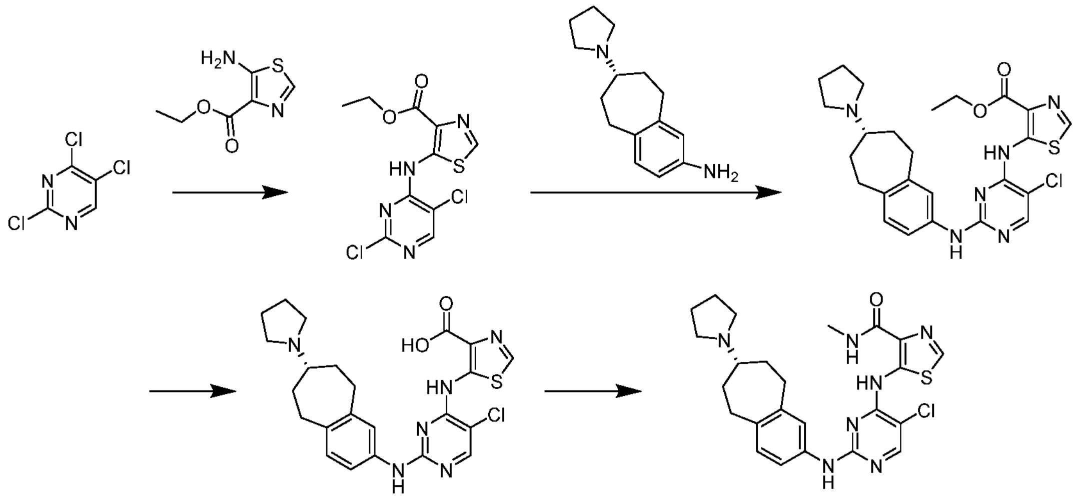

a) Preparation of ethyl 5-((2,5-dichloropyrimidin-4-yl)amino)thiazole-4-carboxylate

ethyl 5-aminothiazole-4-carboxylate (1.27 mmol), 2,4,5-trichloropyrimidine (1.90 mmol) and tetrahydrofuran (10 mL) are added to a 100 mL single-necked flask. The mixture is stirred and cooled (the external temperature is -20°C). Next, sodium hydride (3.8 mmol) is added to the reaction solution in batches. The reaction solution is gradually heated to room temperature and stirred for 8 h. After the reaction of the raw materials is completed, the reaction is stopped. A saturated ammonium chloride solution is added for quenching. 30 mL of ethyl acetate and 30 mL of water are added to the reaction solution, stirred and extracted. The aqueous layer is extracted again with 30 mL of ethyl acetate. Organic phases are merged, washed sequentially with water and saturated sodium chloride solution, and dried with anhydrous sodium sulfate. After filtration, the filtrate is concentrated to dryness to obtain the title compound (236 mg).

b) Preparation of ethyl (S)-5-((5-chloro-2-((7-(pyrrolidin-1-yl)-6,7,8,9-tetrahydro-5H-benzo[7]annulen-2-yl)amino)pyrimidin-4-yl)amino)thiazole-4-carboxylate

ethyl 5-((2,5-dichloropyrimidin-4-yl)amino)thiazole-4-carboxylate (0.31 mmol), (S)-7-(pyrrolidin-1-yl)-6,7,8,9-tetrahydro-5H-benzo[7]annulen-2-amine (0.34 mmol), n-butanol (4 mL) and trifluoroacetic acid (0.1 mL) are added to a 10 mL microwave tube. After nitrogen protection, microwave reaction is performed at 120°C for 2 h. The reactant is concentrated to dryness to obtain the title compound (196 mg).

MS(ESI+): 513.1(M+H).

c) Preparation of (S)-5-((5-chloro-2-((7-(pyrrolidin-1-yl)-6,7,8,9-tetrahydro-5H-benzo[7]annulen-2-yl)amino)pyrimidin-4-yl)amino)thiazole-4-carboxylic acid

Ethyl (S)-5-((5-chloro-2-((7-(pyrrolidin-1-yl)-6,7,8,9-tetrahydro-5H-benzo[7]annulen-2-yl]-amino)pyrimidin-4-yl)amino)thiazole-4-carboxylate (0.38 mmol), methanol (5 mL) and tetrahydrofuran (5 mL) are added to a 50 mL three-necked flask and stirred. A lithium hydroxide solution is added dropwise, stirred and stands overnight. After the reaction is completed, the reactant is desolvatized to dryness. 3 mL of water and 2N hydrogen chloride are added to the concentrated residue to adjust the pH to about 2 and freeze-dried to obtain the title compound. Proceed to the next step directly.

d) Preparation of (S)-5-((5-chloro-2-((7-(pyrrolidin-1-yl)-6,7,8,9-tetrahydro-5H-benzo[7]annulen-2-yl)amino)pyrimidin-4-yl)amino)-N-methylthiazole-4-carboxamide

(S)-5-((5-chloro-2-((7-(pyrrolidin-1-yl)-6,7,8,9-tetrahydro-5H-benzo[7]annulen-2-yl)amino)pyrimidin-4-yl)amino)thiazole-4-carboxylic acid (0.16 mmol), a tetrahydrofuran solution of methylamine (0.24 mmol), triethylamine (0.32 mmol) and N,N-dimethyl formamide (5 mL) are added to a 50 mL single-necked flask and stirred at room temperature. Then, a BOP reagent (0.32 mmol) is then added and reacted for 1 h. After the reaction is completed, 30 mL of water and 30 mL of ethyl acetate are added, stirred and extracted. The organic layer is concentrated to dryness, and purified by column chromatography (dichloromethane/methanol=20: 1) to obtain the title compound (37 mg).

$^1$H NMR (400 MHz, DMSO-$d_6$) δ 12.10 (s, 1H), 9.44 (s, 1H), 8.71 (s, 1H), 8.57 (d, J = 4.8 Hz, 1H), 8.32 (s, 1H), 7.47 (s, 1H), 7.33 (d, J = 7.3 Hz, 1H), 7.12 (d, J = 8.1 Hz, 1H), 3.06-2.97 (m, 2H), 2.89 - 2.60 (m, 10H), 2.24 (br, 2H),

1.87 (s, 4H), 1.50 (br, 2H). MS(ESI+): 498.1(M+H).

**Example 27** (S)-3-((5-chloro-2-((7-(pyrrolidin-1-yl)-6,7,8,9-tetrahydro-5H-benzo[7]annulen-2-yl)amino)pyrimidin-4-yl)amino)-N-methylisoniamide

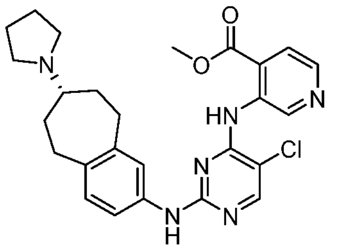

According to the preparation method of Example 1, 2-amino-N,N-dimethyl benzenesulfonamide in step a) is replaced with 3-aminoisonicotinate.

$^1$H NMR (400 MHz, DMSO-$d_6$) δ9.98 (s, 1H), 9.47 (s, 1H), 8.46 (d, J = 5.0 Hz, 1H), 8.25-8.38 (m, 2H), 7.85 (d, J = 4.8 Hz, 1H), 7.40 (s, 1H), 7.25-7.32 (m, 1H), 6.98 (d, J = 8.1 Hz, 1H), 3.91 (s, 3H), 2.81-2.93 (m, 2H), 2.89-2.60 (m, 7H), 1.81-1.96 (m, 2H), 1.65-1.77 (m, 4H), 1.44-1.59 (m, 2H). MS(ESI+): 493.2(M+H).

**Example 28** (S)-7-((5-chloro-2-(((7-(pyrrolidin-1-yl)-6,7,8,9-tetrahydro-5H-benzo[7]annulen-2-yl)amino))pyrimidin-4-yl)amino)-2-methylisoindolin-1-one

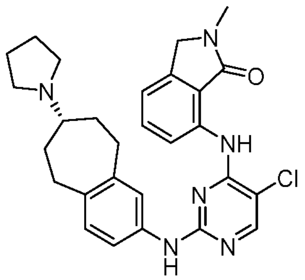

a) Preparation of 7-((2,5-dichloropyrimidin-4-yl)amino)-2-methylisoindolin-1-one

7-amino-2-methylisoindolin-1-one (50 mg) and 2,4,5-trichloropyrimidine (113 mg) are dissolved in tetrahydrofuran (5 mL), cooled to -20°C and then added with sodium hydride (37.2 mg). The reaction ends after 3 h. The reaction solution is poured into ice water for quenching, and then extracted with ethyl acetate. The ethyl acetate phase is concentrated to dryness and purified by column chromatography (dichloromethane/methanol=40: 1) to obtain the title compound (65 mg). MS(ESI+): 308.96(M+H).

b) Preparation of (S)-7-((5-chloro-2-(((7-(pyrrolidin-1-yl)-6,7,8,9-tetrahydro-5H-benzo[7]annulen-2-yl)amino))pyrimidin-4-yl)amino)-2-methylisoindolin-1-one

7-((2,5-dichloropyrimidin-4-yl)amino)-2-methylisoindolin-1-one (55 mg) and (S)-7-(pyrrolidin-1-yl)-6,7,8,9-tetrahydro-5H-benzo[7]annulen-2-amine (41 mg) are dissolved in n-butanol (5 mL), added with trifluoroacetic acid (0.5 mL), heated under the protection of nitrogen at 120°C and reacted for 8 h. After the reaction is completed, the reaction solution is added with 2 mL of saturated sodium bicarbonate solution. The organic phase is taken and concentrated to dryness, and purified by column chromatography (dichloromethane/methanol=20: 1) to obtain the title compound (75 mg).

$^1$H NMR (400 MHz, DMSO-$d_6$) δ 10.67 (s, 1H), 9.41 (s, 1H), 8.73 (d, J = 8.3 Hz, 1H), 8.25 (s, 1H), 7.53-7.41 (m, 2H), 7.34 (d, J = 8.2 Hz, 1H), 7.21 (d, J = 7.5 Hz, 1H), 7.05 (d, J = 8.1 Hz, 1H), 4.49 (s, 2H), 3.09 (s, 3H), 2.97-2.81 (m, 2H), 2.69-2.56 (m, 7H), 1.97 (s, 2H), 1.80-1.66 (m, 4H), 1.53 (s, 2H). MS(ESI+): 503.3(M+H).

**Example 29** (S)-7-((5-chloro-2-(((7-(pyrrolidin-1-yl)-6,7,8,9-tetrahydro-5H-benzo[7]annulen-2-yl)amino))pyrimidin-4-yl)amino)isoindolin-1-one

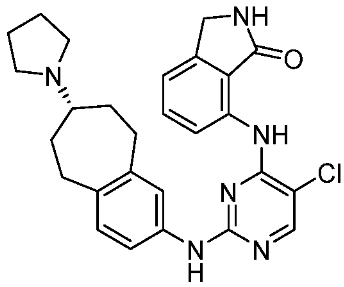

According to the preparation method of Example 1, 2-amino-N,N-dimethyl benzenesulfonamide in step a) is replaced with 7-amino-isoindolin-1-one.

$^1$H NMR (400 MHz, DMSO-$d_6$) δ 10.72 (s, 1H), 9.44 (s, 1H), 8.81 (s, 1H), 8.74 (d, J = 8.3 Hz, 1H), 8.24 (d, J = 8.5 Hz, 2H), 7.49 (d, J = 7.0 Hz, 1H), 7.36 (d, J = 7.7 Hz, 1H), 7.21 (d, J = 7.5 Hz, 1H), 7.07 (d, J = 8.1 Hz, 1H), 4.41 (s, 2H), 2.93-2.75 (m, 7H), 2.73-2.60 (m, 2H), 2.07 (s, 2H), 1.77 (d, J = 6.0 Hz, 4H), 1.50 (s, 2H). MS(ESI+): 489.1(M+H).

**Example 30** (S)-N-(2-((5-chloro-2-((7-(pyrrolidin-1-yl)-6,7,8,9-tetrahydro-5H-benzo[7]annulen-2-yl)amino)pyrimidin-4-yl)amino)phenyl)methanesulfonamide

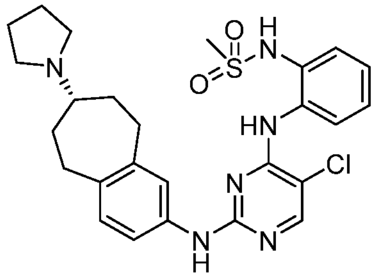

According to the preparation method of Example 1, 2-amino-N,N-dimethyl benzenesulfonamide in step a) is replaced with N-(2-aminophenyl) methane sulfonamide.

$^1$H NMR (400 MHz, DMSO-$d_6$) δ 9.25 (s, 1H), 8.63 (s, 1H), 8.24 (s, 1H), 8.16 (s, 1H), 7.96 (d, *J* = 6.9 Hz, 1H), 7.46-7.34 (m, 3H), 7.31-7.12 (m, 2H), 6.91 (d, *J* = 8.1 Hz, 1H), 3.01-2.72 (m, 8H), 2.59 (d, *J* = 11.3 Hz, 4H), 2.08 (s, 2H), 1.80 (s, 4H), 1.41 (s, 2H).MS(ESI+): 527.2(M+H).

**Example 31** 2-((5-chloro-2-((3-(tetrahydro-2H-pyran-4-yl)-2,3,4,5-tetrahydro-1H-benzo[d]aza-7-yl)amino)pyrimidin-4-yl)amino)-N-methylbenzamide

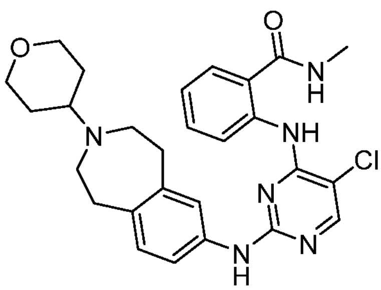

According to the preparation method of Example 1, 2-amino-N,N-dimethyl benzenesulfonamide in step a) is replaced with 2-amino-N-methylbenzamide.

(S)-7-(pyrrolidin-1-yl)-6,7,8,9-tetrahydro-5H-benzo[7]annulen-2-amine in step b) is replaced with 3-(tetrahydro-2H-pyran-4-yl)-2,3,4,5-tetrahydro-1H-benzo[d]azepin-7-amine to obtain the target product.

$^1$H NMR (400 MHz, DMSO-$d_6$) δ 11.58 (s, 1H), 9.35 (s, 1H), 8.74 (dd, J = 12.7, 7.1 Hz, 2H), 8.16 (d, J = 7.2 Hz, 1H), 7.76 (d, J = 7.8 Hz, 1H), 7.52-7.41 (m, 2H), 7.35 (d, J = 8.0 Hz, 1H), 7.15 (t, J = 7.6 Hz, 1H), 7.01 (d, J = 8.1 Hz, 1H), 3.89 (d, J = 8.1 Hz, 4H), 2.91-2.59 (m, 12H), 1.63 (d, J = 11.6 Hz, 2H), 1.52 (dt, J = 11.8, 8.0 Hz, 2H). MS(ESI+): 507.2(M+H).

**Example 32** 2-((5-chloro-2-((3-cyclopentyl-2,3,4,5-tetrahydro-1H-benzo(d)aza-7-yl)amino)pyrimidine-4-yl)amino)-N-methylbenzamide

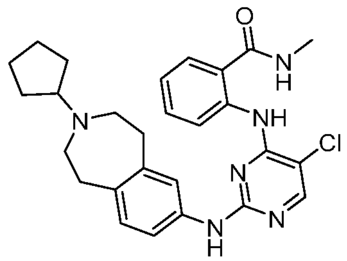

According to the preparation method of Example 1, 2-amino-N,N-dimethyl benzenesulfonamide in step a) is replaced with 2-amino-N-methylbenzamide.

(S)-7-(pyrrolidin-1-yl)-6,7,8,9-tetrahydro-5H-benzo[7]annulen-2-amine in step b) is replaced with 3-cyclopentyl-2,3,4,5-tetrahydro-1H-benzo[d]aza-7-amine to obtain the target product.

$^1$H NMR (400 MHz, DMSO-$d_6$) δ 11.60 (s, 1H), 9.39 (s, 1H), 8.75 (dd, J = 11.5, 6.7 Hz, 2H), 8.24 - 8.19 (m, 1H), 7.76 (d, J = 7.9 Hz, 1H), 7.48 (d, J = 9.1 Hz, 2H), 7.40 (d, J = 8.0 Hz, 1H), 7.15 (t, J = 7.5 Hz, 1H), 7.04 (d, J = 8.0 Hz, 1H), 3.18 (s, 1H), 2.96 - 2.75 (m, 11H), 1.88 (s, 2H), 1.62 (d, J = 26.3 Hz, 2H), 1.51 (s, 4H). MS(ESI+): 491.2(M+H).

**Example 33** (S)-3-((5-chloro-2-((7-(pyrrolidin-1-yl)-6,7,8,9-tetrahydro-5H-benzo[7]annulen-2-yl)amino)pyrimidin-4-yl)amino)-N-methylpicolinamide

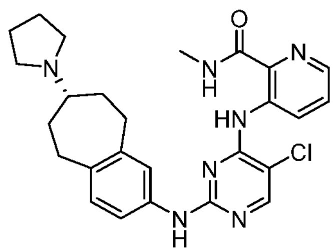

According to the preparation method of Example 1, 2-amino-N,N-dimethyl benzenesulfonamide in step a) is replaced with 3-amino-N-methyl-2-pyridine carboxamide. MS(ESI+): 492.2(M+H).

**Example 34** (S)-2-((5-chloro-2-((7-(pyrrolidin-1-yl)-6,7,8,9-tetrahydro-5H-benzo[7]annulen-2-yl)amino)pyrimidin-4-yl)amino)-5-cyano-N-methylbenzamide

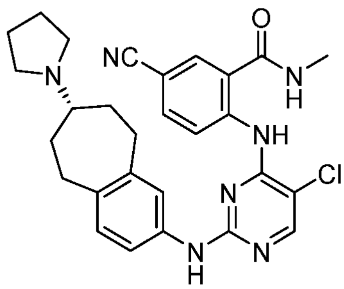

According to the preparation method of Example 1, 2-amino-N,N-dimethyl benzenesulfonamide in step a) is replaced with 2-amino-5-cyano-N-methylbenzamide.

$^1$H NMR (400 MHz, DMSO-$d_6$) δ 11.98 (s, 1H), 9.47 (s, 1H), 8.97 (d, 2H), 8.28 (d, J = 9.4 Hz, 1H), 8.24 (d, J = 2.0 Hz, 1H), 7.83 (dd, J = 8.8, 2.0 Hz, 1H), 7.42 (d, J = 2.2 Hz, 1H), 7.30 (d, J = 8.4 Hz, 1H), 7.04 (d, J = 8.1 Hz, 1H), 2.90 (m, 1H), 2.83 (m, 4H), 2.63 (d, J = 6.2 Hz, 3H), 2.56 (m, 4H), 1.93 (s, 2H), 1.71 (m, 4H), 1.52 (s, 2H). MS(ESI+): 516.1(M+H).

**Example 35** (S)-2-((5-chloro-2-((7-(pyrrolidin-1-yl)-6,7,8,9-tetrahydro-5H-benzo[7]annulen-2-yl)amino)pyrimidin-4-yl)amino)-N-methyl-5-(trifluoromethyl) benzamide

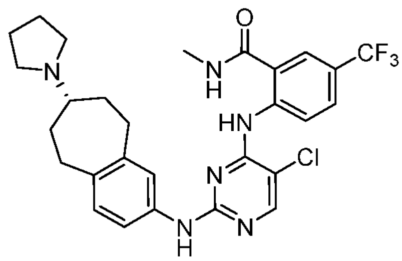

According to the preparation method of Example 1, 2-amino-N,N-dimethyl benzenesulfonamide in step a) is replaced with 2-amino-N-methyl-5-(trifluoromethyl) benzamide.

$^1$H NMR (400 MHz, DMSO-$d_6$) δ 11.92 (s, 1H), 9.48 (s, 1H), 9.01 (m, 2H), 8.30 (s, 1H), 8.16 (d, J = 7.6 Hz, 1H), 7.74 (d, J = 8.9 Hz, 1H), 7.52 (d, J = 1.5 Hz, 1H), 7.34 (d, J = 8.1 Hz, 1H), 7.08 (d, J = 8.2 Hz, 1H), 3.10 (s, 3H), 2.84 (d, J = 4.4 Hz, 3H), 2.78 - 2.57 (m, 4H), 2.33 (s, 2H), 2.18 (s, 2H), 1.85 (s, 4H), 1.46 (s, 2H). MS(ESI+): 559.2(M+H).

**Example 36** (S) -5-chloro-2-((5-chloro-2-((7-(pyrrolidin-1-yl)-6,7,8,9-tetrahydro-5H-benzo[7]annulen-2-yl)amino)pyrimidin-4-yl)amino)-N-methylbenzamide

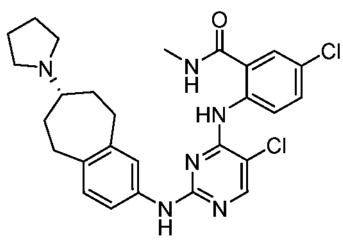

According to the preparation method of Example 1, 2-amino-N,N-dimethyl benzenesulfonamide in step a) is replaced with 2-amino-5-chloro-N-methylbenzamide.

$^1$H NMR (400 MHz, DMSO-$d_6$) δ 11.51 (s, 1H), 9.39 (s, 1H), 8.89 (d, J = 4.5 Hz, 1H), 8.74 (d, J = 8.7 Hz, 1H), 8.22 (d, J = 10.7 Hz, 1H), 7.84 (d, J = 2.5 Hz, 1H), 7.56 - 7.43 (m, 2H), 7.32 (d, J = 7.9 Hz, 1H), 7.05 (d, J = 8.2 Hz, 1H), 2.98 (m, 5H), 2.81 (d, J = 4.5 Hz, 5H), 2.70 - 2.57 (m, 2H), 2.12 (s, 2H), 1.81 (s, 4H), 1.47 (s, 2H). MS(ESI+): 525.1(M+H).

**Example 37** (S)-2-((5-chloro-2-(((7-(pyrrolidin-1-yl)-6,7,8,9-tetrahydro-5H-benzo[7]annulen-2-yl)amino))pyrimidin-4-yl)amino)-5-methoxyl-N-methylbenzamide

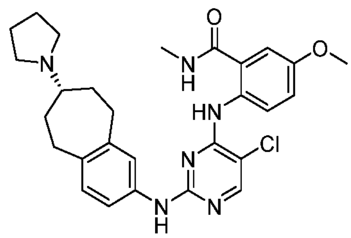

According to the preparation method of Example 1, 2-amino-N,N-dimethyl benzenesulfonamide in step a) is replaced with 2-amino-5-methoxyl-N-methylbenzamide. $^1$H NMR (400 MHz, DMSO-$d_6$) δ 11.13 (s, 1H), 9.26 (s, 1H), 8.75 (d, J = 4.5 Hz, 1H), 8.54 (d, J = 8.7 Hz, 1H), 8.15 (s, 1H), 7.45 (d, J = 1.8 Hz, 1H), 7.33-7.27(m, 2H), 7.04 (dd, J = 9.1, 2.9 Hz, 1H), 7.00 (d, J = 8.2 Hz, 1H), 3.83 (s, 3H), 2.89 - 2.71 (m, 10H), 2.62-2.52 (m, 2H), 2.00 (br, 2H), 1.75 (br, 4H), 1.49 (br, 2H). MS(ESI+): 521.2(M+H).

**Example 38** (S)-2-((5-chloro-2-((7-(pyrrolidin-1-yl)-6,7,8,9-tetrahydro-5H-benzo[7]annulen-2-yl)amino)pyrimidin-4-yl)amino)-N-(2-methoxyethyl) benzamide

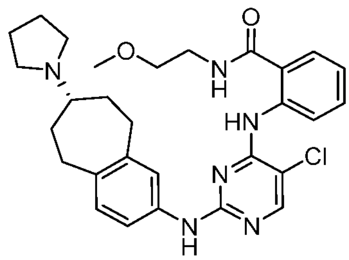

According to the preparation method of Example 1, 2-amino-N,N-dimethyl benzenesulfonamide in step a) is replaced with 2-amino-N-(2-methoxyethyl)benzamide. MS(ESI+): 535.1(M+H).

**Example 39** 2-((5-chloro-2-((S)-7-(pyrrolidin-1-yl)-6,7,8,9-tetrahydro-5H-benzo[7]annulen-2-yl)amino)pyrimidin-4-yl)amino)-N-(tetrahydrofuran-3-yl) benzamide

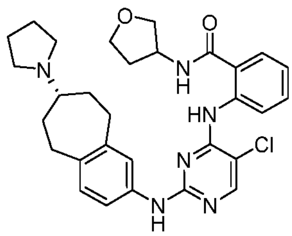

According to the preparation method of Example 1, 2-amino-N,N-dimethyl benzenesulfonamide in step a) is replaced with 2-amino-N-(tetrahydro-3-furyl)benzamide. MS(ESI+): 547.2(M+H).

**Example 40** (S)-5-chloro-N4-(2-(morpholinmethyl)phenyl)-N2-(7-(pyrrolidin-1-yl)-6,7,8,9-tetrahydro-5H-benzo[7]annulen-2-yl)pyrimidine-2,4-diamine

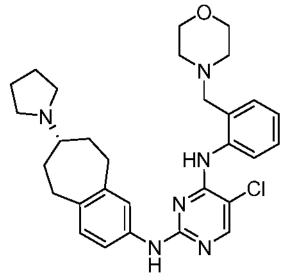

According to the preparation method of Example 1, 2-amino-N,N-dimethyl benzenesulfonamide in step a) is replaced with 2-(morpholin-4-methyl)aniline.

$^1$H NMR (400 MHz, DMSO-$d_6$) δ 10.06 (s, 1H), 9.28 (s, 1H), 8.33 - 8.10 (m, 2H), 7.49 (s, 1H), 7.38 - 7.21 (m, 3H), 7.08 (t, *J* = 7.3 Hz, 1H), 6.97 (d, *J* = 8.1 Hz, 1H), 3.61 (d, *J* = 9.2 Hz, 6H), 3.10 - 2.75 (m, 6H), 2.67 (s, 1H), 2.59 (t, *J* = 11.9 Hz, 2H), 2.40 (s, 4H), 2.09 (s, 2H), 1.79 (s, 4H), 1.44 (s, 2H). MS(ESI+): 533.2(M+H).

**Example 41** (S)-N-(2-((5-chloro-2-((7-(pyrrolidin-1-yl)-6,7,8,9-tetrahydro-5H-benzo[7]annulen-2-yl)amino)pyrimidin-4-yl)amino)phenyl) cyclopropanesulfonamide

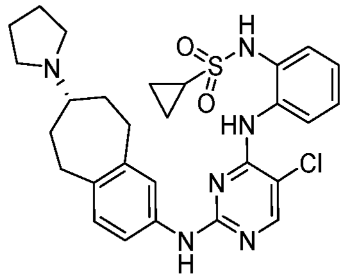

According to the preparation method of Example 1, 2-amino-N,N-dimethyl benzenesulfonamide in step a) is replaced with N-(2-aminophenyl) cyclopropanesulfonamide.

$^1$H NMR (400 MHz, DMSO-$d_6$) δ 9.27 (s, 1H), 8.56 (s, 1H), 8.18 (d, J = 13.3 Hz, 1H), 7.98 (d, J = 7.6 Hz, 1H), 7.48 - 7.36 (m, 2H), 7.31 (t, J = 7.1 Hz, 1H), 7.24 (t, J = 6.9 Hz, 2H), 6.92 (d, 1H), 3.02 (s, 6H), 2.78 (m, 1H), 2.57 (m, 3H), 2.09 (m, 2H), 1.82 (m, 4H), 1.40 (m,2H), 0.84 (m, Hz, 4H). MS(ESI+): 553.2(M+H).

**Example 42** (S)-4-((5-chloro-2-((7-(pyrrolidin-1-yl)-6,7,8,9-tetrahydro-5H-benzo[7]annulen-2-yl)amino)pyrimidin-4-yl)amino)-N-methyl-2-oxo-1,2-dihydropyridine-3-carboxamide

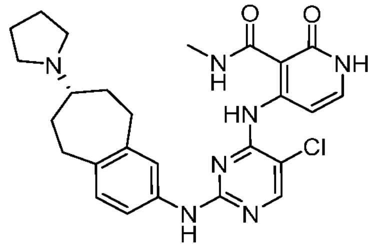

According to the preparation method of Example 1, 2-amino-N,N-dimethyl benzenesulfonamide in step a) is replaced with 4-amino-6-methoxyl-N-methylnicotinamide. This product is a demethyl byproduct of Preparation Example

44.

$^1$H NMR (400 MHz, DMSO-$d_6$) δ 14.30 (s, 1H), 11.98 (s, 1H), 10.69 (d, 1H), 9.53 (s, 1H), 8.36 (s, 1H),8.14 (d, 1H), 7.44 (m, 2H), 7.29 (d, 1H), 7.04 (d, 1H) ,2.92-2.83(m, 5H), 2.68-2.33 (m, 7H), 1.86 (m, 2H), 1.69(m, 4H), 1.54 (m, 2H). MS(ESI+): 508.2(M+H).

**Example 43** (S)-4-((5-chloro-2-((7-(pyrrolidin-1-yl)-6,7,8,9-tetrahydro-5H-benzo[7]annulen-2-yl)amino)pyrimidin-4-yl)amino)-6-methoxyl-N-methylnicotinamide

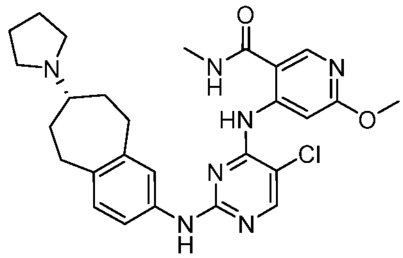

According to the preparation method of Example 1, 2-amino-N,N-dimethyl benzenesulfonamide in step a) is replaced with 4-amino-6-methoxyl-N-methylnicotinamide. $^1$H NMR (400 MHz, DMSO-$d_6$) δ 11.63 (s, 1H), 8.36 (s, 1H), 8.30 (d, 2H), 7.36 (m, 2H), 7.13 (d, 1H), 6.98 (d, 1H) , 6.26 (s, 1H) ,4.00 (s, 3H),3.07(s, 3H), 2.90 (m, 4H), 2.74 (m, 2H), 2.50 (s, 2H), 1.87 (m, 3H), 1.69(m, 4H), 1.54 (m, 2H). MS(ESI+): 522.3(M+H).

**Example 44** (S)-4-((5-chloro-2-((7-(pyrrolidin-1-yl)-6,7,8,9-tetrahydro-5H-benzo[7]annulen-2-yl)amino)pyrimidin-4-yl)amino)-2-methoxyl-N-methylnicotinamide

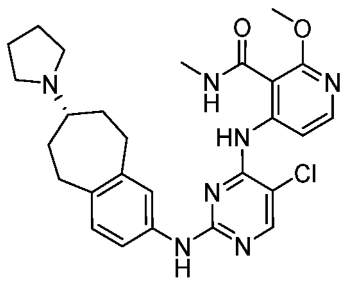

According to the preparation method of Example 1, 2-amino-N,N-dimethyl benzenesulfonamide in step a) is replaced with 4-amino-2-methoxyl-N-methylnicotinamide. $^1$H NMR (400 MHz, DMSO-$d_6$) δ 12.21 (s, 1H), 9.48 (s, 1H), 8.65 (d, 1H), 8.53 (m, 1H), 8.32 (s, 1H),8.12 (d, 1H), 7.44 (m, 1H), 7.33 (d, 1H), 7.04 (d, 1H), 3.96 (s, 3H),3.98-2.83(m, 6H), 2.40-2.61 (m, 5H), 1.85 (m, 3H), 1.69(m, 4H), 1.54 (m, 2H). MS(ESI+): 522.3(M+H).

**Example 45** (S)-2-((5-chloro-2-((7-(pyrrolidin-1-yl)-6,7,8,9-tetrahydro-5H-benzo[7]annulen-2-yl)amino)pyrimidin-4-yl)amino)-5-hydroxyl-N-methylbenzamide

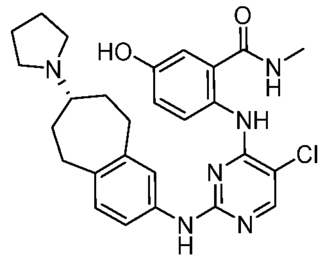

According to the preparation method of Example 1, 2-amino-N,N-dimethyl benzenesulfonamide in step a) is replaced with 2-amino-5-hydroxyl-N-methylbenzamide. $^1$HNMR(400MHz,DMSO$d_6$)δ10.85(s,1H),9.57(s,1H),9.22(s,1H),8.63(d,J=4.6Hz,1H),8.38 (d,J=8.8Hz,1H),8.12(s,1H),7.45(s,1H),7.32(d,J=7.3Hz,1H),7.10(d,J=2.7Hz,1H),6.02-6.91(m,2H),3.00-2.70(m,7H),2.65 -2.56(m,2H),2.14-1.94(m,2H),1.78(s,4H),1.58-1.43(m,2H),1.23(s,3H). MS(ESI+): 507.3(M+H).

**Example 46** 2-((5-chloro-2-((3-(tetrahydro-2H-pyran-4-yl)-2,3,4,5-tetrahydro-1H-benzo[d]aza-7-yl)amino)pyrimidin-4-yl)amino)-5-fluoro-N-methylbenzamide

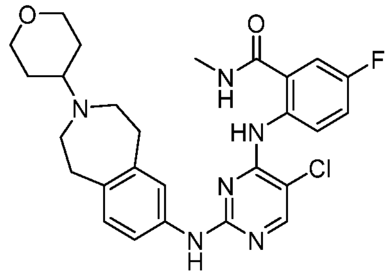

According to the preparation method of Example 1, 2-amino-N,N-dimethyl benzenesulfonamide in step a) is replaced with 2-amino-5-fluoro-N-methylbenzamide.

(S)-7-(pyrrolidin-1-yl)-6,7,8,9-tetrahydro-5H-benzo[7]annulen-2-amine in step b) is replaced with 3-(tetrahydro-2H-pyran-4-yl)-2,3,4,5-tetrahydro-1H-benzo[d]azepin-7-amine to obtain the target product.

$^1$HNMR(400MHz,DMSO-$d_6$)δ11.31(s,1H),9.32(s,1H),8.82(s,1H),8.65(s,1H),8.19(s,1H),7.63(d,1H),.39(d,1H),7.30(d, 2H),7.00(d,1H),3.87(m,2H),3.87(m,2H),2.81-2.78(m,5H),2.70(m,3H),2.65(m,4H),1.63-1.56(m,2H),1.54-1.45(m,2H). MS(ESI+): 525.2(M+H).

**Example 47** (S)-(6-((5-chloro-2-((7-(pyrrolidin-1-yl)-6,7,8,9-tetrahydro-5H-benzo[7]annulen-2-yl)amino)pyrimidin-4-yl)amino)quinoxalin-5-yl) dimethyl phosphine oxide

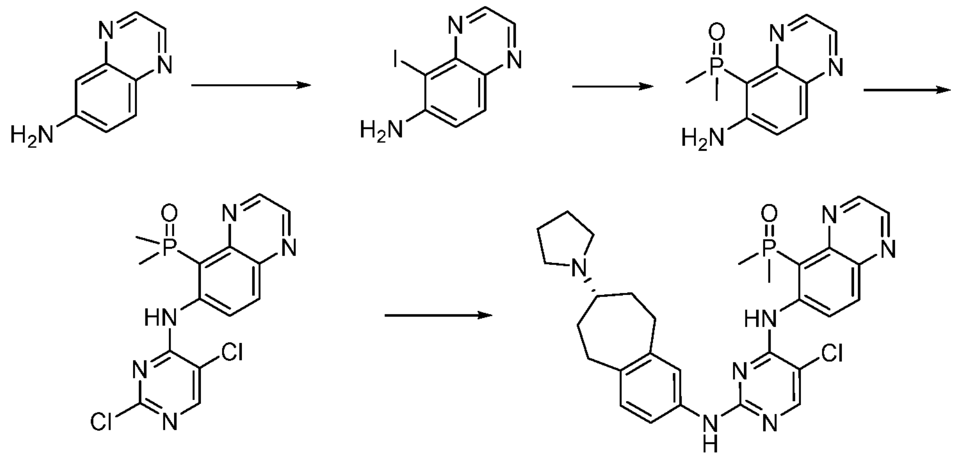

a) Preparation of 5-iodoquinoxaline-6-amine

Quinoxaline-6-amine (2900 mg) is dissolved in a mixture solution of 50 ml of dichloromethane and 80 ml of saturated sodium bicarbonate, added dropwise with iodine chloride (3900 mg) at 0°C, and reacted for 1 h. Liquid separation is then performed. The organic phase is subjected to rotary evaporation under reduced pressure. The resulting crude product is purified by silica-gel column chromatography to obtain the title compound (3 g).

b) Preparation of (6-aminoquinoxalin-5-yl) dimethyl phosphine oxide

5-iodoquinoxaline-6-amine (2710 mg), dimethyl phosphine oxide (780 mg), potassium phosphate (3180 mg), palladium acetate (112 mg), 4,5-bisdiphenyl phosphine-9,9-dimethylxanthene (518 mg), 30 ml of N,N-dimethylformamide and 6 ml of water are sequentially added to a 100 ml three-necked flask. Nitrogen replacement is performed for 3 times. The reaction is then performed at 120°C for 24 h. After the reaction is completed, 200 ml of dichloromethane and 100 ml of water are added. Liquid separation is performed. The resulting organic phase is filtered, concentrated under reduced pressure to dryness, and purified by reverse-phase column chromatography (acetonitrile/water) to obtain the title compound (1.3 g).

c) Preparation of 6-((2,5-dichloropyrimidin-4-yl)amino)quinoxalin-5-yl) dimethyl phosphine oxide

(6-aminoquinoxalin-5-yl) dimethyl phosphine oxide (183 mg) and 2,4,5-trichloropyrimidine (221 mg) are dissolved in THF (5mL), cooled to -20°C and then added with sodium hydride (80 mg, 60%). The mixture is transfered to room temperature after 30 min and continued to react for 5 h. The reaction solution is quenched by adding 5 mL of saturated ammonium chloride solution, and then extracted with 10 mL of ethyl acetate. The organic phase is concentrated to dryness, and purified by silica-gel column chromatography to obtain the title compound (120 mg).

d) Preparation of (S)-(6-((5-chloro-2-((7-(pyrrolidin-1-yl)-6,7,8,9-tetrahydro-5H-benzo[7]annulen-2-yl)amino)pyrimidin-4-yl)amino)quinoxalin-5-yl) dimethyl phosphine oxide

6-((2,5-dichloropyrimidin-4-yl)amino)quinoxalin-5-yl)dimethyl phosphine oxide (25 mg) and (S)-7-(pyrrolidin-1-yl)-6,7,8,9-tetrahydro-5H-benzo[7]annulen-2-amine (16.5 mg) are dissolved in n-butanol (3 mL), added with trifluoroacetic acid (0.3 mL), and reacted at 120°C for 4 h. The reaction solution is neutralized by adding 2M sodium hydroxide solution (2 mL), and separated. The aqueous phase is extracted with ethyl acetate. The organic phases are merged, and concentrated to dryness and purified by column chromatography (dichloromethane/methanol=20: 1) to obtain the title compound (35 mg).

$^1$H NMR (400 MHz, CDCl3) $\delta$ 12.78 (d, *J* = 3.0 Hz, 1H), 9.17 (dd, *J* = 9.6, 4.1 Hz, 1H), 8.83-8.65 (m, 2H), 8.17 (d, *J* = 2.6 Hz, 1H), 8.09 (d, *J* = 9.5 Hz, 1H), 7.36 - 7.28 (m, 2H), 7.07 (d, *J* = 8.2 Hz, 2H), 3.09 (m, 4H), 2.89 (m, 1H), 2.82 - 2.60 (m, 4H), 2.33 (m, 2H), 2.20 - 2.03 (m, 6H), 1.96 (m, 4H), 1.55 (m, 2H). MS(ESI+): 562.2(M+H).

**Example 48** (S)-6-((5-chloro-2-((7-(pyrrolidin-1-yl)-6,7,8,9-tetrahydro-5H-benzo[7]annulen-2-yl)amino)pyrimidin-4-yl)amino)-N-methylquinoxaline-5-carboxamide

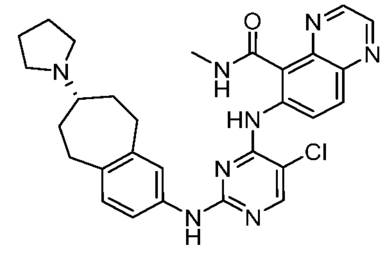

a) Preparation of 6-((2,5-dichloropyrimidin-4-yl)amino)-N-methylquinoxaline-5-carboxamide

6-amino-N-methylquinoxaline-5-carboxamide (25 mg) and 2,4,5-trichloropyrimidine (68 mg) are dissolved in tetrahydrofuran (5 mL), cooled to -20°C and then added with sodium hydride (15 mg). The mixture is transfered to room temperature after 30 min and continued to react for 5 h. The reaction solution is quenched by adding 5 mL of saturated ammonium chloride solution. A tetrahydrofuran layer is taken. The aqueous phase is extracted with ethyl acetate. The organic phases are merged, and concentrated to dryness and purified by column chromatography (dichloromethane/methanol=20: 1) to obtain the title compound (27 mg).

MS: 1/2$[M+H]^+$: 175.1.

b) Preparation of (S)-6-((5-chloro-2-((7-(pyrrolidin-1-yl)-6,7,8,9-tetrahydro-5H-benzo[7]annulen-2-yl)amino)pyrimidin-4-yl)amino)-N-methylquinoxaline-5-carboxamide 6-((2,5-dichloropyrimidin-4-yl)amino)-N-methylquinoxaline-5-carboxamide (25 mg) and (S)-7-(pyrrolidin-1-yl) -6,7,8,9-tetrahydro-5H-benzo[7]annulen-2-amine (16.5 mg) are dissolved in n-butanol (3 mL), added with trifluoroacetic acid (0.3 mL), and reacted at 120°C for 4 h. The reaction solution is neutralized by adding 2M sodium hydroxide solution (2 mL), and separated. The aqueous phase is extracted with ethyl acetate. The organic phases are merged, and concentrated to dryness and purified by column chromatography (dichloromethane/methanol=20: 1) to obtain the title compound (1.5 mg). MS(ESI+): 543.3(M+H).

**Example 49** 7-((5-chloro-2-((3-(tetrahydro-2H-pyran-4-yl)-2,3,4,5-tetrahydro-1H-benzo [d] aza-7-yl)amino)pyrimidin-4-yl)amino)-2-methylisoindolin-1-one

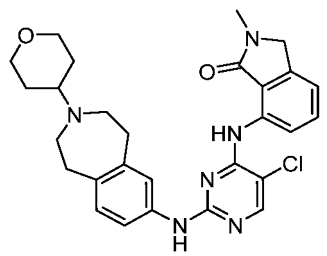

According to the preparation method of Example 1, 2-amino-N,N-dimethyl benzenesulfonamide in step a) is replaced with 7-amino-2-methylisoindolin-1-one.

(S)-7-(pyrrolidin-1-yl)-6,7,8,9-tetrahydro-5H-benzo[7]annulen-2-amine in step b) is replaced with 3-(tetrahydro-2H-pyran-4-yl)-2,3,4,5-tetrahydro-1H-benzo[d]azepin-7-amine to obtain the target product.

$^1$H NMR (400 MHz, DMSO-$d_6$) $\delta$ 10.67 (s, 1H), 9.45 (s, 1H), 8.25 (s, 1H), 7.48 (d, *J* = 8.3 Hz, 2H), 7.36 (dd, J = 8.1, 2.2 Hz, 1H), 7.21 (d, *J* = 7.5 Hz, 1H), 7.14 - 7.07 (m, 1H), 7.06 (s, 1H), 4.49 (s, 2H), 3.90 (dd, *J* = 11.0, 4.1 Hz, 2H), 3.28 (td, *J* = 11.7, 2.0 Hz, 3H), 3.09 (s, 3H), 2.89 - 2.74 (m, 8H), 1.75 - 1.60 (m, 2H), 1.54 (qd, *J* = 11.9, 4.4 Hz, 2H). MS(ESI+): 519.2(M+H).

**Example 50** (2-((5-chloro-2-(((S)-7-(pyrrolidin-1-yl)-6,7,8,9-tetrahydro-5H-benzo[7]annulen-2-yl)amino)pyrimidin-4-yl)amino)phenyl)(imino)(methyl)-$\lambda^6$-sulfanone

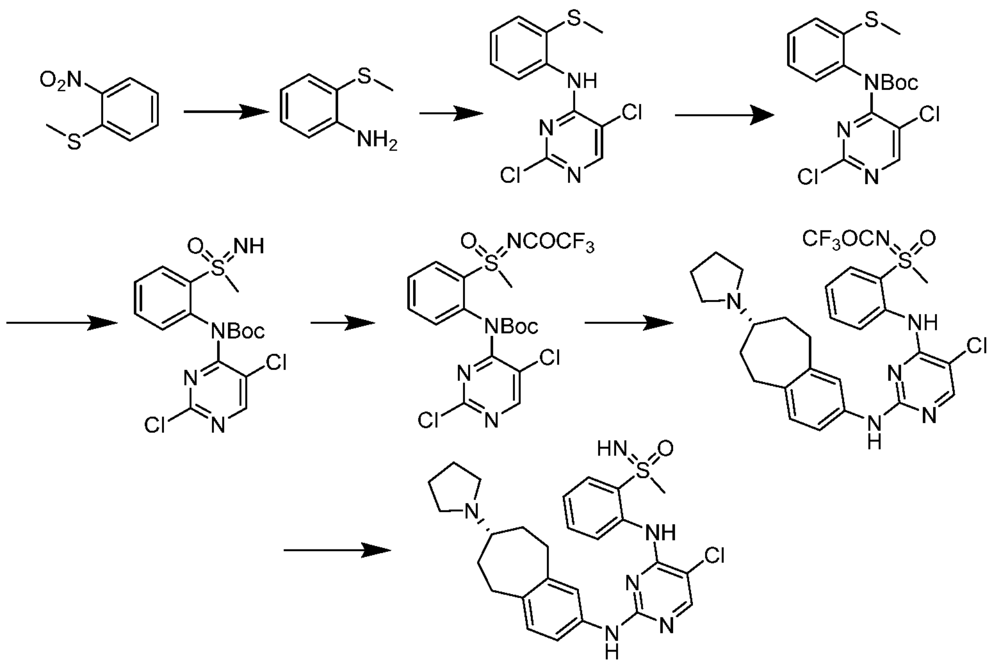

a) Preparation of 2-(methylthio)aniline

At 0°C, titanium trichloride (5 ml) is added dropwise to a tetrahydrofuran (5 ml) solution of 2-nitrobenzene sulfide (169 mg), and after dropping, transferred to room temperature and reacted for 4.0 h. After the reaction is completed, the reactant is added with sodium hydroxide (2M) to adjust the pH to 9. The organic layers are extracted with ethyl acetate, merged, and dried with anhydrous sodium sulfate. A solvent is removed by rotary evaporation under reduced pressure to obtain the title compound (125 mg). MS(ESI+): 140.1(M+H).

b) Preparation of 2,5-dichloro-N-(2-(methylthio)phenyl)pyrimidin-4-amine

At 0°C, sodium hydride (72 mg) is added to an N,N-dimethylformamide (3 ml) solution of 2-aminoanisole (120 mg), stirred for 10 min and added with 2,4,5-trichloropyrimidine (329 mg), and then transfered to room temperature and reacted for 3.0 h. After the reaction is completed, the reactant is quenched with 1 ml of ammonium chloride. Liquid separation is performed. Organic layers are extracted with ethyl acetate, merged, and dried with anhydrous sodium sulfate. A solvent is removed by rotary evaporation under reduced pressure. The reactant is purified by column chromatography (petroleum ether/ethyl acetate=50/1) to obtain the title compound (65 mg). MS(ESI+): 286.2(M+H).

c) Preparation of tert-butyl (2,5-dichloropyrimidin-4-yl)(2-(methylthio)phenyl) carbamate

Di-tert-butyl dicarbonate (99 mg) and 4-dimethylaminopyridine (14 mg) are added to a dichloromethane (3 ml) solution of 2,5-dichloro-N-(2-(methylthio)phenyl)pyrimidin-4-amine (65 mg), and reacted at room temperature for 2 h. After the reaction is completed, the solvent is removed by rotary evaporation under reduced pressure. The resulting product is purified by column chromatography (petroleum ether/ethyl acetate=20: 1) to obtain the title product (72 mg). MS(ESI+): 386.1(M+H).

d) Preparation of tert-butyl (2,5-dichloropyrimidin-4-yl)(2-(S-methylsulfonimidoyl)phenyl) carbamate

Ammonium acetate (57 mg) and iodobenzene diacetate (122mg) are added to an ethanol (3 ml) solution of tert-butyl (2,5-dichloropyrimidin-4-yl)(2-(methylthio)phenyl) carbamate (72 mg), and reacted at room temperature for 2 h. After the reaction is completed, the solvent is removed by rotary evaporation under reduced pressure. The crude product is purified by column chromatography (petroleum ether/ethyl acetate=20/1) to obtain the title product (55 mg). MS(ESI+): 417.1(M+H).

e) Preparation of tert-butyl (2,5-dichloropyrimidin-4-yl)(2-(S-methyl-N-(2,2,2-trifluoroacetyl)sulfonimidoyl)phenyl) carbamate

Trifluoroacetic acid (55 mg), 4-dimethylaminopyridine (2 mg) and triethylamine (33 mg) are added to a dichloromethane (3 ml) solution of tert-butyl(2,5-dichloropyrimidin-4-yl)(2-(S-methylsulfonimido)phenyl)carbamate (55 mg), and reacted at room temperature for 3.0 h. After the reaction is completed, a solvent is removed by rotary evaporation under reduced pressure. The crude product is purified by column chromatography (petroleum ether/ethyl acetate = 20/1) to obtain the title compound (42 mg). MS(ESI+): 513.1(M+H).

f) N-((2-((5-chloro-2-(((S)-7-(pyrrolidin-1-yl)-6,7,8,9-tetrahydro-5H-benzo[7]annulen-2-yl)amino)pyrimidin-4-yl)amino)phenyl)(methyl)(oxo)-$\lambda^6$-sulfonamidoyl)-2,2,2-trifluoroacetamide

(S)-7-(pyrrolidin-1-yl)-6,7,8,9-tetrahydro-5H-benzo[7]annulen-2-amine (28 mg) and trifluoroacetic acid (0.1 ml) are added to a n-butanol (3 ml) solution of tert-butyl(2,5-dichloropyrimidin-4-yl)(2-(S-methyl-N-(2,2,2-trifluoroacetyl)sulfonimide)phenyl)carbamate (42 mg), and reacted in microwaves at 120°C for 2.0 h. After the reaction is completed, a solvent is removed by rotary evaporation under reduced pressure to obtain the title compound (25 mg). MS(ESI+): 607.1(M+H).

g) (2-((5-chloro-2-((S)-7-(pyrrolidin-1-yl)-6,7,8,9-tetrahydro-5H-benzo[7]annulen-2-yl)amino)pyrimidin-4-yl)amino)phenyl)(imino)(methyl)-$\lambda^6$-sulfanone

N-((2-((5-chloro-2-((S)-7-(pyrrolidin-1-yl)-6,7,8,9-tetrahydro-5H-benzo[7]annulen-2-yl)amino)pyrimidin-4-yl)amino)phenyl)(methyl)(oxo)-$\lambda^6$-sulfonamidoyl)-2,2,2-trifluoroacetamide, ethanol (3 ml) and potassium carbonate (41 mg) are sequentially added to a reaction flask, and reacted at room temperature for 3 h. After the reaction is completed, a solvent is removed by rotary evaporation under reduced pressure. The resulting product is purified by silica-gel column chromatography to obtain the title product (6 mg).

$^1$H NMR (400 MHz, DMSO-$d_6$) $\delta$ 10.82 (s, 1H), 9.39 (s, 1H), 8.66 (d, *J* = 8.3 Hz, 1H), 8.26 (s, 1H), 7.93 (dd, *J* = 7.9, 1.6 Hz, 1H), 7.71 - 7.58 (m, 1H), 7.46 - 7.26 (m, 3H), 7.00 (d, *J* = 8.2 Hz, 1H), 5.15 (s, 1H), 3.12 (s, 4H), 2.85 (s, 3H), 2.67 (m, 3H), 2.33 (m, 2H), 2.04 - 1.88 (m, 2H), 1.72 (m, 4H), 1.58 - 1.43 (m, 2H). MS(ESI+): 511.3(M+H).

**Example 51** (S)-(2-((5-chloro-2-((7-(pyrrolidin-1-yl)-6,7,8,9-tetrahydro-5H-benzo[7]annulen-2-yl)amino)pyrimidin-4-yl)amino)-5-fluorophenyl)dimethyl phosphine oxide

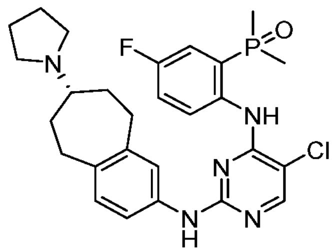

According to the preparation method of Example 1, 2-amino-N,N-dimethyl benzenesulfonamide in step a) is replaced with (2-amino-5-fluorophenyl) dimethyl phosphine oxide.

$^1$H NMR (400 MHz, DMSO-$d_6$) $\delta$ 10.84 (s, 1H), 9.28 (s, 1H), 8.50 (s, 1H), 8.17 (s, 1H), 7.53 (ddd, *J* = 13.9, 8.8, 3.1 Hz, 1H), 7.41 - 7.29 (m, 2H), 7.26 (dd, *J* = 8.2, 2.2 Hz, 1H), 6.97 (d, *J* = 8.2 Hz, 1H), 2.84 (m, 2H), 2.56 (s, 7H), 1.87 (s, 2H), 1.81 (s, 3H), 1.78 (s, 3H), 1.70 (s, 4H), 1.50 (s, 2H). MS(ESI+): 528.2(M+H).

**Example 52** (S)-(5-chloro-2-((5-chloro-2-((7-(pyrrolidin-1-yl)-6,7,8,9-tetrahydro-5H-benzo[7]annulen-2-yl)amino)pyrimidin-4-yl)amino)-5-fluorophenyl) dimethyl phosphine oxide

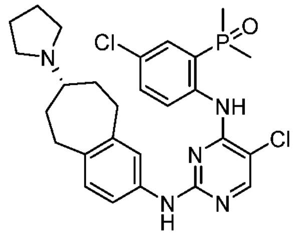

According to the preparation method of Example 1, 2-amino-N,N-dimethyl benzenesulfonamide in step a) is replaced with (2-amino-5-chlorophenyl) dimethyl phosphine oxide.

$^1H$ NMR (400 MHz, DMSO-$d_6$) δ 11.12 (s, 1H), 9.33 (s, 1H), 8.57 (s, 1H), 8.19 (s, 1H), 7.69 (dd, *J* = 13.7, 2.4 Hz, 1H), 7.49 (dd, *J* = 9.0, 2.4 Hz, 1H), 7.42 (d, *J* = 2.1 Hz, 1H), 7.25 (d, *J* = 8.1 Hz, 1H), 6.99 (d, *J* = 8.1 Hz, 1H), 3.17 (s, 2H), 2.95 - 2.75 (m, 2H), 2.64-2.53 (m, 5H), 1.91 (s, 2H), 1.84 (s, 3H), 1.80 (s, 3H), 1.71 (s, 4H), 1.52 (s, 2H). MS(ESI+): 544.2(M+H).

**Example 53** (S)-(2-((5-chloro-2-((7-(pyrrolidin-1-yl)-6,7,8,9-tetrahydro-5H-benzo[7]annulen-2-yl)amino)pyrimidin-4-yl)amino)-5-methylphenyl) dimethyl phosphine oxide

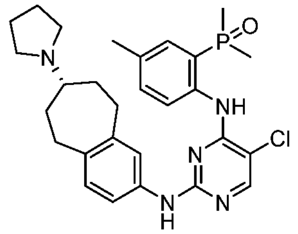

According to the preparation method of Example 1, 2-amino-N,N-dimethyl benzenesulfonamide in step a) is replaced with (2-amino-5-methylphenyl) dimethyl phosphine oxide.

$^1H$ NMR (400 MHz, DMSO-$d_6$) δ 10.92 (s, 1H), 9.25 (s, 1H), 8.39 (s, 1H), 8.14 (s, 1H), 7.55 - 7.37 (m, 2H), 7.29 (ddd, *J* = 14.0, 8.1, 2.1 Hz, 2H), 6.96 (d, *J* = 8.2 Hz, 1H), 2.96 - 2.76 (m, 2H), 2.70 - 2.54 (m, 4H), 2.44 (s, 3H), 2.34 (s, 3H), 1.89 (d, *J* = 11.2 Hz, 2H), 1.78 (s, 3H), 1.74 (s, 3H), 1.70 (s, 4H), 1.51 (s, 2H). MS(ESI+): 524.2(M+H).

**Example 54** (S)-4-((5-chloro-2-((7-(pyrrolidin-1-yl)-6,7,8,9-tetrahydro-5H-benzo[7]annulen-2-yl)amino)pyrimidin-4-yl)amino)-3-(dimethylphosphoryl)benzonitrile

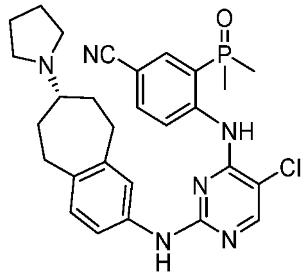

According to the preparation method of Example 1, 2-amino-N,N-dimethyl benzenesulfonamide in step a) is replaced with (2-amino-5-cyanophenyl) dimethyl phosphine oxide.

$^1H$ NMR (400 MHz, DMSO-*d6*) δ 11.72 (s, 1H), 9.43 (s, 1H), 8.85 (s, 1H), 8.26 (s, 1H), 8.17 - 8.09 (m, 1H), 7.83 (d, J = 9.0 Hz, 1H), 7.40 (s, 1H), 7.27 (d, J = 8.2 Hz, 1H), 7.02 (d, J = 8.1 Hz, 1H), 2.88 (s, 2H), 2.66 (m, 1H), 2.51 (s, 4H),2.32 (p, J = 1.8 Hz, 2H), 2.00 (s, 2H), 1.86 (s, 3H), 1.84 (s, 3H),1.68 (s, 4H), 1.52 (s, 2H).MS(ESI+): 535.3(M+H).

**Example 55** (S)-(2-((5-chloro-2-((7-(pyrrolidin-1-yl)-6,7,8,9-tetrahydro-5H-benzo[7]annulen-2-yl)amino)pyrimidin-4-yl)amino)-5-methoxyphenyl) dimethyl phosphine oxide

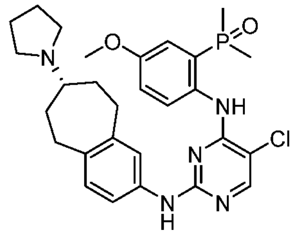

According to the preparation method of Example 1, 2-amino-N,N-dimethyl benzenesulfonamide in step a) is replaced with (2-amino-5-methoxyphenyl) dimethyl phosphine oxide.

$^1H$ NMR (400 MHz, DMSO-$d_6$) δ 10.53 (s, 1H), 9.21 (s, 1H), 8.27 (d, *J* = 6.9 Hz, 1H), 8.12 (s, 1H), 7.38 (d, *J* = 2.3 Hz, 1H), 7.23 (dd, *J* = 8.1, 2.2 Hz, 1H), 7.16 (dd, *J* = 14.4, 3.0 Hz, 1H), 7.09 (dd, *J* = 9.1, 2.9 Hz, 1H), 6.93 (d, *J* = 8.1 Hz, 1H), 3.82 (s, 3H), 2.83 (m, 2H), 2.55 (d, *J* = 6.1 Hz, 4H), 2.47-2.4 (m, 3H), 1.87 (m, 2H), 1.77 (s, 3H), 1.74 (s, 3H), 1.68 (d, *J* = 6.2 Hz, 4H), 1.50 (s, 2H). MS(ESI+): 540.2(M+H).

**Example 56** (S)-(2-((5-chloro-2-((7-(pyrrolidin-1-yl)-6,7,8,9-tetrahydro-5H-benzo[7]annulen-2-yl)amino)pyrimidin-4-yl)amino)phenyl)bis(methyl-$d_3$) phosphine oxide

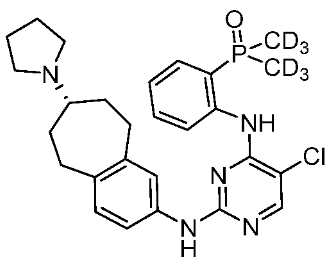

According to the preparation method of Example 1, 2-amino-N,N-dimethyl benzenesulfonamide in step a) is replaced with (2-aminophenyl)bis(methyl-$d_3$) phosphine oxide.

$^1H$ NMR (400 MHz, DMSO-$d_6$) δ 11.11 (s, 1H), 9.28 (s, 1H), 8.57 (d, *J* = 6.1 Hz, 1H), 8.17 (s, 1H), 7.60 (ddd, *J* = 13.9, 7.7, 1.6 Hz, 1H), 7.49 (t, *J* = 7.9 Hz, 1H), 7.39 (d, *J* = 2.3 Hz, 1H), 7.31 (dd, *J* = 8.1, 2.3 Hz, 1H), 7.19 (td, *J* = 7.6, 2.0 Hz, 1H), 6.97 (d, *J* = 8.1 Hz, 1H), 4.20 - 3.94 (m, 1H), 3.17 (d, *J* = 4.8 Hz, 2H), 2.87 (s, 2H), 2.53 (s, 4H), 1.85 (s, 2H), 1.73 - 1.64 (m, 4H), 1.51 (s, 2H). MS(ESI+):516.3(M+H).

**Example 57** (S)-(2-((5-chloro-2-((7-(pyrrolidin-1-yl)-6,7,8,9-tetrahydro-5H-benzo[7]annulen-2-yl)amino)pyrimidin-4-yl)amino)-5-fluorophenyl)bis(methyl-$d_3$) phosphine oxide

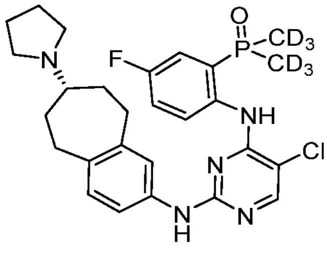

According to the preparation method of Example 1, 2-amino-N,N-dimethyl benzenesulfonamide in step a) is

replaced with (2-amino-5-fluorophenyl)bis(methyl-$d_3$) phosphine oxide.

$^1$H NMR (400 MHz, DMSO-$d_6$) δ 10.80 (s, 1H), 9.27 (s, 1H), 8.49 (s, 1H), 8.16 (s, 1H), 7.52 (ddd, *J* = 14.0, 8.7, 3.1 Hz, 1H), 7.39 - 7.30 (m, 2H), 7.26 (dd, *J* = 8.1, 2.2 Hz, 1H), 6.97 (d, *J* = 8.2 Hz, 1H), 2.93 - 2.66 (m, 2H), 2.62 - 2.53 (m, 7H), 1.88 (d, *J* = 2.2 Hz, 2H), 1.68 (q, *J* = 3.9 Hz, 4H), 1.44 (s, 2H). MS(ESI+): 534.2(M+H).

**Example 58** Preparation of compounds 58-1 to 58-4

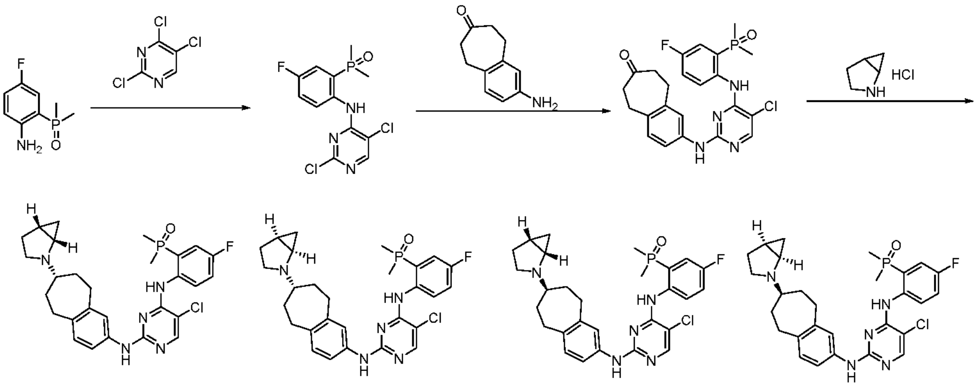

a) (2-((2,5-dichloropyrimidin-4-yl)amino)-5-fluorophenyl) dimethyl phosphine oxide

Compounds (2-amino-5-fluorophenyl) dimethyl phosphine oxide (1.1 g) and 2,4,5-trichloropyrimidine (1.18 g) are dissolved in N,N-dimethylformamide (15 mL), added with N,N-diisopropylethylamine (1.52 g), heated to 70°C and reacted for 5 h. The reaction solution is diluted with 50 mL of water. The diluted solution is extracted with ethyl acetate (80 mL×3). An ethyl acetate layer is washed for three times with a saturated sodium chloride solution (50 mL×3). The organic layer is dried with anhydrous sodium sulfate and concentrated to dryness to obtain a crude product. The crude product is slurried with ethanol (20 mL) to obtain the title compound (1.08 g). MS(ESI+): 334.06(M+H).

b) 2-((5-chloro-4-((2-(dimethylphosphoryl)-4-fluorophenyl)amino)pyrimidin-2-yl)amino)-5,6,8,9-tetrahydro-7H-benzo[7]annulen-7-one

N,N-dimethylformamide (5 ml), 2-amino-5,6,8,9-tetrahydrobenzo[7]annulen-7-one (200.00 mg) and 2, 5-dichloro-N-[2-(dimethylphosphoryl)-4-fluorophenyl]pyrimidin-4-amine (343.20 mg) are added to a reaction flask, and added with a 1,4-dioxane solution (80.00 mg) of 4M hydrochloric acid under stirring under the protection of nitrogen. The reaction mixture is irradiated with microwave radiation at 130°C for 10 min, concentrated under reduced pressure, and diluted with N,N-dimethylformamide (5 ml). The precipitated solid is collected by filtration and washed with petroleum ether/ethyl acetate (5: 1) (2×5 mL) to obtain filtrate of the title product. The filtrate is concentrated under reduced pressure to 5 ml. A preparation solution phase is purified by column chromatography (column: XBridge Prep OBD C18 column, 30×150 mm, with a filler particle size of 5 μm; mobile phase A: water (10 mmol/L ammonium bicarbonate), mobile phase B: acetonitrile; flow rate: 60 ml/min; gradient: 25%B to 65%B within 10 min; UV detection wavelength: 220nm; product retention time: 7.53 min) to obtain the pure title product (100 mg). MS(ESI+): 473.05(M+H).

c) Preparation of compounds 58-1, 58-2, 58-3 and 58-4

2-azabicyclo[3.1.0]hexane hydrochloride (75.87 mg) and magnesium sulfate (101.81 mg) are stirred and mixed, add to dichloromethane (5 mL), and added with triethylamine (85.59 mg) at room temperature in the atmosphere of nitrogen. 2-[(5-chloro-4-[[2-(dimethylphosphoryl)-4-fluorophenyl]amino]pyrimidin-2-yl)amino]-5,6,8,9-tetrahydrobenzo[7]annulen-7-one (100.00 mg) is added to the mixture after stirring for 10 min, and stirred at 35°C for 1 h. Sodium cyanoborohydride (39.87 mg) is added to the mixture, and stirred at 35°C for 3 h. The resulting mixture is filtered, and a filter cake is washed with dichloromethane (3×10 mL). The mother liquor is concentrated under reduced pressure, and the resulting residue is purified by reversed-phase high-performance liquid chromatography (column: XBridge-Prep-OBD C18 column, 30×150 mm, with a filler particle size of 5 μm; mobile phase A: 10 mmol/L ammonium bicarbonate aqueous solution, and mobile phase B: acetonitrile; flow rate: 60 mL/min; gradient: 25%B to 65%B, 10 min; detection wavelength: 220nm; isomer mixture retention time: 8.62 min) to obtain the title product (a mixture of four isomers).

Chiral resolution is performed by chiral liquid chromatography (Chiralpak IA, 2×25 cm, with a filler particle size of 5 μm; mobile phase A: n-hexane (10 mM ammonia-methanol), and mobile phase B: ethanol; flow rate: 18 ml/min; gradient: 50%B within 16 min, isogradient; detection wavelength: 220/254 nm; column temperature: 25°C) .

Isomer 58-1 (12.2 mg) having a chiral HPLC retention time of 1.96 min.

$^1$H NMR (400 MHz, DMSO-$d_6$): δ 10.84 (s, 1H), 9.27 (s, 1H), 8.50 (d, *J* = 8.4 Hz, 1H), 8.17 (s, 1H), 7.53 (ddd, *J* = 13.9, 8.7, 3.0 Hz, 1H), 7.38 (d, *J* = 2.3 Hz, 1H), 7.39 - 7.30 (m, 1H), 7.26 (d, *J* = 8.0 Hz, 1H), 6.97 (d, *J* = 8.1 Hz, 1H), 2.95 - 2.82 (m, 3H), 2.71 - 2.65 (m, 1H), 2.48 - 2.41 (m, 2H), 2.26 (t, *J* = 6.6 Hz, 1H), 1.99 - 1.84 (m, 3H), 1.82 (s, 3H), 1.80 (s, 1H), 1.78 (s, 3H), 1.77 - 1.71 (m, 1H), 1.69 - 1.62 (m, 1H), 1.47 (s, 1H), 1.35 (s, 1H), 0.62 (q, *J* = 4.2, 3.6 Hz, 1H), 0.09 (q, *J* = 5.6 Hz, 1H). MS(ESI+): 540.15(M+H).

Isomer 58-2 (10.6 mg) having a chiral HPLC retention time of 3.58 min.

$^1$H NMR (400 MHz, DMSO-$d_6$): δ 10.83 (s, 1H), 9.27 (s, 1H), 8.50 (s, 1H), 8.17 (s, 1H), 7.53 (ddd, *J* = 14.0, 8.8, 3.0 Hz, 1H), 7.39 - 7.30 (m, 2H), 7.27 (d, *J* = 7.9 Hz, 1H), 6.98 (d, *J* = 8.1 Hz, 1H), 2.91 (t, *J* = 8.3 Hz, 2H), 2.85 - 2.78 (m, 1H), 2.68 (s, 1H), 2.46 (s, 2H), 2.26 (t, *J* = 6.6 Hz, 1H), 1.86 (s, 3H), 1.80 (d, *J* = 13.7 Hz, 8H), 1.70 - 1.63 (m, 1H), 1.46 (s, 1H), 1.34 (s, 1H), 0.61 (d, *J* = 6.8 Hz, 1H), 0.09 (q, *J* = 5.6 Hz, 1H). MS(ESI+): 540.15(M+H).

Isomer 58-3 (12.7 mg) having a chiral HPLC retention time of 1.39 min.

$^1$H NMR (400 MHz, DMSO-$d_6$): δ 10.82 (s, 1H), 9.27 (s, 1H), 8.49 (s, 1H), 8.17 (s, 1H), 7.53 (ddd, *J* = 13.9, 8.8, 3.0 Hz, 1H), 7.39 - 7.30 (m, 2H), 7.30 - 7.23 (m, 1H), 6.98 (d, *J* = 8.1 Hz, 1H), 2.96 - 2.87 (m, 2H), 2.78 (s, 1H), 2.68 (s, 1H), 2.46 (s, 2H), 2.26 (t, *J* = 6.6 Hz, 1H), 1.99 - 1.85 (m, 3H), 1.80 (d, *J* = 13.7 Hz, 8H), 1.65 (s, 1H), 1.48 (s, 1H), 1.35 (s, 1H), 0.62 (s, 1H), 0.09 (d, *J* = 8.0 Hz, 1H). MS(ESI+): 540.15(M+H).

Isomer 58-4 (12.2 mg) having a chiral HPLC retention time of 1.58 min.

$^1$H NMR (400 MHz, DMSO-$d_6$): δ 10.84 (s, 1H), 9.27 (s, 1H), 8.50 (s, 1H), 8.17 (s, 1H), 7.53 (ddd, *J* = 14.0, 8.8, 3.1 Hz, 1H), 7.41 - 7.30 (m, 2H), 7.26 (d, *J* = 7.8 Hz, 1H), 6.97 (d, *J* = 8.1 Hz, 1H), 2.94 - 2.82 (m, 3H), 2.69 (s, 1H), 2.46 (s, 3H), 2.05 - 1.84 (m, 3H), 1.80 (d, J = 13.7 Hz, 8H), 1.66 (s, 1H), 1.47 (s, 1H), 1.36 (s, 1H), 0.63 (s, 1H), 0.10 (s, 1H). MS(ESI+): 540.15(M+H).

**Example 59** Preparation of compounds 59-1 and 59-2

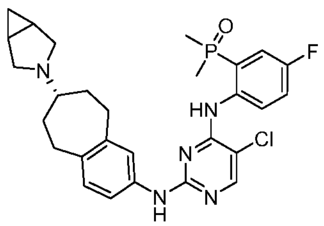
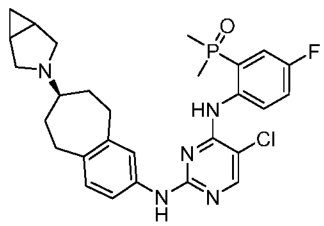

According to the preparation method of Example 58, 2-azabicyclo[3.1.0]hexane hydrochloride in step b) is replaced with 3-azabicyclo[3.1.0]hexane hydrochloride. The crude product is purified by reversed-phase high-performance liquid chromatography (column: XBridge-Prep-OBD C18 column, 30×150 mm, with a filler particle size of 5 μm; mobile phase A: 10 mmol/L ammonium bicarbonate aqueous solution, and mobile phase B: acetonitrile; flow rate: 60 mL /min; gradient: 25Bto 65B, 10 min; detection wavelength: 220 nm; isomer mixture retention time: 8.62 min) to obtain a mixture of isomers.

Chiral resolution is performed on the mixture of isomers by chiral liquid chromatography (CHIRALPAK IF, 2×25 cm, with a filler particle size of 5 μm; mobile phase A: n-hexane (0.1% diethanolamine), and mobile phase B: ethanol; flow rate: 20 ml/min; gradient: 20%B within 21 min, isogradient; detection wavelength: 220/254 nm; column temperature: 25°C) to obtain:
isomer 59-1 (16.8 mg) having a chiral HPLC retention time of 8.10 min.

$^1$H NMR (400 MHz, DMSO-$d_6$, *ppm):* δ10.83 (s, 1H), 9.26 (s, 1H), 8.49 (s, 1H), 8.17 (s, 1H), 7.53 (ddd, *J* = 13.9, 8.7, 3.0 Hz, 1H), 7.33 (dq, *J* = 8.7, 3.0 Hz, 2H), 7.26 (dd, *J* = 8.2, 2.2 Hz, 1H), 6.96 (d, *J* = 8.1 Hz, 1H), 2.95 - 2.80 (m, 4H), 2.39 (s, 3H), 2.31- 2.21 (m, 1H), 1.81 (s, 3H), 1.78 (s, 3H), 1.77 - 1.61 (m, 2H), 1.51 (s, 2H), 1.38 (s, 2H), 1.24 (s, 1H), 0.60 (q, *J* = 3.7 Hz, 1H), 0.31 (s, 1H). MS(ESI+): 540.15(M+H)

Isomer 59-2 (15.3 mg) having a chiral HPLC retention time of 10.29 min.

$^1$H NMR (400 MHz, DMSO-$d_6$, *ppm):* δ 10.83 (s, 1H), 9.26 (s, 1H), 8.49 (s, 1H), 8.17 (s, 1H), 7.53 (ddd, *J* = 13.9, 8.7, 3.0 Hz, 1H), 7.33 (dq, *J* = 8.8, 3.1 Hz, 2H), 7.26 (dd, *J* = 8.2, 2.3 Hz, 1H), 6.96 (d, *J* = 8.1 Hz, 1H), 2.89 (m, 4H), 2.43 (m, 3H), 2.26 (t, *J* = 6.7 Hz, 1H), 1.81 (s, 3H), 1.78 (s, 4H), 1.76 - 1.72 (m, 1H), 1.69 - 1.61 (m, 1H), 1.51 (s, 2H), 1.38 (s, 2H), 0.59 (q, *J* = 3.7 Hz, 1H), 0.31 (s, 1H). MS(ESI+): 540.15(M+H)

**Example 60** Preparation of compounds 60-1 and 60-2

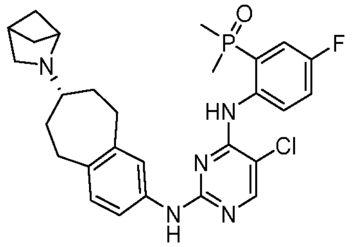 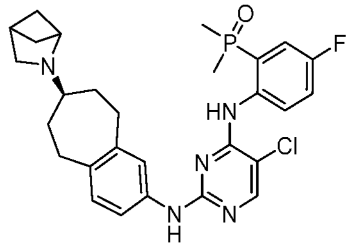

According to the preparation method of Example 58, 2-azabicyclo[3.1.0]hexane hydrochloride in step b) is replaced with 2-azabicyclo[2.1.1]hexane hydrochloride. The crude product is purified by reversed-phase high-performance liquid chromatography (column: XBridge-Prep-OBD C18 column, 30×150 mm, with a filler particle size of 5 μm; mobile phase A: 10 mmol/L ammonium bicarbonate aqueous solution, and mobile phase B: acetonitrile; flow rate: 60 mL/min; gradient: 25%B-65%B, 10 min; detection wavelength: 220 nm; isomer mixture retention time: 8.62 min) to obtain an isomer mixture.

Chiral resolution is performed on the isomer mixture by chiral liquid chromatography (column: CHIRALPAK IG, 2 cm×25 cm, with a filler particle size of 5 μm; mobile phase A: methyl tert-butyl ether (10 mM ammonia-methanol), and mobile phase B: ethanol; flow rate: 20 ml/min;
gradient: 20%B within 10 min, isogradient; detection wavelength: 220/254 nm; column temperature: 25°C) to obtain: isomer 60-1 (28.7 mg) having a chiral HPLC retention time of 1.67 min.

$^1$H NMR (400 MHz, DMSO-$d_6$, *ppm):* δ 10.84 (s, 1H), 9.27 (s, 1H), 8.50 (s, 1H), 8.17 (s, 1H), 7.53 (ddd, *J* = 14.0, 8.8, 3.1 Hz, 1H), 7.39 - 7.30 (m, 2H), 7.27 (dd, *J* = 8.2, 2.3 Hz, 1H), 6.97 (d, *J* = 8.2 Hz, 1H), 3.65 (d, *J* = 6.5 Hz, 1H), 2.85 (d, *J* = 37.1 Hz, 2H), 2.73 - 2.62 (m, 3H), 2.57 (d, *J* = 14.5 Hz, 3H), 1.92 (d, *J* = 12.8 Hz, 2H), 1.82 (s, 3H), 1.78 (s, 3H), 1.61 (s, 2H), 1.48 - 1.20 (m, 4H). MS(ESI+): 540.15(M+H)

Isomer 60-2 (27.7 mg) having a chiral HPLC retention time of 2.31 min.

$^1$H NMR (400 MHz, DMSO-$d_6$, *ppm):* δ 10.84 (s, 1H), 9.27 (s, 1H), 8.51 (s, 1H), 8.17 (s, 1H), 7.53 (ddd, *J* = 13.9, 8.8, 3.0 Hz, 1H), 7.41 - 7.30 (m, 2H), 7.27 (dd, *J* = 8.1, 2.2 Hz, 1H), 6.97 (d, *J* = 8.1 Hz, 1H), 3.65 (d, *J* = 6.5 Hz, 1H), 2.86 (m, 2H), 2.74 - 2.62 (m, 3H), 2.57 (d, *J* = 14.5 Hz, 3H), 1.92 (d, *J* = 14.3 Hz, 2H), 1.82 (s, 3H), 1.78 (s, 3H), 1.61 (d, *J*= 4.3 Hz, 2H), 1.48 - 1.21 (m, 4H). MS(ESI+): 540.15(M+H)

**Example 61** (S)-(2-((5-chloro-2-((7-(pyrrolidin-1-yl)-6,7,8,9-tetrahydro-5H-benzo[7]annulen-2-yl)amino)pyrimidin-4-yl)amino)-5-hydroxyphenyl) dimethyl phosphine oxide

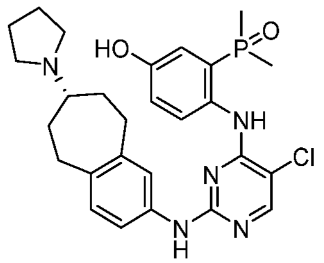

(S)-(2-((5-chloro-2-((7-(pyrrolidin-1-yl)-6,7,8,9-tetrahydro-5H-benzo[7]annulen-2-yl)amino)pyrimidin-4-yl)amino)-5-methoxyphenyl) dimethyl phosphine oxide (140 mg) and dichloromethane (10 mL) are added to a 25 mL single-necked flask and stirred. The temperature is then cooled to -20°C. Boron tribromide (150 mg) is added dropwise to the reaction solution. After the addition is completed, the temperature gradually rises to room temperature and the reaction solution is stirred and stands overnight. The temperature is then cooled to -20°C, sodium bicarbonate is added dropwise to obtain a saturated solution, and the saturated solution is extracted with dichloromethane (10 mL). The organic layers are merged, concentrated to dryness, and purified by silica-gel column chromatography (dichloromethane: methanol = 20: v/v) to obtain 16 mg of the title product.

$^1$H NMR (400 MHz, DMSO-$d_6$, *ppm):* δ10.19 (s, 1H), 9.69 (s, 1H), 9.17 (s, 1H), 8.07 (d, J = 16.1 Hz, 2H), 7.37

(d, J = 2.3 Hz, 1H), 7.28 - 7.16 (m, 1H), 7.03 (dd, J = 14.3, 2.9 Hz, 1H), 6.95 (dd, J = 8.9, 2.8 Hz, 1H), 6.90 (d, J = 8.2 Hz, 1H), 2.82 (m, 2H), 2.55 (m, 4H), 2.44 (d, J = 12.7 Hz, 3H), 1.84 (s, 2H), 1.70 (s, 3H), 1.69 (s, 4H), 1.67 (s, 3H), 1.50 (s, 2H). MS(ESI+): 526.2(M+H).

**Example 62** Preparation of compounds 62-1 and 62-2

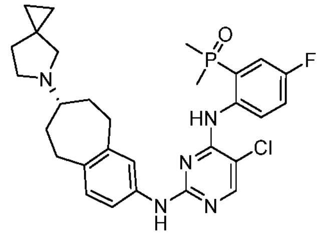

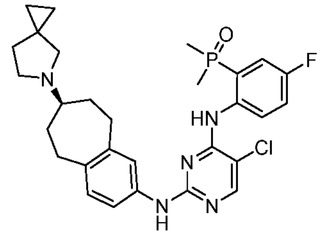

According to the preparation method of Example 58, 2-azabicyclo[3.1.0]hexane hydrochloride in step c) is replaced with 5-azaspiro[2.4]heptane hydrochloride. The crude product is purified by reversed-phase high-performance liquid chromatography (column: Xselect CSH OBD column, 30×150 mm, with a filler particle size of 5 μm; mobile phase A: 10 mmol/L ammonium bicarbonate aqueous solution, and mobile phase B: acetonitrile; flow rate: 60 mL /min; gradient: 25%B-55%B, 8 min; detection wavelength: 254/220 nm) to obtain 60 mg of an isomer mixture.

Chiral resolution is performed on the isomer mixture by chiral liquid chromatography (CHIRALPAK IC, 2 cm×25 cm, with a filler particle size of 5 μm; mobile phase A: n-hexane (10 mM ammonia-methanol), and mobile phase B: ethanol; flow rate: 20 ml/min; gradient: 50%B within 12 min, isogradient; detection wavelength: 220/254 nm; column temperature: 25°C) to obtain:

Isomer 62-1 (27.2 mg) having a HPLC retention time of 7.9 min.

$^1$H NMR (400 MHz, DMSO-$d_6$, *ppm):* δ10.84 (s, 1H), 9.27 (s, 1H), 8.50 (s, 1H), 8.17 (s, 1H), 7.53 (m, 1H), 7.39 - 7.29 (m, 2H), 7.27 (d, *J* = 8.1 Hz, 1H), 6.97 (d, *J* = 8.2 Hz, 1H), 2.88 (m, 2H), 2.73 (m, 2H), 2.48 (m, 2H), 2.43 (m, 2H), 1.90 - 1.68 (m, 11H), 1.50 (s, 2H), 0.51 (m, 4H). MS(ESI+): 554.2(M+H)

Isomer 62-2 (28.2 mg) having a HPLC retention time of 9.8 min.

$^1$H NMR (400 MHz, DMSO-$d_6$, *ppm*)*:* δ 10.84 (s, 1H), 9.27 (s, 1H), 8.50 (s, 1H), 8.17 (s, 1H), 7.53 (ddd, *J* = 13.9, 8.7, 3.1 Hz, 1H), 7.39 - 7.30 (m, 2H), 7.27 (dd, *J* = 7.9, 2.0 Hz, 1H), 6.97 (d, *J* = 8.1 Hz, 1H), 2.87 (s, 2H), 2.73 (t, *J* = 6.8 Hz, 2H), 2.59 (s, 2H), 2.51- 2.41 (m,2H), 1.92 - 1.68 (m, 11H), 1.50 (s, 2H), 0.56 - 0.45 (m, 4H). MS(ESI+): 554.2(M+H)

**Example 63** Preparation of compounds 63-1, 63-2, 63-3 and 63-4

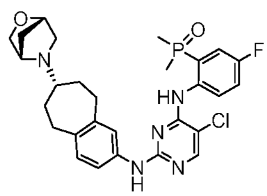

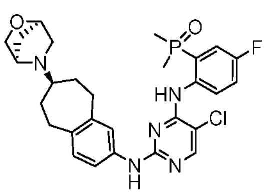

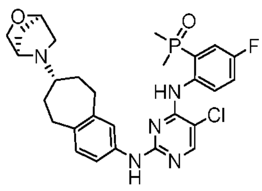

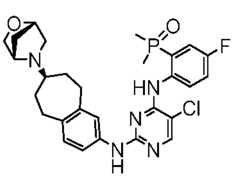

According to the preparation method of Example 58, 2-azabicyclo[3.1.0]hexane hydrochloride in step c) is replaced with 2-oxa-5-azabicyclo[2.2.1]heptane hydrochloride. The crude product is purified by a preparative high-performance liquid phase under the following conditions (column: Xselect CSH-OBD column 30×150 mm, with a filler particle size of 5 μm; mobile phase A: water (10 mmol/L ammonium bicarbonate), mobile phase B: acetonitrile; flow rate: 60 ml/min; gradient: 20%B-53%B within 8 min; wavelength: 210 nm; retention time: 7.02 min) to obtain a white mixture of four isomers.

The mixture of four isomers is purified by a chiral high-performance liquid chromatography column (IC column: CHIRALPAK ID, 2×25 cm (filler 5 μm); mobile phase A: methyl tert-butyl ether (10 mM ammonia-methanol), and mobile phase B: ethanol; flow rate: 20 ml/min; gradient: 10%B within 27 min, isogradient; detection wavelength: 220/254 nm; and column temperature: 25°C) to obtain:

isomer 63-1 (20 mg) having a HPLC retention time of 13.5 min.

$^1$H NMR (400 MHz, DMSO-$d_6$, *ppm):* δ 10.83 (s, 1H), 9.26 (s, 1H), 8.50 (s, 1H), 8.17 (s, 1H), 7.53 (t, *J* = 11.4 Hz, 1H), 7.35 (s, 2H), 7.30 - 7.23 (m, 1H), 6.96 (d, *J* = 8.2 Hz, 1H), 4.33 (s, 1H), 3.89 (d, *J* = 7.5 Hz, 1H), 3.69 (s, 1H), 3.55 (s, 1H), 3.44 (s, 2H),2.99 (d, *J* = 9.3 Hz, 1H), 2.87 (s, 2H), 2.68 (s, 1H), 2.33 (m, 1H), 1.80 (m, 8H), 1.72 (d, *J* = 9.4 Hz, 1H), 1.61 (m, 1H), 1.40 (m, 2H). MS(ESI+): 556.2(M+H);

isomer 63-2 (14.7 mg) having a HPLC retention time of 16.8 min.

$^1$H NMR (400 MHz, DMSO-$d_6$, *ppm*): δ 10.83 (s, 1H), 9.26 (s, 1H), 8.50 (s, 1H), 8.17 (s, 1H), 7.53 (m, 1H), 7.35 (m, 2H), 7.29 - 7.24 (m, 1H), 6.96 (d, *J* = 8.2 Hz, 1H), 4.33 (s, 1H), 3.89 (d, *J* = 7.5 Hz, 1H), 3.69 (s, 1H), 3.55 (s, 2H), 2.99 (m, 1H),2.87 (m, 2H), 2.68 (m, 2H), 2.33 (m, 1H), 1.80 (m, 8H), 1.72 (m, 1H), 1.61 (m, 1H), 1.40 (m, 2H). MS(ESI+): 556.2(M+H);

and a mixture (42 mg) of the isomer 63-3 and the isomer 63-4;

the mixture of the isomer 63-3 and the isomer 63-4 is purified by chiral liquid chromatography (column: Chiralpak IC, 2×25 cm, with a filler particle size of 5 μm; mobile phase A: methyl tert-butyl ether (10 mM ammonia-methanol), mobile phase B: isopropanol; flow rate: 20 ml/min; gradient: 30%B within 21 min, isogradient; detection wavelength: 220/254 nm; column temperature: 25°C)) again to obtain:

isomer 63-3 (14.7 mg) having a HPLC retention time of 9.2min.

$^1$H NMR: (400 MHz, DMSO-$d_6$, *ppm*): δ 10.84 (s, 1H), 9.26 (s, 1H), 8.50 (s, 1H), 8.17 (s, 1H), 7.57 - 7.47 (m, 1H), 7.39 - 7.24 (m, 3H), 6.97 (m, 1H), 4.33 (m, 1H), 3.90 (m, 1H), 3.69 (m, 1H), 3.53 (m, 1H), 3.00 (m, 1H), 2.86 (s, 3H),2.70 (m, 2H), 2.33 (m, 1H), 1.80 (m, 8H), 1.72 (m, 1H), 1.61 (m, 1H), 1.40 (m, 2H). MS(ESI+): 556.2(M+H);

isomer 63-4 (15 mg) having a HPLC retention time of 18.9min.

$^1$H NMR: (400 MHz, DMSO-$d_6$, *ppm*): δ 10.84 (s, 1H), 9.26 (s, 1H), 8.50 (s, 1H), 8.17 (s, 1H), 7.53 (m, 1H), 7.39 - 7.22 (m, 3H), 6.97 (d, *J* = 8.2 Hz, 1H), 4.33 (d, *J* = 2.1 Hz, 1H), 3.89 (m, 1H), 3.71 - 3.67 (m, 1H), 3.53 (m, 1H), 3.00 (m, 1H), 2.87 (m, 2H), 2.70 (m, 1H), 2.55 (m, 2H), 2.33 (m, 1H), 1.80 (m, 8H), 1.72 (m, 1H), 1.61 (m, 1H), 1.39 (m, 2H). MS(ESI+): 556.2(M+H).

**Example 64** Preparation of (2-((5-chloro-2-(((S)-7-(pyrrolidin-1-yl)-6,7,8,9-tetrahydro-5H-benzo[7]annulen-2-yl)amino)pyrimidin-4-yl)amino)phenyl)(methyl)(methylimino)-λ$^6$-sulfanone

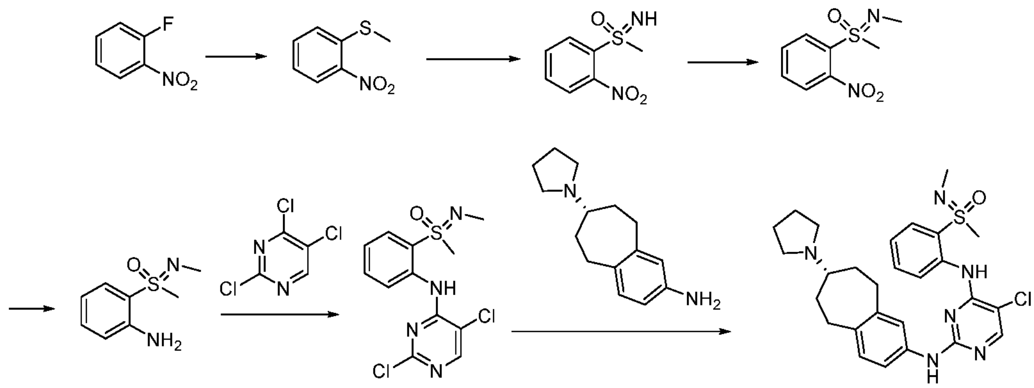

a) 2-(Methylthio)nitrobenzene

1-fluoro-2-nitrobenzene (1.4 g), isopropanol (15 ml), and sodium thiomethoxide(2.1 g) are sequentially added to a reaction flask, and reacted for 3 h at room temperature. After the reaction is completed, the reaction solution is cooled to 0°C, and filtered by suction. The filter cake is washed with water (10 ml*2), and dried to obtain 1.62 g of the title product.

b) Preparation of imino(methyl)(2-nitrophenyl)-λ$^6$-sulfanone

A compound 2-methylthionitrobenzene (400 mg), ethanol (15 ml), iodobenzenediacetic acid (1.5 g), and ammonium acetate (0.74 g) are sequentially added to a reaction flask, and reacted at room temperature for 2 h. After the reaction is completed, a solvent is removed by concentration under reduced pressure, and purified by silica-gel column

chromatography (dichloromethane/methanol=50/1 v/v) to obtain 374 mg of the title product.

c) Preparation of methyl(methylimino)(2-nitrophenyl)-λ$^6$-sulfanone

Imino(methyl)(2-nitrophenyl)-λ$^6$-sulfanone (370 mg), N,N-dimethylformamide (2 ml), and sodium hydride (89 mg, 60% w /w) are sequentially added to a reaction flask, added dropwise with iodomethane (315 mg), and reacted at room temperature for 1 h. After the reaction is completed, a solvent is removed by concentration under reduced pressure, and purified by silica-gel column chromatography (dichloromethane/methanol=50/1 v/v) to obtain 289 mg of the title product.

d) Preparation of (2-aminophenyl)(methyl)(methylimino)-λ$^6$-sulfanone

Titanium trichloride (6 ml) is added dropwise to a tetrahydrofuran (5 ml) solution of methyl(methylimino)(2-nitrophenyl)-λ$^6$-sulfanone (235 mg) under an ice bath condition, gradually heated to room temperature and reacted for 2 h. After the reaction is completed, the reaction solution is neutralized by adding dropwise 2M sodium hydroxide to weak alkaline and filtered by suction. The filter cake is washed with ethyl acetate (10 ml*3). The organic layers are merged, dried and then concentrated to obtain 182 mg of the title product.

e) Preparation of (2-((2,5-dichloropyrimidin-4-yl)amino)phenyl)(methyl)(methylimino)-λ$^6$-sulfanone

At 0°C, 2,4,5-trichloropyrimidine (217 mg), (2-aminophenyl)(methyl)(methylimino)-λ$^6$-sulfanone (182 mg), N, N-dimethylformamide (5 ml), and sodium hydride (48 mg, 60% w/w) are sequentially added to a reaction flask. The mixture is gradually heated to room temperature and reacted for 2 h. After the reaction is completed, the reaction solution is quenched with a saturated ammonium chloride solution and separated. The aqueous phase is extracted with ethyl acetate. The organic layers are merged, dried with anhydrous sodium sulfate, concentrated, and purified by silica-gel column chromatography (dichloromethane/methanol=20/1 v /v) to obtain 170 mg of the title product.

f) (2-((5-chloro-2-(((S)-7-(pyrrolidin-1-yl)-6,7,8,9-tetrahydro-5H-benzo[7]annulen-2-yl)amino)pyrimidin-4-yl)amino)phenyl)(methyl)(methylimino)-λ$^6$-sulfanone

(2-((2,5-dichloropyrimidin-4-yl)amino)phenyl)(methyl)(methylimino)-λ$^6$-sulfanone (170 mg), N, N-dimethylformamide (5 ml), (S)-7-(pyrrolidin-1-yl)-6,7,8,9-tetrahydro-5H-benzo[7]annulen-2-amine (118 mg), and p-toluenesulfonic acid monohydrate (194 mg) are sequentially added to a reaction flask, and reacted in 400W microwaves at 120°C for 1 h. After the reaction is completed, a solvent is removed by concentration under reduced pressure, and purified by silica-gel column chromatography (dichloromethane/methanol=20: 1 v/v) to obtain 46 mg of the title product.

$^1$H NMR (400 MHz, DMSO-$d_6$, *ppm*): δ 10.83 (s, 1H), 9.39 (s, 1H), 8.75 (d, J = 6.8 Hz, 1H), 8.26 (s, 1H), 7.89 (dd, J = 7.9, 1.5 Hz, 1H), 7.64 (t, J = 7.9 Hz, 1H), 7.40 (s, 1H), 7.32 (t, J = 7.6 Hz, 2H), 7.00 (d, J = 8.1 Hz, 1H), 3.18 (s, 3H), 2.88 (s, 2H), 2.71 (s, 3H), 2.67 - 2.52 (m, 7H), 1.92 (s, 2H), 1.71 (s, 4H), 1.51 (s, 2H). MS(ESI+): 525.2(M+H).

**Example 65** (S)-(2-((5-chloro-2-((7-(pyrrolidin-1-yl)-6,7,8,9-tetrahydro-5H-benzo[7]annulen-2-yl)amino)pyrimidin-4-yl)amino)-3-fluoro-5-methylphenyl) dimethyl phosphine oxide

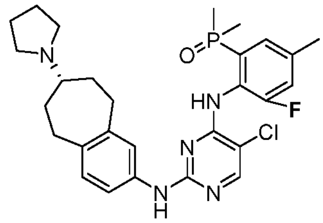

According to the preparation method of Example 47, quinoxaline-6-amine in step a) is replaced with 2-fluoro-4-methylaniline.

$^1$H NMR (400 MHz, DMSO-$d_6$, *ppm):* δ8.96 (s, 1H), 7.52 (d, J = 12.2 Hz, 1H), 7.42 (d, J = 10.7 Hz, 1H), 7.22 (s, 1H), 6.98 (d, J = 8.1 Hz, 1H), 6.74 (dd, J = 13.7, 8.0 Hz, 2H), 6.29 (d, J = 28.4 Hz, 1H), 2.85 - 2.67 (m, 2H), 2.42 (d, J = 12.2 Hz, 7H), 1.89 (s, 2H), 1.80 (s, 2H), 1.68 (d, J = 6.1 Hz, 4H), 1.62 (s, 3H), 1.59 (s, 3H), 1.45 (s, 2H). MS(ESI+):

542.2(M+H).

**Example 66** 2-((5-chloro-2-((3-(tetrahydrofuran-3-yl)-2,3,4,5-tetrahydro-1H-benzo[d]azepin-7-yl)amino)pyrimidin-4-yl)amino)-5-fluorophenyl)dimethyl phosphine oxide

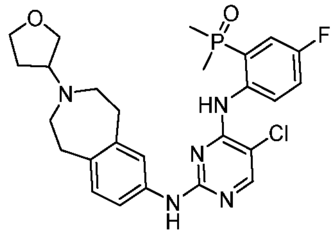

According to the preparation method of Example 1, 2-amino-N,N-dimethyl benzenesulfonamide in step a) is replaced with (2-amino-5-fluorophenyl) dimethyl phosphine oxide.

(S)-7-(pyrrolidin-1-yl)-6,7,8,9-tetrahydro-5H-benzo[7]annulen-2-amine in step b) is replaced with 3- (tetrahydrofuran-3-yl)-2,3,4,5-tetrahydro-1H-benzo[d]azepin-7-amine to obtain the target product.

$^1$H NMR (400 MHz, DMSO-$d_6$, *ppm)* : δ 10.87 (s, 1H), 9.31 (s, 1H), 8.50 (s, 1H), 8.17 (s, 1H), 7.56 - 7.49 (m, 1H), 7.37 - 7.28 (m, 3H), 6.98 (d, *J* = 7.9 Hz, 1H), 3.86 - 3.71 (m, 3H), 3.67-3.47 (m, 3H), 3.22 (br, 1H), 2.85-2.68 (m, 5H), 2.64 - 2.57 (m, 2H), 1.01-1.94 (m, 1H), 1.81 (s, 3H), 1.78 (s, 3H). MS(ESI+): 530.1(M+H).

**Example 67** Preparation of compounds 67-1 and 67-2

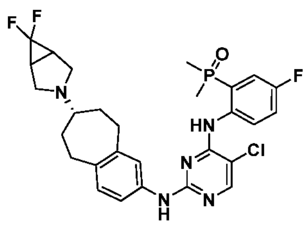
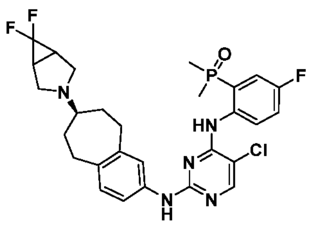

According to the preparation method of Example 58, 2-azabicyclo[3.1.0]hexane hydrochloride in step c) is replaced with 6,6-difluoro-3-azabicyclo[3.1.0]hexane hydrochloride. The crude product is purified by a preparative high-performance liquid phase under the following conditions (column: Xselect CSH-OBD column 30×150 mm, with a filler particle size of 5 μm; mobile phase A: water (10 mmol/L ammonium bicarbonate), mobile phase B: acetonitrile; flow rate: 60 ml/min; gradient: 40%B-70%B within 8 min; wavelength: 254/220 nm) to obtain 200 mg of a mixture of two isomers.

The mixture of two isomers is purified by a chiral high-performance liquid chromatography column (IC column: CHIRALPAK IC, 2×25 cm (filler 5 μm); mobile phase A: n-hexane (10 mM ammonia-methanol), and mobile phase B: ethanol; flow rate: 20 ml/min; gradient: 30%B within 12 min, isogradient; detection wavelength: 220/254 nm; and column temperature: 25°C) to obtain:

isomer 67-1 (91 mg, 36.98%) having a HPLC retention time of 11.2 min:

$^1$HNMR (400 MHz, DMSO-$d_6$, *ppm):* δ 10.84 (s, 1H), 9.27 (s, 1H), 8.50 (s, 1H), 8.17 (s, 1H), 7.53 (m, 1H), 7.38 - 7.29 (m, 2H), 7.27 (d, *J* = 8.2 Hz, 1H), 6.97 (d, *J* = 8.1 Hz, 1H), 3.31 (s, 3H), 3.03 (m, 2H), 2.84 (m, 4H), 2.32 (m, 2H), 1.80 (m, 6H), 1.74 (m, 2H), 1.55 (m, 2H). MS(ESI+): 576.2(M+H).

Isomer 67-2 (93.4mg, 37.96%) having a HPLC retention time of 8.6min:

$^1$HNMR (400 MHz, DMSO-$d_6$, *ppm*) : δ 10.84 (s, 1H), 9.27 (s, 1H), 8.50 (s, 1H), 8.17 (s, 1H), 7.53 (ddd, *J* = 13.9, 8.8, 3.1 Hz, 1H), 7.38 - 7.29 (m, 2H), 7.27 (d, *J* = 7.9 Hz, 1H), 6.97 (d, *J* = 8.2 Hz, 1H), 3.31 (s, 3H), 3.03 (m, 2H), 2.84 (m, 4H), 2.32 (m, 2H), 1.80 (m, 6H), 1.74 (m, 2H), 1.55 (m, 2H). MS(ESI+): 576.2(M+H).

**Example 68** Preparation of compounds 68-1 and 68-2

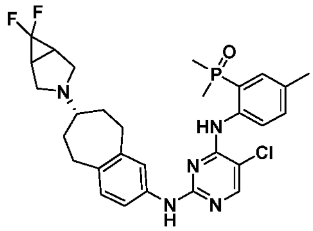 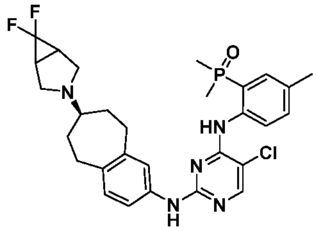

According to the preparation method of Example 58, (2-amino-5-fluorophenyl) dimethyl phosphine oxide in step a) is replaced with (2-amino-5-methylphenyl) dimethyl phosphine oxide, and 2-azabicyclo[3.1.0]hexane hydrochloride in step c) with 6,6-difluoro-3-azabicyclo[3.1.0]hexane hydrochloride. The crude product is purified by a preparative high-performance liquid phase under the following conditions (column: Xselect CSH-OBD column 30×150 mm, with a filler particle size of 5 μm; mobile phase A: water (10 mmol/L ammonium bicarbonate), mobile phase B: acetonitrile; flow rate: 60 ml/min; gradient: 45%B-75%B within 8 min; wavelength: 210 nm; retention time of isomer mixture: 6.57 min) to obtain a mixture of two isomers.

The mixture of two isomers is purified by a chiral high-performance liquid chromatography column (column: CHIRALPAK IE, 2×25 cm (filler 5 μm); mobile phase A: n-hexane (10 mM ammonia-methanol), and mobile phase B: ethanol; flow rate: 15 ml/min; gradient: 50%B within 25 min, isogradient; detection wavelength: 220/254 nm; and column temperature: 25°C)

isomer 68-1 (85 mg) having a HPLC retention time of 19.4 min were obtained:

$^1$H NMR: (400 MHz, DMSO-*$d_6$*, *ppm*): δ 10.86 (s, 1H),9.24 (s, 1H),8.38 (s, 1H),8.15 (s, 1H),7.43 (d, *J* = 15.2 Hz, 2H), 7.29 (m, 2H), 6.96 (m, 1H), 3.41 (s, 3H), 3.05 (s, 2H), 2.86 (m, 4H), 2.34 (m, 5H), 1.76 (m, 8H), 1.54 (m 2H). MS(ESI+): 572.2(M+H).

Isomer 68-2 (85 mg) having a HPLC retention time of 14.9min were obtained :

$^1$H NMR: (400 MHz, DMSO-$d_6$, *ppm):* δ 10.83 (s, 1H), 9.22 (s, 1H), 8.37 (s, 1H), 8.14 (d, *J* = 0.7 Hz, 1H), 7.47 - 7.36 (m, 2H), 7.28 (m, 2H), 6.96 (m, 1H), 3.50 (s, 3H),3.03 (m, 2H), 2.85 (m, 4H), 2.34 (m, 5H), 1.76 (m, 8H), 1.55 (m, 2H). MS(ESI+): 572.2(M+H).

**Example 69** Preparation of compounds 69-1 and 69-2

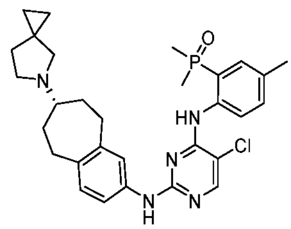 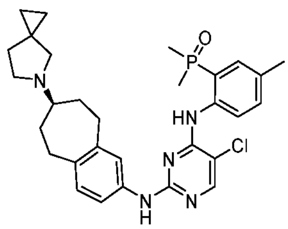

According to the preparation method of Example 58, (2-amino-5-fluorophenyl) dimethyl phosphine oxide in step a) is replaced with (2-amino-5-methylphenyl) dimethyl phosphine oxide, and 2-azabicyclo[3.1.0]hexane hydrochloride in step c) is replaced with 5-azaspiro[2.4]heptane hydrochloride. The crude product is purified by reversed-phase high-performance liquid chromatography (column: Xselect CSH OBD column, 30×150 mm, with a filler particle size of 5 μm; mobile phase A: 10 mmol/L ammonium bicarbonate aqueous solution, and mobile phase B: acetonitrile; flow rate: 60 mL /min; gradient: 45%B-75%B, 8 min; detection wavelength: 210 nm; retention time: 6.57 min) to obtain 60 mg of an isomer mixture.

Chiral resolution is performed on the isomer mixture by chiral liquid chromatography (column: CHIRALPAK IF, 2 cm×25 cm, with a filler particle size of 5 μm; mobile phase A: methyl tert-butyl ether (10 mM ammonia-methanol), and mobile phase B: ethanol; flow rate: 15 ml/min;

gradient: 30%B within 24 min, isogradient; detection wavelength: 220/254 nm; column temperature: 25°C) to obtain:

isomer 69-1 (76mg) having a HPLC retention time of 10.8min.

$^1$H NMR (400 MHz, DMSO-*$d_6$*, ppm): δ 10.93 (s, 1H), 9.26 (s, 1H), 8.40 (s, 1H), 8.15 (s, 1H), 7.49 - 7.38 (m, 2H), 7.34 -7.23 (m, 2H), 6.97 (d, J = 8.1 Hz, 1H),3.31 (s, 5H), 2.84 (m, 4H), 2.34 (s, 3H), 2.01-1.88 (m, 1H), 1.77 (m, 9H), 1.47 (m, 2H), 0.53 (m, 4H).MS(ESI+): 550.2(M+H).Isomer 69-2 (90 mg) having a HPLC retention time of 19.6 min.

$^1$H NMR (400 MHz, DMSO-*$d_6$*, *ppm*): δ 10.93 (s, 1H), 9.26 (s, 1H), 8.40 (s, 1H), 8.15 (s, 1H), 7.49-7.38 (m,

2H), 7.34-7.23 (m, 2H), 6.97 (d, *J* = 8.1 Hz, 1H),3.31 (m, 5H), 2.84 (m, 4H), 2.34 (s, 3H), 2.01-1.88 (m, 1H), 1.77 (m, 9H), 1.47 (m, 2H), 0.53 (m, 4H).MS(ESI+): 550.2(M+H).

**Example 70** Preparation of compounds 70-1 and 70-2

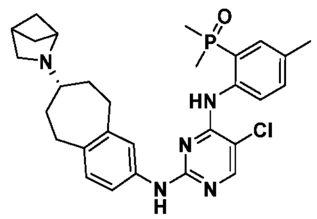

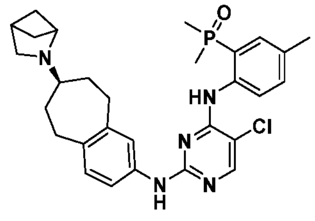

According to the preparation method of Example 58, (2-amino-5-fluorophenyl) dimethyl phosphine oxide in step a) is replaced with (2-amino-5-methylphenyl) dimethyl phosphine oxide, and 2-azabicyclo[3.1.0]hexane hydrochloride in step c) is replaced with 2-azabicyclo[2.1.1]hexane hydrochloride. The crude product is purified by a preparative high-performance liquid phase under the following conditions (column: Xselect CSH-OBD column 30×150 mm, with a filler particle size of 5 μm; mobile phase A: water (10 mmol/L ammonium bicarbonate), mobile phase B: acetonitrile; flow rate: 60 ml/min; gradient: 10%B-40%B within 10 min; wavelength: 254/220 nm) to obtain 160 mg of a mixture of two isomers.

The mixture of two isomers is purified by a chiral high-performance liquid chromatography column (column: CHIRALPAK IG, 2×25 cm (filler 5 μm); mobile phase A: methyl tert-butyl ether (10 nM ammonia-methanol solution), mobile phase B: ethanol; flow rate: 20 ml/min; gradient: 30%B within 11 min, isogradient; detection wavelength: 220/254 nm; and column temperature: 25°C) to obtain:

isomer 70-1 (mg) having a HPLC retention time of 6.5 min.

$^1$H-NMR (400MHz, DMSO-$d_6$, *ppm*): δ 10.92 (s, 1H), 9.25 (s, 1H), 8.40 (s, 1H), 8.15 (s, 1H), 7.50 - 7.36 (m, 2H), 7.30 (ddd, *J* = 10.8, 8.2, 2.1 Hz, 2H), 6.98 (d, *J* = 8.1 Hz, 1H), 3.70 (s, 1H), 2.86 (d, *J* = 35.4 Hz, 2H), 2.72 (dt, *J* = 6.5, 3.0 Hz, 3H), 2.64 - 2.53 (m, 3H), 2.34 (s, 3H), 1.92 (d, *J* = 18.7 Hz, 2H), 1.77 (d, *J*= 13.5 Hz, 6H), 1.64 (s, 2H), 1.54 - 1.19 (m, 4H). MS(ESI+): 536.2(M+H).

Isomer 70-2 (mg) having a HPLC retention time of 9.2 min;

$^1$HNMR (400 MHz, DMSO-$d_6$, *ppm):* δ 10.92 (s, 1H), 9.25 (s, 1H), 8.40 (s, 1H), 8.15 (s, 1H), 7.44 (dd, *J* = 12.6, 2.2 Hz, 2H), 7.30 (ddd, *J* = 11.0, 8.1, 2.2 Hz, 2H), 6.98 (d, *J* = 8.1 Hz, 1H), 3.70 (s, 1H), 2.89 (s, 2H), 2.77 - 2.65 (m, 3H), 2.65 - 2.54 (m, 3H), 2.34 (s, 3H), 1.92 (d, *J* = 18.1 Hz, 2H), 1.77 (d, *J* = 13.5 Hz, 6H), 1.64 (s, 2H), 1.54 - 1.21 (m, 4H). MS(ESI+): 536.2(M+H).

**Example 71** Preparation of compounds 71-1 and 71-2

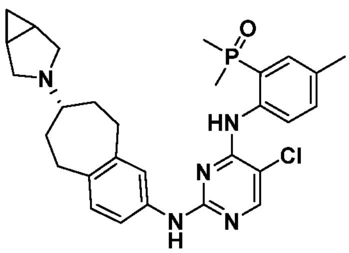

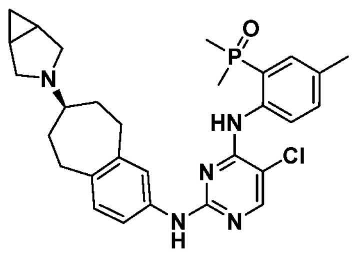

According to the preparation method of Example 58, (2-amino-5-fluorophenyl) dimethyl phosphine oxide in step a) is replaced with (2-amino-5-methylphenyl) dimethyl phosphine oxide, and 2-azabicyclo[3.1.0]hexane hydrochloride in step c) is replaced with 3-azabicyclo[3.1.0]hexane hydrochloride. The crude product is purified by a preparative high-performance liquid phase under the following conditions (column: Xselect CSH-OBD column 30×150 mm, with a filler particle size of 5 μm; mobile phase A: water (10 mmol/L ammonium bicarbonate), mobile phase B: acetonitrile; flow rate: 60 ml/min; gradient: 35%B-85%B within 8 min; wavelength: 254/220 nm) to obtain 140 mg of a mixture of two isomers.

The mixture of two isomers is purified by a chiral high-performance liquid chromatography column (column: CHIRALPAK IE, 2×25 cm (filler 5 μm); mobile phase A: methyl tert-butyl ether (0.1% diethylamine), mobile phase B: ethanol; flow rate: 18 ml/min; gradient: 20%B within 17 min, isogradient; detection wavelength: 220/254 nm and column temperature: 25°C) to obtain:
isomer 71-1 (45.8 mg) having a HPLC retention time of 12.4 min:
$^1$HNMR (400 MHz, DMSO-$d_6$, *ppm*): δ 10.90 (s, 1H), 9.24 (s, 1H), 8.37 (d, *J* = 5.8 Hz, 1H), 8.15 (s, 1H), 7.47 - 7.37 (m, 2H), 7.28 (m, 2H), 6.95 (d, *J* = 8.2 Hz, 1H), 3.32 (s, 2H), 2.95 (d, *J* = 8.3 Hz, 2H), 2.87 (s, 2H), 2.42 - 2.36 (m, 3H), 2.34 (s, 3H), 1.76 (m, 8H), 1.52 (s, 2H), 1.38 (s, 2H), 0.60 (m, 1H), 0.32 (m, 1H). MS(ESI+): 536.2(M+H).
Isomer 71-2 (46.2 mg) having a HPLC retention time of 14.5 min:
$^1$HNMR (400 MHz, DMSO-$d_6$, *ppm*): δ 10.90 (s, 1H), 9.24 (s, 1H), 8.38 (s, 1H), 8.15 (s, 1H), 7.47 - 7.37 (m, 2H), 7.34 - 7.22 (m, 2H), 6.95 (d, *J* = 8.1 Hz, 1H), 3.33 (s, 2H), 2.95 (d, *J* = 8.3 Hz, 2H), 2.90 - 2.82 (m, 2H), 2.40 (s, 3H), 2.34 (s, 3H), 1.76 (m, 8H), 1.52 (m, 2H), 1.41 - 1.35 (m, 2H), 0.60 (m, 1H), 0.32 (m, 1H). MS(ESI+): 536.2(M+H).

**Example 72** Preparation of compounds 72-1, 72-2, 72-3 and 72-4

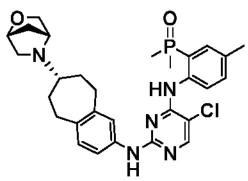 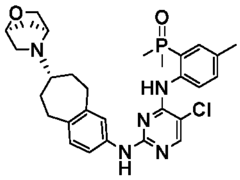 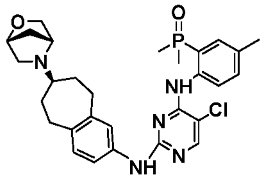 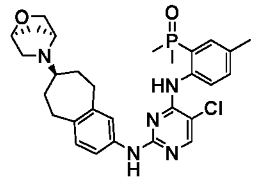

According to the preparation method of Example 58, 2-azabicyclo[3.1.0]hexane hydrochloride in step c) is replaced with 2-oxa-5-azabicyclo[2.2.1]heptane hydrochloride. The crude product is purified by reversed-phase high-performance liquid chromatography (column: XBridge-Prep-OBD C18 column, 30×150 mm, with a filler particle size of 5 μm; mobile phase A: 10 mmol/L ammonium bicarbonate aqueous solution, mobile phase B: acetonitrile; flow rate: 60 mL /min; gradient: 25%B-50%B, 8 min; detection wavelength: 254/220 nm) to obtain 275 mg of an isomer mixture.
Chiral resolution is performed on the isomer mixture by chiral liquid chromatography (column: CHIRALPAK IG, 2 cm×25 cm, with a filler particle size of 5 μm; mobile phase A: methyl tert-butyl ether (10 mM ammonia-methanol), mobile phase B: ethanol; flow rate: 20 ml/min; gradient: 15%B within 45 min, isogradient; detection wavelength: 220/254 nm; column temperature: 25°C) to obtain:
isomer 72-1 (36.4 mg) having a HPLC retention time of 1.67 min.
$^1$H NMR (400 MHz, DMSO-$d_6$, *ppm*): (400 MHz, DMSO-$d_6$, *ppm*): δ 10.90 (s, 1H), 9.24 (s, 1H), 8.40 (dd, *J* = 8.6, 4.4 Hz, 1H), 8.15 (s, 1H), 7.47 - 7.38 (m, 2H), 7.29 (ddd, *J* = 12.4, 8.3, 2.2 Hz, 2H), 6.96 (d, *J* = 8.1 Hz, 1H), 4.33 (t, *J* = 1.9 Hz, 1H), 3.90 (d, *J* = 7.5 Hz, 1H), 3.70 (s, 1H), 3.59 - 3.50 (m, 1H), 2.99 (dd, *J* = 9.6, 1.8 Hz, 1H), 2.86 (s, 2H), 2.71 (s, 1H), 2.53 (s, 1H), 2.48 (s, 1H), 2.34 (s, 4H), 1.83 - 1.71 (m, 9H), 1.65 - 1.58 (m, 1H), 1.54 - 1.27 (m, 2H). MS(ESI+): 552(M+H).
Isomer 72-2 (56.8 g) having a HPLC retention time of 6.119 min.
$^1$H NMR (400 MHz, DMSO-$d_6$, *ppm*): (400 MHz, DMSO-$d_6$, *ppm*): δ 10.90 (s, 1H), 9.25 (s, 1H), 8.39 (dd, *J* = 9.0, 4.5 Hz, 1H), 8.15 (s, 1H), 7.43 (dd, *J* = 14.2, 2.2 Hz, 2H), 7.29 (ddd, *J* = 13.1, 8.3, 2.2 Hz, 2H), 6.96 (d, *J* = 8.1 Hz, 1H), 4.33 (t, *J* = 1.9 Hz, 1H), 3.90 (d, *J* = 7.5 Hz, 1H), 3.70 (s, 1H), 3.53 (dd, *J* = 7.6, 1.7 Hz, 1H), 3.01 (dd, *J* = 9.6, 1.8 Hz, 1H), 2.86 (s, 2H), 2.72 (d, *J* = 8.8 Hz, 1H), 2.57 (d, *J* = 11.9 Hz, 1H), 2.47 (d, *J* = 15.3 Hz, 1H), 2.33 (s, 4H), 1.76 (d, *J* = 13.5 Hz, 9H), 1.65 - 1.58 (m, 1H), 1.39 (s, 2H). MS(ESI+): 552(M+H).
Isomer 72-3 (60.3 g) having a HPLC retention time of 7.224 min.
$^1$H NMR (400 MHz, DMSO-$d_6$, *ppm*): δ 10.92 - 10.87 (m, 1H), 9.24 (s, 1H), 8.40 (d, *J* = 7.6 Hz, 1H), 8.15 (s, 1H), 7.47 - 7.38 (m, 2H), 7.29 (ddd, *J* = 11.7, 8.3, 2.1 Hz, 2H), 6.96 (d, *J* = 8.1 Hz, 1H), 4.33 (s, 1H), 3.90 (d, *J* = 7.5 Hz, 1H), 3.70 (s, 1H), 3.54 (d, *J* = 7.4 Hz, 1H), 2.99 (dd, *J* = 9.5, 1.6 Hz, 1H), 2.90 - 2.83 (m, 2H), 2.72 (d, *J* = 8.5 Hz, 1H), 2.55 (d, *J* = 8.1 Hz, 1H), 2.48 (s, 1H), 2.34 (s, 4H), 1.76 (d, *J* = 13.5 Hz, 9H), 1.62 (d, *J* = 9.3 Hz, 1H), 1.39 (s, 2H). MS(ESI+): 552(M+H)
Isomer 72-4 (32.2 mg) having a HPLC retention time of 9.412 min.
$^1$H NMR (400 MHz, DMSO-$d_6$, *ppm*): (400 MHz, *DMSO-$d_6$, ppm*): δ 10.89 (s, 1H), 9.24 (s, 1H), 8.39 (dd, *J* = 8.3, 4.3 Hz, 1H), 8.15 (s, 1H), 7.43 (dd, *J* = 14.4, 2.1 Hz, 2H), 7.29 (ddd, *J* = 14.3, 8.3, 2.2 Hz, 2H), 6.96 (d, J = 8.1 Hz, 1H), 4.34 (t, *J* = 2.0 Hz, 1H), 3.90 (d, *J* = 7.5 Hz, 1H), 3.71 (s, 1H), 3.54 (dd, *J* = 7.6, 1.7 Hz, 1H), 3.01 (dd, *J* = 9.6, 1.8 Hz, 1H), 2.86 (s, 2H), 2.73 (d, *J* = 8.6 Hz, 1H), 2.61 - 2.54 (m, 1H), 2.47 (s, 1H), 2.36 (d, *J* = 9.2 Hz, 4H), 1.76 (d, *J* = 13.5 Hz, 9H), 1.66 - 1.59 (m, 1H), 1.39 (s, 2H). MS(ESI+): 552(M+H).

**Example 73** (S)-(2-((5-chloro-2-((7-(pyrrolidin-1-yl)-6,7,8,9-tetrahydro-5H-benzo[7] annulen-2-yl)amino)pyrimidin-4-yl)amino)-5-methylpyridin-3-yl)dimethyl phosphine oxide

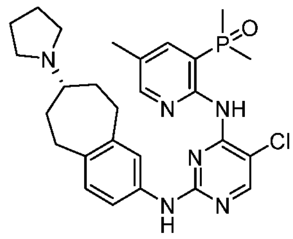

According to the preparation method of Example 47, quinoxaline-6-amine in step a) is replaced with 2-amino-5-methylpyridine.

$^1$H NMR (400 MHz, DMSO-$d_6$, *ppm*): δ 9.32 (s, 1H), 8.47 (s, 1H), 7.95 (d, J = 13.2 Hz, 1H), 7.66 (s, 1H), 7.37 (dd, J = 7.9, 2.3 Hz, 1H), 7.11 (t, J = 7.8 Hz, 1H), 7.40 (s, 1H), 6.96 (t, J = 8.3 Hz, 2H), 2.75 (d, J = 8.1 Hz, 1H), 2.36 (s, 3H), 2.29 (s, 2H), 1.97 (s, 3H), 1.91 - 2.52 (m, 3H), 1.79 (s, 2H), 1.75 (s, 4H), 1.46 (s, 2H). MS(ESI+): 525.4(M+H).

**Example 74** (S)-(2-((5-chloro-2-((7-(pyrrolidin-1-yl)-6,7,8,9-tetrahydro-5H-benzo[7]annulen-2-yl)amino)pyrimidin-4-yl)amino)-5-(trifluoromethyl)phenyl)dimethyl phosphine oxide

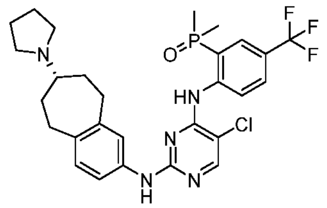

According to the preparation method of Example 47, quinoxaline-6-amine in step a) is replaced with 4-trifluoromethylaniline.

$^1$H NMR (400 MHz, DMSO-*d6*, *ppm*) : δ 9.74 (s, 1H), 9.31 (s, 1H), 8.18 (s, 1H), 7.70 (dd, J = 11.5, 4.3 Hz, 1H), 7.59 (dd, J = 7.5, 3.0 Hz, 1H), 7.10 (t, J = 8.5 Hz, 2H), 7.06 (d, J = 7.6 Hz, 1H), 6.81 (d, J = 7.9 Hz, 1H) , 2.83 (s, 7H), 2.62 (s, 2H), 2.04 (s, 2H), 1.88 (d, J = 13.8 Hz, 6H), 1.77 (s, 4H), 1.49 (dd, J = 14.1, 3.7 Hz, 2H). MS(ESI+): 578.2(M+H).

**Example 75** (S)-(2-((5-chloro-2-((7-(pyrrolidin-1-yl)-6,7,8,9-tetrahydro-5H-benzo[7] annulen-2-yl)amino)pyrimidin-4-yl)amino)-5-(difluoromethoxyl)phenyl)dimethyl phosphine oxide

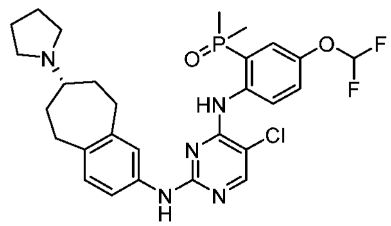

According to the preparation method of Example 10, (2-aminophenyl) dimethyl phosphine oxide in step a) is replaced with (2-amino-5-difluoromethoxyphenyl) dimethyl phosphine oxide.

$^1$H NMR (400 MHz, DMSO-$d_6$, *ppm*) δ 10.96 (s, 1H), 9.30 (s, 1H), 8.55 (s, 1H), 8.28 (s, 0.5H), 8.18 (s, 1H), 7.52 - 7.45 (m, 1H), 7.44 (d, J = 2.9 Hz, 0.25H), 7.39 (d, J = 2.3 Hz, 1H), 7.34 - 7.23 (m, 2H), 7.09 (s, 0.25H), 6.98 (d, J = 8.1 Hz, 1H), 2.87-2.80 (m, 2H), 2.65 (s, 7H), 1.91 (s, 2H), 1.82 (s, 3H), 1.79 (s, 3H), 1.72 (q, J = 3.2 Hz, 4H), 1.51

(s, 2H). MS(ESI+): 576.2(M+H).

**Example 76** (S)-(2-((5-chloro-2-((7-(pyrrolidin-1-yl)-6,7,8,9-tetrahydro-5H-benzo[7]annulen-2-yl)amino)pyrimidin-4-yl)amino)-5-(trifluoromethoxyl)phenyl)dimethyl phosphine oxide

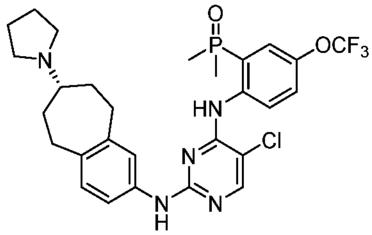

According to the preparation method of Example 10, (2-aminophenyl) dimethyl phosphine oxide in step a) is replaced with (2-amino-5-trifluoromethoxyphenyl) dimethyl phosphine oxide.

$^1$H NMR (400 MHz, DMSO-$d_6$, *ppm*): δ 11.18 (s, 1H), 9.35 (s, 1H), 8.66 (s, 1H), 8.22 (d, *J* = 8.9 Hz, 1H), 7.66 (d, *J* = 14.0 Hz, 1H), 7.52 - 7.37 (m, 2H), 7.30 (d, *J* = 7.3 Hz, 1H), 7.00 (d, *J* = 8.1 Hz, 1H), 2.77 (d, *J* = 75.5 Hz, 7H), 2.57 (d, *J* = 11.6 Hz, 2H), 2.06 (s, 2H), 1.91 - 1.69 (m, 10H), 1.49 (s, 2H). MS(ESI+): 594.2(M+H).

**Example 77** (S)-4-((5-chloro-2-((7-(pyrrolidin-1-yl)-6,7,8,9-tetrahydro-5H-benzo[7]annulen- 2-yl)amino)pyrimidin-4-yl)amino)-3-(dimethylphosphoryl)-N-methylbenzamide

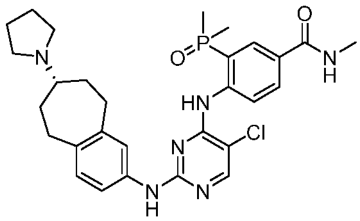

According to the preparation method in step b) to step d) of Example 47, 5-iodoquinoxaline-6-amine in step b) is replaced with 4-amino-3-iodo-N-methylbenzamide.

1H NMR (400 MHz, DMSO-$d_6$, *ppm*): δ9.38 (s, 1H), 8.72 (s, 1H), 8.51 (q, J = 4.5 Hz, 1H), 8.29 (s, 1H), 8.09 - 7.88 (m, 2H), 7.47 (d, J = 2.3 Hz, 1H), 7.29 (d, J = 8.2 Hz, 1H), 7.02 (d, J = 8.1 Hz, 1H), 2.82 (d, J = 4.5 Hz, 5H), 2.76 (s, 5H), 2.58 (t, J = 12.8 Hz, 2H), 2.01 (s, 2H), 1.87 (s, 3H), 1.83 (s, 3H), 1.76 (d, J = 5.9 Hz, 4H), 1.49 (s, 2H). MS(ESI+): 567.2(M+H).

**Example 78** (S)-4-((5-chloro-2-((7-(pyrrolidin-1-yl)-6,7,8,9-tetrahydro-5H-benzo[7]annulen- 2-yl)amino)pyrimidin-4-yl)amino)-3-(dimethylphosphoryl)benzamide

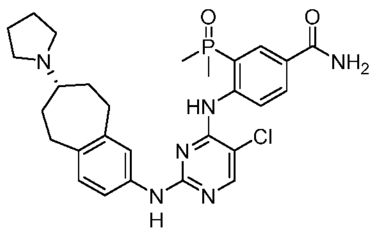

(S)-4-((5-chloro-2-((7-(pyrrolidin-1-yl)-6,7,8,9-tetrahydro-5H-benzo[7]annulen-2-yl)amino)pyrimidin-4-yl)amino)-3-(dimethylphosphoryl)benzonitrile (120 mg), 2N sodium hydroxide (2 ml) and ethanol (10 mL) are added to a 30 mL microwave tube. The microwave reaction is performed at 150°C for 4 h under the protection of nitrogen, and then stopped. The reaction solution is concentrated to dryness, and the residue is purified by silica-gel column chromatography to obtain 40 mg of the title product.

$^1$H NMR (400 MHz, DMSO-*d*$_6$, *ppm*): δ 11.35 (s, 1H), 9.35 (s, 1H), 8.59 (s, 1H), 8.21 (s, 1H), 8.09-7.92 (m, 2H), 7.45 (s, 1H), 7.25 (s, 1H), 7.01 (d, J = 8.0 Hz, 1H), 3.39 (s, 2H), 2.91 - 2.55 (m, 9H), 2.08 (br, 2H), 1.87 - 1.71 (m, 10H), 1.52 (br, 2H). MS(ESI+): 553.2(M+H).

**Example 79** (S)-2-((5-chloro-2-((7-(pyrrolidin-1-yl)-6,7,8,9-tetrahydro-5H-benzo[7]annulen- 2-yl)amino)pyrimidin-4-yl)amino)-5-(dimethylphosphoryl)-N-methylbenzamide

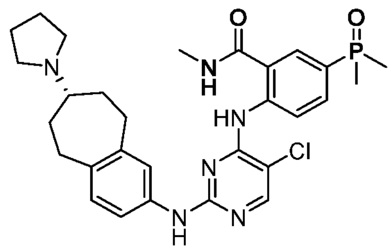

According to the preparation method in step b) to step d) of Example 47, 5-iodoquinoxaline-6-amine in step b) is replaced with 2-amino-5-bromo-N-methylbenzamide.

$^1$H NMR (400 MHz, DMSO-*d*$_6$, *ppm*): δ9.41 (s, 1H), 8.95 (q, J = 4.6 Hz, 1H), 8.84 (d, J = 8.6 Hz, 1H), 8.26 (d, J = 2.2 Hz, 2H), 8.07 (d, J = 11.5 Hz, 1H), 7.79 (t, J = 9.8 Hz, 1H), 7.49 (d, J = 2.3 Hz, 1H), 7.26 (s, 1H), 7.03 (d, J = 8.1 Hz, 1H), 2.84 (d, J = 4.4 Hz, 5H), 2.71 (s, 5H), 2.57 (s, 2H), 1.98 (s, 2H), 1.81 - 1.70 (m, 7H), 1.68 (d, J = 1.9 Hz, 3H), 1.53 (s, 2H). MS(ESI+): 567.2(M+H).

**Example 80** (S)-(5-((5-chloro-2-((7-(pyrrolidin-1-yl)-6,7,8,9-tetrahydro-5H-benzo[7]annulen-2-yl)amino)pyrimidin-4-yl)amino)-2-methylpyridin-4-yl)dimethyl phosphine oxide

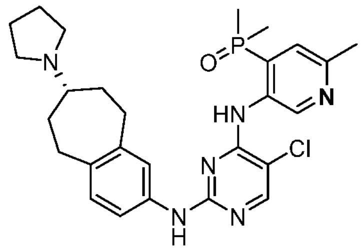

According to the preparation method in step b) to step d) of Example 47, 5-iodoquinoxaline-6-amine in step b) is replaced with 4-iodo-6-methylpyridin-3-amine.

$^1$H NMR (400 MHz,$CDCl_3$,*ppm*) : δ 8.86 (dd, J = 8.8, 5.5 Hz, 1H), 8.44 (s, 1H), 8.06 (s, 1H), 7.44 (s, 1H), 7.34 (dd, J = 8.1, 2.3 Hz, 1H), 7.29 (s, 1H), 7.13 (dd, J = 8.7, 2.0 Hz, 1H), 7.06 (d, J = 8.1 Hz, 1H), 3.40 (m, 2H), 3.28 (m, 3H), 2.87 (m, 1H), 2.78 (m, 3H), 2.52 (s, 3H), 2.41 (m, 2H), 2.04 (m, 4H), 1.87 (m, 6H), 1.65 -1.51 (m, 2H). MS(ESI+): 525.1(M+H).

**Example 81** (S)-(3-((5-chloro-2-((7-(pyrrolidin-1-yl)-6,7,8,9-tetrahydro-5H-benzo[7] annulen-2-yl)amino)pyrimidin-4-yl)amino)-6-methylpyridin-2-yl)dimethyl phosphine oxide

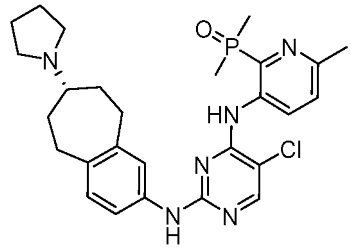

According to the preparation method in step b) to step d) of Example 47, 5-iodoquinoxaline-6-amine in step b) is replaced with 4-iodo-6-methylpyridin-3-amine.

$^1H$ NMR (400 MHz, DMSO-$d_6$, *ppm*) δ 9.37 (s, 1H), 8.97 (s, 1H), 8.29 (s, 1H), 8.20 (s, 1H), 7.42 (d, J = 2.3 Hz, 1H), 7.32 (td, J = 5.4, 2.6 Hz, 2H), 7.04 (d, J = 8.1 Hz, 1H), 2.93 (m, 4H), 2.74 (m, 1H), 2.63 (m, 2H), 2.51 (m, 5H), 2.12 (m, 2H), 1.78 (m, 10H), 1.49 (m, 2H). MS(ESI+): 525.1(M+H).

**Example 82** (S)-(2-((5-chloro-2-((7-(pyrrolidin-1-yl)-6,7,8,9-tetrahydro-5H-benzo[7]annulen-2-yl)amino)pyrimidin-4-yl)amino)-5-(trifluoromethyl)pyridin-3-yl)dimethyl phosphine oxide

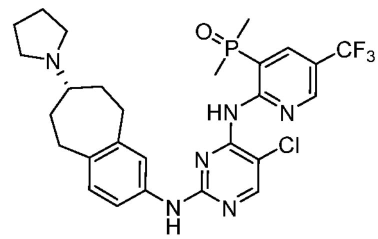

According to the preparation method of Example 47, quinoxaline-6-amine in step a) is replaced with 5-trifluoromethylpyridinediamine.

$^1H$ NMR (400 MHz, DMSO-$d_6$, *ppm*): δ 9.47 (s, 1H), 8.85 (s, 1H), 8.39 (dd, J = 13.3, 2.5 Hz, 1H), 8.33 (s, 1H), 8.25 (s, 1H), 7.60 - 7.49 (m, 2H), 6.97 (d, J = 8.1 Hz, 1H), 2.84 (m, 2H), 2.70 (m, 5H), 1.89 (m, 8H), 1.73 (m, 4H), 1.49 (m, 2H). MS(ESI+): 579.2(M+H).

**Example 83 Preparation of compounds 83-1 and 83-2**

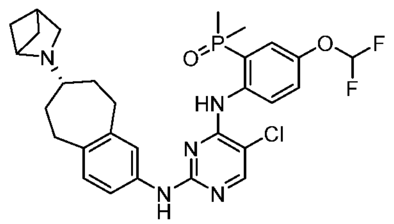

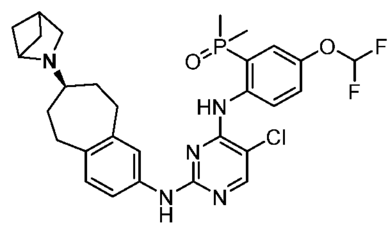

According to the preparation method of Example 58, (2-amino-5-fluorophenyl) dimethyl phosphine oxide in step a) is replaced with (2-amino-5-difluoromethoxyphenyl) dimethyl phosphine oxide, and 2-azabicyclo[3.1.0]hexane hydrochloride in step c) is replaced with 2-azabicyclo[2.1.1]hexane hydrochloride.

The crude product is purified by a preparative high-performance liquid phase under the following conditions (column: XBridge Prep OBD C18 column, 30×150 mm, with a filler particle size of 5 μm; mobile phase A: water (10 mmol/L ammonium bicarbonate), mobile phase B: acetonitrile; flow rate: 60 ml/min; gradient: 15%B-47%B within 8 min; wavelength: 254/220 nm; and column temperature: 25°C) to obtain 87 mg of a mixture of two isomers.

The mixture of two isomers is purified by a chiral high-performance liquid chromatography column (column: CHIRALPAK IC, 2×25 cm (filler 5 μm); mobile phase A: methyl tert-butyl ether (0.1% diethylamine), mobile phase B: ethanol; flow rate: 20 ml/min; gradient: 20%B within 12 min, isogradient; detection wavelength: 220/254 nm; and column

temperature: 25°C) to obtain:

isomer 83-1 (23.8 mg) having a HPLC retention time of 6.4 min:

$^1$H NMR (400MHz, DMSO-$d_6$, *ppm*):δ 10.96 (s, 1H), 9.28 (s, 1H), 8.56 (s, 1H), 8.18 (s, 1H), 7.50 - 7.42 (m, 1.25H), 7.40 (d, J = 2.3 Hz, 1H), 7.34 - 7.23 (m, 2.5H), 7.08 (s, 0.25H), 6.98 (d, *J* = 8.1 Hz, 1H), 3.68 (s, 1H), 2.90 (s, 2H), 2.76 - 2.63 (m, 2H), 2.58 (s, 2H), 1.92 (s, 2H), 1.81 (d, *J* = 13.7 Hz, 6H), 1.63 (s, 2H), 1.35 (d, *J* = 4.0 Hz, 4H), 1.24 (s, 2H). MS(ESI+): 588.2(M+H).

Isomer 83-2 (23.8 mg) having a HPLC retention time of 10.4 min:

$^1$H NMR (400MHz, DMSO-$d_6$, *ppm*): δ10.96 (s, 1H), 9.28 (s, 1H), 8.56 (s, 1H), 8.18 (s, 1H), 7.50 - 7.43 (m, 1H), 7.40 (d, *J* = 2.3 Hz, 0.25H), 7.32 - 7.25 (m, 2.5H), 7.08 (s, 0.25H), 6.98 (d, *J* = 8.2 Hz, 1H), 3.71 (s, 1H), 2.89 (s, 2H), 2.75 - 2.69 (m, 2H), 2.58 (d, *J* = 11.5 Hz, 2H), 2.05 - 1.86 (m, 2H), 1.81 (d, *J* = 13.7 Hz, 6H), 1.64 (s, 2H), 1.35 (d, *J* = 4.6 Hz, 4H), 1.24 (s, 2H). MS(ESI+): 588.2(M+H).

**Example 84** Preparation of compounds 84-1 and 84-2

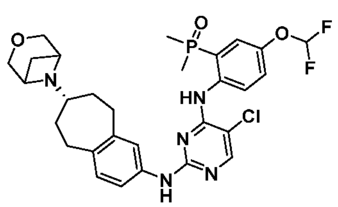 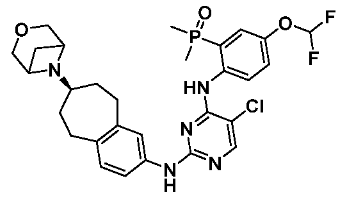

According to the preparation method of Example 58, (2-amino-5-fluorophenyl)dimethyl phosphine oxide in step a) is replaced with (2-amino-5-difluoromethoxyphenyl)dimethyl phosphine oxide, and 2-azabicyclo[3.1.0]hexane hydrochloride in step c) is replaced with 3-oxa-6-azabicyclo[3.1.1]heptane hydrochloride. The crude product is purified by a preparative high-performance liquid phase under the following conditions (column: XBridge Prep OBD C18 column, 30×150 mm, with a filler particle size of 5 μm; mobile phase A: water (10 mmol/L ammonium bicarbonate), mobile phase B: acetonitrile; flow rate: 60 ml/min; gradient: 17%B-45%B within 12 min; isomer mixture retention time: 11.57 min; wavelength: 254/220 nm; column temperature: 25° C) to obtain a mixture of two isomers. The mixture of two isomers is purified by a chiral high-performance liquid chromatography column (column: CHIRALPAK IG, 2×25 cm (filler 5 μm); mobile phase A: n-hexane (0.1% diethylamine), mobile phase B: ethanol; flow rate: 20 ml/min; gradient: 50%B within 18 min, isogradient; detection wavelength: 220/254 nm; and column temperature: 25°C) to obtain:

isomer 84-1 (24 mg) having a HPLC retention time of 12.1 min:

$^1$H NMR (400 MHz, DMSO-d$_6$, *ppm*):*δ* 10.94 (s, 1H), 9.29 (s, 1H), 8.54 (s, 1H), 8.18 (s, 1H), 7.54 - 7.06 (m,5H), 6.97 (d, *J* = 8.1 Hz, 1H), 4.13 (dd, *J* = 10.6, 2.8 Hz, 2H), 3.58 (d, *J* = 10.9 Hz, 2H), 3.52 (d, *J* = 6.0 Hz, 2H), 3.11 (s, 1H), 2.80 (t, *J* = 11.1 Hz,1H), 2.65 (s, 3H), 2.40 - 2.29 (m, 1H), 1.80 (d, *J* = 13.6 Hz, 8H), 1.68 (d, *J* = 7.8 Hz, 1H), 1.09 (s, 2H) . MS(ESI+): 604.2(M+H).

Isomer 84-2 (23 mg) having a HPLC retention time of 15.5 min:

$^1$H NMR (400 MHz, DMSO-$d_6$, *ppm*): δ 10.94 (s, 1H), 9.29 (s, 1H), 8.54 (s, 1H), 8.18 (s, 1H), 7.54 - 7.06 (m,5H), 6.97 (d, *J* = 8.1 Hz, 1H), 4.13 (dd, *J* = 10.6, 2.8 Hz, 2H), 3.58 (d, *J* = 10.9 Hz, 2H), 3.52 (d, *J* = 6.0 Hz, 2H), 3.11 (s, 1H), 2.80 (t, *J* = 11.1 Hz,1H), 2.65 (s, 3H), 2.40 - 2.29 (m, 1H), 1.80 (d, *J* = 13.6 Hz, 8H), 1.68 (d, *J* = 7.8 Hz, 1H), 1.09 (s, 2H) . MS(ESI+): 604.2(M+H).

**Example 85** Preparation of compounds 85-1 and 85-2

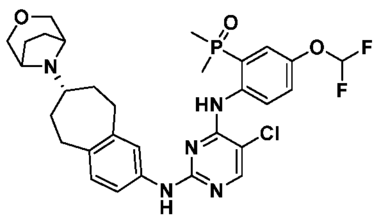 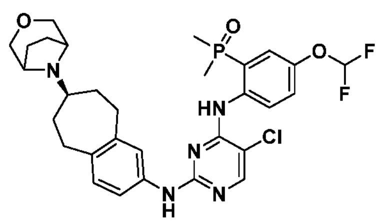

According to the preparation method of Example 58, (2-amino-5-fluorophenyl) dimethyl phosphine oxide in step a) is replaced with (2-amino-5-difluoromethoxyphenyl) dimethyl phosphine oxide, and 2-azabicyclo[3.1.0]hexane hydrochloride in step c) is replaced with 3-oxa-8-azabicyclo[3.2.1]octane hydrochloride. The crude product is purified by reversed-phase high-performance liquid chromatography (column: XBridge-Prep-OBD, C18 column, 30×150 mm, with a filler particle size of 5 μm; mobile phase A: 10 mmol/L ammonium bicarbonate aqueous solution, mobile phase B: acetonitrile (0.1% formic acid); flow rate: 60 mL/min; gradient: 20%B-60%B, 8 min; detection wavelength: 220 nm; isomer mixture retention time: 6.12 min; and column temperature: 25°C) to obtain 75 mg of isomer mixture. Chiral resolution is performed on the isomer mixture by chiral liquid chromatography (column: CHIRALPAK IF, 2 cm×25 cm, with a filler particle size of 5 μm; mobile phase A: n-hexane (0.1% diethylamine), mobile phase B: ethanol; flow rate: 20 ml/min; gradient: 30%B within 23 min, isogradient; detection wavelength: 220/254 nm; column temperature: 25°C) to obtain:

isomer 85-1 (22.3 mg) having a HPLC retention time of 16.0 min.

$^1$H NMR (400 MHz, DMSO-$d_6$, *ppm*): δ 10.95 (s, 1H), 9.27 (s, 1H), 8.55 (s, 1H), 8.18 (d, J = 1.1 Hz, 1H), 7.51 - 7.42 (m, 1.3H), 7.39 (s, 1H), 7.34 - 6.98 (m, 2.5H), 7.07 (s, 0.3H), 6.96(d, J = 8.0 Hz, 1H), 3.63 - 3.53 (m, 2H), 3.45 (d, J = 10.2 Hz, 2H), 3.06 (s, 2H), 2.51 - 2.39 (m, 3H) 1.81 (m, 7H), 1.79 -1.64 (m, 5H), 1.63 - 1.25 (m, 3H), 1.24 (s, 1H). MS(ESI+): 618.2(M+H).

Isomer 85-2 (22.0 mg) having a HPLC retention time of 20.2 min.

$^1$H NMR (400 MHz, DMSO-$d_6$, *ppm*): δ 10.95 (s, 1H), 9.27 (s, 1H), 8.55 (s, 1H), 8.18 (s, 1H), 7.52 - 7.42 (m, 1H), 7.39 (d, J = 2.2 Hz, 1H), 7.33 - 7.06 (m, 3H), 7.08 (s, 0.4H), 6.97 (d, J = 8.1 Hz, 1H), 3.57 (s, 2H), 3.45 (d, J = 10.2 Hz, 2H), 3.05 (s, 2H), 2.51 (s, 3H),1.82 (m, 7H), 1.79-1.67(m,5H), 1.67 - 1.39 (m, 3H), 1.24 (s, 1H). MS(ESI+): 618.2(M+H).

**Example 86** Compounds 86-1 and 86-2

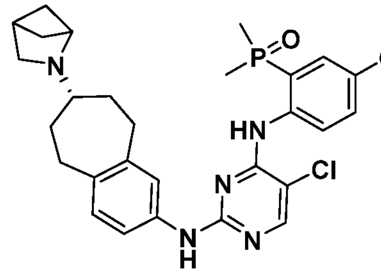
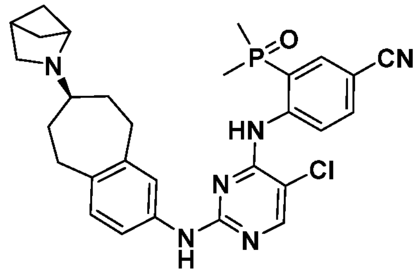

According to the preparation method of Example 58, (2-amino-5-fluorophenyl) dimethyl phosphine oxide in step a) is replaced with (2-amino-5-cyanophenyl) dimethyl phosphine oxide, and 2-azabicyclo[3.1.0]hexane hydrochloride in step c) is replaced with 2-azabicyclo[2.1.1]hexane hydrochloride. The crude product is purified by reversed-phase high-performance liquid chromatography (column: XBridge-Prep-OBD C18 column, 30×150 mm, with a filler particle size of 5 μm; mobile phase A: water (0.05% hydrochloric acid); mobile phase B: acetonitrile; flow rate: 60 mL/min; gradient: 5%B-50%B, 8 min; detection wavelength: 210 nm; isomer mixture retention time: 6.02 min) to obtain 70 mg of an isomer mixture.

Chiral resolution is performed on the isomer mixture by chiral liquid chromatography (column: CHIRALPAK IG, 3 cm×25 cm, with a filler particle size of 5 μm; mobile phase A: n-hexane (0.1% diethylamine), mobile phase B: ethanol; flow rate; gradient: 50%B within 21 min, isogradient; detection wavelength: 220/254 nm; column temperature: 25°C) to obtain:

isomer 86-1 (24.8 mg) having a HPLC retention time of 14.6 min.

$^1$H NMR (400 MHz, DMSO-$d_6$, *ppm)*: δ 11.74 (s, 1H), 9.44 (s, 1H), 8.87 (s, 1H), 8.27 (s, 1H), 8.13 (d, *J* = 13.8 Hz, 1H), 7.84 (d, *J* = 8.9 Hz, 1H), 7.42 (s, 1H), 7.30 (d, *J* = 7.7 Hz, 1H), 7.04 (d, *J* = 8.1 Hz, 1H), 3.73 (s, 1H), 3.02-2.76 (m, 3H), 2.73 (s, 2H), 2.58 (d, *J* = 11.4 Hz, 2H), 2.00 (d, *J* = 14.2 Hz, 2H), 1.87 (m, 7H), 1.62 (m, 2H), 1.36 (s, 3H), 1.24 (s, 1H). MS(ESI+): 547.2(M+H).

Isomer 86-2 (24.9 mg) having a HPLC retention time of 18.4 min.

$^1$H NMR (400 MHz, DMSO-$d_6$, *ppm):* δ 11.74 (s, 1H), 9.45 (s, 1H), 8.88 (s, 1H), 8.27 (s, 1H), 8.14 (dd, *J* = 13.9, 2.0 Hz, 1H), 7.85 (d, *J* = 9.0 Hz, 1H), 7.43 (d, *J* = 2.3 Hz, 1H), 7.38 - 7.21 (m, 1H), 7.06 (d, *J* = 8.1 Hz, 1H), 3.81 (s, 1H), 2.81 (d, *J* = 48.5 Hz, 5H), 2.62 (s, 2H), 2.06 (d, *J* = 18.6 Hz, 2H), 1.87 (d, *J* = 13.8 Hz, 7H), 1.66 (d, *J* = 51.2 Hz, 2H), 1.54 - 1.27 (m, 4H). MS(ESI+): 547.2(M+H).

**Example 87** Compounds 87-1 and 87-2

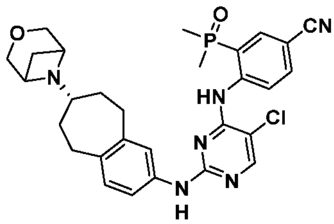

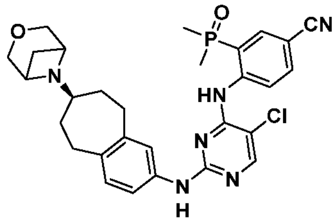

According to the preparation method of Example 58, (2-amino-5-fluorophenyl) dimethyl phosphine oxide in step a) is replaced with (2-amino-5-cyanophenyl) dimethyl phosphine oxide, and 2-azabicyclo[3.1.0]hexane hydrochloride in step c) is replaced with 3-oxa-6-azabicyclo[3.1.1]heptane hydrochloride. The crude product is purified by reversed-phase high-performance liquid chromatography (column: XBridge-Prep-OBD C18 column, 30×150 mm, with a filler particle size of 5 μm; mobile phase A: water (0.05% hydrochloric acid); mobile phase B: acetonitrile; flow rate: 60 mL/min; gradient: 5%B-30%B, 8 min; detection wavelength: 210 nm; isomer mixture retention time: 7.05 min; column temperature: 25°C) to obtain an isomer mixture.

Chiral resolution is performed on the isomer mixture by chiral liquid chromatography (column: CHIRALPAK IG, 2 cm×25 cm, with a filler particle size of 5 μm; mobile phase A: n-hexane (0.1% diethylamine), mobile phase B: ethanol; flow rate: 20 ml/min; gradient: 30%B within 21 min, isogradient; detection wavelength: 220/254 nm; column temperature: 25°C) to obtain:

isomer 87-1 (23 mg) having a HPLC retention time of 16.4 min.

$^1$H NMR (400 MHz, DMSO-$d_6$, *ppm*): δ 11.71 (s, 1H), 9.44 (s, 1H), 8.84 (s, 1H), 8.27 (s, 1H), 8.13 (dd, *J* = 13.9, 2.0 Hz, 1H), 7.88 - 7.76 (m, 1H), 7.42 (d, *J* = 2.3 Hz, 1H), 7.33 - 7.23 (m, 1H), 7.04 (d, *J* = 8.2 Hz, 1H), 4.15 (d, *J* = 10.6 Hz, 2H), 3.59 (d, *J* = 10.8 Hz, 4H), 3.14 (s, 1H), 2.88 - 2.75 (m, 1H), 2.67 (d, *J* = 13.8 Hz, 3H), 2.36 (s, 1H), 1.87 (d, *J* = 13.7 Hz, 8H), 1.69 (d, *J* = 7.7 Hz, 1H), 1.11 (s, 2H). MS(ESI+): 563.2(M+H).

Isomer 87-2 (23.5 mg) having a HPLC retention time of 18.9 min.

$^1$H NMR (400 MHz, DMSO-$d_6$, *ppm*):δ 11.71 (s, 1H), 9.44 (s, 1H), 8.85 (s, 1H), 8.27 (s, 1H), 8.13 (dd, *J* = 13.9, 2.0 Hz, 1H), 7.84 (dd, *J* = 9.1, 2.0 Hz, 1H), 7.42 (d, *J* = 2.3 Hz, 1H), 7.35 - 7.25 (m, 1H), 7.04 (d, *J* = 8.1 Hz, 1H), 4.15 (d, *J* = 10.6 Hz, 2H), 3.60 (d, *J* = 11.6 Hz, 4H), 3.15 (s, 1H), 2.88 - 2.77 (m, 1H), 2.69 (s, 3H), 2.37 (s, 1H), 1.87 (d, *J* = 13.7 Hz, 8H), 1.70 (s, 1H), 1.11 (s, 2H). MS(ESI+): 563.2(M+H).

**Example 88** Compounds 88-1 and 88-2

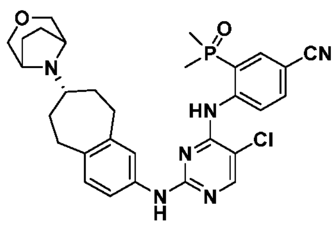

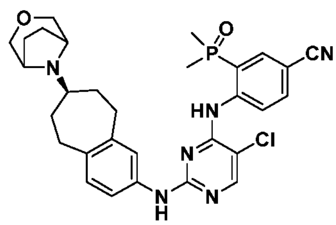

According to the preparation method of Example 58, (2-amino-5-fluorophenyl) dimethyl phosphine oxide in step a) is replaced with (2-amino-5-cyanophenyl) dimethyl phosphine oxide, and 2-azabicyclo[3.1.0]hexane hydrochloride in step c) is replaced with 3-oxa-8-azabicyclo[3.2.1]octane hydrochloride. The crude product is purified by reversed-phase high-performance liquid chromatography (column: XBridge-Prep-OBD C18 column, 30×150 mm, with a filler particle size of 5 μm; mobile phase A: water (10 mmol/L ammonium bicarbonate aqueous solution), mobile phase B: acetonitrile; flow rate: 60 mL /min; gradient: 20%B-60%B, 6 min; detection wavelength: 220 nm; column temperature: 25°C) to obtain 65 mg of an isomer mixture.

Chiral resolution is performed on the isomer mixture by chiral liquid chromatography (column: CHIRALPAK IA, 2 cm×25 cm, with a filler particle size of 5 μm; mobile phase A: n-hexane (0.1% diethylamine), mobile phase B: ethanol; flow rate; gradient: 50%B within 16 min, isogradient; detection wavelength: 220/254 nm; column temperature: 25°C) to

obtain:
isomer 88-1 (22.6 mg) having a HPLC retention time of 9.55 min.
$^1$H NMR (400 MHz, DMSO-$d_6$, *ppm*): δ 11.71 (s, 1H), 9.44 (s, 1H), 8.84 (s, 1H), 8.27 (s, 1H), 8.13 (dd, *J* = 13.9, 2.0 Hz, 1H), 7.88 - 7.76 (m, 1H), 7.42 (d, *J* = 2.3 Hz, 1H), 7.33 - 7.23 (m, 1H), 7.04 (d, *J* = 8.2 Hz, 1H), 4.15 (d, *J* = 10.6 Hz, 2H), 3.59 (d, *J* = 10.8 Hz, 4H), 3.14 (s, 1H), 2.88 - 2.75 (m, 1H), 2.67 (d, *J* = 13.8 Hz, 3H), 2.36 (s, 1H), 1.87 (m, 8H), 1.69 (d, *J* = 7.7 Hz, 1H), 1.11 (s, 2H). MS(ESI+): 577.2(M+H).
Isomer 88-2 (23.5 mg) having a HPLC retention time of 12.7 min.
$^1$H NMR (400 MHz, DMSO-$d_6$, *ppm*): δ 11.71 (s, 1H), 9.44 (s, 1H), 8.85 (s, 1H), 8.27 (s, 1H), 8.13 (dd, *J* = 13.9, 2.0 Hz, 1H), 7.84 (dd, *J* = 9.1, 2.0 Hz, 1H), 7.42 (d, *J* = 2.3 Hz, 1H), 7.35 - 7.25 (m, 1H), 7.04 (d, *J* = 8.1 Hz, 1H), 4.15 (d, *J* = 10.6 Hz, 2H), 3.60 (d, *J* = 11.6 Hz, 4H), 3.15 (s, 1H), 2.88 - 2.77 (m, 1H), 2.69 (s, 3H), 2.37 (s, 1H), 1.87 (m, 8H), 1.70 (s, 1H), 1.11 (s, 2H). MS(ESI+): 577.2(M+H).

**Example 89** Compounds 89-1 and 89-2

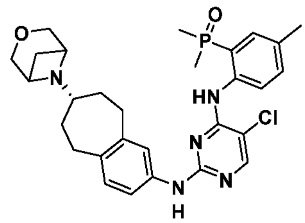
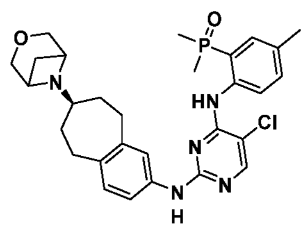

According to the preparation method of Example 58, (2-amino-5-fluorophenyl) dimethyl phosphine oxide in step a) is replaced with (2-amino-5-methylphenyl) dimethyl phosphine oxide, and 2-azabicyclo[3.1.0]hexane hydrochloride in step c) is replaced with 3-oxa-6-azabicyclo[3.1.1]heptane hydrochloride. The crude product is purified by reversed-phase high-performance liquid chromatography (column: XBridge-Prep-OBD C18 column, 30×150 mm, with a filler particle size of 5 μm; mobile phase A: 10 mmol/L ammonium bicarbonate aqueous solution, mobile phase B: acetonitrile; flow rate: 60 mL/min; gradient: 15%B-50%B, 8 min; detection wavelength: 220 nm; isomer mixture retention time: 8.1 min; column temperature: 25°C) to obtain 92 mg of an isomer mixture.
Chiral resolution is performed on the isomer mixture by chiral liquid chromatography (column: CHIRALPAK IF, 5 cm×25 cm, with a filler particle size of 5 μm; mobile phase A: methyl tert-butyl ether (0.1% diethylamine), mobile phase B: ethanol; flow rate: 15 ml/min; gradient: 25%B within 15 min, isogradient; detection wavelength: 220/254 nm; column temperature: 25°C) to obtain:
isomer 89-1 (21.7 mg) having a HPLC retention time of 13.7 min.
$^1$H NMR (400 MHz, DMSO-$d_6$, *ppm*): δ 10.93 (s, 1H), 9.25 (s, 1H), 8.40 (s, 1H), 8.15 (s, 1H), 7.48 - 7.31 (m, 2H), 7.30 - 7.28 (m, 2H), 6.98 (d, J = 8.2 Hz, 1H), 4.15 (d, J = 10.8 Hz, 2H), 3.56 (d, J = 22.1 Hz, 3H), 3.13 (s, 2H), 2.78 (d, J = 8.0 Hz, 1H), 2.66 (s, 3H), 2.33 (s, 4H), 1.77 (d, J = 13.5 Hz, 9H), 1.09 (s, 2H). MS(ESI+): 552.2 (M+H).
Isomer 89-2 (21.1 mg) having a HPLC retention time of 19.5 min.
$^1$H NMR (400 MHz, DMSO-$_{d6, ppm}$) : δ 10.92 (s, 1H), 9.25 (s, 1H), 8.40 (s, 1H), 8.15 (dd, J = 11.5, 4.3 Hz, 1H), 7.43 (dd, J = 14.1, 2.1 Hz, 1H), 7.30 (t, J = 7.5 Hz, 2H), 6.98 (d, J = 8.1 Hz, 1H), 4.15 (d, J = 10.7 Hz, 1H), 3.59 (s, 3H), 3.21 (s, 2H), 2.78 (s, 2H), 1.88 (d, J = 13.8 Hz, 3H), 2.33 (s, 4H), 1.76 (dd, J = 13.7, 1.1 Hz, 2H). MS(ESI+): 552.2(M+H).

**Example 90** Compounds 90-1 and 90-2

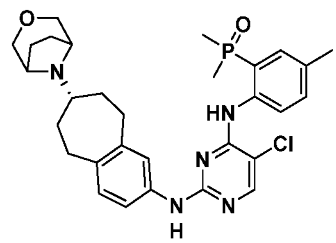
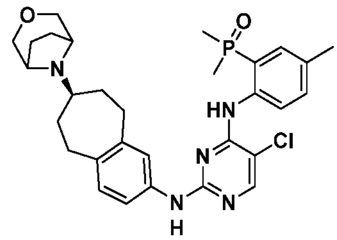

According to the preparation method of Example 58, (2-amino-5-fluorophenyl) dimethyl phosphine oxide in step a) is replaced with (2-amino-5-methylphenyl) dimethyl phosphine oxide, and 2-azabicyclo[3.1.0]hexane hydrochloride in step c) is replaced with 3-oxa-8-azabicyclo[3.2.1]octane hydrochloride. The crude product is purified by reversed-phase high-performance liquid chromatography (column: XBridge-Prep-OBD C18 column, 30×150 mm, with a filler particle size of 5 μm; mobile phase A: 10 mmol/L ammonium bicarbonate aqueous solution, mobile phase B: acetonitrile; flow rate: 60 mL/min; gradient: 25%B-60%B, 8 min; detection wavelength: 220 nm; isomer mixture retention time: 5.15 min) to obtain 63 mg of an isomer mixture.

Chiral resolution is performed on the isomer mixture by chiral liquid chromatography (column: CHIRALPAK IA, 2 cm×25 cm, with a filler particle size of 5 μm; mobile phase A: n-hexane (0.1% diethylamine), mobile phase B: ethanol; flow rate: 20 ml/min; gradient: 30%B within 20 min, isogradient; detection wavelength: 220/254 nm; column temperature: 25°C) to obtain:

isomer 90-1 (29.5 mg) having a HPLC retention time of 13.7 min.

1H NMR ( 400 MHz, DMSO-$d_6$, *ppm*): δ 10.92 (s, 1H), 9.23 (s, 1H), 8.40 (s, 1H), 8.15 (s, 1H), 7.50 - 7.34 (m, 2H), 7.29 (dd, J = 16.0, 8.4 Hz, 2H), 6.96 (d, J = 8.1 Hz, 1H), 3.58 (d, J = 7.4 Hz, 2H), 3.47 (s, 2H), 3.30 (s, 4H), 3.04 (s, 2H), 2.34 (s, 4H), 1.77 (d, J = 13.5 Hz, 12H), 1.58 (s, 2H). MS(ESI+): 566.2 (M+H).

Isomer 90-2 (20.2 mg) having a HPLC retention time of 16.6 min.

1H NMR (400 MHz, DMSO-$d_6$, *ppm*) δ 10.92 (s, 1H), 9.23 (s, 1H), 8.40 (s, 1H), 8.15 (s, 1H), 7.47 - 7.35 (m, 2H), 7.29 (dd, J = 15.5, 8.3 Hz, 2H), 6.96 (d, J = 8.1 Hz, 1H), 3.58 (d, J = 9.9 Hz, 2H), 3.47 (s, 2H), 3.31(s, 4H), 3.04 (s, 2H), 2.34 (s, 4H), 1.77 (d, J = 13.5 Hz, 12H), 1.59 (s, 2H). MS(ESI+): 566.2 (M+H).

**Example 91** Compounds 91-1 and 91-2

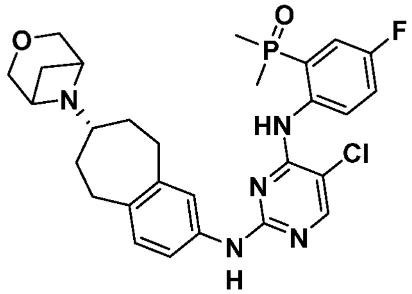

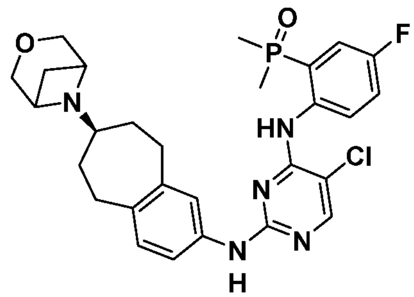

According to the preparation method of Example 58, 2-azabicyclo[3.1.0]hexane hydrochloride in step c) is replaced with 3-oxo-6-azabicyclo[3.1.1]heptane hydrochloride. The crude product is purified by reversed-phase high-performance liquid chromatography (column: Xselect CSH OBD C18 column, 30×150 mm, with a filler particle size of 5 μm; mobile phase A: 10 mmol/L ammonium bicarbonate aqueous solution, mobile phase B: acetonitrile; flow rate: 60 mL /min; gradient: 15%B-52%B, 8 min; detection wavelength: 220 nm) to obtain 70 mg of an isomer mixture.

Chiral resolution is performed on the isomer mixture by chiral liquid chromatography (CHIRALPAK IA, 5 cm×25 cm, with a filler particle size of 5 μm; mobile phase A: n-hexane (0.1% diethylamine), mobile phase B: ethanol; flow rate: 20 ml/min; gradient: 20%B within 29 min, isogradient; detection wavelength: 220/254 nm; column temperature: 25°C) to obtain:

isomer 91-1 (25.5 mg) having a HPLC retention time of 17.9 min.

$^1$H NMR ( 400 MHz, DMSO-$d_6$, *ppm*) δ 10.87 (s, 1H), 9.28 (s, 1H), 8.52 (s, 1H), 8.17 (s, 1H), 7.53 (ddd, J = 13.9, 8.8, 3.0 Hz, 1H), 7.33 (dt, J = 18.0, 6.2 Hz, 3H), 6.98 (d, J = 8.1 Hz, 1H), 4.15 (d, J = 10.8 Hz, 2H), 3.56 (m, 3H), 3.13 (s, 2H), 2.78 (d, J = 9.6 Hz, 1H), 2.67 (s, 3H), 2.37 (s, 1H), 1.80 (m, 9H), 1.08 (s, 2H). MS(ESI+): 556.2 (M+H).

Isomer 91-2 (30.3 mg) having a HPLC retention time of 24.2 min.

$^1$H NMR (400 MHz, DMSO-$d_6$, *ppm*) δ 10.87 (s, 1H), 9.28 (s, 1H), 8.52 (s, 1H), 8.17 (s, 1H), 7.53 (ddd, J = 13.9, 8.8, 3.0 Hz, 1H), 7.33 (dt, J = 18.0, 6.2 Hz, 3H), 6.98 (d, J = 8.1 Hz, 1H), 4.15 (d, J = 10.8 Hz, 2H), 3.56 (m, 3H), 3.13 (s, 2H), 2.78 (d, J = 9.6 Hz, 1H), 2.67 (s, 3H), 2.37 (s, 1H), 1.80 (m, 9H), 1.08 (s, 2H). MS(ESI+): 556.2 (M+H).

**Example 92** Compounds 92-1 and 92-2

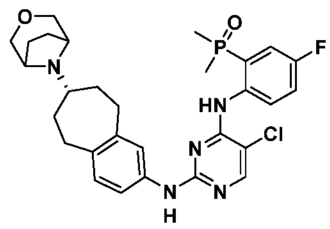
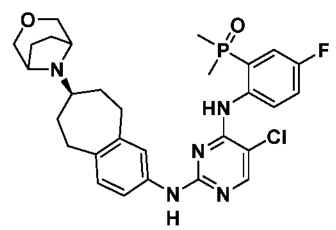

According to the preparation method of Example 58, 2-azabicyclo[3.1.0]hexane hydrochloride in step c) is replaced with 3-oxa-8-azabicyclo[3.2.1]octane hydrochloride. The crude product is purified by reversed-phase high-performance liquid chromatography (column: Xselect CSH OBD C18 column, 30×150 mm, with a filler particle size of 5 μm; mobile phase A: 10 mmol/L ammonium bicarbonate aqueous solution, mobile phase B: acetonitrile; flow rate: 60 mL /min; gradient: 15%B-50%B, 8 min; detection wavelength: 220 nm) to obtain 130 mg of an isomer mixture.

Chiral resolution is performed on the isomer mixture by chiral liquid chromatography (CHIRALPAK IF, 2 cm×25 cm, with a filler particle size of 5 μm; mobile phase A: n-hexane (0.1% diethylamine), mobile phase B: ethanol; flow rate: 15 ml/min; gradient: 30%B within 20 min, isogradient; detection wavelength: 220/254 nm; column temperature: 25°C) to obtain:

isomer 92-1 (28.9 mg) having a HPLC retention time of 3.65 min.

$^1$H NMR ( 400 MHz, DMSO-d6, *ppm*) δ 10.86 (s, 1H), 9.26 (s, 1H), 8.51 (s, 1H), 8.17 (s, 1H), 7.53 (ddd, J = 14.0, 8.8, 3.1 Hz, 1H), 7.39 - 7.21 (m, 3H), 6.97 (d, J = 8.1 Hz, 1H), 3.57 (d, J = 10.1 Hz, 2H), 3.45 (d, J = 10.0 Hz, 2H), 3.30 (s, 4H), 3.04 (s, 2H), 2.41 (d, J = 20.5 Hz, 1H), 1.90 - 1.56 (m, 14H). MS(ESI+): 570.2 (M+H).

Isomer 92-2 (28.9 mg) having a HPLC retention time of 4.85 min.

$^1$H NMR (400 MHz, DMSO-d6, *ppm*) δ 10.86 (s, 1H), 9.26 (s, 1H), 8.51 (s, 1H), 8.17 (s, 1H), 7.53 (ddd, J = 14.0, 8.8, 3.1 Hz, 1H), 7.39 - 7.31 (m, 2H), 7.27 (d, J=8Hz, 1H), 6.97 (d, J = 8.1 Hz, 1H), 3.57 (d, J = 10.1 Hz, 2H), 3.45 (d, J = 10.0 Hz, 2H), 3.30 (s, 4H), 3.04 (s, 2H), 2.41 (d, J = 20.5 Hz, 1H), 1.90 - 1.56 (m, 14H). MS(ESI+): 570.2 (M+H).

**Example 93** Compounds 93-1 and 93-2

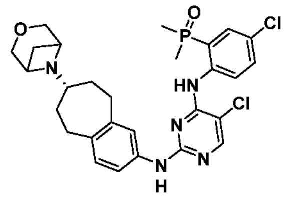
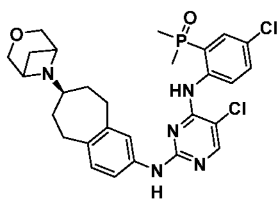

According to the preparation method of Example 58, (2-amino-5-fluorophenyl) dimethyl phosphine oxide in step a) is replaced with (2-amino-5-chlorophenyl) dimethyl phosphine oxide, and 2-azabicyclo[3.1.0]hexane hydrochloride in step c) is replaced with 3-oxa-6-azabicyclo[3.1.1]heptane hydrochloride. The crude product is purified by reversed-phase high-performance liquid chromatography (column: Xselect CSH OBD C18 column, 30×150 mm, with a filler particle size of 5 μm; mobile phase A: 10 mmol/L ammonium bicarbonate aqueous solution, mobile phase B: acetonitrile; flow rate: 60 mL /min; gradient: 20%B-55%B, 8 min; detection wavelength: 220 nm) to obtain 60 mg of an isomer mixture. Chiral resolution is performed on the isomer mixture by chiral liquid chromatography (column: CHIRALPAK IE, 3 cm×25 cm, with a filler particle size of 5 μm; mobile phase A: n-hexane (0.1% diethylamine), mobile phase B: ethanol; flow rate: 15 ml/min;

gradient: 50%B within 32 min, isogradient; detection wavelength: 220/254 nm; column temperature: 25°C) to obtain:

isomer 93-1 (23.7 mg) having a HPLC retention time of 23.7 min.

$^1$HNMR (400MHz, DMSO-$d_6$, *ppm*): δ 11.11 (s, 1H), 9.31 (s, 1H), 8.57 (s, 1H), 8.19 (s, 1H), 7.69 (dd, *J* = 13.6, 2.6 Hz, 1H), 7.49 (dd, *J* = 9.0, 2.6 Hz, 1H), 7.41 (d, *J* = 2.3 Hz, 1H), 7.30 - 7.23 (m, 1H), 6.99 (d, *J* = 8.1 Hz, 1H), 4.14 (d, *J* = 10.6 Hz, 2H), 3.63 - 3.51 (m, 4H), 3.13 (s, 1H), 2.80 (t, *J* = 11.3 Hz, 1H), 2.66 (s, 3H), 2.36 (d, *J* = 6.9 Hz, 1H), 1.82 (d, *J* = 13.7Hz, 8H), 1.68 (d, *J* = 7.8 Hz, 1H), 1.23-1.01(m,2H). MS(ESI+): 572.2 (M+H). Isomer 93-2 (22.3 mg) having a HPLC retention time of 27.9 min.

$^1$H HNMR (400MHz, DMSO-$d_6$, *ppm*):δ 11.11 (s, 1H), 9.31 (s, 1H), 8.57 (s, 1H), 8.19 (s, 1H), 7.69 (dd, *J* = 13.6, 2.6 Hz, 1H), 7.49 (dd, *J* = 9.0, 2.6 Hz, 1H), 7.41 (d, *J* = 2.3 Hz, 1H), 7.30 - 7.23 (m, 1H), 6.99 (d, *J* = 8.1 Hz, 1H), 4.14 (d, *J* = 10.6 Hz, 2H), 3.63 (d, *J* = 11.6 Hz, 4H), 3.13 (s, 1H), 2.80 - 2.77 (m, 1H), 2.66 (s, 3H), 2.36 (s, 1H), 1.82 (d,

$J$ = 13.7Hz, 8H), 1.68 (s, 1H), 1.11 (s, 2H). MS(ESI+): 572.2(M+H).

**Example 94** Compounds 94-1 and 94-2

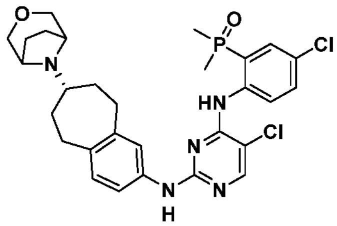

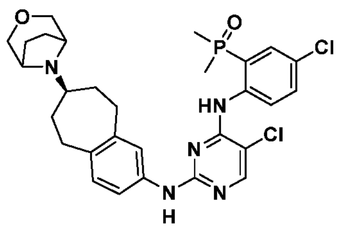

According to the preparation method of Example 58, (2-amino-5-fluorophenyl) dimethyl phosphine oxide in step a) is replaced with (2-amino-5-chlorophenyl) dimethyl phosphine oxide, and 2-azabicyclo[3.1.0]hexane hydrochloride in step c) is replaced with 3-oxa-8-azabicyclo[3.2.1]octane hydrochloride. The crude product is purified by reversed-phase high-performance liquid chromatography (column: Xselect CSH OBD column, 30×150 mm, with a filler particle size of 5 μm; mobile phase A: 10 mmol/L ammonium bicarbonate aqueous solution, mobile phase B: acetonitrile; flow rate: 60 mL /min; gradient: 25%B-65%B, 8 min; detection wavelength: 254 nm; column temperature: 25°C) to obtain 65 mg of an isomer mixture.

Chiral resolution is performed on the isomer mixture by chiral liquid chromatography (CHIRALPAK IA, 2 cm×25 cm, with a filler particle size of 5 μm; mobile phase A: n-hexane (0.1% diethylamine), mobile phase B: ethanol; flow rate: 15 ml/min; gradient: 50%B within 14 min, isogradient; detection wavelength: 254 nm; column temperature: 25°C) to obtain: isomer 94-1 (25.1 mg) having a HPLC retention time of 1.85 min.

$^1$H NMR (400 MHz, DMSO-$d_6$, *ppm):* δ 11.10 (s, 1H), 9.30 (s, 1H), 8.56 (s, 1H), 8.19 (s, 1H), 7.69 (dd, J = 13.6, 2.5 Hz, 1H), 7.49 (dd, J = 9.1, 2.5 Hz, 1H), 7.40 (d, J = 2.2 Hz, 1H), 7.27 - 7.20 (m, 1H), 6.98 (d, J = 8.1 Hz, 1H), 3.58 (d, J = 10.1 Hz, 2H), 3.45 (d, J = 10.2 Hz, 2H), 3.28(s,4H),3.04 (s, 2H), 2.39 (s, 1H), 1.82 (m, 12H), 1.77 (s, 2H). MS(ESI+): 586 (M+H).

Isomer 94-2 (23.4 mg) having a HPLC retention time of 2.73 min.

$^1$H NMR (400 MHz, DMSO-$d_6$, *ppm*): δ 11.10 (s, 1H), 9.30 (s, 1H), 8.56 (s, 1H), 8.19 (s, 1H), 7.69 (dd, J = 13.6, 2.5 Hz, 1H), 7.49 (dd, J = 9.1, 2.5 Hz, 1H), 7.40 (d, J = 2.2 Hz, 1H), 7.27 - 7.20 (m, 1H), 6.98 (d, J = 8.1 Hz, 1H), 3.58 (d, J = 10.1 Hz, 2H), 3.45 (d, J = 10.2 Hz, 2H), 3.28(s,4H),3.04 (s, 2H), 2.39 (s, 1H), 1.82 (m, 12H), 1.77 (s, 2H). MS(ESI+): 586 (M+H).

**Example 95** Preparation of compounds 95-1 and 95-2

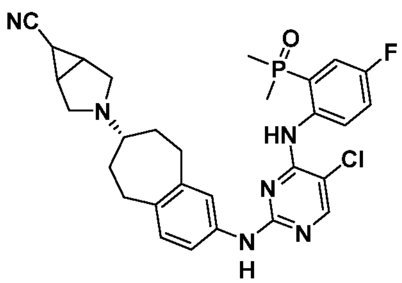

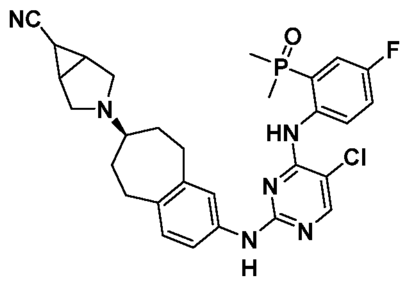

According to the preparation method of Example 58, 2-azabicyclo[3.1.0]hexane hydrochloride in step b) is replaced with 3-azabicyclo[3.1.0]hexane-6-carbonitrile. The crude product is purified by reversed-phase high-performance liquid chromatography (column: XBridge-Prep-OBD C18 column, 30×150 mm, with a filler particle size of 5 μm; mobile phase A: 10 mmol/L ammonium bicarbonate aqueous solution, mobile phase B: acetonitrile; flow rate: 60 mL/min; gradient: 30%B-68%B, 8 min; detection wavelength: 220 nm; isomer mixture retention time: 7.85 min) to obtain an isomer mixture.

Chiral resolution is performed on the isomer mixture by chiral liquid chromatography (column: CHIRALPAK IF, 5 cm×25 cm, with a filler particle size of 5 μm; mobile phase A: methyl tert-butyl ether (0.1% diethylamine), mobile

phase B: ethanol; flow rate: 15 ml/min; gradient: 20%B within 17.5 min, isogradient; detection wavelength: 220/254 nm; column temperature: 25°C) to obtain:
isomer 95-1 (21.7 mg) having a HPLC retention time of 13.4 min.
$^1$H NMR (400 MHz, DMSO-d6$_{,ppm}$) δ 10.82 (s, 1H), 9.25 (s, 1H), 8.49 (s, 1H), 8.16 (s, 1H), 7.53 (ddd, J = 13.9, 8.8, 3.1 Hz, 1H), 7.38 - 7.30 (m, 2H), 7.26 (d, J=2.2 Hz, 1H), 6.96 (d, J = 8.1 Hz, 1H), 3.06 (d, J = 10.1 Hz, 2H), 2.43 (d, J = 9.0 Hz, 2H), 2.15 (s, 2H), 1.87 (s, 2H), 1.79 (d, J = 13.7 Hz, 1H), 1.73 - 1.45 (m, 2H). MS(ESI+): 565.2 (M+H).
Isomer 95-2 (21.1 mg) having a HPLC retention time of 16.2 min.
$^1$H NMR (400 MHz, DMSO-*$d_6$*, *ppm):* δ 10.82 (s, 1H), 9.25 (s, 1H), 8.49 (s, 1H), 8.16 (s, 1H), 7.53 (ddd, J = 14.0, 8.7, 3.0 Hz, 1H), 7.37 - 7.29 (m, 2H), 7.26 (dd, J = 8.1, 2.2 Hz, 1H), 6.96 (d, J = 8.2 Hz, 1H), 3.07 - 2.97 (m, 2H), 2.94 - 2.69 (m, 2H), 2.54 (s, 1H), 2.43 (d, J = 9.0 Hz, 4H), 2.15 (t, J = 2.6 Hz, 2H), 1.87 (t, J = 3.2 Hz, 1H), 1.79 (d, J = 13.6 Hz, 6H), 1.73 (s, 2H), 1.45 (d, J = 16.2 Hz, 2H). MS(ESI+): 565.2 (M+H).

**Example 96** Preparation of compounds 96-1 and 96-2

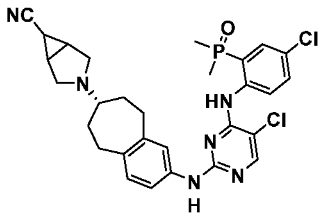
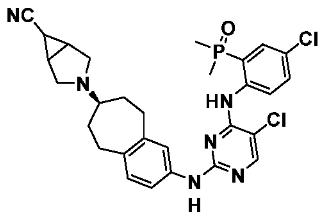

According to the preparation method of Example 58, (2-amino-5-fluorophenyl) dimethyl phosphine oxide in step a) is replaced with (2-amino-5-chlorophenyl) dimethyl phosphine oxide, and 2-azabicyclo[3.1.0]hexane hydrochloride in step c) is replaced with 3-azabicyclo[3.1.0]hexane-6-carbonitrile. The crude product is purified by reversed-phase high-performance liquid chromatography (column: Xselect CSH OBD C18 column, 30×150 mm, with a filler particle size of 5 μm; mobile phase A: 10 mmol/L ammonium bicarbonate aqueous solution, mobile phase B: acetonitrile; flow rate: 60 mL /min; gradient: 45%B-85%B, 8 min; detection wavelength: 254 nm) to obtain 58 mg of an isomer mixture. Chiral resolution is performed on the isomer mixture by chiral liquid chromatography (column: CHIRALPAK IA, 2 cm×25 cm, with a filler particle size of 5 μm; mobile phase A: n-hexane (0.1% diethylamine), mobile phase B: ethanol; flow rate: 20 ml/min;
gradient: 30%B within 37 min, isogradient; detection wavelength: 220/254 nm; column temperature: 25°C) to obtain:
isomer 96-1 (17.4 mg) having a HPLC retention time of 23 min.
$^1$H NMR (400 MHz, DMSO-*$d_6$*, *ppm):* δ 11.10 (s, 1H), 9.30 (s, 1H), 8.55 (s, 1H), 8.19 (s, 1H), 7.69 (dd, J = 13.6, 2.5 Hz, 1H), 7.48 (dd, J = 9.0, 2.6 Hz, 1H), 7.39 (d, J = 2.3 Hz, 1H), 7.27 - 7.20 (m, 1H), 6.97 (d, J = 8.2 Hz, 1H), 3.03 (d, J = 9.2 Hz, 2H), 2.83 (s,2H) , 2.54 (s, 1H), 2.43 (d, J = 9.0 Hz, 4H), 2.15 (d, J = 2.7 Hz, 2H), 1.82 (m, 9H), 1.49 (s, 2H). MS(ESI+): 581.2 (M+H).
Isomer 96-2 (23.1 mg) having a HPLC retention time of 30.8 min.
$^1$H NMR (400 MHz, DMSO-*$d_6$*, *ppm*): δ 11.10 (s, 1H), 9.30 (s, 1H), 8.55 (s, 1H), 8.19 (s, 1H), 7.69 (dd, J = 13.6, 2.5 Hz, 1H), 7.48 (dd, J = 9.0, 2.6 Hz, 1H), 7.39 (d, J = 2.3 Hz, 1H), 7.27-7.20 (m, 1H), 6.97 (d, J = 8.2 Hz, 1H), 3.03 (d, J = 9.2 Hz, 2H), 2.83 (s,2H) , 2.54 (s, 1H), 2.43 (d, J = 9.0 Hz, 4H), 2.15 (d, J = 2.7 Hz, 2H), 1.87 (s, 1H), 1.82 (d, J = 13.7 Hz, 6H),1.77-1.64(m,2H) 1.49 (s, 2H). MS(ESI+): 581.2 (M+H).

**Example 97** Compounds 97-1 and 97-2

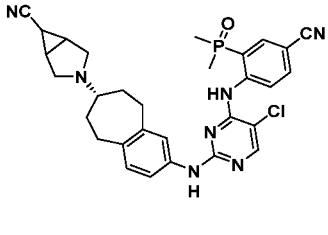
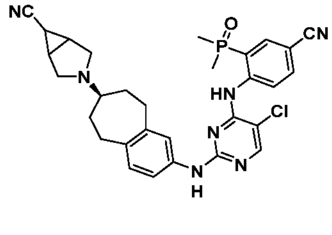

According to the preparation method of Example 58, (2-amino-5-fluorophenyl) dimethyl phosphine oxide in

step a) is replaced with (2-amino-5-cyanophenyl) dimethyl phosphine oxide, and 2-azabicyclo[3.1.0]hexane hydrochloride in step c) is replaced with 3-azabicyclo[3.1.0]hexane-6-carbonitrile. The crude product is purified by reversed-phase high-performance liquid chromatography (column: YMC-Actus Triart C18 column, 30×150 mm, with a filler particle size of 5 μm; mobile phase A: 10 mmol/L ammonium bicarbonate aqueous solution, mobile phase B: acetonitrile; flow rate: 60 mL/min; gradient: 45%B-75%B, 10 min; detection wavelength: 220 nm; isomer mixture retention time: 8.72 min) to obtain 48 mg of an isomer mixture.

Chiral resolution is performed on the isomer mixture by chiral liquid chromatography (column: CHIRALPAK IF, 3 cm×25 cm, with a filler particle size of 5 μm; mobile phase A: methyl tert-butyl ether (0.1% diethylamine), mobile phase B: ethanol; flow rate: 20 ml/min; gradient: 10%B within 26 min, isogradient; detection wavelength: 220/254 nm; column temperature: 25°C) to obtain:
isomer 97-1 (20 mg) having a HPLC retention time of 16.2 min.

$^1H$ NMR (400 MHz, DMSO-$d_6$, *ppm*): δ 11.72 (s, 1H), 9.42 (s, 1H), 8.85 (s, 1H), 8.27 (s, 1H), 8.13 (dd, J = 13.8, 2.0 Hz, 1H), 7.90 - 7.77 (m, 1H), 7.38 (d, J = 2.3 Hz, 1H), 7.29 (d, J = 8.1 Hz, 1H), 7.02 (d, J = 8.1 Hz, 1H), 3.03 (dd, J = 9.3, 2.9 Hz, 2H), 2.86 (s, 2H), 2.69 - 2.66 (m, 1H), 2.44 (d, J = 9.2 Hz, 4H), 2.15 (d, J = 2.8 Hz, 2H), 1.87 (d, J = 13.7 Hz, 7H), 1.75 (s, 2H), 1.49 (s, 2H). MS(ESI+): 572.2 (M+H).

Isomer 97-2 (20.2 mg) having a HPLC retention time of 23 min.

$^1H$ NMR (400 MHz, DMSO-$d_6$, *ppm*): δ 11.72 (s, 1H), 9.42 (s, 1H), 8.85 (s, 1H), 8.27 (s, 1H), 8.13 (dd, J = 14.0, 2.0 Hz, 1H), 7.83 (d, J = 9.0 Hz, 1H), 7.38 (d, J = 2.2 Hz, 1H), 7.32 - 7.25 (m, 1H), 7.02 (d, J = 8.1 Hz, 1H), 3.03 (d, J = 9.1 Hz, 2H), 2.84 (s, 2H), 2.69 - 2.66(m,1H), 2.44 (d, J = 9.0 Hz, 4H), 2.15 (d, J = 2.7 Hz, 2H), 1.87 (d, J = 13.8 Hz, 7H), 1.75 (s, 2H), 1.49 (s, 2H). MS(ESI+): 572.2 (M+H).

**Example 98** Compounds 98-1 and 98-2

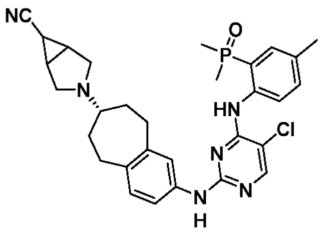

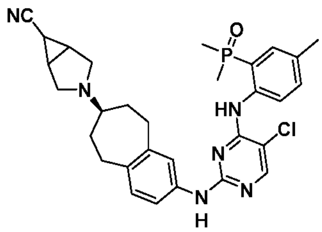

According to the preparation method of Example 58, (2-amino-5-fluorophenyl) dimethyl phosphine oxide in step a) is replaced with (2-amino-5-methylphenyl) dimethyl phosphine oxide, and 2-azabicyclo[3.1.0]hexane hydrochloride in step c) is replaced with 3-azabicyclo[3.1.0]hexane-6-carbonitrile. The crude product is purified by reversed-phase high-performance liquid chromatography (column: YMC-Actus Triart C18 column, 30×150 mm, with a filler particle size of 5 μm; mobile phase A: 10 mmol/L ammonium bicarbonate aqueous solution, mobile phase B: acetonitrile; flow rate: 60 mL/min; gradient: 50%B-66%B, 10 min; detection wavelength: 220 nm; isomer mixture retention time: 8.72 min) to obtain 63 mg of an isomer mixture.

Chiral resolution is performed on the isomer mixture by chiral liquid chromatography (column: CHIRALPAK IF, 3 cm×25 cm, with a filler particle size of 5 μm; mobile phase A: methyl tert-butyl ether (0.1% diethylamine), mobile phase B: ethanol; flow rate: 15 ml/min; gradient: 20%B within 27 min, isogradient; detection wavelength: 220/254 nm; column temperature: 25°C) to obtain:
isomer 98-1 (20.5 mg) having a HPLC retention time of 19.1 min.

$^1H$ NMR (400 MHz, DMSO-$d_6$, *ppm*): δ 10.92 (s, 1H), 9.23 (s, 1H), 8.39 (s, 1H), 8.15 (s, 1H), 7.47 - 7.37 (m, 2H), 7.34 - 7.24 (m, 2H), 6.95 (d, J = 8.1 Hz, 1H), 3.03 (d, J = 9.2 Hz, 2H), 2.83 (s, 2H), 2.69 - 2.66(m,1H), 2.44 (d, J = 9.2 Hz, 4H), 2.34 (s, 3H), 2.16 (s, 2H), 1.88 (s, 1H), 1.77 (d, J = 13.5 Hz, 8H), 1.49 (s, 2H). MS(ESI+): 561.2 (M+H).

Isomer 98-2 (20.4 mg) having a HPLC retention time of 25.1 min.

$^1H$ NMR (400 MHz, DMSO-$d_6$, *ppm*): δ 10.92 (s, 1H), 9.24 (s, 1H), 8.39 (s, 1H), 8.15 (d, J = 2.2 Hz, 1H), 7.47 - 7.37 (m, 2H), 7.34 - 7.24 (m, 2H), 6.95 (d, J = 8.3 Hz, 1H), 3.02 (d, J = 9.3 Hz, 2H), 2.93 - 2.74 (m, 3H), 2.34 (s, 4H), 2.21 (s, 3H), 2.16 (s, 2H), 1.88 (s, 1H), 1.84 - 1.66 (m, 8H), 1.49 (s, 2H). MS(ESI+): 561.2 (M+H).

**Example 99** Compounds 99-1 and 99-2

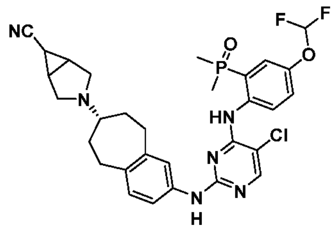
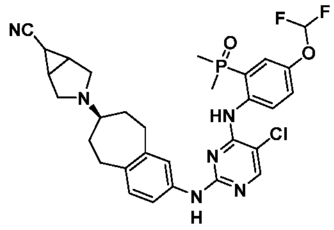

According to the preparation method of Example 58, (2-amino-5-fluorophenyl) dimethyl phosphine oxide in step a) is replaced with (2-amino-5-difluoromethoxyphenyl) dimethyl phosphine oxide, and 2-azabicyclo[3.1.0]hexane hydrochloride in step c) is replaced with 3-azabicyclo[3.1.0]hexane-6-carbonitrile. The crude product is purified by reversed-phase high-performance liquid chromatography (column: Xselect CSH OBD C18 column, 30×150 mm, with a filler particle size of 5 μm; mobile phase A: 10 mmol/L ammonium bicarbonate aqueous solution, mobile phase B: acetonitrile; flow rate: 60 mL/min; gradient: 35%B-64%B, 10 min; detection wavelength: 220 nm; isomer mixture retention time: 9.6 min) to obtain an isomer mixture.

Chiral resolution is performed on the isomer mixture by chiral liquid chromatography (column: CHIRALPAK IE, 2 cm×25 cm, with a filler particle size of 5 μm; mobile phase A: methyl tert-butyl ether (0.1% diethylamine), mobile phase B: ethanol; flow rate: 20 ml/min; gradient: 10%B within 24 min, isogradient; detection wavelength: 220/254 nm; column temperature: 25°C) to obtain:
isomer 99-1 (22.5 mg) having a HPLC retention time of 14.3 min.

$^1$H NMR (400 MHz, DMSO-$d_6$, *ppm*) : δ 10.96 (s, 1H), 9.27 (s, 1H), 8.55 (s, 1H), 8.18 (s, 1H), 7.49 - 7.42 (m, 1H), 7.37 (d, J = 2.3 Hz, 1H), 7.29 (d, J = 8.6 Hz, 2H), 7.26 - 7.20 (m, 1H), 6.96 (d, J = 8.1 Hz, 1H), 3.03 (d, J = 9.2 Hz, 2H), 2.84 (s, 2H),2.55 (s, 1H), 2.42 (d, J = 9.2 Hz, 4H), 2.16 (t, J = 2.6 Hz, 2H), 1.87 (s, 1H), 1.81 (d, J = 13.6 Hz, 6H), 1.71 (s, 2H), 1.50 (s, 2H). MS(ESI+): 613.2 (M+H).

Isomer 99-2 (22.4 mg) having a HPLC retention time of 21.25 min.

$^1$H NMR (400 MHz, DMSO-$d_6$, *ppm*): δ 10.96 (s, 1H), 9.27 (s, 1H), 8.55 (s, 1H), 8.18 (s, 1H), 7.49 - 7.42 (m, 1 H), 7.37 (d, J = 2.3 Hz, 1H), 7.32 - 7.21 (m, 3H), 6.96 (d, J = 8.1 Hz, 1H), 3.03 (d, J = 9.2 Hz, 2H), 2.84 (s, 2H), 2.55 (s, 1H), 2.42 (d, J = 9.2 Hz, 4H), 2.15 (d, J = 2.6 Hz, 2H), 1.87 (s, 1H), 1.81 (d, J = 13.7 Hz, 6H), 1.71 (s, 2H), 1.51 (s, 2H). MS(ESI+):613.2 (M+H).

**Example 100** Preparation of compounds 100-1 and 100-2

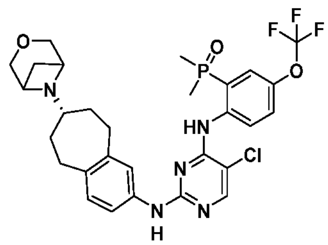
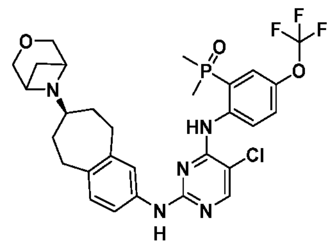

According to the preparation method of Example 58, (2-amino-5-fluorophenyl) dimethyl phosphine oxide in step a) is replaced with (2-amino-5-trifluoromethoxyphenyl) dimethyl phosphine oxide, and 2-azabicyclo[3.1.0]hexane hydrochloride in step c) is replaced with 3-oxa-6-azabicyclo[3.1.1]heptane hydrochloride. The crude product is purified by reversed-phase high-performance liquid chromatography (column: YMC-Actus Triart C18 column, 30×150 mm, with a filler particle size of 5 μm; mobile phase A: 10 mmol/L ammonium bicarbonate aqueous solution, mobile phase B: acetonitrile; flow rate: 60 mL /min; gradient: 30%B-60%B, 8 min; detection wavelength: 220 nm) to obtain 70 mg of an isomer mixture. Chiral resolution is performed on the isomer mixture by chiral liquid chromatography (column: CHIRALPAK IG, 2 cm×25 cm, with a filler particle size of 5 μm; mobile phase A: n-hexane (0.1% diethylamine), mobile phase B: ethanol; flow rate: 20 ml/min; gradient: 30%B within 25 min, isogradient; detection wavelength: 220/254 nm; column temperature: 25°C) to obtain:
isomer 100-1 (18.4 mg) having a HPLC retention time of 17.1 min.

$^1$H NMR (400 MHz, DMSO-$d_6$, *ppm):* δ 11.15 (s, 1H), 9.32 (s, 1H), 8.65 (s, 1H), 8.21 (s, 1H), 7.66 (dd, *J* = 13.8, 2.9 Hz, 1H), 7.46(d, *J*=12.0 Hz,1H), 7.40(d, *J*=4.0 Hz, 1H), 7.27 (d, *J* = 8.0 Hz, 1H), 6.98 (d, *J* = 8.1 Hz, 1H), 4.13 (dd, *J* = 10.5, 5.5 Hz, 2H), 3.67 - 3.46 (m, 4H), 3.12 (s, 1H), 2.91 - 2.77 (m, 1H), 2.67 (d, *J* = 11.1 Hz, 3H), 2.35 (d, *J* = 7.1

Hz, 1H), 1.85 - 1.78 (m, 8H),1.65(d, *J*=8 Hz, 1H) ,1.10 (s, 2H). MS(ESI+): 622.2 (M+H).

Isomer 100-2 (18.2 mg) having a HPLC retention time of 22.4 min.

$^1H$ NMR (400 MHz, DMSO-$d_6$, *ppm*): δ 11.15 (s, 1H), 9.32 (s, 1H), 8.65 (s, 1H), 8.21 (s, 1H), 7.66 (dd, *J* = 13.8, 2.9 Hz, 1H), 7.46(d, *J*=12.0 Hz,1H), 7.40(d, *J*=4.0 Hz, 1H), 7.27 (d, *J* = 8.0 Hz, 1H), 6.98 (d, *J* = 8.1 Hz, 1H), 4.13 (dd, *J* = 10.5, 5.5 Hz, 2H), 3.67 - 3.46 (m, 4H), 3.12 (s, 1H), 2.91 - 2.77 (m, 1H), 2.67 (d, *J* = 11.1 Hz, 3H), 2.35 (d, *J* = 7.1 Hz, 1H), 1.85 - 1.78 (m, 8H),1.65(d, *J*=8 Hz, 1H) ,1.10 (s, 2H). MS(ESI+): 622.2 (M+H).

**Example 101** Compounds 101-1 and 101-2

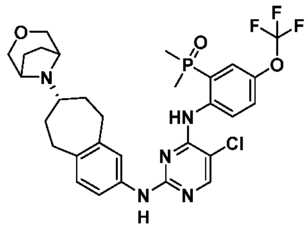

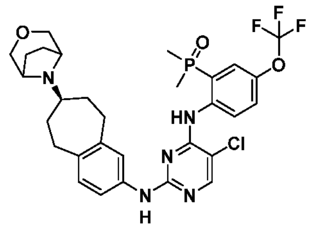

According to the preparation method of Example 58, (2-amino-5-fluorophenyl) dimethyl phosphine oxide in step a) is replaced with (2-amino-5-trifluoromethoxyphenyl) dimethyl phosphine oxide, and 2-azabicyclo[3.1.0]hexane hydrochloride in step c) is replaced with 3-oxa-8-azabicyclo[3.2.1]octane hydrochloride. The crude product is purified by reversed-phase high-performance liquid chromatography (column: YMC-Actus Triart C18 column, 30×150 mm, with a filler particle size of 5 μm; mobile phase A: 10 mmol/L ammonium bicarbonate aqueous solution, mobile phase B: acetonitrile; flow rate: 60 mL /min; gradient: 40%B-70%B, 8 min; detection wavelength: 220 nm) to obtain 63 mg of an isomer mixture. Chiral resolution is performed on the isomer mixture by chiral liquid chromatography (column: CHIRALPAK IG, 2 cm×25 cm, with a filler particle size of 5 μm; mobile phase A: n-hexane (0.1% diethylamine), mobile phase B: ethanol; flow rate: 20 ml/min; gradient: 30%B within 23 min, isogradient; detection wavelength: 220/254 nm; column temperature: 25°C) to obtain:
isomer 101-1 (23.8 mg) having a HPLC retention time of 15.3 min.

$^1H$ NMR (400 MHz, DMSO-$d_6$, *ppm*): δ 11.16 (s, 1H), 9.30 (s, 1H), 8.65 (s, 1H), 8.21 (s, 1H), 7.66 (dd, J = 13.9, 2.8 Hz, 1H), 7.44 (d, J=12.0 Hz,1H), 7.37 (s, 1H), 7.24 (d, J = 7.9 Hz, 1H), 6.97 (d, J = 8.1 Hz, 1H), 3.58 (d, J = 10.1 Hz, 2H), 3.45 (d, J = 10.2 Hz, 2H), 3.30 (s, 2H), 3.07 (s, 2H), 2.47-2.30 (m, 2H), 1.85 (s, 7H), 1.82 (s. 4H), 1.76-1.65 (m, 4H). MS(ESI+): 636.2 (M+H).

Isomer 101-2 (18.6 mg) having a HPLC retention time of 19.7 min.

$^1H$ NMR (400 MHz, DMSO-$d_6$, *ppm):* δ 11.16 (s, 1H), 9.30 (s, 1H), 8.65 (s, 1H), 8.21 (s, 1H), 7.66 (dd, J = 13.9, 2.8 Hz, 1H), 7.44 (d, J=12.0 Hz,1H), 7.37 (s, 1H), 7.24 (d, J = 7.9 Hz, 1H), 6.97 (d, J = 8.1 Hz, 1H), 3.58 (d, J = 10.1 Hz, 2H), 3.45 (d, J = 10.2 Hz, 2H), 3.30 (s, 2H), 3.07 (s, 2H), 2.47-2.30 (m, 2H), 1.85 (s, 7H), 1.82 (s. 4H), 1.76-1.65 (m, 4H). MS(ESI+): 636.2 (M+H).

**Example 102** Compounds 102-1 and 102-2

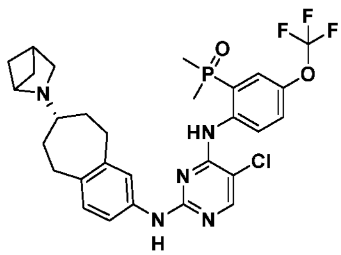

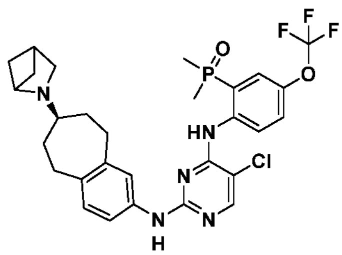

According to the preparation method of Example 58, (2-amino-5-fluorophenyl) dimethyl phosphine oxide in step a) is replaced with (2-amino-5-trifluoromethoxyphenyl) dimethyl phosphine oxide, and 2-azabicyclo[3.1.0]hexane hydrochloride in step c) is replaced with 3-azabicyclo[2.1.1]hexane hydrochloride. The crude product is purified by

reversed-phase high-performance liquid chromatography (column: YMC-Actus Triart C18 column, 30×150 mm, with a filler particle size of 5 μm; mobile phase A: 10 mmol/L ammonium bicarbonate aqueous solution, mobile phase B: acetonitrile; flow rate: 60 mL /min; gradient: 20%B-60%B, 8 min; detection wavelength: 254 nm) to obtain 75 mg of an isomer mixture. Chiral resolution is performed on the isomer mixture by chiral liquid chromatography (column: CHIRALPAK IG, 3 cm×25 cm, with a filler particle size of 5 μm; mobile phase A: n-hexane (0.1% diethylamine), mobile phase B: ethanol; flow rate: 20 ml/min; gradient: 20%B within 20 min, isogradient; detection wavelength: 220/254 nm; column temperature: 25°C) to obtain:

isomer 102-1 (18.6 mg) having a HPLC retention time of 15.7 min.

$^1$H NMR (400 MHz, DMSO-$d_6$, *ppm*): δ 11.16 (s, 1H), 9.31 (s, 1H), 8.66 (s, 1H), 8.21 (s, 1H), 7.66 (dd, *J* = 13.9, 2.8 Hz, 1H), 7.45 (d, *J* = 9.3 Hz, 1H), 7.40 (d, *J* = 2.2 Hz, 1H), 7.26 (d, *J* = 8.2 Hz, 1H), 6.98 (d, *J* = 8.1 Hz, 1H), 3.73 - 3.57 (m, 1H), 2.90 (s, 2H), 2.68 (d, *J* = 18.6 Hz, 3H), 2.55 (s, 3H), 1.83 (d, *J* = 13.7 Hz, 8H), 1.61 (s, 2H), 1.34 (s, 4H). MS(ESI+): 606.05(M+H).

Isomer 102-2 (18.7 mg) having a HPLC retention time of 18.3 min.

$^1$H NMR (400 MHz, DMSO-$d_6$, *ppm*): δ 11.16 (s, 1H), 9.31 (s, 1H), 8.66 (s, 1H), 8.21 (s, 1H), 7.66 (dd, *J* = 13.9, 2.9 Hz, 1H), 7.45 (d, *J* = 9.5 Hz, 1H), 7.40 (d, *J* = 2.2 Hz, 1H), 7.29 - 7.23 (m, 1H), 6.98 (d, *J* = 8.1 Hz, 1H), 3.65 (s, 1H), 2.90 (s, 2H), 2.68 (d, *J* = 19.3 Hz, 3H), 2.55 (s, 3H), 1.83 (d, *J* = 13.7 Hz, 8H), 1.63 (d, *J* = 15.2 Hz, 2H), 1.34 (s, 4H). MS(ESI+): 606.10(M+H).

**Example 103** Compounds 103-1 and 103-2

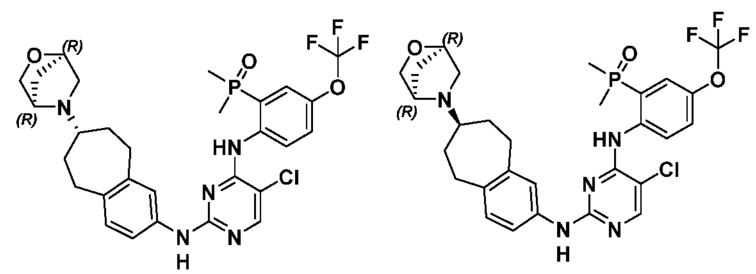

According to the preparation method of Example 58, (2-amino-5-fluorophenyl) dimethyl phosphine oxide in step a) is replaced with (2-amino-5-trifluoromethoxyphenyl) dimethyl phosphine oxide, and 2-azabicyclo[3.1.0]hexane hydrochloride in step c) is replaced with (1*R*, 4*R*)-2-oxa-5-azabicyclo[2.2.1]heptane hydrochloride. The crude product is purified by reversed-phase high-performance liquid chromatography (column: Xselect CSH OBD chromatographic column, 30×150 mm, with a filler particle size of 5 μm; mobile phase A: 10 mmol/L ammonium bicarbonate aqueous solution, mobile phase B: acetonitrile; flow rate: 60 mL/min; gradient: 30%B-65%B, 8 min; detection wavelength: 220 nm; isomer mixture retention time: 6.65 min) to obtain 70 mg of an isomer mixture.

Chiral resolution is performed on the isomer mixture by chiral liquid chromatography (column: CHIRALPAK IG, 2 cm×25 cm, with a filler particle size of 5 μm; mobile phase A: methyl tert-butyl ether (0.1% diethylamine); mobile phase B: ethanol; flow rate: 20 ml/min;

gradient: 10%B within 21 min, isogradient; detection wavelength: 220/254 nm; column temperature: 25°C) to obtain:

isomer 103-1 (21.2 mg) having a HPLC retention time of 13.5 min.

$^1$H-NMR (400MHz, DMSO-$d_6$, *ppm*): δ 11.14 (s, 1H), 9.30 (s, 1H), 8.65 (d, *J* = 9.3 Hz, 1H), 8.20 (s, 1H), 7.65 (dd, *J* = 13.8, 2.9 Hz, 1H), 7.44 (dd, *J* = 8.4, 2.6 Hz, 1H), 7.38 (d, *J* = 2.3 Hz, 1H), 7.26 (dd, *J* = 8.0, 2.3 Hz, 1H), 6.97 (d, *J* = 8.2 Hz, 1H), 4.33 (t, *J* = 1.9 Hz, 1H), 3.89 (d, *J* = 7.5 Hz, 1H), 3.71 - 3.66 (m, 1H), 3.53 (dd, *J* = 7.6, 1.6 Hz, 1H), 2.99 (dd, *J* = 9.5, 1.8 Hz, 1H), 2.87 (s, 2H), 2.69 (d, *J* = 6.9 Hz, 1H), 2.55 (s, 1H), 2.46 (s, 1H), 2.32 (d, *J* = 9.5 Hz, 1H), 1.83 (d, *J* = 13.8 Hz, 8H), 1.72 (dd, *J* = 9.3, 2.1 Hz, 1H), 1.65 - 1.58 (m, 1H), 1.55 - 1.28 (m, 2H). MS(ESI+): 622.2 (M+H).

Isomer 103-2 (21.7 mg) having a HPLC retention time of 17.8 min.

$^1$H NMR (400MHz, DMSO-$d_6$, *ppm*): δ 11.11 (s, 1H), 9.29 (s, 1H), 8.63 (t, *J* = 6.6 Hz, 1H), 8.20 (s, 1H), 7.65 (dd, *J* = 13.9, 2.8 Hz, 1H), 7.45 (dd, *J* = 9.2, 2.8 Hz, 1H), 7.39 (d, *J* = 2.3 Hz, 1H), 7.25 (dd, *J* = 8.0, 2.3 Hz, 1H), 6.96 (d, *J* = 8.1 Hz, 1H), 4.33 (t, *J* = 2.0 Hz, 1H), 3.89 (d, *J* = 7.5 Hz, 1H), 3.68 (d, *J* = 2.1 Hz, 1H), 3.53 (dd, *J* = 7.5, 1.6 Hz, 1H), 2.99 (dd, *J* = 9.6, 1.8 Hz, 1H), 2.88 (s, 2H), 2.68 (dt, *J* = 14.3, 7.9 Hz, 1H), 2.59 - 2.52 (m, 1H), 2.48 (s, 1H), 2.33 (d, *J* = 9.4 Hz, 1H), 1.83 (d, *J* = 13.7 Hz, 8H), 1.72 (dd, *J* = 9.4, 2.1 Hz, 1H), 1.61 (dd, *J* = 9.4, 2.4 Hz, 1H), 1.43 (s, 2H). MS(ESI+): 622.2(M+H).

**Example 104** Compounds 104-1 and 104-2

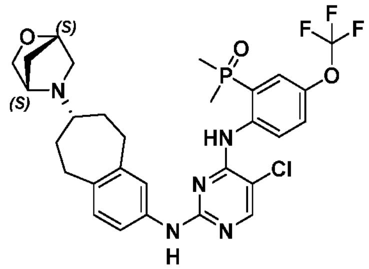

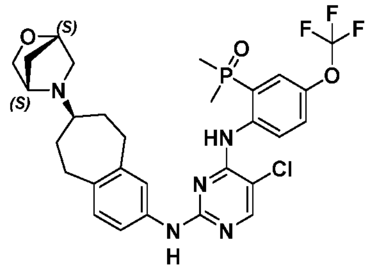

According to the preparation method of Example 58, (2-amino-5-fluorophenyl) dimethyl phosphine oxide in step a) is replaced with (2-amino-5-trifluoromethoxyphenyl) dimethyl phosphine oxide, and 2-azabicyclo[3.1.0]hexane hydrochloride in step c) is replaced with (1*S*, 4*S*)-2-oxa-5-azabicyclo[2.2.1]heptane hydrochloride. The crude product is purified by reversed-phase high-performance liquid chromatography (column: Xselect CSH OBD chromatographic column, 30×150 mm, with a filler particle size of 5 μm; mobile phase A: 10 mmol/L ammonium bicarbonate aqueous solution, mobile phase B: acetonitrile; flow rate: 60 mL/min; gradient: 30%B-65%B, 8 min; detection wavelength: 220 nm; isomer mixture retention time: 6.65 min) to obtain 75 mg of an isomer mixture.

Chiral resolution is performed on the isomer mixture by chiral liquid chromatography (column: CHIRALPAK ID, 2 cm×25 cm, with a filler particle size of 5 μm; mobile phase A: methyl tert-butyl ether (0.1% diethylamine); mobile phase B: ethanol; flow rate: 20 ml/min;
gradient: 10%B within 24 min, isogradient; detection wavelength: 220/254 nm; column temperature: 25°C) to obtain: isomer 104-1 (21.6 mg) having a HPLC retention time of 11.4 min.

$^1$H NMR (400MHz, DMSO-$d_6$, *ppm*): δ 11.29 (s, 1H),,9.51 (s, 1H), 8.66 (s, 1H), 8.24 (d, *J* = 1.4 Hz, 1H), 7.68 (dd, *J* = 13.8, 2.9 Hz, 1H), 7.53 -7.47 (m, 1H), 7.39 (q, *J* = 7.9, 6.3 Hz, 2H), 7.06 (t, *J* = 8.3 Hz, 1H), 4.64 (dd, *J* = 41.1, 26.0 Hz, 2H), 4.22 (t, *J* = 9.7 Hz, 1H), 3.66 (dd, *J* = 18.8, 9.5 Hz, 1H), 3.53 - 3.28 (m, 2H), 3.18 (d, *J* = 11.4 Hz, 1H), 2.85 - 2.66 (m, 4H), 2.27 (s, 2H), 2.17 (t, *J* = 9.6 Hz, 1H), 2.08 (d, *J* = 11.1 Hz, 1H), 1.84 (d, *J* = 13.7 Hz, 6H), 1.66 - 1.49 (m, 1H), 1.42 (p, *J* = 11.7 Hz, 1H). MS(ESI+): 622.1 (M+H).

Isomer 104-2 (22.8 mg) having a HPLC retention time of 21.3 min.

$^1$H NMR (400MHz, DMSO-$d_6$, *ppm*): 11.15 (s, 1H), 9.30 (s, 1H), 8.65 (s, 1H), 8.20 (s, 1H), 7.65 (dd, *J* = 13.8, 2.8 Hz, 1H), 7.47 - 7.42 (m, 1H), 7.39 (d, *J* = 2.2 Hz, 1H), 7.26 (dd, *J* = 8.2, 2.3 Hz, 1H), 6.97 (d, *J* = 8.1 Hz, 1H), 4.33 (t, *J* = 2.0 Hz, 1H), 3.90 (d, *J* = 7.5 Hz, 1H), 3.69 (d, *J* = 2.2 Hz, 1H), 3.53 (dd, *J* = 7.6, 1.7 Hz, 1H), 2.99 (dd, *J* = 9.4, 1.8 Hz, 1H), 2.88 (s, 2H), 2.69 (d, *J* = 7.1 Hz, 1H), 2.55 (s, 2H), 2.32 (d, *J* = 9.5 Hz, 1H), 1.83 (d, *J* = 13.7 Hz, 8H), 1.73 (d, *J* = 2.1 Hz, 1H), 1.65 - 1.58 (m, 1H), 1.56 - 1.33 (m, 2H). MS(ESI+): 622.1 (M+H).

**Example 105** Compounds 105-1 and 105-2

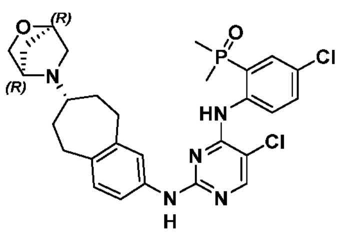

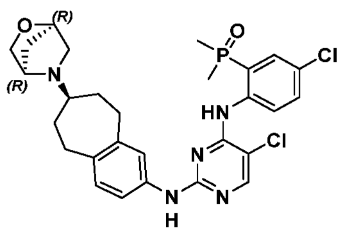

According to the preparation method of Example 58, (2-amino-5-fluorophenyl) dimethyl phosphine oxide in step a) is replaced with (2-amino-5-chlorophenyl) dimethyl phosphine oxide, and 2-azabicyclo[3.1.0]hexane hydrochloride in step c) is replaced with (1*R*, 4*R*)-2-oxa-5-azabicyclo[2.2.1]heptane hydrochloride. The crude product is purified by reversed-phase high-performance liquid chromatography (column: Xselect CSH OBD chromatographic column, 30×150 mm, with a filler particle size of 5 μm; mobile phase A: 10 mmol/L ammonium bicarbonate aqueous solution, mobile phase B: acetonitrile; flow rate: 60 mL/min; gradient: 3%B-73%B, 6 min; detection wavelength: 220 nm; isomer mixture retention time: 7.05 min) to obtain 70 mg of an isomer mixture.

Chiral resolution is performed on the isomer mixture by chiral liquid chromatography (column: CHIRALPAK IF, 2 cm×25 cm, with a filler particle size of 5 μm; mobile phase A: methyl tert-butyl ether (0.1% diethylamine); mobile phase B: ethanol; flow rate: 15 ml/min; gradient: 30%B within 18 min, isogradient; detection wavelength: 220/254 nm; column temperature: 25°C) to obtain:

isomer 105-1 (26.4 mg) having a HPLC retention time of 11.7 min.

$^1$H NMR (400MHz, DMSO-$d_6$, *ppm*): δ 11.10 (s, 1H), 9.31 (s, 1H), 8.56 (s, 1H), 8.19 (s, 1H), 7.69 (dd, *J* = 13.6, 2.6 Hz, 1H), 7.49 (dd, *J* = 9.0, 2.5 Hz, 1H), 7.41 (d, *J* = 2.3 Hz, 1H), 7.28 - 7.21 (m, 1H), 6.99 (d, *J* = 8.1 Hz, 1H), 4.34 (d, *J* = 2.5 Hz, 1H), 3.90 (d, *J* = 7.5 Hz, 1H), 3.70 (s, 1H), 3.54 (dd, *J* = 7.5, 1.6 Hz, 1H), 3.01 (dd, *J* = 9.4, 1.8 Hz, 1H), 2.88 (s, 2H), 2.71 (s, 1H), 2.64 - 2.54 (m, 2H), 2.34 (d, *J* = 9.3 Hz, 1H), 1.82 (d, *J* = 13.7 Hz, 8H), 1.73 (d, *J* = 9.9 Hz, 1H), 1.62 (d, *J* = 8.6 Hz, 1H), 1.41 (s, 2H). MS(ESI+): 572.10(M+H).

Isomer 105-2 (23.0 mg) having a HPLC retention time of 16.0 min.

$^1$H NMR (400MHz, DMSO-$d_6$, *ppm*): δ 11.09 (s, 1H), 9.30 (s, 1H), 8.56 (s, 1H), 8.19 (s, 1H), 7.69 (dd, *J* = 13.6, 2.5 Hz, 1H), 7.50 (dd, *J* = 9.0, 2.5 Hz, 1H), 7.41 (d, *J* = 2.3 Hz, 1H), 7.24 (dd, *J* = 7.7, 1.9 Hz, 1H), 6.98 (d, *J* = 8.1 Hz, 1H), 4.33 (t, *J* = 1.9 Hz, 1H), 3.90 (d, *J* = 7.4 Hz, 1H), 3.71 (s, 1H), 3.54 (dd, *J* = 7.5, 1.6 Hz, 1H), 3.00 (dd, *J* = 9.5, 1.8 Hz, 1H), 2.85 (d, *J* = 31.5 Hz, 2H), 2.71 (s, 1H), 2.55 (s, 2H), 2.34 (d, *J* = 9.4 Hz, 1H), 1.82 (d, *J* = 13.7 Hz, 8H), 1.73 (d, *J* = 9.1 Hz, 1H), 1.66 - 1.59 (m, 1H), 1.43 (s, 2H). MS(ESI+):572.10(M+H).

**Example 106** Compounds 106-1 and 106-2

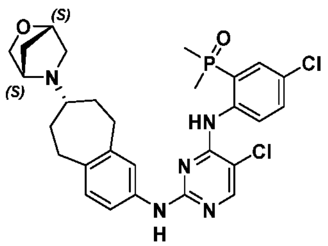

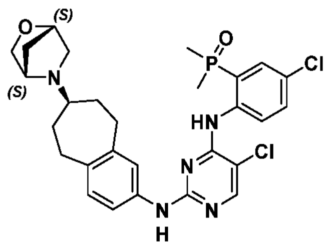

According to the preparation method of Example 58, (2-amino-5-fluorophenyl) dimethyl phosphine oxide in step a) is replaced with (2-amino-5-chlorophenyl) dimethyl phosphine oxide, and 2-azabicyclo[3.1.0]hexane hydrochloride in step c) is replaced with (1*S*, 4*S*)-2-oxa-5-azabicyclo[2.2.1]heptane hydrochloride. The crude product is purified by reversed-phase high-performance liquid chromatography (column: Xselect CSH OBD column, 30×150 mm, with a filler particle size of 5 μm; mobile phase A: 10 mmol/L ammonium bicarbonate aqueous solution, mobile phase B: acetonitrile; flow rate: 60 mL/min; gradient: 3%B-73%B, 6 min; detection wavelength: 220 nm; isomer mixture retention time: 7.05 min) to obtain 80 mg of an isomer mixture.

Chiral resolution is performed on the isomer mixture by chiral liquid chromatography (column: CHIRALPAK IF, 2 cm×25 cm, with a filler particle size of 5 μm; mobile phase A: methyl tert-butyl ether (0.1% diethylamine); mobile phase B: ethanol; flow rate: 15 ml/min; gradient: 30%B within 18 min, isogradient; detection wavelength: 220/254 nm; column temperature: 25°C) to obtain:

isomer 106-1 (25.6 mg) having a HPLC retention time of 11.7 min.

$^1$H NMR (400 MHz, DMSO-$d_6$, *ppm*): δ 11.10 (s, 1H), 9.31 (s, 1H), 8.56 (s, 1H), 8.20 (s, 1H), 7.69 (dd, *J* = 13.7, 2.6 Hz, 1H), 7.50 (dd, *J* = 9.0, 2.5 Hz, 1H), 7.42 (d, *J* = 2.3 Hz, 1H), 7.25 (dd, *J* = 8.0, 2.2 Hz, 1H), 6.98 (d, *J* = 8.1 Hz, 1H), 4.41 - 4.30 (m, 1H), 3.91 (d, *J* = 7.6 Hz, 1H), 3.72 (s, 1H), 3.60 - 3.50 (m, 1H), 3.01 (d, *J* = 9.4 Hz, 1H), 2.80 (d, *J* = 61.2 Hz, 3H), 2.54 (s, 2H), 2.37-2.33(m,1H),1.82 (d, *J* = 13.7 Hz, 8H), 1.74 (d, *J* = 9.3 Hz, 1H), 1.63 (d, *J* = 9.3 Hz, 1H), 1.43 (s, 2H). MS(ESI+): 572.10(M+H).

Isomer 106-2 (27.9 mg) having a HPLC retention time of 16.0 min.

$^1$H NMR (400 MHz, DMSO-$d_6$, *ppm*): δ 11.10 (s, 1H), 9.31 (s, 1H), 8.56 (s, 1H), 8.20 (s, 1H), 7.69 (dd, *J* = 13.7, 2.6 Hz, 1H), 7.50 (dd, *J* = 9.0, 2.5 Hz, 1H), 7.42 (d, *J* = 2.3 Hz, 1H), 7.25 (dd, *J* = 8.0, 2.2 Hz, 1H), 6.98 (d, *J* = 8.1 Hz, 1H), 4.41 - 4.30 (m, 1H), 3.91 (d, *J* = 7.6 Hz, 1H), 3.72 (s, 1H), 3.60 - 3.50 (m, 1H), 3.01 (d, *J* = 9.4 Hz, 1H), 2.80 (d, *J* = 61.2 Hz, 3H), 2.54 (s, 2H), 2.37-2.33(m,1H),1.82 (d, *J* = 13.7 Hz, 8H), 1.74 (d, *J* = 9.3 Hz, 1H), 1.63 (d, *J* = 9.3 Hz, 1H), 1.43 (s, 2H). MS(ESI+):572.10(M+H).

**Example 107** Preparation of (S)-(4-((5-chloro-2-((7-(pyrrolidin-1-yl)-6,7,8,9-tetrahydro-5H-benzo[7]annulen-2-yl)amino)pyrimidin-4-yl)amino)pyridin-3-yl)dimethyl phosphine oxide

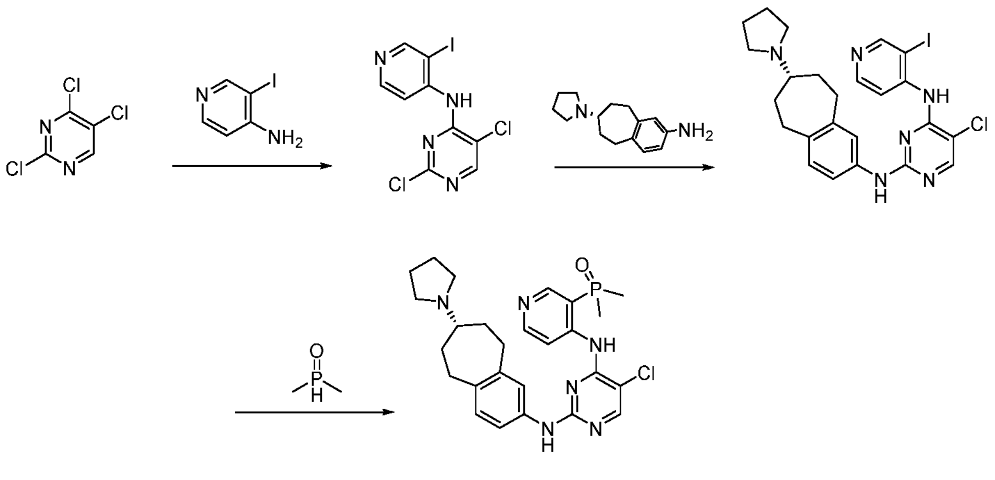

a) Preparation of 2,5-dichloro-N-(3-iodopyridin-4-yl)pyrimidin-4-amine

2,4,5-trichloropyrimidine (200 mg), 3-iodopyridine-4-amine (254 mg), and tetrahydrofuran (10 ml) are sequentially added to a reaction flask, added dropwise with lithium bis(trimethylsilyl)amide (2.2 ml, 1M) at -10°C, and reacted for 1 h. After the reaction is completed, the mixture is quenched by adding a saturated ammonium chloride solution (5 ml), and extracted with ethyl acetate (5 ml*2). After the solution is separated, the organic layers are merged, and dried with anhydrous sodium sulfate. The organic phase is concentrated to dryness. The residue is purified by silica-gel column chromatography (mobile phase: dichloromethane/methanol = 50/1) to obtain 216 mg of the title product.

b) Preparation of (S)-5-chloro-$N^4$-(3-iodopyridin-4-yl)-$N^2$-(7-(pyrrolidin-1-yl)-6,7,8,9-tetrahydro-5H-benzo[7]annulen-2-yl)pyrimidine-2,4-diamine

A compound 2,5-dichloro-N-(3-iodopyridin-4-yl)pyrimidin-4-amine (176 mg), (S)-7-(pyrrolidin-1-yl)-6,7,8,9-tetrahydro-5H-benzo[7]annulen-2-amine (110 mg), n-butanol (5 ml), and trifluoroacetic acid (0.1 ml) are sequentially added to a reaction flask, and reacted in 400W microwaves at 120°C for 5 h. After the reaction is completed, the reactant is concentrated to dryness, and purified by column chromatography (mobile phase: dichloromethane/methanol=50/1) to obtain 86 mg of the title product.

c) Preparation of (S)-(4-((5-chloro-2-((7-(pyrrolidin-1-yl)-6,7,8,9-tetrahydro-5H-benzo[7]annulen-2-yl)amino)pyrimidin-4-yl)amino)pyridin-3-yl)dimethyl phosphine oxide

(S)-5-chloro-$N^4$-(3-iodopyridin-4-yl)-$N^2$-(7-(pyrrolidin-1-yl)-6,7,8,9-tetrahydro-5H-benzo[7]annulen-2-yl)pyrimidine-2,4-diamine (40 mg), dimethyl phosphine oxide (11 mg), potassium phosphate (22 mg), palladium acetate (2 mg), 4,5-bis(diphenylphosphine)-9,9-dimethylxanthene (4 mg), and N,N-dimethylformamide (10 ml) are sequentially added to a reaction flask, heated to 100°C in the atmosphere of nitrogen, and reacted for 3 h. After the reaction is completed, the reactant is concentrated to dryness, and purified by silica-gel column chromatography (dichloromethane/methanol=20: 1 v/v) to obtain 40 mg of the title product.

$^1$H NMR (400 MHz, DMSO-$d_6$, *ppm*): δ 9.49 (s, 1H), 8.77 (s, 1H), 8.67 (d, J = 8.4 Hz, 1H), 8.47 (d, J = 5.6 Hz, 1H), 8.28 (s, 1H), 8.22 (s, 1H), 7.43 (s, 1H), 7.34 (d, J = 7.0 Hz, 1H), 7.07 (d, J = 8.1 Hz, 1H), 2.83 (s, 7H), 2.62 (s, 2H), 2.04 (s, 2H), 1.88 (d, J = 13.8 Hz, 6H), 1.77 (s, 4H), 1.49 (dd, J = 14.1, 3.7 Hz, 2H). MS(ESI+): 511.2(M+H).

**Example 108** (S)-(4-((5-chloro-2-((7-(pyrrolidin-1-yl)-6,7,8,9-tetrahydro-5H-benzo[7] annulen-2-yl)amino)pyrimidin-4-yl)amino)-1,3-phenylene)bis(dimethyl phosphine oxide)

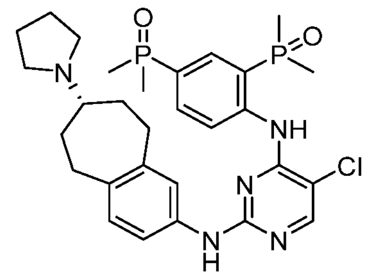

According to the preparation method of Example 58, (2-amino-5-fluorophenyl) dimethyl phosphine oxide in step a) is replaced with (4-amino-1,3-phenylene)bis(dimethyl phosphine oxide).

$^1H$ NMR (400 MHz,$CDCl_3$): $\delta$8.81 (ddd, *J* = 8.7, 4.0, 2.4 Hz, 1H), 7.81 (ddd, *J* = 13.7, 11.4, 1.9 Hz, 1H), 7.74 - 7.67 (m, 1H), 7.63 - 7.54 (m, 1H), 7.33 (dd, *J* = 14.2, 2.3 Hz, 2H), 7.17 -7.11 (m, 2H), 7.03 (d, *J* = 8.1 Hz, 1H), 3.19 (m, 4H), 2.91 - 2.78 (m, 2H), 2.71 (m, 2H), 2.32 (m, 4H), 2.01 (m, 4H), 1.91 (s, 3H), 1.88 (s, 3H), 1.77 (s, *J* = 1.7 Hz, 3H), 1.74 (s, *J* = 1.7 Hz, 3H), 1.61 (m, 2H). MS(ESI+): 586.3(M+H).

**Example 109** (S)-(2-((5-chloro-2-((7-(pyrrolidin-1-yl)-6,7,8,9-tetrahydro-5H-benzo[7]annulen -2-yl)amino)pyrimidin-4-yl)amino)-5-(1H-tetrazol-1-yl)phenyl)dimethyl phosphine oxide

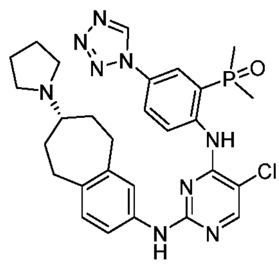

According to the preparation method of Example 1, 2-amino-N,N-dimethyl benzenesulfonamide in step a) is replaced with (2-amino-5-(1H-tetrazol-1-yl)phenyl)dimethyl phosphine oxide.

$^1H$ NMR (400 MHz, $CDCl_3$): $\delta$ 11.23 (s, 1H), 9.47 (s, 1H), 8.91 (dd, J = 8.9, 4.4 Hz, 1H), 8.15 (s, 1H), 7.81 - 7.66 (m, 2H), 7.43 (d, J = 2.3 Hz, 1H), 7.20 - 7.08 (m, 2H), 7.04 (d, J = 8.1 Hz, 1H), 3.13 (m, 4H), 2.89 - 2.84 (m, 1H), 2.78 - 2.64 (m, 4H), 2.33 (m, 2H), 2.02 (m, 4H), 1.96 (s, 3H), 1.93 (s, 3H), 1.66 (m, 2 H).MS(ESI+): 578.2 (M+H).

**Example 110** (S)-(5-bromo 2-((5-chloro-2-((7-(pyrrolidin-1-yl)-6,7,8,9-tetrahydro-5H-benzo[7]annulen-2-yl)amino)pyrimidin-4-yl)amino)phenyl)dimethyl phosphine oxide

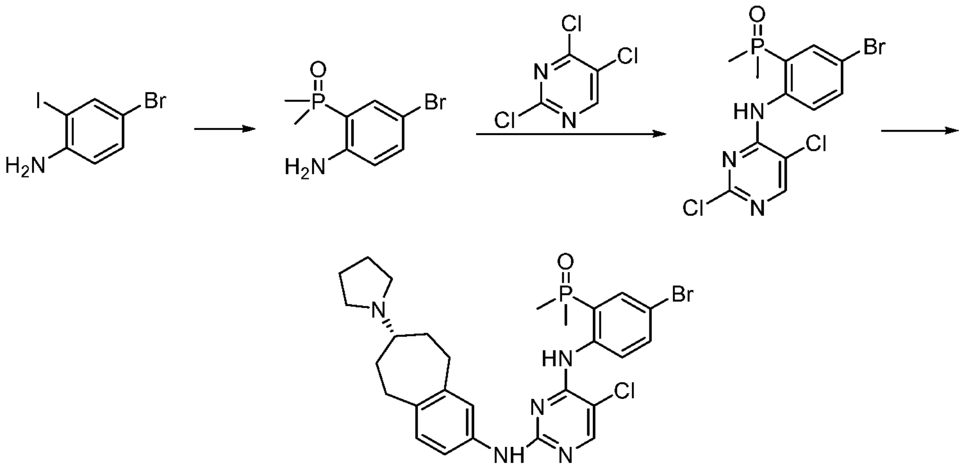

a) (2-amino-5-bromophenyl) dimethyl phosphine oxide

An intermediate 4-bromo-2-iodoaniline (1.5 g), dimethyl phosphine oxide (500 mg), tris(dibenzylideneacetone) dipalladium (480 mg), 4,5-bis(diphenylphosphine)-9,9-dimethylxanthene (900 mg), triethylamine (101 mg) and dioxane (30 mL) are added to a 100 mL single-necked bottle. The reaction solution is placed at 60°C and reacted for 3 h under the protection of nitrogen. The reaction solution is concentrated to dryness, and the residue is purified by silica-gel column chromatography (dichloromethane/methanol=20: 1 v/v) to obtain 927 mg of the title product.

b) (5-bromo-2-((2,5-dichloropyrimidin-4-yl)amino)phenyl)dimethyl phosphine oxide

An intermediate (2-amino-5-bromophenyl) dimethyl phosphine oxide (1.36 g), 2,4,5-trichloropyrimidine (1.3 g), *N,N*-dimethylformamide (15 mL) and *N,N*-diisopropylethylamine (600 mg) are placed in a 30 mL microwave tube under the protection of nitrogen. After the reaction solution is subjected to a microwave reaction at 110°C for 1.5 h, the reaction is stopped. The reaction solution is concentrated to dryness, and the residue is slurried with 10 mL of methanol and filtered by suction to obtain 411 mg of the title product.

c) (S)-(5-bromo -2-((5-chloro-2-((7-(pyrrolidin-1-yl)-6,7,8,9-tetrahydro-5H-benzo[7]annulen-2-yl)amino)pyrimidin-4-yl)amino)phenyl)dimethyl phosphine oxide

An intermediate (5-bromo-2-((2,5-dichloropyrimidin-4-yl)amino)phenyl)dimethyl phosphine oxide (120 mg), (S)-7-(pyrrolidine-1-yl)-6,7,8,9-tetrahydro-5H-benzo[7]annulen-2-amine (69 mg), n-butanol (4 mL) and p-toluenesulfonic acid monohydrate (120 mg) are added to 10 mL microwave tube. The microwave reaction is performed at 120°C for 1h under the protection of nitrogen. The reaction solution is concentrated to dryness, and the residue is added with ethyl acetate (20 mL) and then washed with 2N sodium hydroxide (10 mL) for three times. The organic layer is concentrated to dryness, and the residue is purified by silica-gel column chromatography (dichloromethane/methanol=20: 1 v/v) to obtain 200 mg of the title product.

$^1$H NMR (400 MHz, DMSO-$d_6$, *ppm*): δ 11.11 (s, 1H), 9.31 (s, 1H), 8.49 (s, 1H), 8.19 (s, 1H), 7.79 (dd, J = 13.5, 2.4 Hz, 1H), 7.60 (dd, J = 9.0, 2.4 Hz, 1H), 7.43 (d, J = 2.3 Hz, 1H), 7.22 (d, J = 8.1 Hz, 1H), 6.98 (d, J = 8.1 Hz, 1H), 2.96-2.66 (m, 5H), 2.59 - 2.54 (m, 4H), 1.87 (s, 2H), 1.83 (s, 3H), 1.80 (s, 3H), 171-1.67 (m, 4H), 1.53 (br, 2H). MS(ESI+): 588.1 (M+H).

**Example 111** (S)-(2-((5-chloro-2-((7-(pyrrolidin-1-yl)-6,7,8,9-tetrahydro-5H-benzo[7] annulen-2-yl)amino)pyrimidin-4-yl)amino)phenyl-5-d)dimethyl phosphine oxide

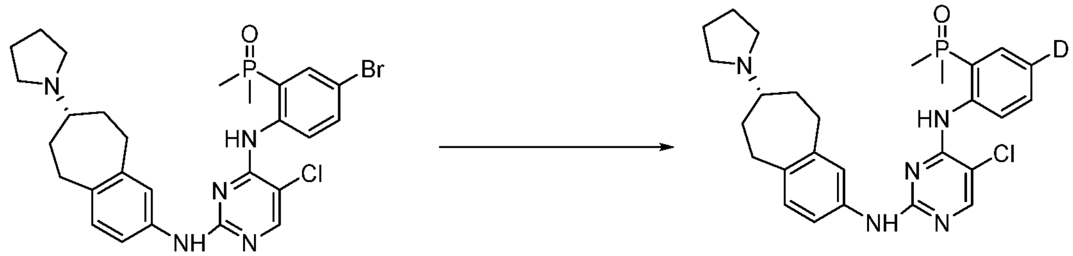

An intermediate (S)-(5-bromo-2-((5-chloro-2-((7-(pyrrolidin-1-yl)-6,7,8,9-tetrahydro-5H-benzo[7] annulen-2-yl)amino)pyrimidin-4-yl)amino)phenyl)dimethyl phosphine oxide (100 mg), deuterated methanol (0.5 mL), palladium acetate (4 mg), butyldi-1-adamantylphosphine (12 mg), potassium phosphate (72 mg) and toluene (4 mL) are added to a 25 mL single-necked bottle. The reaction solution is placed at 80°C and reacted for 8 h under the protection of nitrogen. The reaction solution is desolventized to dryness. The reaction solution is concentrated to dryness. The residue is purified by silica-gel column chromatography (dichloromethane/methanol=20: 1 v/v) to obtain 40 mg of the title product. $^1$H NMR (400 MHz, DMSO-$d_6$, *ppm*): δ 11.10 (s, 1H), 9.27 (s, 1H), 8.57 (s, 1H), 8.17 (s, 1H), 7.69-7.57 (m, 1H), 7.49 (d, J = 8.6 Hz, 1H), 7.38 (s, 1H), 7.31 (d, J = 8.6 Hz, 1H), 6.97 (d, J = 8.0 Hz, 1H), 2.94 - 2.74 (m, 5H), 2.61 - 2.55 (m, 4H), 1.94 - 1.73 (m, 8H), 1.73 - 1.62(s, 4H),1.54-1.47 (m, 2H). MS(ESI+): 511.1(M+H).

**Example 112** (S)-(2-((5-chloro-2-((7-(pyrrolidin-1-yl)-6,7,8,9-tetrahydro-5H-benzo[7] annulen-2-yl)amino)pyrimidin-4-yl)amino)-5-cyclopropylphenyl) dimethyl phosphine oxide

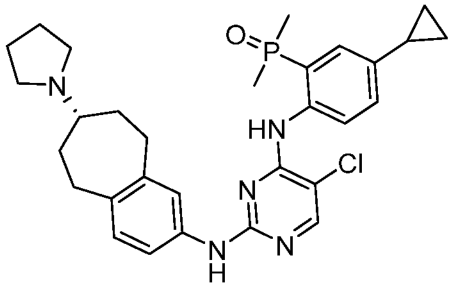

According to the preparation method of Example 1, 2-amino-N,N-dimethyl benzenesulfonamide in step a) is replaced with (2-amino-5-cyclopropylphenyl) dimethyl phosphine oxide.

$^1$H NMR (400 MHz, DMSO-*d*$_6$, *ppm*): δ 10.91 (s, 1H), 9.24 (s, 1H), 8.25 (s, 1H), 8.14 (s, 1H), 7.44 (d, J = 2.3 Hz, 1H), 7.31 (dd, J = 14.3, 2.2 Hz, 1H), 7.25 (dd, J = 8.1, 2.3 Hz, 1H), 7.14 (dd, J = 8.6, 2.2 Hz, 1H), 6.97 (d, J = 8.2 Hz, 1H), 2.87 (m, 1H), 2.73 (m, 6H), 2.62 - 2.52 (m, 2H), 2.05 - 1.92 (m, 3H), 1.77 (m, 10H), 1.51 (s, 2H), 1.01 - 0.95 (m, 2H), 0.76 - 0.70 (m, 2H). MS(ESI+): 550.2(M+H).

**Example 113** (S)-(2-((5-chloro-2-((7-(pyrrolidin-1-yl)-6,7,8,9-tetrahydro-SH-benzo[7] annulen-2-yl)amino)pyrimidin-4-yl)amino)-6-methylphenyl) dimethyl phosphine oxide

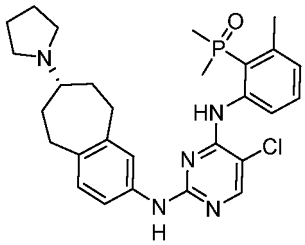

According to the preparation method of Example 1, 2-amino-N,N-dimethyl benzenesulfonamide in step a) is replaced with (2-amino-6-methylphenyl) dimethyl phosphine oxide.

$^1$H NMR (400 MHz, Chloroform-d) δ 11.96 (s, 1H), 8.38 (dd, *J* = 8.5, 3.9 Hz, 1H), 8.06 (s, 1H), 7.40 - 7.33 (m, 1H), 7.31 - 7.27 (m, 2H), 7.01 (d, *J* = 8.7 Hz, 1H), 6.93 (dd, *J* = 7.5, 3.9 Hz, 1H), 6.84 (s, 1H), 2.93 - 2.72 (m, 6H), 2.66 (t, *J* = 12.8 Hz, 2H), 2.46 (s, 3H), 2.17 (s, 2H), 1.98 (s, 3H), 1.94 (s, 3H), 1.86 (s, 5H), 1.57 - 1.50 (m, 2H). MS(ESI+): 524.2(M+H).

**Example 114** (S)-(2-((5-chloro-2-((7-(pyrrolidin-1-yl)-6,7,8,9-tetrahydro-5H-benzo[7] annulen-2-yl)amino)pyrimidin-4-yl)amino)-5-(isopropyloxyl)phenyl dimethyl phosphine oxide

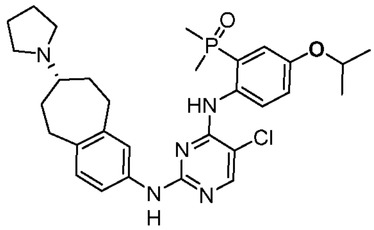

According to the preparation method of Example 1, 2-amino-N,N-dimethyl benzenesulfonamide in step a) is replaced with (2-amino-5-isopropyloxylphenyl) dimethyl phosphine oxide.

$^1H$ NMR (400 MHz, DMSO-$d_6$, *ppm):* δ 10.42 (s, 1H), 8.43 (dd, J = 9.2, 5.1 Hz, 1H), 8.05 (s, 1H), 7.29 (d, J = 7.8 Hz, 2H), 7.06 - 6.98 (m, 2H), 6.89 (s, 1H), 6.81 (dd, J = 15.0, 2.9 Hz, 1H), 4.54 (p, J = 6.0 Hz, 1H), 2.86 (s, 7H), 2.71 - 2.63 (m, 2H), 2.19 (s, 2H), 1.88 (d, J = 6.2 Hz, 4H), 1.83 (s, 3H), 1.80 (s, 3H), 1.57 (s, 2H), 1.38 (s, 3H), 1.36 (s, 3H). MS(ESI+):

568.3(M+H).

**Example 115** (S)-(2-((5-chloro-2-((7-(pyrrolidin-1-yl)-6,7,8,9-tetrahydro-5H-benzo[7]annulen-2-yl)amino)pyrimidin-4-yl)amino)-5-(methoxymethyl)phenyl)dimethylphosphine oxide

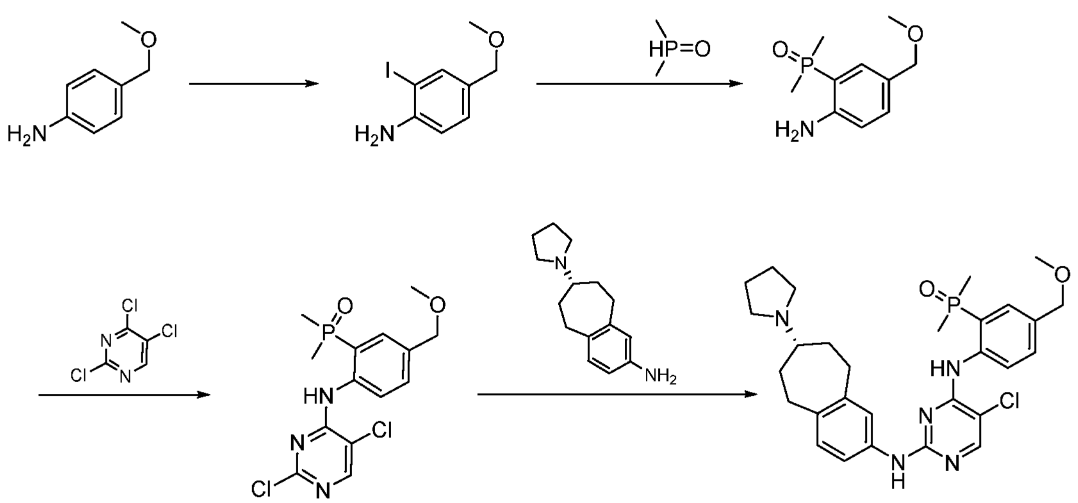

a) 2-iodo-4-(methoxymethyl)aniline

4-(methoxymethyl)aniline (9 g), iodine (16.65 g) and sodium bicarbonate (16.53 g) are added to a dichloromethane (261 mL)/water (135 mL) solution, and stirred at 22°C for 16 h. The reaction solution is quenched with saturated sodium thiosulfate (10 ml) at room temperature. The resulting mixture is extracted with dichloromethane (3×100 mL). The merged organic layer is then washed with a saturated aqueous sodium chloride solution (1×100 mL). The organic layer is then dried with anhydrous sodium sulfate. After filtration, the filtrate is concentrated under reduced pressure. The residue is purified by silica-gel column chromatography (petroleum ether: ethyl acetate = 1: 1 v/v) to obtain the title product (16 g). MS(ESI+): 264.0(M+H).

b) (2-amino-5-(methoxymethyl)phenyl)dimethyl phosphine oxide

Dimethyl phosphine oxide (5.22 g) is added to N,N-dimethylformamide (224 mL) which was added with 2-iodo-4-(methoxymethyl)aniline (16 g, 60.82 mmol, 1.00 equivalent), potassium phosphate (14.20 g), palladium acetate (0.68 g) and 4,5-bisdiphenylphosphine-9,9-dimethylxanthene (1.76 g) in the atmosphere of nitrogen and stirred, reacted under stirring at 120°C for 2 h. The mixture is cooled to room temperature. The mixture is filtered, and a filter cake is washed with N,N-dimethylformamide (3×5 mL). The filtrate is concentrated under reduced pressure. The residue is purified by silica-gel column chromatography (dichloromethane/methanol=20: 1 v/v) to obtain the title product (12.9 g). MS(ESI+): 214.1(M+H).

c) (2-((2,5-dichloropyrimidin-4-yl)amino)-5-(methoxymethyl)phenyl)dimethyl phosphine oxide

(2-((2,5-dichloropyrimidin-4-yl)amino)-5-(methoxymethyl)phenyl)dimethyl phosphine oxide (1.10 g), 2,4,5-trichloropyrimidine (1.23 g) and N,N-diisopropylethylamine (2.00 g) are added to N,N-dimethylformamide (22 mL) at room temperature, and stirred for 3 h. The resulting mixture is diluted with dichloromethane (30 mL). The reaction is quenched by adding water (10 ml) at 0°C. The resulting mixture is extracted with dichloromethane (3×50 mL). The merged organic layer is washed with saturated sodium chloride (1×50 mL) and dried with anhydrous sodium sulfate. After filtration, the filtrate is concentrated under reduced pressure. The residue is purified by silica-gel column chromatography (dichloromethane/methanol=20: 1 v/v) to obtain the title product (1.28 g).

MS(ESI+): 360.0(M+H).

d) (S)-(2-((5-chloro-2-((7-(pyrrolidin-1-yl)-6,7,8,9-tetrahydro-5H-benzo[7]annulen-2-yl)amino)pyrimidin-4-yl)amino)-5-(methoxymethyl)phenyl) dimethyl phosphine oxide

(S)-(2-((5-chloro-2-((7-(pyrrolidin-1-yl)-6,7,8,9-tetrahydro-5H-benzo[7]annulen-2-yl)amino)pyrimidin-4-yl)amino)-5-(methoxymethyl)phenyl) dimethyl phosphine oxide (50.00 mg) and (S)-7-(pyrrolidin-1-yl)-6,7,8,9-tetrahydro-5H-benzo[7]annulen-2-amine (31.98 mg) are added to isopropanol (2 mL), then added with a 1,4-dioxane solution (10 drops, 4M) of hydrogen chloride, and irradiated with microwaves at 130°C for 3.5 h. The mixture is cooled to room temperature and concentrated under reduced pressure. The crude product is purified by reversed-phase high-performance liquid chromatography (column: YMC Actus Triart C18, 30*150 mm, with a filler particle size of 5 μm; mobile phase A: water (10 mmol/L ammonium bicarbonate), mobile phase B: acetonitrile; flow rate: 60 mL/min; gradient: 20%B to 50%B within 8 min; wavelength: 220 nm; retention time: 6.83 min; column temperature: 25°C) to obtain the title product (20.2 mg).

$^1$H NMR (400 MHz, DMSO-$d_6$, *ppm*): δ 11.07 (s, 1H), 9.26 (s, 1H), 8.52 (d, *J* = 4.6 Hz, 1H), 8.17 (s, 1H), 7.53 (dd, *J* = 14.0, 2.0 Hz, 1H), 7.44 (q, *J* = 3.1 Hz, 2H), 7.26 (dd, *J* = 8.1, 2.3 Hz, 1H), 6.97 (d, *J* = 8.1 Hz, 1H), 4.42 (s, 2H), 3.31 (s, 3H), 3.01 - 2.75 (m, 2H), 2.55 (s, 5H), 2.50 (s, 2H), 1.84 (s, 2H), 1.81 (s, 3H), 1.77 (s, 3H), 1.70 (q, *J* = 3.6, 3.2 Hz, 4H), 1.54 (s, 2H). MS(ESI+): 554.2(M+H).

**Example 116** Compounds 116-1 and 116-2

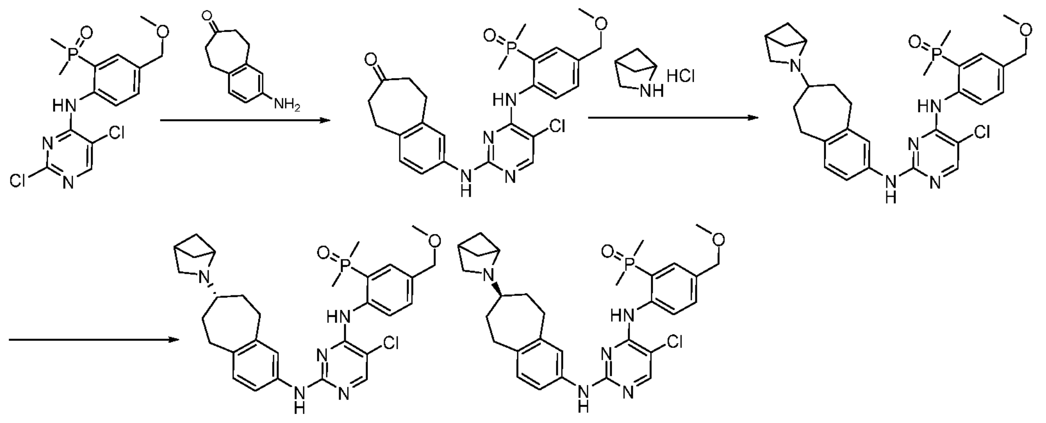

a) 2-[(5-chloro-4-[[2-(dimethylphosphoryl)-4-(methoxymethyl)phenyl]amino]pyrimidin-2-yl)amino]-5,6,8,9-tetrahydrobenzo[7]annulen-7-one

(2-((2,5-dichloropyrimidin-4-yl)amino)-5-(methoxymethyl)phenyl)dimethyl phosphine oxide (1.10 g) and 2-amino-5,6,8,9-tetrahydrobenzo[7]annulen-7-one (0.54 g) are added to isopropanol (20 mL), then added with a 1,4-dioxane solution (10 drops, 4M) of hydrogen chloride, and irradiated with microwaves at 130°C for 0.5 h. The mixture is then cooled to room temperature, and filtered, and the precipitated solid is collected and washed with isopropanol (1×10 mL) to obtain the title product (1 g). MS(ESI+): 499.2(M+H).

b) (2-((2-((7-(2-azabicyclo[2.1.1]hexan-2-yl)-6,7,8,9-tetrahydro-5H-benzo[7]annulen-2-yl)amino)-5-chloropyrimidin-4-yl)amino)-5-(methoxymethyl)phenyl)dimethyl phosphine oxide

According to the preparation method in step c) of Example 58, 2-azabicyclo[3.1.0]hexane hydrochloride in step c) is replaced with 2-azabicyclo[2.1.1]hexane hydrochloride. The crude product is purified by reversed-phase high-performance liquid chromatography (column: XBridge-Prep-OBD C18 column, 30×150 mm, with a filler particle size of 5 μm; mobile phase A: 10 mmol/L ammonium bicarbonate aqueous solution, mobile phase B: acetonitrile (0.1% formic acid)); flow rate: 60 mL /min; gradient: 5%B-45%B, 8 min; detection wavelength: 220 nm; column temperature: 25°C) to obtain an isomer mixture.

c) Chiral resolution is performed on the isomer mixture by chiral liquid chromatography (column: CHIRALPAK ID, 2 cm×25 cm, with a filler particle size of 5 μm; mobile phase A: methyl tert-butyl ether (0.1% diethylamine), mobile phase

B: ethanol; flow rate: 20 ml/min; gradient: 25%B within 13 min, isogradient; detection wavelength: 220/254 nm; column temperature: 25°C) to obtain:
isomer 116-1 (47.4 mg) having a HPLC retention time of 8.6 min.

$^1$H NMR (400 MHz, DMSO-$d_6$, *ppm*): δ 11.10 (s, 1H), 9.31 (s, 1H), 8.56 (s, 1H), 8.20 (s, 1H), 7.69 (dd, J= 13.7, 2.6 Hz, 1H), 7.50 (dd, *J* = 9.0, 2.5 Hz, 1H), 7.42 (d, *J* = 2.3 Hz, 1H), 7.25 (dd, *J* = 8.0, 2.2 Hz, 1H), 6.98 (d, *J* = 8.1 Hz, 1H), 4.41 - 4.30 (m, 1H), 3.91 (d, *J* = 7.6 Hz, 1H), 3.72 (s, 1H), 3.60 - 3.50 (m, 1H), 3.01 (d, *J* = 9.4 Hz, 1H), 2.80 (d, *J* = 61.2 Hz, 3H), 2.54 (s, 2H), 2.37-2.33(m,1H),1.82 (d, *J* = 13.7 Hz, 8H), 1.74 (d, *J* = 9.3 Hz, 1H), 1.63 (d, *J* = 9.3 Hz, 1H), 1.43 (s, 2H). MS(ESI+): 566.2(M+H).

Isomer 116-2 (38.2 mg) having a HPLC retention time of 10.8 min.

$^1$H NMR (400 MHz, DMSO-$d_6$, *ppm*): δ 11.07 (s, 1H), 9.27 (s, 1H), 8.53 (d, *J* = 8.3 Hz, 1H), 8.18 (s, 1H), 7.54 (dd, *J* = 14.0, 2.0 Hz, 1H), 7.45 (dt, *J* = 5.0, 2.6 Hz, 2H), 7.27 (d, *J* = 8.1 Hz, 1H), 6.98 (d, *J* = 8.1 Hz, 1H), 4.43 (s, 2H), 3.66 (s, 1H), 3.32 (s, 3H), 2.89 (s, 2H), 2.75 - 2.62 (m, 3H), 2.52 (s, 2H), 2.50 (s, 1H), 1.91 (s, 2H), 1.79 (d, *J* = 13.5 Hz, 6H), 1.62 (s, 2H), 1.34 (s, 4H). MS(ESI+): 566.2(M+H).

**Example 117** (S)-(4-chloro-2-((5-chloro-2-((7-(pyrrolidin-1-yl)-6,7,8,9-tetrahydro-5H-benzoannulen-2-yl)amino)pyrimidin-4-yl)amino)-6-methylphenyl)dimethyl phosphine oxide

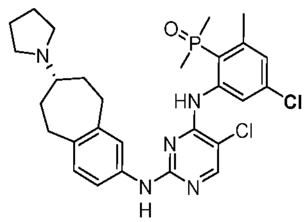

According to the preparation method of Example 115, 4-(methoxymethyl)aniline in step a) is replaced with 5-chloro-3-methylaniline.

$^1$H NMR (400 MHz, DMSO-$d_6$, *ppm*): δ 12.11 (s, 1H), 9.30 (s, 1H), 8.29 (dd, J = 6.2, 3.7 Hz, 1H), 8.17 (s, 1H), 7.40 (dd, J = 8.1, 2.3 Hz, 1H), 7.22 (d, J = 2.4 Hz, 1H), 7.12 (s, 1H), 6.97 (d, J = 8.2 Hz, 1H), 2.87 - 2.76 (m, 2H), 2.68 (d, J = 6.0 Hz, 4H), 2.62 (s, 1H), 2.54 (s, 2H), 2.45 (s, 3H), 1.93 (s, 2H), 1.90 (s, 3H), 1.87 (s, 3H), 1.78 - 1.66 (m, 4H), 1.47 (s, 2H). MS(ESI+): 558.1(M+H).

**Example 118** (S)-(4-chloro-2-((5-chloro-2-((7-(pyrrolidin-1-yl)-6,7,8,9-tetrahydro-5H-benzoannulen-2-yl)amino)pyrimidin-4-yl)amino)-5-fluorophenyl)dimethyl phosphine oxide

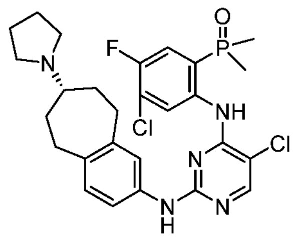

According to preparation method (steps b~d) of Example 115, 2-iodo-4-(methoxymethyl)aniline in step b) is replaced with 5-chloro-4-fluoro-2-iodoaniline.

$^1$H NMR (400 MHz, DMSO-$d_6$, *ppm*): δ 11.09 (s, 1H), 9.36 (s, 1H), 8.70 (s, 1H), 8.20 (s, 1H), 7.73 (dd, J = 13.7, 9.3 Hz, 1H), 7.36 (dd, J = 8.1, 2.2 Hz, 1H), 7.26 - 7.22 (m, 1H), 7.01 (d, J = 8.2 Hz, 1H), 2.92-2.75 (m, 3H), 2.70 - 2.64 (m, 4H), 2.59-2.53 (m, 2H), 1.93 (br, 2H), 1.83 (s, 3H), 1.80 (s, 3H), 1.72 (br, 4H), 1.49 (br, 2H). MS(ESI+): 562.1(M+H).

**Example 119** (S)-(2-((5-chloro-2-((7-(pyrrolidin-1-yl)-6,7,8,9-tetrahydro-5H-benzo[7]annulen-2-yl)amino)pyrimidin-4-yl)amino)-5-(2-oxa-6-azaspiro[3.3]heptan-6-yl)phenyl)dimethyl phosphine oxide

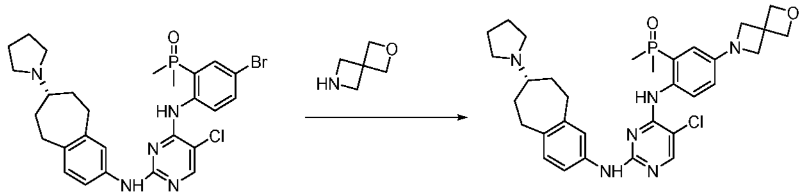

(S)-(5-bromo-2-((5-chloro-2-((7-(pyrrolidin-1-yl)-6,7,8,9-tetrahydro-5H-benzo[7]annulen-2-yl)amino)pyrimidin-4-yl)amino)phenyl)dimethyl phosphine oxide (50 mg), 2-oxo-6-azaspiro[3.3]heptane (18 mg), 2-bicyclohexylphosphino-2',6'-diisopropoxybiphenyl (6 mg), tris(dibenzylideneacetone)dipalladium (6 mg), and cesium carbonate (55 mg) are added to 1,4-dioxane (5 ml) under the protection of nitrogen, and reacted at 100°C for 3 h. After the reaction is completed, the solid is removed by suction filtration. The filter cake is washed twice with dichloromethane (10 ml×2). Organic layers are merged. The reaction solution is desolventized to dryness. The reaction solution is concentrated to dryness, and the residue is purified by silica-gel column chromatography (dichloromethane/methanol=20:1 v/v) to obtain 35 mg of the title product.

$^1$H NMR (400 MHz, DMSO-$d_6$, *ppm*): δ10.23 (s, 1H), 9.15 (s, 1H), 8.25 (s, 1H), 8.10-8.02 (m, 1H), 7.41 (d, J = 2.3 Hz, 1H), 7.17 (d, J = 8.1 Hz, 1H), 6.91 (d, J = 8.2 Hz, 1H), 6.66 - 6.59 (m, 2H), 4.73 (s, 4H), 4.02 (s, 4H), 2.91 - 2.73 (m, 3H), 2.59 (br, 4H), 2.44 - 2.36 (m, 2H), 1.85 (br, 2H), 1.75-1.65 (m, 10H), 1.52 (br, 2H). MS(ESI+): 607.3(M+H).

**Example 120** Preparation of compounds 120-1 and 120-2

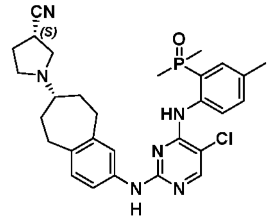

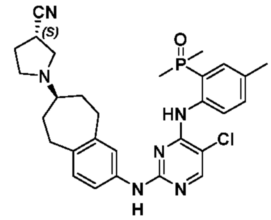

According to the preparation method of Example 58, (2-amino-5-fluorophenyl) dimethyl phosphine oxide in step a) is replaced with (2-amino-5-methylphenyl) dimethyl phosphine oxide, and 2-azabicyclo[3.1.0]hexane hydrochloride in step c) is replaced with (3S)-pyrrolidine-3-carbonitrile hydrochloride. The crude product is purified by a preparative high-performance liquid phase under the following conditions (column: XBridge Prep OBD column, 30×150 mm, with a filler particle size of 5 μm; mobile phase A: water (10 mmol/L ammonium bicarbonate), mobile phase B: acetonitrile; flow rate: 60 ml/min; gradient: 30%B-68%B within 8 min; wavelength: 254/220 nm) to obtain of a mixture of two isomers. The mixture of two isomers is purified by a chiral high-performance liquid chromatography column (column: CHIRALPAK IF, 5×25 cm (filler 5 μm); mobile phase A: methyl tert-butyl ether (0.1% diethylamine), mobile phase B: ethanol; flow rate: 15 ml/min; gradient: 20%B within 17.5 min, isogradient; detection wavelength: 220/254 nm; and column temperature: 25°C) to obtain:

isomer 120-1 (20 mg) having a HPLC retention time of 13.4 min:
$^1$HNMR (400 MHz, DMSO-$d_6$, *ppm*):δ10.92 (s, 1H), 9.25 (s, 1H), 8.40 (s, 1H), 8.15 (s, 1H), 7.46 - 7.39 (m, 2H), 7.34 - 7.25 (m, 2H), 6.98 (d, J = 8.2 Hz, 1H), 3.29 - 3.20 (m, 1H), 2.94 - 2.69 (m, 5H), 2.62 - 2.54 (m, 3H),2.46 (s, 1H), 2.34 (s, 3H), 2.23 - 2.11 (m, 1H), 1.99 - 1.81 (m, 3H), 1.77 (d, J = 13.5 Hz, 6H), 1.52 (s, 2H). MS(ESI+): 549.2(M+H).
isomer 120-2 (20 mg) having a HPLC retention time of 16.2 min:
$^1$HNMR (400 MHz, DMSO-$d_6$,*ppm*):δ10.93 (s, 1H), 9.25 (s, 1H), 8.40 (s, 1H), 8.15 (s, 1H), 7.47 - 7.39 (m, 2H), 7.35 - 7.25 (m, 2H), 6.98 (d, J = 8.2 Hz, 1H),3.23 - 3.25 (m, 1H), 2.95 - 2.70 (m, 5H), 2.57 (d, J = 6.7 Hz, 4H), 2.34 (s, 3H), 2.23 - 2.11 (m, 1H), 2.00 - 1.81 (m, 3H), 1.77 (d, J = 13.5 Hz, 6H), 1.51 (s, 2H). MS(ESI+): 549.2(M+H).

**Example 121** Preparation of compounds 121-1 and 121-2

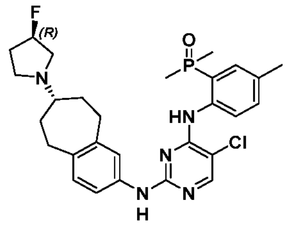 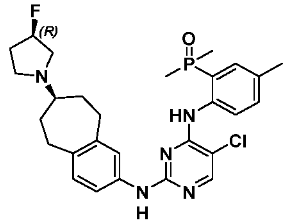

According to the preparation method of Example 58, (2-amino-5-fluorophenyl) dimethyl phosphine oxide in step a) is replaced with (2-amino-5-methylphenyl) dimethyl phosphine oxide, and 2-azabicyclo[3.1.0]hexane hydrochloride in step c) is replaced with (3R)-3-fluoropyrrolidine hydrochloride. The crude product is purified by a preparative high-performance liquid phase under the following conditions (column: XBridge Prep OBD column, 30×150 mm, with a filler particle size of 5 μm; mobile phase A: water (10 mmol/L ammonium bicarbonate), mobile phase B: acetonitrile; flow rate: 60 ml/min; gradient: 30%B-68%B within 8 min; wavelength: 254/220 nm) to obtain of a mixture of two isomers. The mixture of two isomers is purified by a chiral high-performance liquid chromatography column (column: CHIRALPAK IF, 5×25 cm (filler 5 μm); mobile phase A: methyl tert-butyl ether (0.1% diethylamine), mobile phase B: ethanol; flow rate: 15 ml/min; gradient: 20%B within 17.5 min, isogradient; detection wavelength: 220/254 nm; and column temperature: 25°C) to obtain:

isomer 121-1 (20 mg) having a HPLC retention time of 13.4 min:

$^1$HNMR (400 MHz, DMSO-$d_6$, *ppm*):δ10.92 (s, 1H), 9.25 (s, 1H), 8.40 (s, 1H), 8.15 (s, 1H), 7.51 - 7.38 (m, 2H), 7.35 - 7.22 (m, 2H), 6.98 (d, *J* = 8.2 Hz, 1H), 5.20 (m, 1H), 2.99 - 2.69 (m, 5H), 2.57 (s, 2H),2.45 (s, 2H),2.34 (s, 3H), 2.09 (ddd, *J* = 18.3, 14.3, 7.0 Hz, 1H), 1.91 (d, *J* = 26.4 Hz, 3H), 1.77 (d, *J* = 13.5 Hz, 6H), 1.49 (s, 2H).

MS(ESI+): 542.2(M+H).

Isomer 121-2 (20 mg) having a HPLC retention time of 16.2 min:

$^1$HNMR (400 MHz, DMSO-$d_6$, *ppm*):δ10.92 (s, 1H), 9.25 (s, 1H), 8.40 (s, 1H), 8.15 (s, 1H), 7.48 - 7.39 (m, 2H), 7.35 - 7.24 (m, 2H), 6.98 (d, J = 8.1 Hz, 1H), 5.20 (d, J = 56.2 Hz, 1H), 2.97 - 2.70 (m, 5H), 2.57 (s, 3H),2.46 (s, 1H),2.34 (s, 3H), 2.19 - 2.04 (m, 1H), 1.87 (s, 3H), 1.77 (d, J = 13.5 Hz, 6H), 1.49 (s, 2H).

MS(ESI+): 542.2(M+H).

**Example 122** Preparation of compounds 122-1 and 122-2

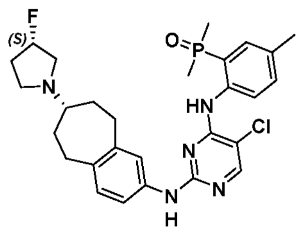 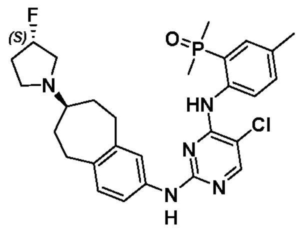

According to the preparation method of Example 58, (2-amino-5-fluorophenyl) dimethyl phosphine oxide in step a) is replaced with (2-amino-5-methylphenyl) dimethyl phosphine oxide, and 2-azabicyclo[3.1.0]hexane hydrochloride in step c) is replaced with (3S)-3-fluoropyrrolidine hydrochloride. The crude product is purified by a preparative high-performance liquid phase under the following conditions (column: YMC-Actus Triart C18 column, 30×150 mm, with a filler particle size of 5 μm; mobile phase A: water (10 mmol/L ammonium bicarbonate), mobile phase B: acetonitrile; flow rate: 60 ml/min; gradient: 20%B-58%B within 8 min; wavelength: 254/220 nm) to obtain a mixture of two isomers (63 mg).

The mixture of two isomers is purified by a chiral high-performance liquid chromatography column (column: CHIRALPAK IE, 2×25 cm (filler 5 μm); mobile phase A: methyl tert-butyl ether (0.1% diethylamine), mobile phase B: ethanol; flow rate: 19 ml/min; gradient: 35%B within 11 min, isogradient; detection wavelength: 220/254 nm; and column temperature: 25°C) to obtain:

isomer 122-1 (15.2 mg) having a HPLC retention time of 5.9 min:
$^1$HNMR (400 MHz, DMSO-$d_6$, *ppm*):$\delta$10.92 (s, 1H), 9.25 (s, 1H), 8.40 (s, 1H), 8.15 (s, 1H), 7.51 - 7.38 (m, 2H), 7.35 - 7.22 (m, 2H), 6.98 (d, *J* = 8.2 Hz, 1H), 5.20 (m, 1H), 2.99 - 2.69 (m, 5H), 2.57 (s, 2H),2.45 (s, 2H),2.34 (s, 3H), 2.09 (ddd, *J* = 18.3, 14.3, 7.0 Hz, 1H), 1.91 (d, *J* = 26.4 Hz, 3H), 1.77 (d, *J* = 13.5 Hz, 6H), 1.49 (s, 2H). MS(ESI+): 542.2(M+H).

Isomer 122-2 (20.2 mg) having a HPLC retention time of 9.0 min:
$^1$HNMR (400 MHz, DMSO-$d_6$, *ppm*):$\delta$10.92 (s, 1H), 9.25 (s, 1H), 8.40 (s, 1H), 8.15 (s, 1H), 7.48 - 7.39 (m, 2H), 7.35 - 7.24 (m, 2H), 6.98 (d, J = 8.1 Hz, 1H), 5.20 (d, J = 56.2 Hz, 1H), 2.97 - 2.70 (m, 5H), 2.57 (s, 3H),2.46 (s, 1H),2.34 (s, 3H), 2.19 - 2.04 (m, 1H), 1.87 (s, 3H), 1.77 (d, J = 13.5 Hz, 6H), 1.49 (s, 2H). MS(ESI+): 542.2(M+H).

**Example 123** Preparation of compounds 123-1 and 123-2

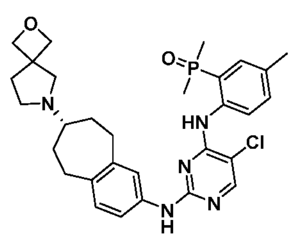 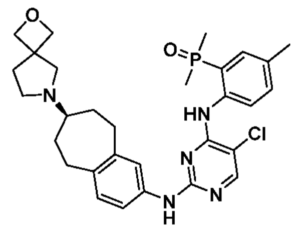

According to the preparation method of Example 58, (2-amino-5-fluorophenyl) dimethyl phosphine oxide in step a) is replaced with (2-amino-5-methylphenyl) dimethyl phosphine oxide, and 2-azabicyclo[3.1.0]hexane hydrochloride in step c) is replaced with 2-oxo-6-azaspiro[3.4]octane oxalate. The crude product is purified by a preparative high-performance liquid phase under the following conditions (column: YMC-Actus Triart C18 column, 30×150 mm, with a filler particle size of 5 μm; mobile phase A: water (10 mmol/L ammonium bicarbonate), mobile phase B: acetonitrile; flow rate: 60 ml/min; gradient: 20%B-58%B within 8 min; wavelength: 254/220 nm) to obtain a mixture of two isomers (63 mg).

The mixture of two isomers is purified by a chiral high-performance liquid chromatography column (column: CHIRALPAK IE, 2×25 cm (filler 5 μm); mobile phase A: methyl tert-butyl ether (0.1% diethylamine), mobile phase B: ethanol; flow rate: 19 ml/min; gradient: 35%B within 12 min, isogradient; detection wavelength: 220/254 nm; and column temperature: 25°C) to obtain:
isomer 123-1 (18.9 mg) having a HPLC retention time of 9.1 min:
$^1$HNMR (400 MHz, DMSO-$d_6$, *ppm*):$\delta$10.92 (s, 1H), 9.24 (s, 1H), 8.40 (s, 1H), 8.15 (s, 1H), 7.43 (d, *J* = 14.8 Hz, 2H), 7.29 (dd, *J* = 14.5, 8.4 Hz, 2H), 6.97 (d, *J*= 8.1 Hz, 1H), 4.57 - 4.34 (m, 4H), 2.97 - 2.63 (m, 4H), 2.62 - 2.52 (m, 3H), 2.47 (s, 2H), 2.34 (d, *J* = 3.1 Hz, 3H), 2.11 - 1.92 (m, 2H), 1.77 (d, *J* = 13.5 Hz, 8H), 1.51 (s, 2H). MS(ESI+): 566.2(M+H).

Isomer 123-2 (19.6 mg) having a HPLC retention time of 10.6 min:
$^1$HNMR (400 MHz, DMSO-$d_6$, *ppm*):$\delta$10.92 (s, 1H), 9.24 (s, 1H), 8.40 (s, 1H), 8.15 (s, 1H), 7.53 - 7.40 (m, 2H), 7.29 (dd, *J* = 14.4, 8.6 Hz, 2H), 6.97 (d, *J* = 8.1 Hz, 1H), 4.68 - 4.30 (m, 4H), 2.98 - 2.64 (m, 4H), 2.62 - 2.53 (m, 3H), 2.42 (s, 2H), 2.34 (d, *J* = 3.0 Hz, 3H), 2.03 (t, *J* = 7.0 Hz, 2H), 1.77 (d, *J* = 13.5 Hz, 8H), 1.52 (s, 2H). MS(ESI+): 566.2(M+H).

**Example 124** Preparation of compounds 124-1 and 124-2

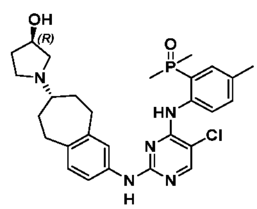 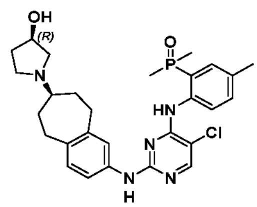

According to the preparation method of Example 58, (2-amino-5-fluorophenyl) dimethyl phosphine oxide in step a) is replaced with (2-amino-5-methylphenyl) dimethyl phosphine oxide, and 2-azabicyclo[3.1.0]hexane hydrochloride in step c) is replaced with (3R)-pyrrolidin-3-ol hydrochloride. The crude product is purified by a preparative high-performance liquid phase under the following conditions (column: XBridge Prep OBD C18 column, 30×150 mm, with a

filler particle size of 5 μm; mobile phase A: water (10 mmol/L ammonium bicarbonate), mobile phase B: acetonitrile; flow rate: 60 ml/min; gradient: 10%B-45%B within 8 min; wavelength: 254/220 nm) to obtain a mixture of two isomers (71mg).

The mixture of two isomers is purified by a chiral high-performance liquid chromatography column (column: CHIRALPAK IE, 2×25 cm (filler 5 μm); mobile phase A: methyl tert-butyl ether (0.1% diethylamine), mobile phase B: ethanol; flow rate: 20 ml/min; gradient: 25%B within 12 min, isogradient; detection wavelength: 220/254 nm; and column temperature: 25°C) to obtain:

isomer 124-1 (15.7 mg) having a HPLC retention time of 7.9 min:

$^1$HNMR (400 MHz, DMSO-$d_6$, *ppm*):δ10.92 (s, 1H), 9.25 (s, 1H), 8.40 (s, 1H), 8.15 (s, 1H), 7.54 - 7.37 (m, 2H), 7.36 - 7.16 (m, 2H), 6.97 (d, J= 8.1 Hz, 1H), 4.66 (s, 1H), 4.18 (s, 1H), 2.85 (d, J = 29.2 Hz, 3H), 2.73 - 2.60 (m, 1H), 2.55 (s, 2H), 2.42 (d, J = 40.0 Hz, 3H), 2.34 (s, 3H), 2.12 -1.81 (m, 3H), 1.77 (d, J = 13.5 Hz, 6H), 1.52 (d, J = 32.5 Hz, 3H). MS(ESI+): 540.2(M+H).

Isomer 124-2 (18.3 mg) having a HPLC retention time of 10.0 min:

$^1$HNMR (400 MHz, DMSO-$d_6$, *ppm*): δ10.92 (s, 1H), 9.25 (s, 1H), 8.40 (s, 1H), 8.15 (s, 1H), 7.49 - 7.38 (m, 2H), 7.35 - 7.21 (m, 2H), 6.97 (d, J = 8.1 Hz, 1H), 4.65 (d, J = 4.6 Hz, 1H), 4.18 (s, 1H), 2.82 (dd, J = 20.0, 11.6 Hz, 3H), 2.67 (dt, J = 6.1, 3.1 Hz, 2H), 2.54 (s, 1H), 2.45 (s, 3H), 2.34 (s, 3H), 1.95 (dt, J = 15.2, 7.6 Hz, 3H), 1.77 (d, J = 13.5 Hz, 6H), 1.53 (s, 3H). MS(ESI+): 540.2(M+H).

**Example 125** Preparation of compounds 125-1 and 125-2

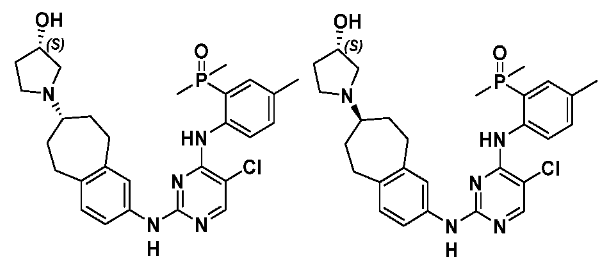

According to the preparation method of Example 58, (2-amino-5-fluorophenyl) dimethyl phosphine oxide in step a) is replaced with (2-amino-5-methylphenyl) dimethyl phosphine oxide, and 2-azabicyclo[3.1.0]hexane hydrochloride in step c) is replaced with (3S)-pyrrolidin-3-ol hydrochloride. The crude product is purified by a preparative high-performance liquid phase under the following conditions (column: YMC-Actus Triart C18 column, 30×150 mm, with a filler particle size of 5 μm; mobile phase A: water (10 mmol/L ammonium bicarbonate), mobile phase B: acetonitrile; flow rate: 60 ml/min; gradient: 15%B-60%B within 8 min; wavelength: 254/220 nm) to obtain a mixture of two isomers (56 mg).

The mixture of two isomers is purified by a chiral high-performance liquid chromatography column (column: CHIRALPAK IE, 2×25 cm (filler 5 μm); mobile phase A: methyl tert-butyl ether (0.1% diethylamine), mobile phase B: ethanol; flow rate: 15 ml/min; gradient: 30%B within 20 min, isogradient; detection wavelength: 220/254 nm; and column temperature: 25°C) to obtain:

isomer 125-1 (21.1 mg) having a HPLC retention time of 13.2 min:

$^1$HNMR (400 MHz, DMSO-$d_6$, *ppm*):δδ 10.92 (s, 1H), 9.25 (s, 1H), 8.40 (s, 1H), 8.15 (s, 1H), 7.57 - 7.37 (m, 2H), 7.35 - 7.20 (m, 2H), 6.97 (d, *J* = 8.1 Hz, 1H), 4.65 (d, *J* = 4.6 Hz, 1H), 4.18 (s, 1H), 2.89 (s, 1H), 2.82 (d, *J* = 9.3 Hz, 2H), 2.66 (s, 2H),2.52 (s, 1H), 2.40 (d, *J* = 9.6 Hz, 3H), 2.34 (s, 3H), 1.97 (dt, *J* = 13.1, 7.0 Hz, 1H), 1.84 (s, 2H), 1.77 (d, *J* = 13.5 Hz, 6H), 1.53 (d, *J* = 30.2 Hz, 3H). MS(ESI+): 540.2(M+H).

Isomer 125-2 (21.1 mg) having a HPLC retention time of 17.9 min:

$^1$HNMR (400 MHz, DMSO-$d_6$, *ppm*): δ10.92 (s, 1H), 9.25 (s, 1H), 8.40 (s, 1H), 8.15 (s, 1H), 7.48 - 7.37 (m, 2H), 7.34 - 7.22 (m, 2H), 6.97 (d, J = 8.2 Hz, 1H), 4.65 (s, 1H), 4.26 - 4.10 (m, 1H), 2.89 (s, 1H), 2.82 (d, J = 9.3 Hz, 2H), 2.66 (s, 2H),2.52 (s, 1H), 2.40 (d, J = 9.6 Hz, 3H), 2.34 (s, 3H), 1.97 (dt, J = 13.1, 7.0 Hz, 1H), 1.84 (s, 2H), 1.77 (d, J = 13.5 Hz, 6H), 1.52 (d, J = 30.2 Hz, 3H). MS(ESI+): 540.2(M+H).

**Example 126** Preparation of compounds 126-1 and 126-2

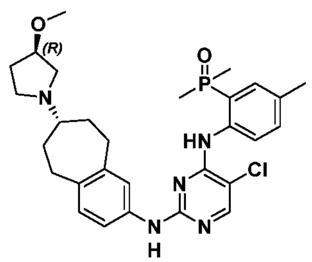
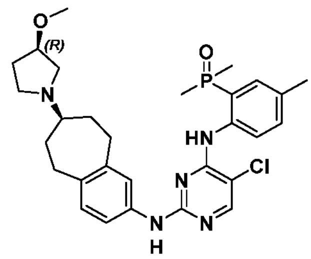

According to the preparation method of Example 58, (2-amino-5-fluorophenyl) dimethyl phosphine oxide in step a) is replaced with (2-amino-5-methylphenyl) dimethyl phosphine oxide, and 2-azabicyclo[3.1.0]hexane hydrochloride in step c) is replaced with (3R)-3-methoxypyrrolidine hydrochloride. The crude product is purified by a preparative high-performance liquid phase under the following conditions (column: XBridge Prep OBD C18 column, 30×150 mm, with a filler particle size of 5 μm; mobile phase A: water (10 mmol/L ammonium bicarbonate), mobile phase B: acetonitrile; flow rate: 60 ml/min; gradient: 10%B-55%B within 8 min; wavelength: 254/220 nm) to obtain a mixture of two isomers. The mixture of two isomers is purified by a chiral high-performance liquid chromatography column (column: CHIRALPAK IE, 2×25 cm (filler 5 μm); mobile phase A: methyl tert-butyl ether (0.1% diethylamine), mobile phase B: ethanol; flow rate: 20 ml/min; gradient: 30%B within 20 min, isogradient; detection wavelength: 220/254 nm; and column temperature: 25°C) to obtain:

isomer 126-1 (18.3 mg) having a HPLC retention time of 6.7 min:

$^1$HNMR (400 MHz, DMSO-$d_6$, *ppm*):δ 10.92 (s, 1H), 9.25 (s, 1H), 8.40 (s, 1H), 8.15 (s, 1H), 7.43 (dd, J = 13.4, 2.2 Hz, 2H), 7.29 (ddd, J= 15.0, 8.4, 2.0 Hz, 2H), 6.97 (d, J = 8.1 Hz, 1H), 3.86 (s, 1H), 3.18 (s, 3H), 2.95 - 2.72 (m, 3H), 2.69 - 2.58 (m, 1H), 2.55 (s, 1H), 2.46 (s, 4H), 2.34 (s, 3H), 2.04 - 1.80 (m, 3H), 1.77 (d, J = 13.5 Hz, 6H), 1.66 (s, 1H), 1.49 (s, 2H). MS(ESI+): 554.2(M+H).

Isomer 126-2 (16.7 mg) having a HPLC retention time of 8.45 min:

$^1$HNMR (400 MHz, DMSO-$d_6$, *ppm*): δ10.92 (s, 1H), 9.25 (s, 1H), 8.40 (d, *J* = 8.3 Hz, 1H), 8.15 (s, 1H), 7.49 - 7.39 (m, 2H), 7.35 - 7.23 (m, 2H), 6.97 (d, *J* = 8.1 Hz, 1H), 3.87 (s, 1H), 3.18 (s, 3H), 2.80 (s, 3H), 2.69 - 2.59 (m, 1H), 2.55 (s, 2H), 2.44 (s, 3H), 2.34 (s, 3H), 2.02 - 1.81 (m, 3H), 1.78 (s, 3H), 1.75 (s, 3H), 1.67 (s, 1H), 1.49 (s, 2H). MS(ESI+): 554.2(M+H).

**Example 127** Preparation of compounds 127-1 and 127-2

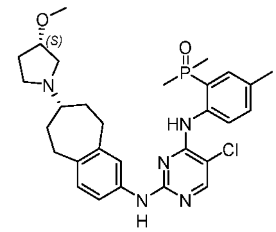
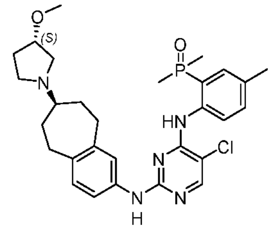

According to the preparation method of Example 58, (2-amino-5-fluorophenyl) dimethyl phosphine oxide in step a) is replaced with (2-amino-5-methylphenyl) dimethyl phosphine oxide, and 2-azabicyclo[3.1.0]hexane hydrochloride in step c) is replaced with (3S)-3-methoxypyrrolidine hydrochloride. The crude product is purified by a preparative high-performance liquid phase under the following conditions (column: YMC-Actus Triart C18 column, 30×150 mm, with a filler particle size of 5 μm; mobile phase A: water (10 mmol/L ammonium bicarbonate), mobile phase B: acetonitrile; flow rate: 60 ml/min; gradient: 15%B-60%B within 8 min; wavelength: 254/220 nm) to obtain a mixture of two isomers (56 mg).

The mixture of two isomers is purified by a chiral high-performance liquid chromatography column (column: CHIRALPAK IF, 2×25 cm (filler 5 μm); mobile phase A: methyl tert-butyl ether (0.1% diethylamine), mobile phase B: ethanol; flow rate: 15 ml/min; gradient: 30%B within 20 min, isogradient; detection wavelength: 220/254 nm; and column temperature: 25°C) to obtain:

isomer 127-1 (24.5 mg) having a HPLC retention time of 13.2 min:

$^1$HNMR (400 MHz, DMSO-$d_6$, *ppm*):δ 10.92 (s, 1H), 9.25 (s, 1H), 8.40 (s, 1H), 8.15 (s, 1H), 7.48 - 7.38 (m, 2H), 7.34 - 7.22 (m, 2H), 6.97 (d, *J* =8.2 Hz, 1H), 3.87 (s, 1H), 3.18 (s, 3H), 2.82 (q, *J* =14.8, 8.1 Hz, 3H), 2.64 (d, *J* =7.8 Hz, 1H),

2.54 (s, 2H), 2.44 (s, 3H), 2.34 (s, 3H), 2.03 - 1.80 (m, 3H), 1.77 (d, *J* = 13.5 Hz, 6H), 1.66 (s, 1H), 1.49 (s, 2H). MS(ESI+): 554.2(M+H).

Isomer 127-2 (22.3 mg) having a HPLC retention time of 17.9 min:
$^1$HNMR (400 MHz, DMSO-$d_6$, *ppm*): δ10.92 (s, 1H), 9.25 (s, 1H), 8.39 (s, 1H), 8.15 (s, 1H), 7.43 (dd, *J* =13.5, 2.2 Hz, 2H), 7.29 (ddd, *J* =14.9, 8.5, 2.0 Hz, 2H), 6.97 (d, *J* =8.1 Hz, 1H), 3.86 (dt, *J* =7.2, 3.6 Hz, 1H), 3.18 (s, 3H),2.82 (q, *J* =14.8, 8.1 Hz, 3H), 2.64 (d, *J* =7.8 Hz, 1H), 2.54 (s, 2H), 2.44 (s, 3H), 2.34 (s, 3H), 2.03 - 1.80 (m, 3H), 1.77 (d, *J* =13.5 Hz, 6H), 1.65 (t, *J* = 11.1 Hz, 1H), 1.49 (s, 2H). MS(ESI+): 554.2(M+H).

**Example 128** (S)-(2-((5-chloro-2-((7-(pyrrolidin-1-yl)-6,7,8,9-tetrahydro-5H-benzo[7]annulen-2-yl)amino)pyrimidin-4-yl)amino)-5-cyclopropoxyphenyl)dimethyl phosphine oxide

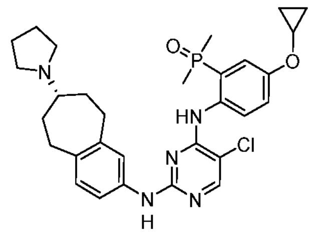

According to the preparation method of Example 1, 2-amino-N,N-dimethyl benzenesulfonamide in step a) is replaced with (2-amino-5-cyclopropoxyphenyl) dimethyl phosphine oxide.

$^1$HNMR (400 MHz, DMSO-$d_6$, *ppm*):δ 10.56 (s, 1H), 9.22(s, 1H), 8.32 (s, 1H), 8.13 (s, 1H), 7.38 (m, 1H), 7.32 (m, 3H), 6.94 (d, *J* =8.4 Hz, 1H), 3.93 (m, 1H), 2.87 (m, 2H), 2.53 (m, 4H),2.42 (m, 3H), 1.83 (m, 2H), 1.77(m, 3H), 1.73 (m, 3H) 1.69 (m, 4H),1.53(m, 2H), 0.82 (m, 2H), 0.70 (m, 2H). MS(ESI+): 566.2(M+H).

**Example 129** (S)-(2-((5-chloro-2-((7-(pyrrolidin-1-yl)-6,7,8,9-tetrahydro-5H-benzo[7]annulen-2-yl)amino)pyrimidin-4-yl)amino)-5-fluoro-4-methylphenyl)dimethyl phosphine oxide

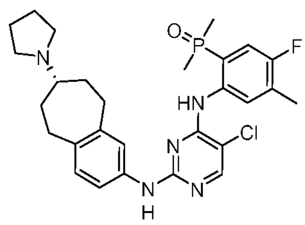

According to the preparation method of Example 115, 4-(methoxymethyl)aniline in step a) is replaced with 4-fluoro-2-iodo-5-methylaniline.

$^1$H NMR (400 MHz, DMSO-$d_6$, *ppm*): δ10.79 (s, 1H), 9.24 (s, 1H), 8.36 (s, 1H), 8.16 (s, 1H), 7.45 (dd, J = 13.8, 9.5 Hz, 1H), 7.37 (dd, J = 8.0, 2.3 Hz, 1H), 7.22 (s, 1H), 6.95 (d, J = 8.1 Hz, 1H), 2.89 (s, 1H), 2.80 (s, 2H), 2.56 - 2.48 (m,4H), 2.43 (d, J = 9.4 Hz, 2H), 2.23 (s, 3H), 1.76 (d, J = 13.6 Hz, 8H), 1.72 - 1.64 (m, 4H), 1.51 (s, 2H). MS(ESI+): 542,2 (M+H).

**Example 130** (S)-(2-((5-chloro-2-((7-(pyrrolidin-1-yl)-6,7,8,9-tetrahydro-5H-benzo[7]annulen-2-yl)amino)pyrimidin-4-yl)amino)-4-methylphenyl)dimethyl phosphine oxide

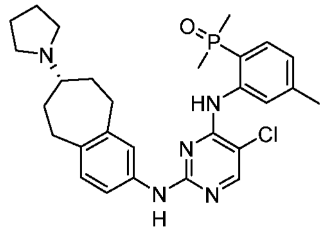

According to the preparation method of Example 115, 4-(methoxymethyl)aniline in step a) is replaced with 2-iodo-5-methylaniline.

$^1$H NMR (400 MHz, DMSO-$d_6$, *ppm*): δ 10.78 (s, 1H), 8.59 (s, 1H), 8.39 - 8.32 (m, 1H), 8.08 (s, 1H), 7.44 (dd, J = 8.1, 2.3 Hz, 1H), 7.18 (s, 1H), 7.06 - 6.98 (m, 2H), 6.94 (dt, J = 7.9, 2.0 Hz, 1H), 3.09 (s, 4H), 2.90 - 2.65 (m, 5H), 2.32 (s, 5H), 1.95 (t, J = 3.9 Hz, 3H), 1.81 (d, J = 13.1 Hz, 6H), 1.58 - 1.46 (m, 2H), 1.26 (p, J = 7.7, 7.0 Hz, 1H). MS(ESI+): 524.2(M+H).

**Example 131** (S)-(2-((5-chloro-2-((7-(pyrrolidin-1-yl)-6,7,8,9-tetrahydro-5H-benzo[7]annulen-2-yl)amino)pyrimidin-4-yl)amino)-5-fluoro-4-methoxylphenyl)dimethyl phosphine oxide

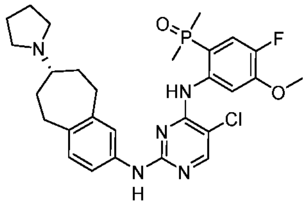

According to the preparation method of Example 1, 2-amino-N,N-dimethyl benzenesulfonamide in step a) is replaced with (2-amino-4-methoxyl-5-fluorophenyl)dimethyl phosphine oxide. The crude product is purified by a preparative high-performance liquid phase under the following conditions (column: Xselect CSH-OBD column, 30×150 mm, with a filler particle size of 5 μm; mobile phase A: water (10 mmol/L ammonium bicarbonate), mobile phase B: acetonitrile; flow rate: 60 ml/min; gradient: 15%B-50%B within 8 min; wavelength: 254/220 nm) to obtain the title product (65 mg). The HPLC retention time is 7.65 min.

$^1$HNMR (400 MHz, DMSO-$d_6$, *ppm*):δ10.92 (s, 1H), 9.24 (s, 1H), 8.19 (s, 1H), 8.10 (dd, *J* = 8.1, 4.1 Hz, 1H), 7.50 (dd, *J* = 13.4, 11.5 Hz, 1H), 7.33 (d, *J* = 2.4 Hz, 1H), 7.25 (dd, *J* = 8.0, 2.3 Hz, 1H), 6.93 (d, *J* = 8.1 Hz, 1H), 3.59 (s, 3H), 2.89 (s, 1H), 2.77 (s, 1H), 2.54 (s, 4H), 2.42 (s, 3H), 1.82 (s, 2H), 1.76 (d, *J* = 13.6 Hz, 6H), 1.69 (d, *J* = 6.3 Hz, 4H), 1.51 (s, 2H). MS(ESI+): 558.2(M+H).

**Example 132** (S)-(2-chloro-6-((5-chloro-2-((7-(pyrrolidin-1-yl)-6,7,8,9-tetrahydro-5H-benzo [7]annulen-2-yl)amino)pyrimidin-4-yl)amino)-3-fluorophenyl)dimethyl phosphine oxide

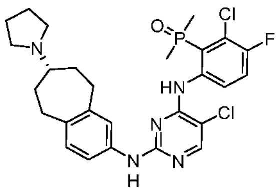

According to the preparation method of Example 1, 2-amino-N,N-dimethyl benzenesulfonamide in step a) is replaced with (2-amino-5-fluoro-6-chlorophenyl)dimethyl phosphine oxide. The crude product is purified by a preparative high-performance liquid phase under the following conditions (column: XBridge Prep OBD C18 column, 30×150 mm, with a filler particle size of 5 μm; mobile phase A: water (10 mmol/L ammonium bicarbonate), mobile phase B: acetonitrile; flow rate: 60 ml/min; gradient: 15%B-55%B within 8 min; wavelength: 254/220 nm) to obtain the title product (28.3 mg).

The HPLC retention time is 7.07 min.

$^1$HNMR (400 MHz, DMSO-$d_6$, *ppm*):δ12.20 (s, 1H), 9.28 (s, 1H), 8.65 (s, 1H), 8.18 (s, 1H), 7.53 (dd, J = 9.4, 8.3 Hz, 1H), 7.35 (d, J = 2.3 Hz, 1H), 7.29 (dd, J = 8.2, 2.2 Hz, 1H), 6.99 (d, J=8.2 Hz, 1H), 2.87 (s, 2H), 2.55 - 2.53 (m, 4H), 2.49 - 2.29 (m, 3H), 2.04 (d, J = 13.9 Hz, 6H), 1.86 (s, 2H), 1.69 (s, 4H), 1.52 (s, 2H). MS(ESI+): 562.2(M+H).

**Example 133** Compounds 133-1 and 133-2

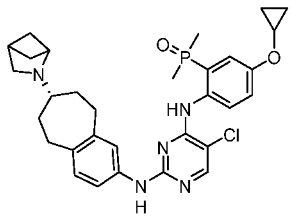
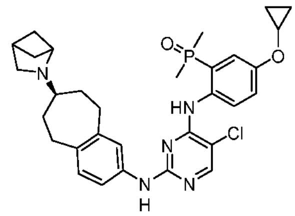

According to the preparation method of Example 58, (2-amino-5-fluorophenyl) dimethyl phosphine oxide in step a) is replaced with (2-amino-5-cyclopropoxyphenyl)dimethyl phosphine oxide, and 2-azabicyclo[3.1.0]hexane hydrochloride in step c) is replaced with 2-azabicyclo[2.1.1]hexane hydrochloride. The crude product is purified by a preparative high-performance liquid phase under the following conditions (column: YMC-Actus Triart C18 column, 30×150 mm, with a filler particle size of 5 μm; mobile phase A: water (10 mmol/L ammonium bicarbonate), mobile phase B: acetonitrile; flow rate: 60 ml/min; gradient: 20%B-55%B within 8 min; wavelength: 254/220 nm) to obtain a mixture of two isomers (65 mg).

The mixture of two isomers is purified by a chiral high-performance liquid chromatography column (column: CHIRALPAK IG, 2×25 cm (filler 5 μm); mobile phase A: methyl tert-butyl ether (0.1% diethylamine), mobile phase B: ethanol; flow rate: 20 ml/min; gradient: 25%B within 9 min, isogradient; detection wavelength: 220/254 nm; and column temperature: 25°C) to obtain:

isomer 133-1 (21.5 mg) having a HPLC retention time of 7.5 min:

$^1$HNMR (400 MHz, DMSO-$d_6$, *ppm*):δ 10.53 (s, 1H), 9.21 (s, 1H), 8.32 (s, 1H), 8.12 (s, 1H), 7.38 (d, *J* = 2.3 Hz, 1H), 7.28 - 7.19 (m, 3H), 6.95 (d, *J* = 8.1 Hz, 1H), 3.92 (tt, *J* = 6.0, 2.9 Hz, 1H), 3.66 (d, *J* = 6.5 Hz, 1H), 2.89 (s, 2H), 2.71 (dt, *J* = 6.0, 2.8 Hz, 1H), 2.67 (s, 2H), 2.54 (s, 2H), 1.90 (s, 2H), 1.75 (d, *J* = 13.5 Hz, 6H), 1.62 (s, 2H), 1.40 - 1.30 (m, 5H), 0.83 (tt, *J* = 4.7, 2.4 Hz, 2H), 0.69 (p, *J* = 3.7, 3.0 Hz, 2H). MS(ESI+): 578.2(M+H).

Isomer 133-2 (21.8 mg) having a HPLC retention time of 11.2 min:

$^1$HNMR (400 MHz, DMSO-$d_6$, *ppm*): δ10.55 (s, 1H), 9.21 (s, 1H), 8.32 (s, 1H), 8.13 (s, 1H), 7.39 (d, *J* = 2.3 Hz, 1H), 7.29 - 7.19 (m, 3H), 6.95 (d, *J* = 8.1 Hz, 1H), 3.92 (tt, *J* = 6.0, 2.9 Hz, 1H), 3.66 (s, 1H), 2.89 (s, 2H), 2.74 - 2.64 (m, 3H), 2.56 (d, *J* = 12.5 Hz, 2H), 1.89 (s, 2H), 1.75 (d, *J* = 13.5 Hz, 6H), 1.62 (s, 2H), 1.37-1.30 (m, 5H), 0.82 (td, *J* = 5.5, 4.6, 2.2 Hz, 2H), 0.69 (p, *J* = 3.8, 3.0 Hz, 2H). MS(ESI+): 578.2(M+H).

**Example 134** (S)-(6-((5-chloro-2-((7-(pyrrolidin-1-yl)-6,7,8,9-tetrahydro-5H-benzo[7]annulen-2-yl)amino)pyrimidin-4-yl)amino)-3-fluoro-2-methoxyphenyl)dimethyl phosphine oxide

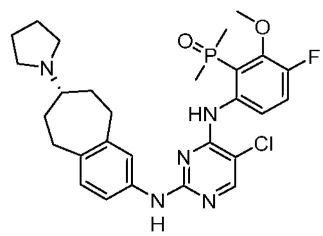

According to the preparation method of Example 1, 2-amino-N,N-dimethyl benzenesulfonamide in step a) is replaced with (6-amino-3-fluoro-2-methoxyphenyl)dimethyl phosphine oxide. The crude product is purified by a preparative high-performance liquid phase under the following conditions (column: XBridge Prep OBD column, 30×150 mm, with a filler particle size of 5 μm; mobile phase A: water (10 mmol/L ammonium bicarbonate), mobile phase B: acetonitrile;

flow rate: 60 ml/min; gradient: 15%B-60%B within 8 min; wavelength: 254/220 nm) to obtain the title product (26 mg). The HPLC retention time is 7.33 min.

$^1$HNMR (400 MHz, DMSO-$d_6$, *ppm*):δ12.06 (s, 1H), 9.27 (s, 1H), 8.45 (s, 1H), 8.15 (s, 1H), 7.43 - 7.27 (m, 3H), 7.01 (d, *J* = 8.1 Hz, 1H), 4.01 (d, *J* = 3.3 Hz, 3H), 2.89 (s, 2H), 2.54 (d, *J* = 5.2 Hz, 4H), 2.44 (s, 3H), 1.84 (d, *J* = 14.1 Hz, 8H), 1.70 (d, *J* = 5.8 Hz, 4H), 1.53 (s, 2H). MS(ESI+): 558.2(M+H).

**Example 135** (S)-(6-((5-chloro-2-((7-(pyrrolidin-1-yl)-6,7,8,9-tetrahydro-5H-benzo[7]annulen-2-yl)amino)pyrimidin-4-yl)amino)-3-fluoro-2-methylphenyl)dimethyl phosphine oxide

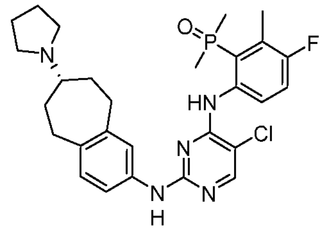

According to the preparation method of Example 47, 5-iodoquinoxaline-6-amine in step a) is replaced with 3-methyl-4-fluoroaniline.

The crude product is purified by a preparative high-performance liquid phase under the following conditions (column: Xselect CSH-OBD column, 30×150 mm, with a filler particle size of 5 μm; mobile phase A: water (10 mmol/L ammonium bicarbonate), mobile phase B: acetonitrile; flow rate: 60 ml/min; gradient: 15%B-50%B within 8 min; wavelength: 254/220 nm) to obtain the title product (12.2 mg). The HPLC retention time is 7.65 min.

$^1$HNMR (400 MHz, DMSO-$d_6$, *ppm*):δ11.29 (s, 1H), 9.21 (s, 1H), 8.10 (d, *J*=20.0 Hz, 2H), 7.38 - 7.29 (m, 2H), 7.20 (dd, *J* = 8.2, 2.2 Hz, 1H), 6.92 (d, *J* = 8.2 Hz, 1H), 2.86 (s, 1H), 2.72 (s, 1H), 2.60 (s, 4H), 2.48 (s, 2H), 2.40 (d, *J* = 2.8 Hz, 3H), 1.87 (d, *J* = 13.4 Hz, 8H), 1.72 (d, *J* = 5.9 Hz, 4H), 1.48 (s, 2H). MS(ESI+): 542.2(M+H).

**Example 136** (S)-(2-((5-chloro-2-((7-(pyrrolidin-1-yl)-6,7,8,9-tetrahydro-5H-benzo[7]annulen-2-yl)amino)pyrimidin-4-yl)amino)-4-cyano-5-methoxyphenyl)dimethyl phosphine oxide

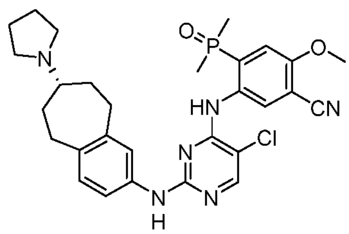

According to the preparation method of Example 47, 5-iodoquinoxaline-6-amine in step a) is replaced with 3-cyano-4-methoxyaniline.

The crude product is purified by a preparative high-performance liquid phase under the following conditions (column: YMC-Actus Triart C18 column, 30×150 mm, with a filler particle size of 5 μm; mobile phase A: water (10 mmol/L ammonium bicarbonate), mobile phase B: acetonitrile; flow rate: 60 ml/min; gradient: 20%B-45%B within 8 min; wavelength: 254/220 nm) to obtain the title product (9.6 mg). The HPLC retention time is 6.95 min.

$^1$HNMR (400 MHz, DMSO-$d_6$, *ppm*):δ10.71 (s, 1H), 9.33 (s, 1H), 8.64 (s, 1H), 8.17 (s, 1H), 7.36(d, *J* =60.0 Hz,1H), 7.31 (d, *J* = 4.0 Hz, 1H), 7.21 (s, 1H), 6.98 (d, *J* = 8.1 Hz, 1H), 3.99 (s, 3H), 2.84 (d, *J* = 16.5 Hz, 2H), 2.51- 2.45 (m, 4H), 2.42 (s, 3H), 1.84-1.80 (m, 8H), 1.69 (p, *J* = 2.9 Hz, 4H), 1.50 (s, 2H). MS(ESI+): 565.2(M+H).

**Example 137** Compounds 137-1 and 137-2

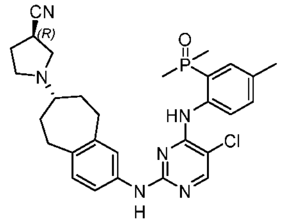

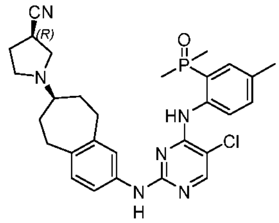

According to the preparation method of Example 58, (2-amino-5-fluorophenyl) dimethyl phosphine oxide in step a) is replaced with (2-amino-5-methylphenyl) dimethyl phosphine oxide, and 2-azabicyclo[3.1.0]hexane hydrochloride in step c) is replaced with (3R)-pyrrolidine-3-cyanohydrochloride. The crude product is purified by a preparative high-performance liquid phase under the following conditions (column: XBridge Prep OBD C18 column, 30×150 mm, with a filler particle size of 5 μm; mobile phase A: water (10 mmol/L ammonium bicarbonate), mobile phase B: acetonitrile; flow rate: 60 ml/min; gradient: 20%B-65%B within 8 min; wavelength: 254/220 nm) to obtain a mixture of two isomers (50 mg).

The mixture of two isomers is purified by a chiral high-performance liquid chromatography column (column: CHIRALPAK IE, 2×25 cm (filler 5 μm); mobile phase A: methyl tert-butyl ether (0.1% diethylamine), mobile phase B: ethanol; flow rate: 19 ml/min; gradient: 30%B within 9 min, isogradient; detection wavelength: 220/254 nm; and column temperature: 25°C) to obtain:

isomer 137-1 (10.5 mg) having a HPLC retention time of 6.7 min:

$^1$HNMR (400 MHz, DMSO-$d_6$, *ppm*): δ 10.93 (s, 1H), 9.25 (s, 1H), 8.40 (s, 1H), 8.15 (s, 1H), 7.48 - 7.39 (m, 2H), 7.35 - 7.25 (m, 2H), 6.98 (d, *J* = 8.1 Hz, 1H), 3.23 (s, 2H), 2.89 - 2.70 (m, 5H), 2.55 (d, *J* = 6.3 Hz, 3H), 2.34 (s, 3H), 2.15 (td, *J* = 8.9, 8.3, 5.2 Hz, 1H), 1.94 - 1.86 (m, 3H), 1.77 (d, *J* = 13.5 Hz, 6H), 1.51 (s, 2H).

Isomer 137-2 (10.6 mg) having a HPLC retention time of 7.8 min:

$^1$HNMR (400 MHz, DMSO-$d_6$, *ppm*): δ10.93 (s, 1H), 9.25 (s, 1H), 8.40 (s, 1H), 8.15 (s, 1H), 7.48 - 7.39 (m, 2H), 7.35 - 7.25 (m, 2H), 6.98 (d, *J* = 8.1 Hz, 1H), 3.26-3.20 (m,2H), 2.89 - 2.70 (m, 6H), 2.57 (d, *J* = 6.3 Hz, 2H), 2.34 (s, 3H), 2.15 (td, *J* = 8.9, 8.3, 5.2 Hz, 1H), 1.94 - 1.86 (m, 3H), 1.77 (d, *J* = 13.5 Hz, 6H), 1.51 (s, 2H). MS(ESI+): 549.2(M+H).

**Example 138** (S)-(2-((5-chloro-2-((7-(pyrrolidin-1-yl)-6,7,8,9-tetrahydro-5H-benzo[7]annulen-2-yl)amino)pyrimidin-4-yl)amino)-5-(2-hydroxypropan-2-yl)phenyl))dimethyl phosphine oxide

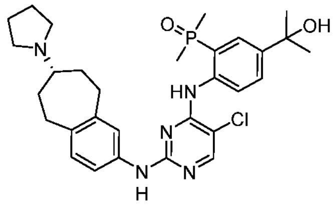

According to the preparation method of Example 47, 5-iodoquinoxaline-6-amine in step a) is replaced with 2-(4-aminophenyl)propan-2-ol.

The crude product is purified by a preparative high-performance liquid phase under the following conditions (column: XBridge Prep OBD C18 column, 30×150 mm, with a filler particle size of 5 μm; mobile phase A: water (10 mmol/L ammonium bicarbonate), mobile phase B: acetonitrile; flow rate: 60 ml/min; gradient: 10%B-50%B within 8 min; wavelength: 254/220 nm) to obtain the title product (11 mg). The HPLC retention time is 6.73 min.

$^1$HNMR (400 MHz, DMSO-$d_6$, *ppm*):δ11.00 (s, 1H), 9.26 (s, 1H), 8.44 (s, 1H), 8.16 (s, 1H), 7.59 (t, *J* = 11.7 Hz, 2H), 7.45 (s, 1H), 7.26 (d, *J* = 8.0 Hz, 1H), 6.97 (d, *J* = 8.1 Hz, 1H), 5.12 (s, 1H), 2.90 (s, 6H), 1.85 (s,3H), 1.79 (s,3H), 1.76(s, 3H), 1.70 (s, 5H), 1.57 (s, 3H), 1.46 (s, 6H). MS(ESI+): 568.2(M+H).

**Example 139** (S)-(2-((5-chloro-2-((7-(pyrrolidin-1-yl)-6,7,8,9-tetrahydro-5H-benzo[7]annulen-2-yl)amino)pyrimidin-4-yl)amino)-5-(2-methoxypropan-2-yl)phenyl)dimethyl phosphine oxide

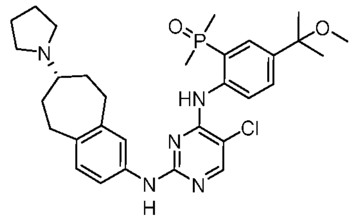

According to the preparation method of Example 47, 5-iodoquinoxaline-6-amine in step a) is replaced with 4-(2-methoxypropan-2-yl)aniline.

The crude product is purified by a preparative high-performance liquid phase under the following conditions (column: XBridge Prep OBD C18 column, 30×150 mm, with a filler particle size of 5 μm; mobile phase A: water (10 mmol/L ammonium bicarbonate), mobile phase B: acetonitrile; flow rate: 60 ml/min; gradient: 15%B-55%B within 8 min; wavelength: 254/220 nm) to obtain the title product (19.3 mg). The HPLC retention time is 6.42 min.

$^1$HNMR (400 MHz, DMSO-$d_6$, *ppm*):δ11.11 (s, 1H), 9.27 (s, 1H), 8.53 (d, *J* = 7.0 Hz, 1H), 8.17 (s, 1H), 7.52 - 7.43 (m, 3H), 7.24 (d, *J* = 8.3 Hz, 1H), 6.97 (d, *J* = 8.1 Hz, 1H), 3.01 (s, 3H), 2.91 (s, 2H), 2.54 (d, *J* = 5.2 Hz, 5H), 2.48 (s, 2H), 1.85(s, 2H), 1.81 (d, *J* = 13.5 Hz, 6H), 1.69 (s, 4H), 1.56 (s, 2H), 1.49 (s, 6H). MS(ESI+): 582.2(M+H).

**Example 140** (S)-1-(4-((5-chloro-2-((7-(pyrrolidin-l-yl)-6,7,8,9-tetrahydro-5H-benzo[7]annulen-2-yl)amino)pyrimidin-4-yl)amino)-3-(dimethylphosphoryl)phenyl)cyclopropane-1-carbonitrile

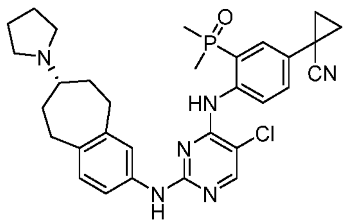

According to the preparation method of Example 47, 5-iodoquinoxaline-6-amine in step a) is replaced with 1-(4-aminophenyl)cyclopropanecarbonitrile.

$^1$HNMR (400 MHz, DMSO-$d_6$,*ppm*):δ11.13 (s, 1H), 9.30 (s, 1H), 8.56 (s, 1H), 8.26 (s, 1H), 7.53 (dd, J = 8.8, 2.3 Hz, 1H), 7.44 (d, J = 2.3 Hz, 1H), 7.36 (dd, J = 14.0, 2.4 Hz, 1H), 7.28 - 7.22 (m, 1H), 6.99 (d, J = 8.2 Hz, 1H), 2.95 - 2.77 (m, 3H), 2.72 - 2.55 (m, 6H), 2.00-1.88 (m, 2H), 1.82 (s, 3H), 1.78 (s, 3H), 1.77 -1.70 (m, 6H), 1.62 -1.48 (m, 4H). MS(ESI+): 575.3(M+H).

**Example 141** (S)-(2-((5-chloro-2-((7-(pyrrolidin-1-yl)-6,7,8,9-tetrahydro-5H-benzo[7]annulen-2-yl)amino)pyrimidin-4-yl)amino)-5-(hydroxymethyl)phenyl)dimethyl phosphine oxide

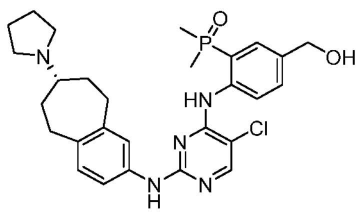

Acetic acid (10 ml) and an acetic acid solution (1 ml, 30% w/w) of hydrogen bromide are added to (S)-(2-((5-chloro-2-((7-(pyrrolidin-1-yl)-6,7,8,9-tetrahydro-5H-benzo[7]annulen-2-yl]amino)pyrimidin-4-yl)amino)-5-methoxymethyl)dimethyl phosphine oxide (500 mg), and reacted hermetically at 100°C for 16 h. after the reaction is completed, a 2N aqueous sodium hydroxide solution is added to adjust the pH=9, and a viscous solid was precipitated. The viscous solid is purified by column chromatography (dichloromethane: methanol = 10: 1 v/v) to obtain the title product (50 mg).

1H NMR (400 MHz, Chloroform-d) δ 10.85 (s, 1H), 8.50 (dd, J = 8.7, 4.4 Hz, 1H), 8.06 (s, 1H), 7.67 (s, 1H), 7.49 (d, J = 8.7 Hz, 1H), 7.20 - 7.12 (m, 1H), 7.09 - 7.00 (m, 2H), 6.90 (dd, J = 8.0, 2.4 Hz, 1H), 4.67 (d, J = 4.9 Hz, 2H), 3.09 (m,4H), 2.88 (dt, J = 13.3, 6.7 Hz, 2H), 2.76 - 2.62 (m, 4H), 2.48 (m, 1H), 2.25 (m,1H), 1.98 (q, J = 3.9 Hz, 4H), 1.83 (dd, J = 13.1, 4.3 Hz, 6H), 1.60 (d, J = 11.1 Hz, 2H). MS(ESI+): 540.3(M+H).

**Part II Biological Activity Test**

The specific structure of the positive drug 1 (BGB324) used in the activity test is as follows:

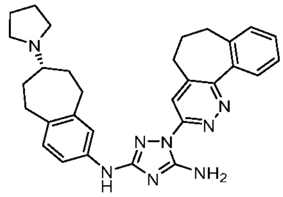
.

The specific structure of the positive drug 2 (TP0903) is as follows:

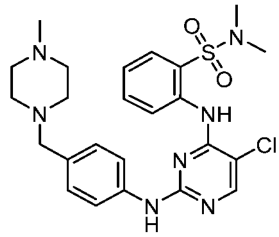
.

The above compounds are purchased from Shanghai Shenghong Biological Technology Co., Ltd.

**Test 1:** Inhibitory activity of compound against AXL kinase

1.Experimental process

a) AXL enzyme (Carna, 08-107) configuration and addition: a 1× enzyme buffer (200 μL of Enzymatic buffer kinase 5X, 10 μL of 500 mM $MgCl_2$, 10 μL of 100 mM DTT and 6.26 μL of 2500 nM SEB are added with 773.75 μL of $H_2O$, and prepared into 1 ml of 1× enzyme buffer) is used. AXL enzyme is diluted from 33.33 ng/uL to 0.027 ng/μL (1.67×, final conc.=0.016 ng/uL), a BioTek (MultiFlo FX) automatic liquid dispenser is used, and 6 μL of enzyme solution having a 1.67-fold final concentration is added respectively to compound wells and positive control wells; and 6 μL of 1×Enzymatic buffer is added to negative control wells.
b) Compound preparation and addition: the compounds and positive drugs prepared in the examples are diluted from 10 mM to 100 μM by DMSO, and titrated with a compound titrator (Tecan, D300e). The titrated solution is automatically sprayed by the titrator to each well to reach a required concentration, wherein the first concentration is 1 μM, and after 1/2 log dilution, a total of 8 concentrations are obtained. The resulting mixture is centrifuged at 2500 rpm for 30 s and incubated at room temperature for 15 min.
c) Preparation and addition of ATP and substrate: ATP (Sigma, A7699) is diluted with 1× enzyme buffer from 10 mM to 75 μM (5×), and the final concentration is 15 μM; the substrate TK Substrate 3-biotin (Cisbio, 61TK0BLC) is diluted with 1× enzyme buffer from 500 μM to 5 μM (5×), and the final concentration is 1 μM; ATP and the substrate are mixed in equal volume to obtain a mixed solution; the mixed solution is added to each well at 4 μL by using a BioTek automatic dispenser; centrifuged at 2500 rpm for 30 s, and reacted at 25°C for 45 min.
d) Preparation and addition of detection reagent: Streptavidin-XL665 (Cisbio, 610SAXLG) is diluted with HTRF KinEASE detection buffer (cisbio) from 16.67 μM to 250 nM (4×), and the final concentration is 62.5 nM; TK Antibody-Cryptate (Cisbio) is diluted with HTRF KinEASE detection buffer (cisbio) from 100× to 5×, and the final concentration is 1×; XI,665 and Antibody are mixed in equal volume; the mixed solution is added to each well at 10 μL by using the BioTek automatic dispenser, centrifuged at 2500 rpm for 30 s, and reacted at 25°C for 1 h. After the reaction is completed, the resulting product is detected by a multi-function plate reader HTRF.

2. Data analysis

GraphPad Prism 5 software log (inhibitor) vs. response-Variable slope is used to fit a dose-response curve to obtain the $IC_{50}$ value of the compound to the inhibition of AXL kinase. The calculation formula of the inhibition rate is as follows:

$$\% \text{ Inhibition} = \frac{\text{Conversion\%_max} - \text{Conversion\%_sample}}{\text{Conversion\%_max} - \text{Conversion\%_min}} \times 100$$

Conversion%_sample: a conversion rate reading of a sample;
Conversion%_min: a conversion rate reading in the absence of enzyme active wells;
Conversion%_max: a conversion rate reading in the absence of compound inhibition wells.

3. Experimental results

The experimental results are shown in Table 1, where the IC50 of the compound is expressed as "<" (less than) a specific value, which means that the IC50 value is lower than a detection limit of the test used.

Table 1. $IC_{50}$ data of AXL

| Title compound of example | Inhibitory activity $IC_{50}$(nM) against AXL | Title compound of example | Inhibitory activity $IC_{50}$(nM) against AXL |
|---|---|---|---|
| Example 1 | 0.33 | Example 2 | 13.40 |
| Example 3 | 23.58 | Example 4 | <0.11 |
| Example 5 | 20.71 | Example 6 | <0.11 |
| Example 7 | 7.74 | Example 8 | 8.28 |
| Example 9 | 21.86 | Example 10 | 2.35 |
| Example 11 | 8.18 | Example 12 | <0.11 |
| Example 13 | <0.11 | Example 15 | 2.41 |
| Example 16 | 0.12 | Example 17 | 27.91 |
| Example 18 | 0.19 | Example 19 | 0.11 |
| Example 20 | 1.93 | Example 21 | 6.53 |
| Example 22 | 0.11 | Example 23 | 1.38 |
| Example 24 | <0.11 | Example 25 | 0.62 |
| Example 26 | 7.21 | Example 27 | 19.03 |
| Example 28 | 0.31 | Example 29 | 0.34 |
| Example 30 | 1.05 | Example 31 | 0.80 |
| Example 32 | 0.75 | Example 33 | 5.15 |
| Example 34 | 1.27 | Example 35 | 1.21 |
| Example 36 | 0.36 | Example 37 | 0.18 |
| Example 38 | 6.24 | Example 39 | 2.59 |
| Example 40 | 0.72 | Example 41 | 2.08 |
| Example 42 | 14.57 | Example 43 | 9.87 |
| Example 44 | 1.47 | Example 45 | 0.50 |
| Example 46 | 0.80 | Example 47 | 1.72 |
| Example 48 | 0.42 | Example 49 | 9.85 |

(continued)

| Title compound of example | Inhibitory activity $IC_{50}$(nM) against AXL | Title compound of example | Inhibitory activity $IC_{50}$(nM) against AXL |
|---|---|---|---|
| Example 50 | 2.16 | Example 51 | 2.60 |
| Example 52 | 1.06 | Example 53 | 1.86 |
| Example 54 | 3.10 | Example 55 | 3.14 |
| Example 56 | 2.04 | Example 57 | 6.27 |
| Example 58 Isomer 58-1 | 1.88 | Example 58 Isomer 58-2 | 6.45 |
| Example 58 Isomer 58-3 | 60.44 | Example 58 Isomer 58-4 | 78.55 |
| Example 59 Isomer 59-1 | 1.39 | Example 59 Isomer 59-2 | 86.45 |
| Example 60 Isomer 60-1 | 1.65 | Example 60 Isomer 60-2 | 106.65 |
| Example 61 | 2.38 | Example 62 Isomer 62-1 | 49.05 |
| Example 62 Isomer 62-2 | 1.12 | Example 63 Isomer 63-1 | 1.93 |
| Example 63 Isomer 63-2 | 61.66 | Example 63 Isomer 63-3 | 2.46 |
| Example 63 Isomer 63-4 | 57.04 | Example 69 Isomer 69-1 | 1.35 |
| Example 69 Isomer 69-2 | 44.72 | Example 70 Isomer 70-1 | 1.23 |
| Example 71 Isomer 71-1 | 0.26 | Example 71 Isomer 71-2 | 26.70 |
| Example 72 Isomer 72-1 | 1.02 | Example 72 Isomer 72-2 | 1.0 |
| Example 79 | 1.05 | Example 84 Isomer 84-1 | 0.98 |
| Example 84 Isomer 84-2 | 28.79 | Example 85 Isomer 85-1 | 0.73 |
| Example 85 Isomer 85-2 | 33.05 | Example 86 Isomer 86-1 | 30.34 |
| Example 86 Isomer 86-2 | 1.57 | Example 87 Isomer 87-1 | 0.53 |
| Example 87 Isomer 87-2 | 36.48 | Example 88 Isomer 88-1 | 39.43 |
| Example 88 Isomer 88-2 | 0.55 | Example 89 Isomer 89-1 | 0.77 |
| Example 89 Isomer 89-2 | 27.38 | Example 90 Isomer 90-1 | 0.57 |

(continued)

| Title compound of example | Inhibitory activity $IC_{50}$(nM) against AXL | Title compound of example | Inhibitory activity $IC_{50}$(nM) against AXL |
|---|---|---|---|
| Example 90 Isomer 90-2 | 45.71 | Example 91 Isomer 91-1 | 1.56 |
| Example 91 Isomer 91-2 | 57.40 | Example 92 Isomer 92-1 | 1.42 |
| Example 92 Isomer 92-2 | 38.16 | Example 93 Isomer 93-1 | 1.08 |
| Example 93 Isomer 93-2 | 14.88 | Example 94 Isomer 94-1 | 0.68 |
| Example 94 Isomer 94-2 | 27.57 | Example 100 Isomer 100-1 | 1.00 |
| Example 100 Isomer 100-2 | 40.01 | Example 101 Isomer 101-1 | 1.20 |
| Example 101 | 55.21 | Example 102 | 1.16 |
| Isomer 101-2 | | Isomer 102-1 | |
| Example 102 Isomer 102-2 | 35.53 | Example 103 Isomer 103-1 | 1.34 |
| Example 103 Isomer 103-2 | 47.51 | Example 104 Isomer 104-1 | 1.33 |
| Example 104 Isomer 104-2 | 62.97 | Example 105 Isomer 105-1 | 0.86 |
| Example 105 Isomer 105-2 | 24.92 | Example 106 Isomer 106-1 | 0.71 |
| Example 106 Isomer 106-2 | 21.81 | Example 110 | 1.70 |
| Example 111 | 1.81 | Example 112 | 2.41 |
| Example 113 | 1.28 | Example 114 | 2.03 |
| Example 115 | 4.89 | Example 116 Isomer 116-1 | 4.94 |
| Example 116 Isomer 116-2 | 96.83 | Example 117 | 1.78 |
| Example 118 | 5.45 | Example 119 | 9.24 |
| Example 120 Isomer 120-1 | 4.01 | Example 120 Isomer 120-2 | 64.16 |
| Example 121 Isomer 121-1 | 3.21 | Example 121 Isomer 121-2 | 50.07 |
| Example 122 Isomer 122-1 | 1.87 | Example 122 Isomer 122-2 | 63.43 |
| Example 123 Isomer 123-1 | 2.04 | Example 123 Isomer 123-2 | 38.18 |
| Example 124 Isomer 124-1 | 8.05 | Example 124 Isomer 124-2 | 74.33 |

(continued)

| Title compound of example | Inhibitory activity $IC_{50}$(nM) against AXL | Title compound of example | Inhibitory activity $IC_{50}$(nM) against AXL |
|---|---|---|---|
| Example 125 | 4.11 | Example 125 | 80.46 |
| Isomer 125-1 | | Isomer 125-2 | |
| Example 126 Isomer 126-1 | 4.87 | Example 126 Isomer 126-2 | 47.35 |
| Example 127 Isomer 127-1 | 3.43 | Example 127 Isomer 127-2 | 66.44 |
| Example 128 | 1.82 | Example 129 | 9.68 |
| Example 130 | 12.28 | Example 131 | 207.20 |
| Example 132 | 2.36 | Example 133 Isomer 133-1 | 1.09 |
| Example 133 Isomer 133-2 | 38.08 | Example 134 | 0.85 |
| Example 135 | 1.35 | Example 136 | 13.08 |
| Example 137 Isomer 137-1 | 6.38 | Example 137 Isomer 137-2 | 38.24 |
| Example 138 | 5.29 | Example 139 | 2.53 |
| Example 140 | 2.91 | Example 141 | 2.43 |
| Positive drug 1 (BGB324) | 2.25 | Positive drug 2 (TP0903) | 16.39 |

**Test 2:** Detection of proliferation inhibition activity of compounds to cells

1.Experimental process

MV-4-11 (human myeloid monocytic leukemia cell line, culture medium: IMDM+10% fetal bovine serum) is purchased from Cobioer Biosciences Co.,Ltd and cultured in a 5% $CO_2$ incubator at 37°C. The cells in a logarithmic growth phase are plated in a 96-well plate at cell densities of 8000 cells/well, 6000 cells/well, 2000 cells/well, 2000 cells/well and 3000 cells/well, and a blank control group is set at the same time.

A compound to be tested and the positive drug are dissolved in dimethyl sulfoxide to prepare a 10 mM stock solution, which is then stored in -80°C refrigerator for long-time storage. After the cells are plated for 24 h, the 10 mM compound stock solution is diluted with dimethyl sulfoxide to obtain a working solution having a 200-fold concentration (the highest concentration is 200 or 2000 μM, a 3-fold gradient, a total of 10 concentrations), and 3 μL of the working solution of each concentration is added to 197 μL of compelte medium, and diluted into a working solution of 3-fold concentration. 50 μL of the working solution is then taken and added to a 100 μL cell culture medium (a final concentration of dimethyl sulfoxide is 0.5%, v/v), and two complex holes are set for each concentration. After 72 h of dosing treatment, 50 μl of CellTiter-Glo® (purchased from Promega) is added to each well. Fluorescence signals are measured on Envision (PerkinElmer) according to an operating procedure of the specification. GraphPad Prism 5 software log(inhibitor) vs.response-Variable slope is used to fit a dose-response curve, so as to obtain the $IC_{50}$ value of the compound to the inhibition of cell proliferation. The calculation formula of the inhibition rate is as follows:

$$\text{Inhibition rate\%} = \frac{1-(\text{Signal value of subject}-\text{signal value of blank group})}{\text{Signal value of negative control group}-\text{signal value of blank group}} \times 100\,\%$$

wherein:

signal value of subject: a mean fluorescence signal of cell + medium + compound group;

signal value of blank group: a mean fluorescence signalof medium group (containing 0.5% DMSO);
signal value of negative control group: a mean fluorescence signal of cell + medium group (containing 0.5% DMSO).

2. Experimental results

The experimental results are shown in Table 2:

Table 2 Anti-proliferative activity of compounds for MV4-11 cells

| **Title compound of examples** | **$IC_{50}$(MV4-11, nM)** |
|---|---|
| Example 1 | 6.83 |
| Example 6 | 3.38 |
| Example 10 | 5.49 |
| Example 12 | 1.44 |
| Example 13 | 0.64 |
| Example 15 | 9.15 |
| Example 16 | 0.97 |
| Example 19 | 1.60 |
| Example 20 | 5.96 |
| Example 22 | 6.76 |
| Example 23 | 7.11 |
| Example 24 | 6.89 |
| Example 25 | 3.23 |
| Example 29 | 3.23 |
| Example 30 | 2.64 |
| Example 31 | 1.46 |
| Example 32 | 1.98 |
| Example 34 | 6.16 |
| Example 36 | 5.12 |
| Example 37 | 3.39 |
| Example 39 | 6.40 |
| Example 40 | 4.93 |
| Example 41 | 4.28 |
| Example 45 | 3.48 |
| Example 46 | 1.63 |
| Example 47 | 5.24 |
| Example 49 | 8.30 |
| Example 51 | 4.53 |
| Example 52 | 5.32 |
| Example 53 | 6.61 |
| Example 61 | 7.10 |
| Example 62<br>Isomer 62-2 | 3.50 |

(continued)

| Title compound of examples | $IC_{50}$(MV4-11, nM) |
|---|---|
| Example 63<br>Isomer 63-1 | 7.02 |
| Example 69 | 3.46 |
| Isomer 69-1 | |
| Example 70<br>Isomer 70-1 | 9.38 |
| Example 71<br>Isomer 71-1 | 7.74 |
| Example 75 | 2.93 |
| Example 76 | 7.98 |
| Example 77 | 10.13 |
| Example 81 | 3.13 |
| Example 84<br>Isomer 84-1 | 2.55 |
| Example 85<br>Isomer 85-1 | 1.93 |
| Example 86<br>Isomer 86-2 | 2.91 |
| Example 87<br>Isomer 87-1 | 3.78 |
| Example 88<br>Isomer 88-2 | 2.06 |
| Example 89<br>Isomer 89-1 | 2.99 |
| Example 90<br>Isomer 90-1 | 2.94 |
| Example 91<br>Isomer 91-1 | 2.25 |
| Example 92<br>Isomer 92-1 | 2.01 |
| Example 93<br>Isomer 93-1 | 2.65 |
| Example 94<br>Isomer 94-1 | 2.42 |
| Example 95<br>Isomer 95-1 | 7.49 |
| Example 96<br>Isomer 96-1 | 9.15 |
| Example 97<br>Isomer 97-1 | 8.88 |
| Example 98<br>Isomer 98-1 | 8.66 |

(continued)

| Title compound of examples | $IC_{50}$(MV4-11, nM) |
|---|---|
| Example 99<br>Isomer 99-1 | 7.52 |
| Example 102<br>Isomer 102-1 | 5.19 |
| Example 103<br>Isomer 103-1 | 6.24 |
| Example 104<br>Isomer 104-1 | 7.21 |
| Example 105<br>Isomer 105-1 | 2.16 |
| Example 106<br>Isomer 106-1 | 3.51 |
| Example 109 | 8.56 |
| Example 115 | 6.97 |
| Example 116<br>Isomer 116-1 | 10.11 |
| Example 118 | 30.29 |
| Example 120<br>Isomer 120-1 | 6.88 |
| Example 121<br>Isomer 121-1 | 3.90 |
| Example 122<br>Isomer 122-1 | 3.59 |
| Example 123<br>Isomer 123-1 | 3.03 |
| Example 125<br>Isomer 125-1 | 4.36 |
| Example 133<br>Isomer 133-1 | 5.42 |
| Example 134 | 3.63 |
| Example 135 | 4.23 |
| Example 138 | 41.26 |
| Example 141 | 13.91 |
| Positive drug 1 (BGB324) | 208.1 |
| Positive drug 2 (TP0903) | 11.97 |

**Test 3:** In-vivo efficacy of compound for MV4-11

Inhibitory effects of a compound and a positive drug on the in-vivo tumor growth of a xenograft tumor model of a nude mouse on human acute monocytic leukemia cell MV-4-11 are tested.

1.Construction of mouse model

MV-4-11 cells in a logarithmic growth phase are harvested, resuspended after cell counting, and adjusted in cell concentration to $7.0\times10^7$ cells/mL. The resuspended solution is injected into the right armpit of the nude mice subcutaneously. Each animal is inoculated with 200 $\mu$L ($14\times10^6$ cells) of the suspended solution to establish a MV-4-11 xenograft tumor model. Afer the tumor volume reaches 100-300 $mm^3$, tumor-bearing mice in good health and similar tumor volume are selected.

2.Compound configuration

The compound and the positive drug are vortexed with a suitable solvent and then ultrasonicated to completely dissolve the compound. Then, an appropriate volume of citric acid buffer is slowly added, and the mixed solution is vortexed to make the solution mixed uniformly to obtain administrative formulations having concentrations of 0.1, 0.5, and 1 $mg\cdot mL^{-1}$.
Solvent control group: PEG400 & citric acid buffer (20:80, v:v).

3.Animal grouping and administration

The modeled mice are randomly divided into groups (n=6), and administrated with the relevant compounds and positive drugs on the day of grouping. After 21 days or after the tumor volume of the solvent control group reaches 2000 $mm^3$, the experiment is terminated (whichever reaches the index first), and the administration volume is $10mL\cdot kg^{-1}$. Both the compound and the positive drug are administered by gavage, once a day. After the experiment starts, the tumor diameter and animal body weight are measured twice a week, and the tumor volume is calculated.

4. Data analysis

The tumor volume (TV) calculation formula is: tumor volume ($mm^3$) = $l \times w^2/2$,
wherein $l$ represents a long diameter (mm) of the tumor; w represents a short diameter (mm) of the tumor.
The calculation formula of relative tumor volume (RTV) is: $RTV = TV_t/TV_{initial}$
wherein $TV_{initial}$ is the tumor volume measured at the time of grouping and first administration; TVt is the tumor volume at each measurementduring administration.
The calculation formula of tumor growth inhibition rate TGI(%) is: $TGI=100\%\times [1-(TV_{t(T)} -TV_{initial}(T))/(TV_{t(C)}-TV_{initial(C)})]$
wherein $TV_{t(T)}$ represents the tumor volume of treatment group at each measurement; $TV_{initial(T)}$ represents the tumor volume of treatment group measured at the time of grouping and first administration; $TV_{t(C)}$ represents the tumor volume of solvent control group at each measurement; $TV_{initial(C)}$ represents the tumor volume of the solvent control group measured at the time of grouping and first administration.
The calculation formula of relative tumor proliferation rate (%T/C) is: $\%T/C =100\%\times (RTV_T/RTV_C)$
wherein $RTV_T$ represents RTV of treatment group; RTVc represents RTV of solvent control group.
The test data is calculated and subjected to related statistical treatment with Microsoft Office Excel 2007 software.

5. Experimental results

The experimental results are shown in Table 3:

Table 3 In-vivo efficacy of compounds for MV4-11

| **Compound** | **Intragastric administration dose (mg/kg/d)** | **Tumor growth inhibition rate (TGI%)** | **Relative tumor proliferation rate (% T/C)** |
|---|---|---|---|
| Example 10 | 5 | 106 | 3.7 |
| Example 18 | 5 | 71 | 37 |
| Example 19 | 1 | 78 | 28 |
| Example 28 | 5 | 104 | 5.9 |
| Example 47 | 5 | 94 | 15 |

(continued)

| Compound | Intragastric administration dose (mg/kg/d) | Tumor growth inhibition rate (TGI%) | Relative tumor proliferation rate (% T/C) |
|---|---|---|---|
| Example 53 | 5 | 79 | 31 |
| Example 115 | 5 | 85 | 23 |
| Positive drug 1 (BGB324) | 20 | 32 | 71 |
| Positive drug 2 (TP0903) | 5 | 62 | 42 |

Notes: the experimental data in the table is relevant data obtained at the end of the experiment (the end of the experiment is defined as: the end of the experiment after 21 days or after the tumor volume of the solvent control group reaches 2000 $mm^3$ (whichever comes first)).

**Test 4:** Pharmacokinetic study of compounds on ICR mice

1. Formulation of gavage prescriptions for compounds

Each compound is prepared with DMSO into a 10 mg/mL stock solution.

Preparation of mixed solvent: Tween 80: PEG400: Water=1:9:90 (v/v/v)

450 μl of the compound DMSO stock solution with a concentration of 10 mg/mL is accurately sucked into a glass bottle, and added with an appropriate volume of DMSO and mixed solvent, wherein a ratio of the solvent in the final formulation is DMSO: mixed solvent (v/v) = 10: 90. The mixed solution is vortexed (or ultrasonically treated), and dispersed uniformly to obtain 4.5 mL administration test solutions with a concentration of 1 mg/mL, respectively.

2. Test plan

Male 6-10 week old ICR mice (sourced from: Beijing Vital River Laboratory Animal Technology Co., Ltd.) are taken, 6 mice in each group. The mice are fasted overnight and fed 4 h after the administration. On the day of the experiment, the mice are administrated with 10 $mg \cdot kg^{-1}$ of compound test solution by gavage. After administration, about 100 μL of blood is harvested from the orbits of the mice at 0 min, 5 min, 15 min, 30 min, 1 h, 2 h, 4 h, 8 h, and 24 h, and placed in EDTA-$K_2$ anticoagulant tubes. The whole blood sample is centrifuged at 1500 to 1600 g for 10 min, and the plasma obtained by separation is stored in a refrigerator at -40 to -20°C for biological sample analysis. The blood drug concentration is determined by an LC-MS/MS method.

3. Data analysis and results

A non-compartmental model in Pharsight Phoenix 7.0 is used to calculate pharmacokinetic parameters. The specific results are shown in the table below.

Table 4 ICR mouse pharmacokinetic results of the compounds

| Compound | Cmax (ng/mL) | Tmax (h) | $AUC_{0-24}$ (ng.h/mL) | T1/2 (h) |
|---|---|---|---|---|
| Example 10 | 349 | 0.25 | 1000 | 3.33 |
| Example 18 | 249 | 2.00 | 2300 | 3.03 |
| Example 19 | 342 | 1.00 | 3540 | 3.05 |
| Example 28 | 226 | 8.00 | 3000 | NR |
| Example 47 | 69 | 1.00 | 307 | 2.06 |
| Example 51 | 419 | 0.50 | 1070 | 2.17 |
| Example 52 | 429 | 0.50 | 1150 | 2.42 |

(continued)

| Compound | Cmax (ng/mL) | Tmax (h) | $AUC_{0-24}$ (ng.h/mL) | T1/2 (h) |
|---|---|---|---|---|
| Example 53 | 676 | 1.00 | 2200 | 1.81 |
| Example 58 | 171 | 0.50 | 822 | 4.04 |
| Isomer 58-1 | | | | |
| Example 58 Isomer 58-2 | 278 | 0.25 | 549 | 3.06 |
| Example 59 Isomer 59-1 | 695 | 0.25 | 2380 | 2.90 |
| Example 60 Isomer 60-1 | 572 | 0.50 | 857 | 1.55 |
| Example 115 | 324 | 2.17 | 1300 | 1.35 |
| Positive drug 2 (TP-0903) | 26.8 | 0.25 | 52.2 | 1.20 |
| NR: Not reported | | | | |

## Claims

**1.** A compound of Formula I, or a pharmaceutically acceptable salt thereof,

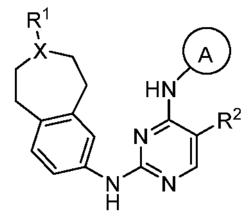

I

wherein X is CH or N;

$R^1$ is a 5-12 membered saturated heterocyclic ring or a 5-8 membered saturated carbocyclic ring, which is optionally substituted by one or more $C_{1-6}$ alkyl, $C_{1-6}$ alkoxyl, halogen, cyano, deuterium or hydroxyl, and $R^1$ is not

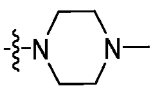 , 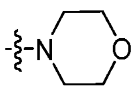 and 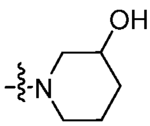 ;

$R^2$ is halogen;

ring A is selected from phenyl, 5-6 membered heteroaryl or 9-12 membered benzoheterocyclyl, wherein phenyl and 5-6 membered heteroaryl are optionally substituted by one or more $R^3$, and 9-12 membered benzoheterocyclyl is optionally substituted by -ξ-O or one or more $R^3$;

$R^3$ is selected from: deuterium, halogen, $C_{1-6}$ alkyl, $C_{1-6}$ alkoxyl, $C_{3-10}$ cycloalkyloxyl,

-ξ-OH , -ξ-CN , -ξ-P(=O)$R^4R^5$ , -ξ-S(=O)$_2R^6$ , -ξ-S(=O)$_2NR^7R^8$ , -ξ-C(=O)$NR^7R^8$ ,

-ξ-$NR^7$S(=O)$_2R^6$ , 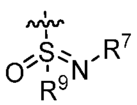 , -ξ-C(=O)$OR^{10}$ , -ξ-C(=O)$R^{11}$ , -ξ-$R^{12}$ , -ξ-NHOH , -ξ-$NO_2$ ,

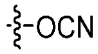 or 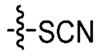 ,

wherein the $C_{1-6}$ alkyl or $C_{1-6}$ alkoxyl is optionally substituted by hydroxyl, halogen, cyano, $C_{1-3}$ alkoxyl or 4-7 membered heterocycloalkyl;

$R^4$ and $R^5$ are independently selected from $C_{1-6}$ alkyl, hydroxyl, $C_{2-6}$ alkenyl, $C_{2-6}$ alkynyl, $C_{1-6}$ alkoxyl or $C_{3-10}$ cycloalkyl, wherein the $C_{1-6}$ alkyl is optionally substituted by deuterium, hydroxyl, halogen, cyano or $C_{1-3}$ alkoxyl; or $R^4$ and $R^5$ can form a 3-6 membered phosphorus-containing saturated monocyclic ring together with adjacent P atom;

$R^6$ is selected from $C_{1-6}$ alkyl, $C_{2-6}$ alkenyl, $C_{2-6}$ alkynyl, $C_{1-6}$ alkoxyl, $C_{3-10}$ cycloalkyl, 4-7 membered heterocycloalkyl or 5-7 membered heteroaryl;

$R^7$ and $R^8$ are independently selected from hydrogen, $C_{1-6}$ alkyl, $C_{1-6}$ alkoxyl, $C_{3-10}$ cycloalkyl, 4-7 membered heterocycloalkyl or 5-7 membered heteroaryl, wherein the $C_{1-6}$ alkyl is optionally substituted by hydroxyl, halogen, cyano or $C_{1-3}$ alkoxyl; or $R^7$ and $R^8$ form a 3-6 membered nitrogen-containing saturated monocyclic ring together with their adjacent N atom;

$R^9$ and $R^{10}$ are independently selected from $C_{1-6}$ alkyl, $C_{2-6}$ alkenyl, $C_{2-6}$ alkynyl or $C_{3-10}$ cycloalkyl;

$R^{11}$ is selected from $C_{1-6}$ alkyl, $C_{2-6}$ alkenyl, $C_{2-6}$ alkynyl, $C_{3-10}$ cycloalkyl, 4-7 membered heterocycloalkyl or 5-7 membered heteroaryl; and

$R^{12}$ is selected from $C_{3-10}$ cycloalkyl, 4-7 membered heterocycloalkyl or 5-7 membered heteroaryl, which is optionally substituted by one or more hydroxyl, halogen, cyano, $C_{1-6}$ alkyl or 3 -7 membered heterocycloalkyl.

2. The compound of Formula I according to claim 1, wherein $R^1$ is a 5-8 membered saturated heterocyclic ring or a 5-8 membered saturated carbocyclic ring, which is optionally substituted by one or more $C_{1-6}$ alkyl, $C_{1-6}$ alkoxyl, halogen, cyano, deuterium or hydroxyl, and $R^1$ is not

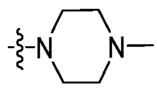 , 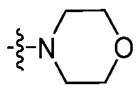 and 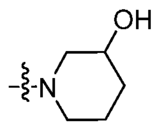 ;

or, $R^1$ is a 5-12 membered saturated heterocyclic ring or a 5-7 membered saturated carbocyclic ring, which is optionally substituted by one or more $C_{1-6}$ alkyl, $C_{1-6}$ alkoxyl, halogen, cyano, deuterium or hydroxyl, and $R^1$ is not

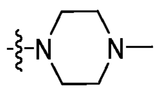 , 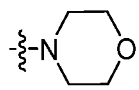

and

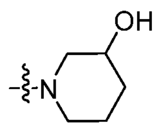 .

3. The compound of Formula I according to claim 1 **characterized in that**:ring A is phenyl, furyl, thienyl, pyrrolyl, pyrazolyl, imidazolyl, thiazolyl, oxazolyl, 1,2,3-triazolyl, 1,2,4-triazolyl, pyridyl, pyrimidinyl, pyridazinyl, pyrazinyl, benzo five-membered heterocyclyl or benzo six-membered heterocyclyl groups, wherein the groups are optionally substituted by one or more $R^3$.

4. The compound of Formula I according to claim 1 **characterized in that**:$R^3$ is selected from: deuterium, halogen, $C_{1-6}$ alkyl, $C_{1-6}$ alkoxyl, $C_{3-10}$ cycloalkyloxyl,

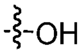, 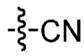,

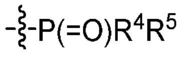, 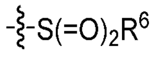, 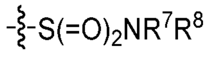, 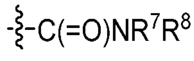, 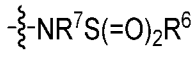,

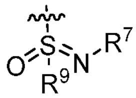, 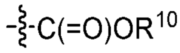 or 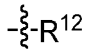,

wherein the $C_{1-6}$ alkyl or $C_{1-6}$ alkoxyl is optionally substituted by hydroxyl, halogen, cyano, $C_{1-3}$ alkoxyl or 4-7 membered heterocycloalkyl; or, $R^3$ is selected from: deuterium, halogen, $C_{1-6}$ alkyl, $C_{1-6}$ alkoxyl,

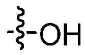, 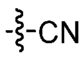,

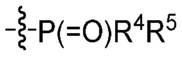, 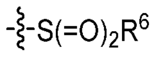, 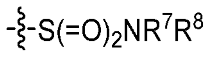, 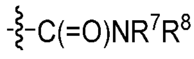, 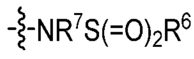,

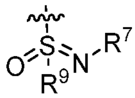, 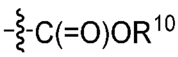, 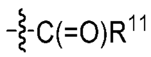, 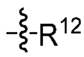, 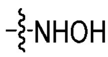, 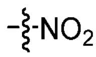, 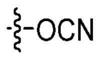 or 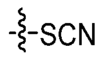,

wherein the $C_{1-6}$ alkyl or $C_{1-6}$ alkoxyl is optionally substituted by hydroxyl, halogen, cyano, $C_{1-3}$ alkoxyl or 4-7 membered heterocycloalkyl; and preferably, $R^3$ is selected from: deuterium, halogen, $C_{1-6}$ alkyl, $C_{1-6}$ alkoxyl,

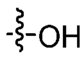, 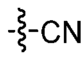,

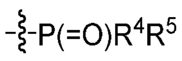, 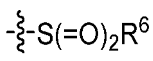, 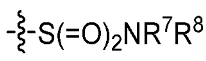, 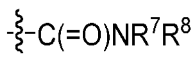, 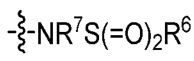,

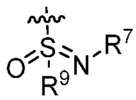, 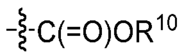 or 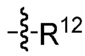,

wherein the $C_{1-6}$ alkyl or $C_{1-6}$ alkoxyl is optionally substituted by hydroxyl, halogen, cyano, $C_{1-3}$ alkoxyl or 4-7 membered heterocycloalkyl.

5. The compound of Formula I according to claim 1 **characterized in that** having a structure shown in the following compound 1-1 or I-2,

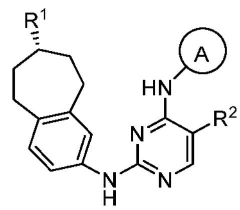

I-1

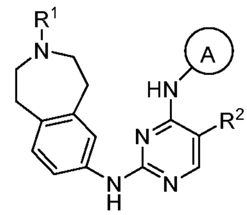

I-2

wherein the definitions of $R^1$, $R^2$ and ring A are as defined in the compound of Formula I.

**6.** A compound of Formula II or a pharmaceutically acceptable salt thereof,

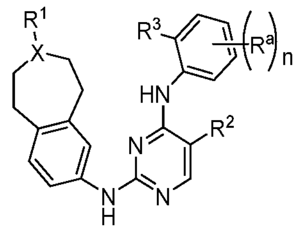

II

wherein the definitions of X, $R^1$, $R^2$ and $R^3$ are consistent with those defined in the compound of Formula I;
n is an integer from 0 to 4;
$R^a$ is deuterium, halogen, $C_{1-6}$ alkyl, $C_{1-6}$ alkoxyl, $C_{3-10}$ cycloalkyloxyl,

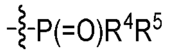 ,

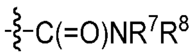 , 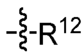 , 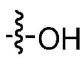 or 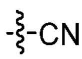 ,

wherein the $C_{1-6}$ alkyl or $C_{1-6}$ alkoxyl is optionally substituted by one or more deuterium, methoxyl, hydroxyl, halogen or cyano; preferably, $R^a$ is deuterium, halogen, $C_{1-6}$ alkyl, $C_{1-6}$ alkoxyl,

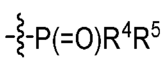 , 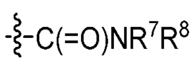 ,

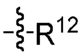 , 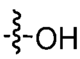 or 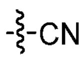 ,

wherein the $C_{1-6}$ alkyl or $C_{1-6}$ alkoxyl is optionally substituted by one or more deuterium, halogen or cyano;

preferably, $R^a$ is deuterium, halogen, $C_{1-6}$ alkyl, $C_{1-6}$ alkoxyl, 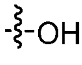 or 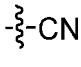, wherein the $C_{1-6}$ alkyl or $C_{1-6}$ alkoxyl is optionally substituted by one or more deuterium, halogen or cyano;
wherein the definitions of $R^4$, $R^5$, $R^7$, $R^8$ and $R^{12}$ are consistent with those defined in the compound of Formula I;
preferably, the compound of Formula II has a structure shown in the following compound II-1,

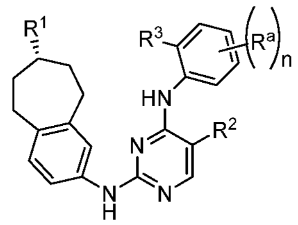

II-1

wherein the definitions of $R^1$, $R^2$, $R^3$, $R^a$ and n are consistent with those defined in the compound of Formula II.

**7.** The compound of Formula II according to claim 6 **characterized in that** having a structure shown in the following compound III,

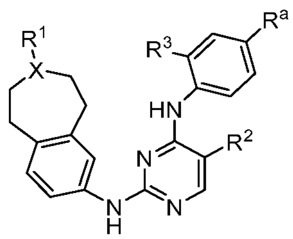

III

wherein the definitions of X, $R^1$, $R^2$, $R^3$ and $R^a$ are consistent with those defined in the compound of Formula II; preferably, the compound of Formula II has a structure shown in the following compound III-1,

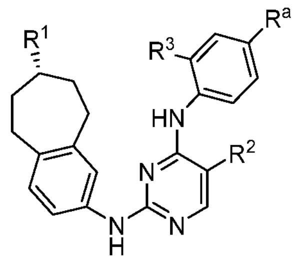

III-1

wherein the definitions of $R^1$, $R^2$, $R^3$ and $R^a$ are consistent with those defined in the compound of Formula II.

**8.** A compound of Formula IV or a pharmaceutically acceptable salt thereof,

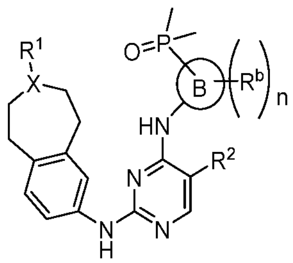

IV

wherein the definitions of X, $R^1$ and $R^2$ are consistent with those defined in the compound of Formula I;
n is an integer from 0 to 4;

ring B is selected from phenyl, 5-6 membered heteroaryl, or 9-12 membered benzoheterocyclyl optionally substituted by

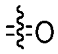;

$R^b$ is deuterium, halogen, $C_{1-6}$ alkyl, $C_{1-6}$ alkoxyl, $C_{3-10}$ cycloalkyloxyl,

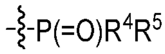,

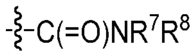, 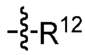, 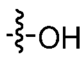 or 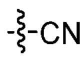,

wherein the $C_{1-6}$ alkyl or $C_{1-6}$ alkoxyl is optionally substituted by one or more methoxyl, hydroxyl, deuterium, halogen or cyano;
preferably, $R^b$ is deuterium, halogen, $C_{1-6}$ alkyl, $C_{1-6}$ alkoxyl,

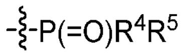,

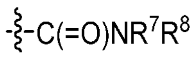, 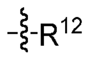, 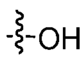 or 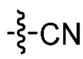,

wherein the $C_{1-6}$ alkyl or $C_{1-6}$ alkoxyl is optionally substituted by one or more deuterium, halogen or cyano;
preferably, $R^b$ is deuterium, halogen, $C_{1-6}$ alkyl, $C_{1-6}$ alkoxyl,

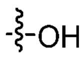 or 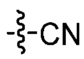,

wherein the $C_{1-6}$ alkyl or $C_{1-6}$ alkoxyl is optionally substituted by one or more deuterium, halogen or cyano;
where in the definitions of $R^4$, $R^5$, $R^7$, $R^8$ and $R^{12}$ are consistent with those defined in the compound of Formula I;
preferably, the compound of Formula IV has a structure shown in the following IV-1:

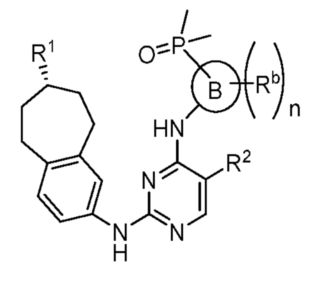

IV-1

wherein the definitions of $R^1$, $R^2$, $R^b$, n and ring B are consistent with those defined in the compound of Formula IV;
preferably, the compound of Formula IV has a structure shown in the following Formula V:

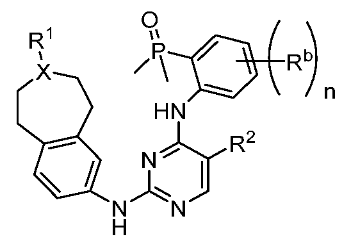

V

wherein the definitions of X, $R^1$, $R^2$, $R^b$ and n are consistent with those defined in the compound of Formula IV; preferably, the compound of Formula IV has a structure shown in the following Formula VI, or a pharmaceutically acceptable salt thereof:

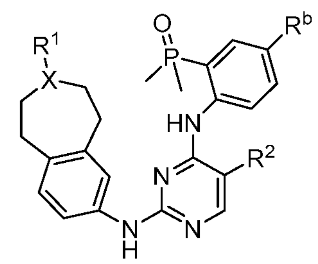

VI

wherein the definitions of X, $R^1$, $R^2$ and $R^b$ are consistent with those defined in the compound of Formula IV.

**9.** The following compounds or pharmaceutically acceptable salts thereof:

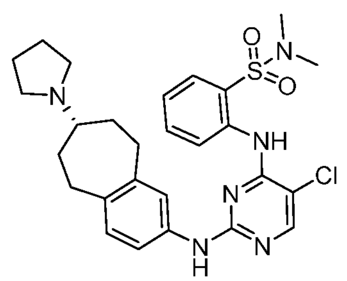
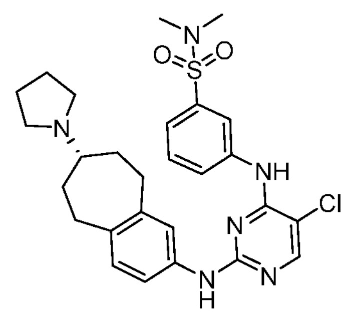
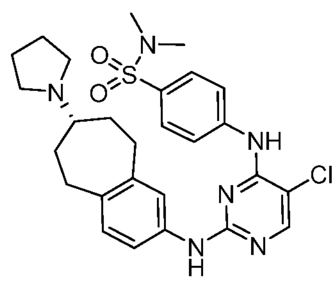

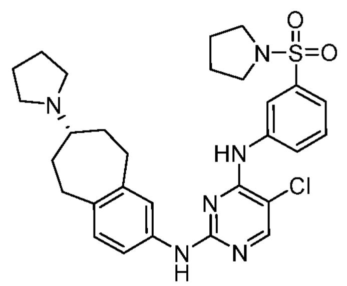
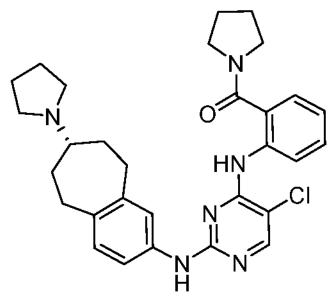
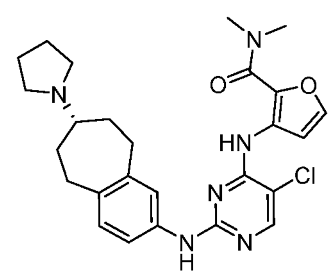

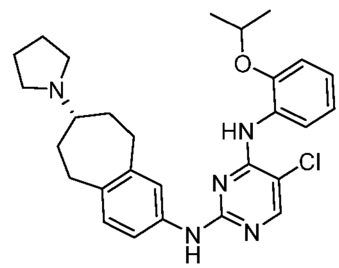
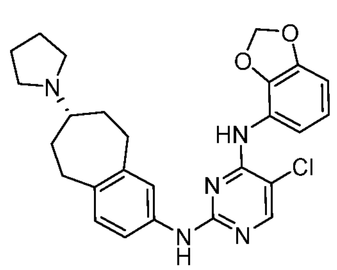
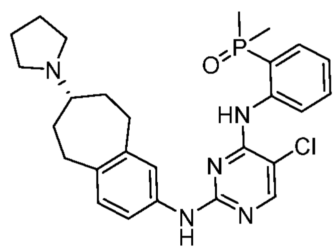
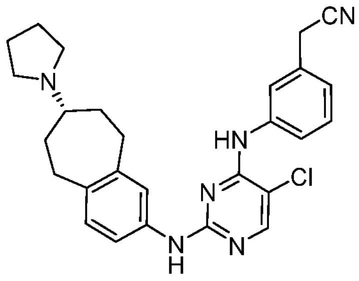
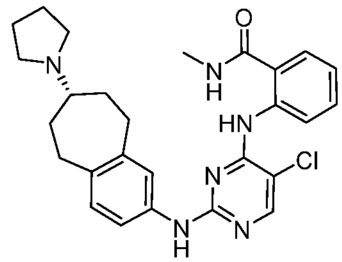
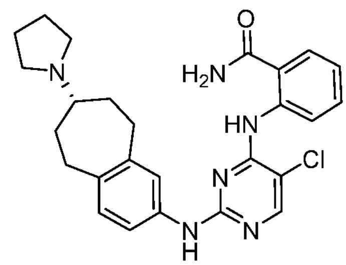
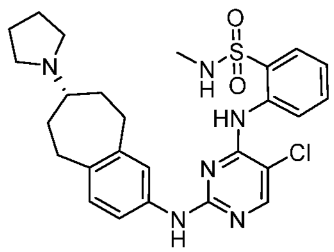
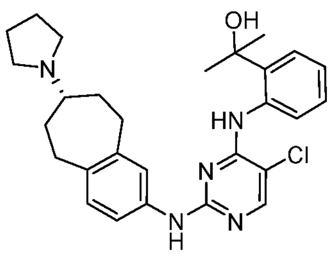
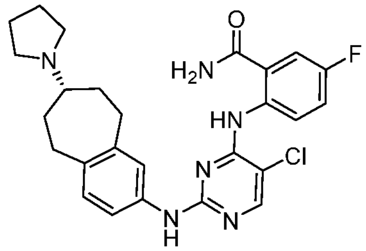
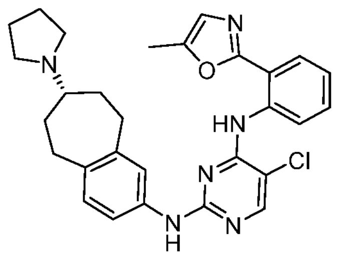
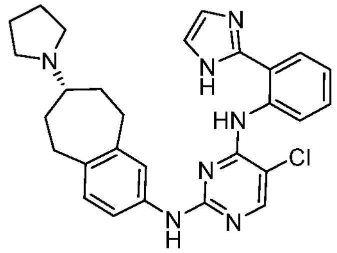
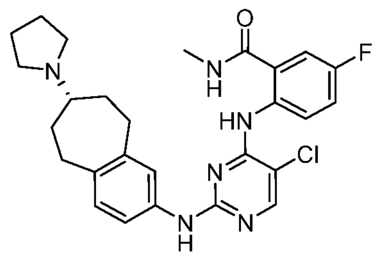
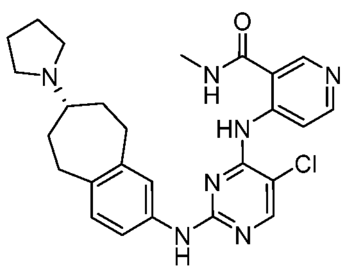
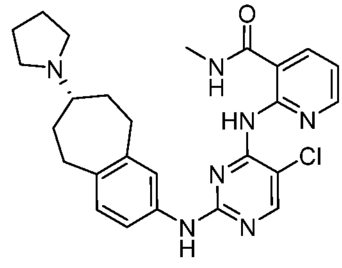
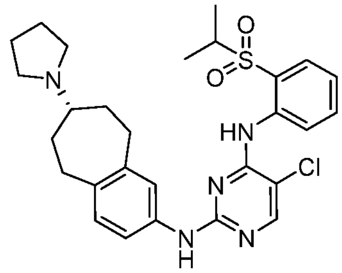

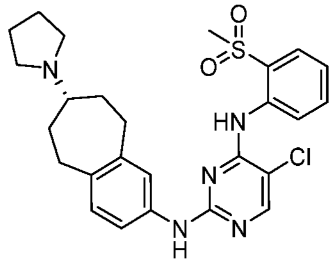
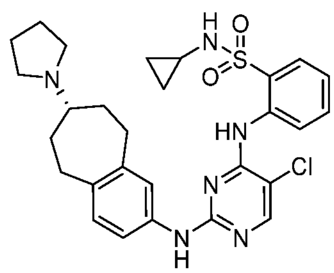
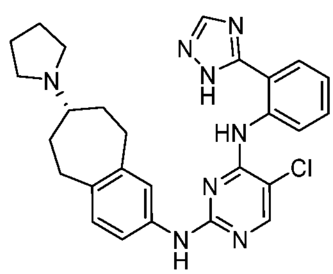

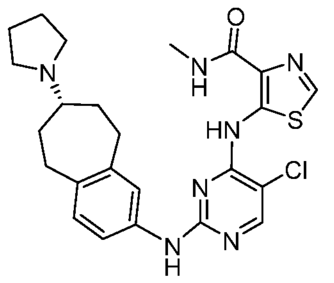
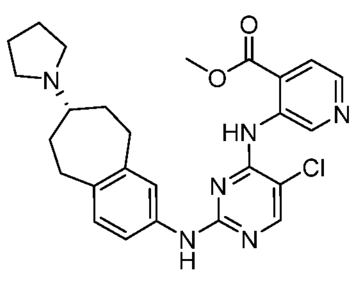
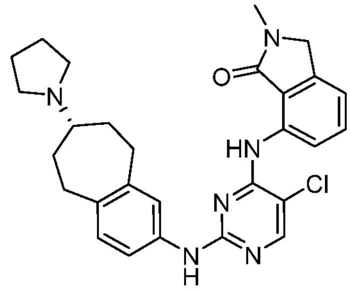

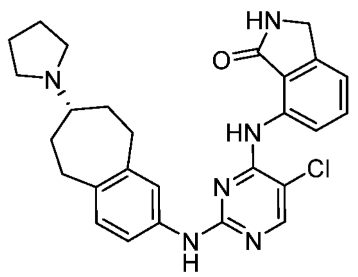
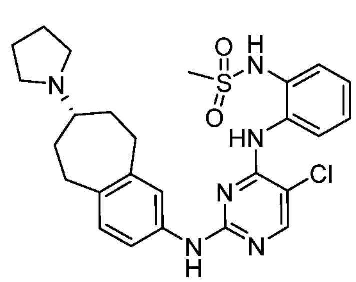
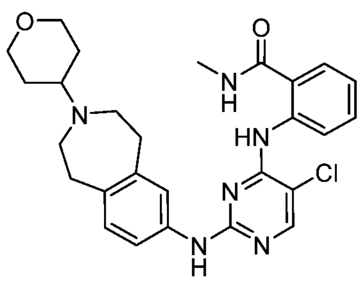

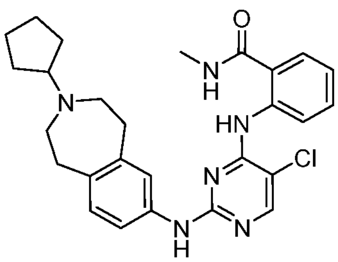
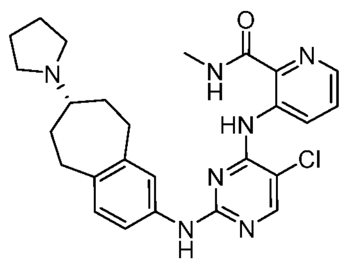
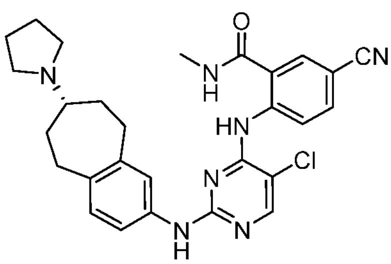

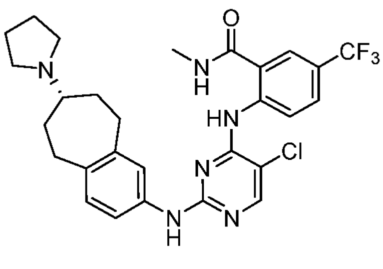
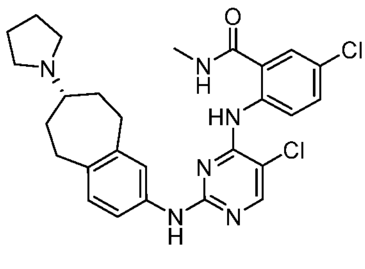
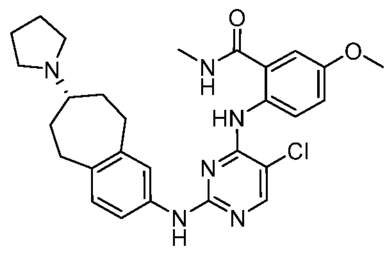

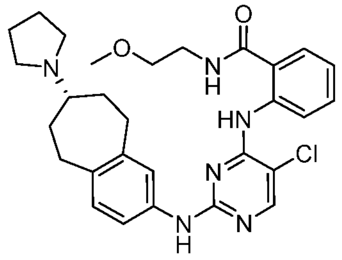
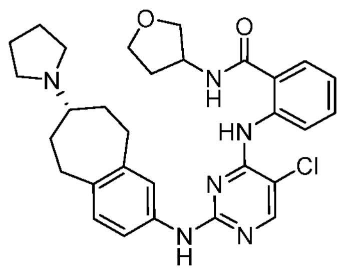
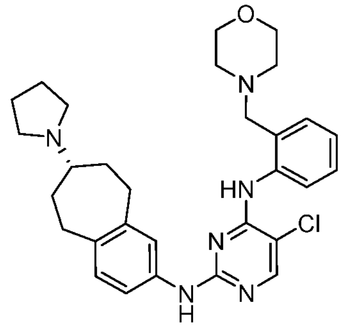

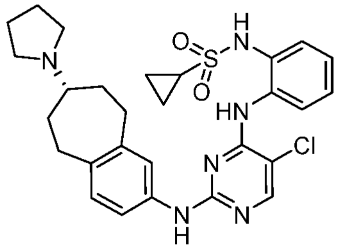
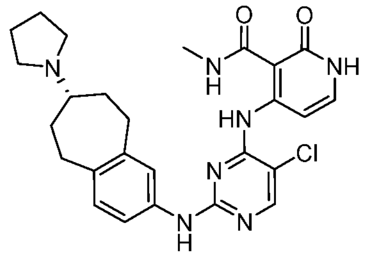
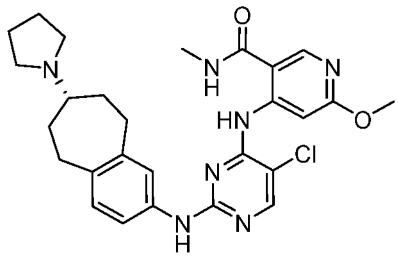

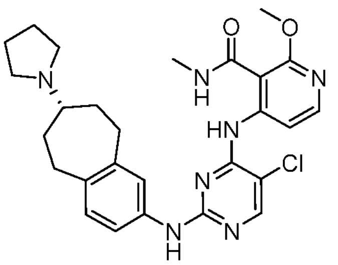
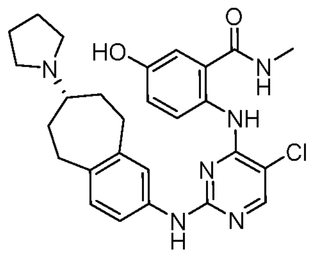
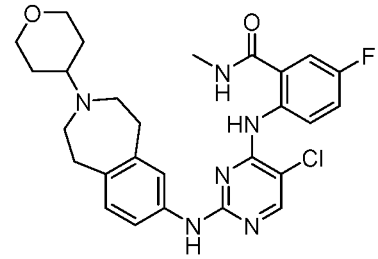

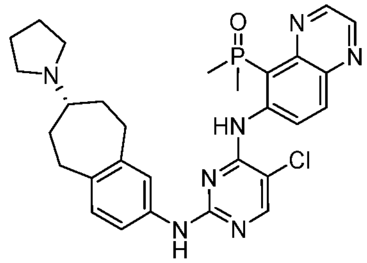
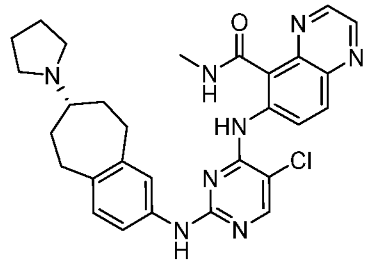
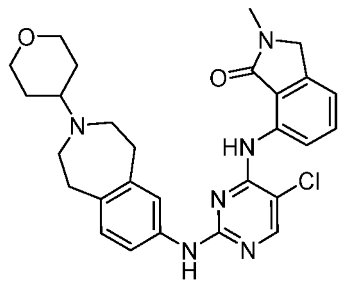

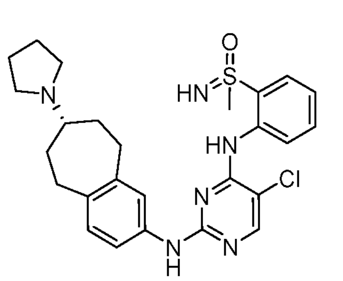
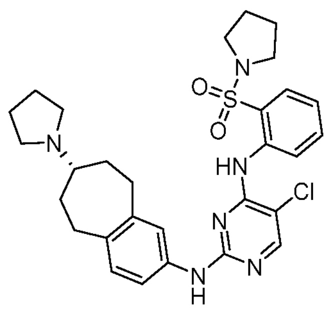
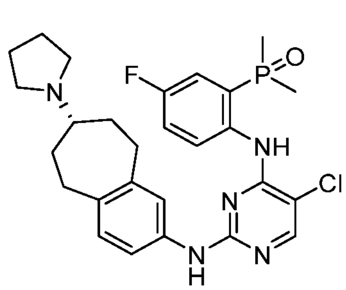

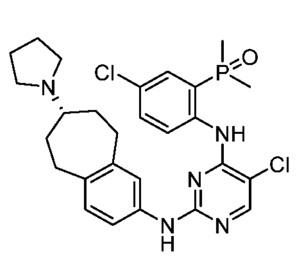
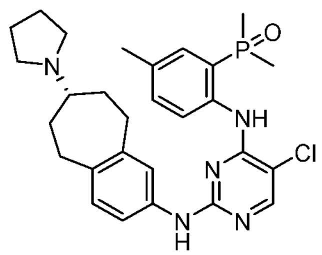
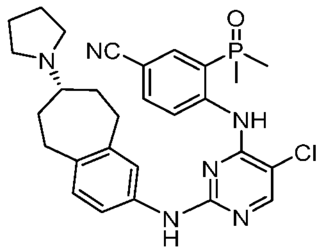

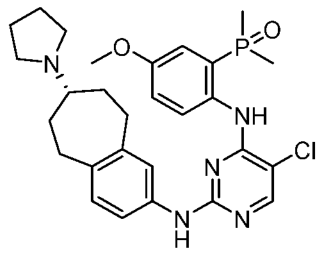
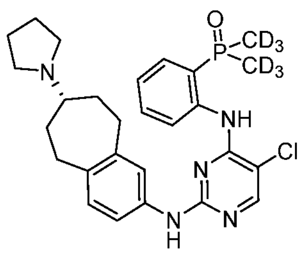
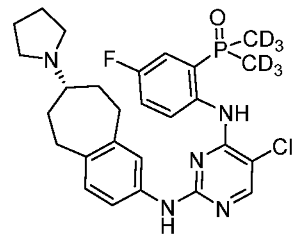

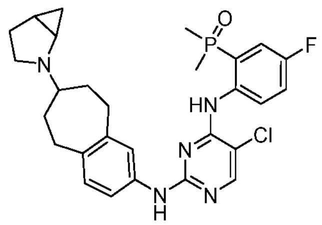
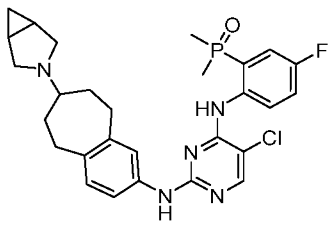
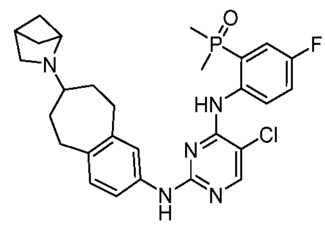

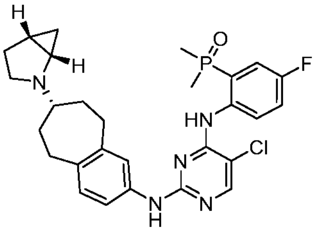
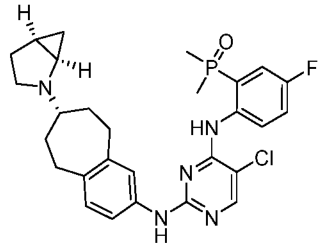
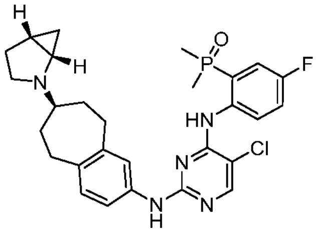

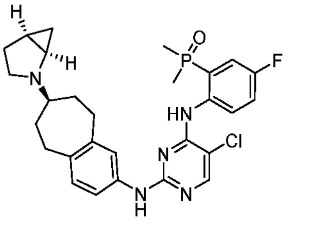
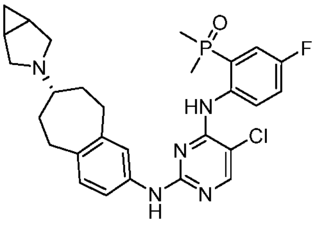
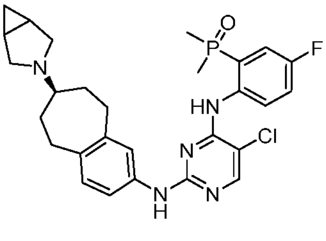

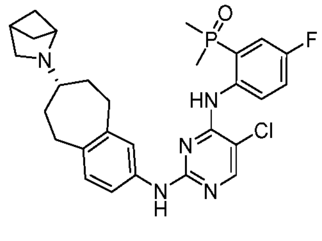
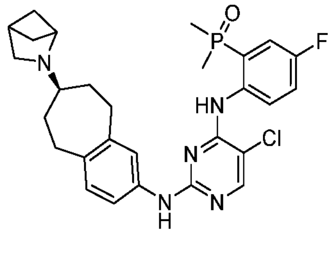
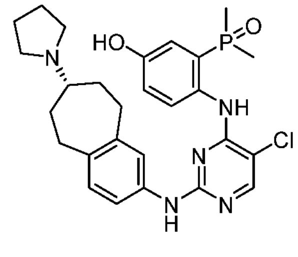

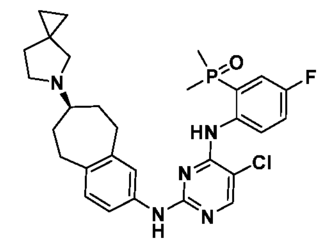
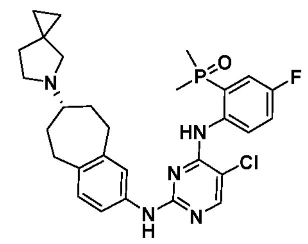
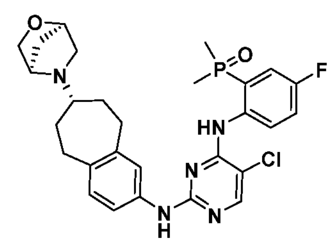

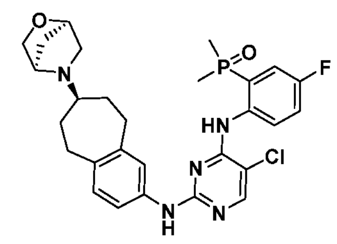
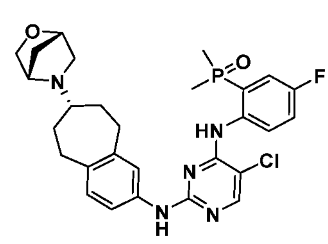
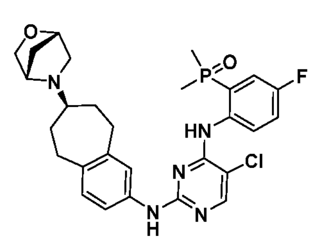

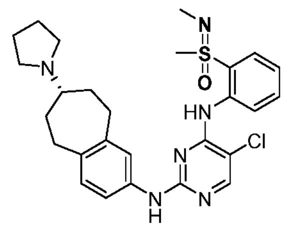
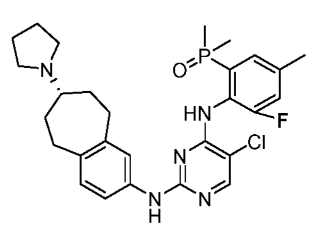
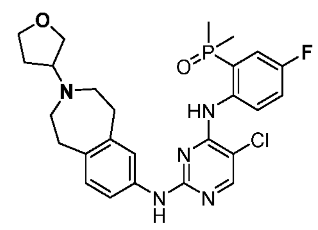

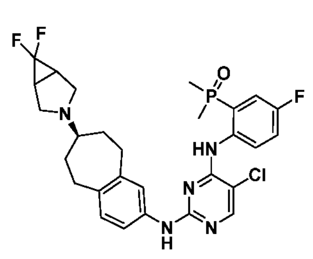
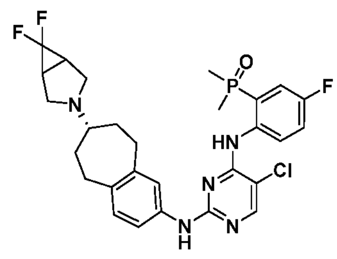
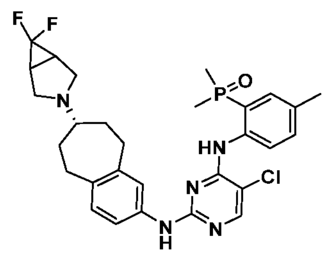

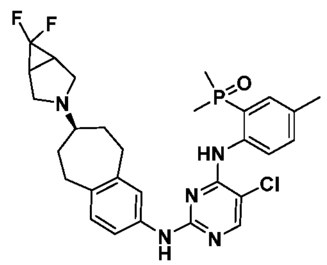
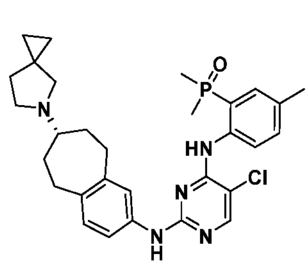
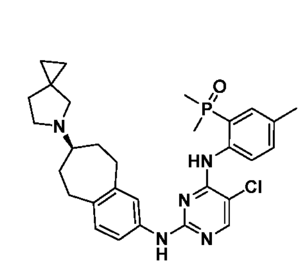

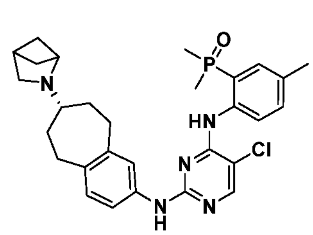
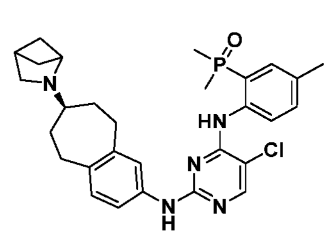
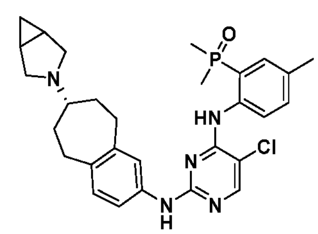

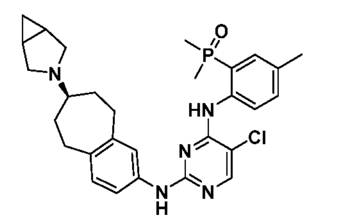
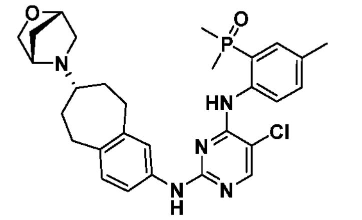
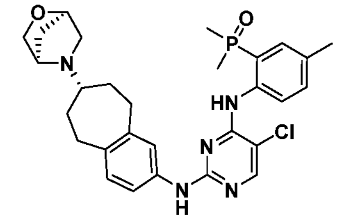
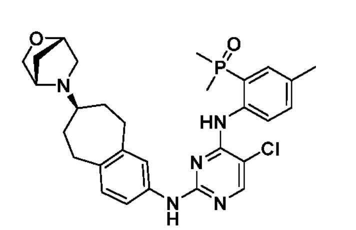
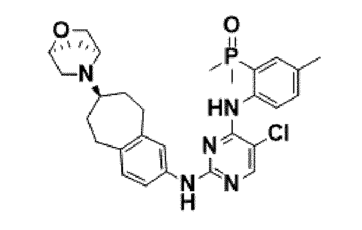
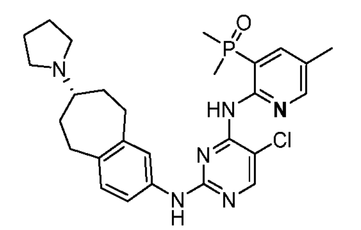
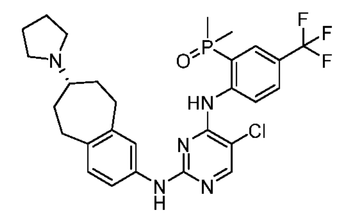
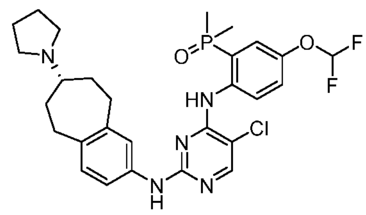
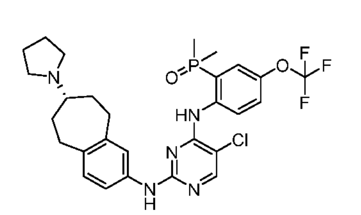
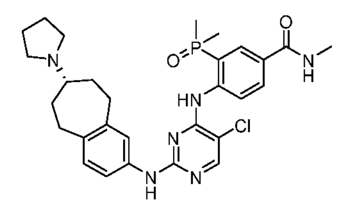
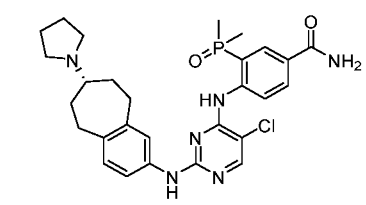
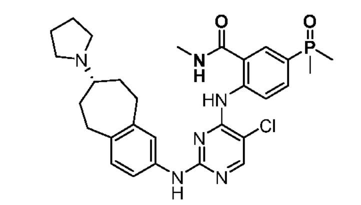
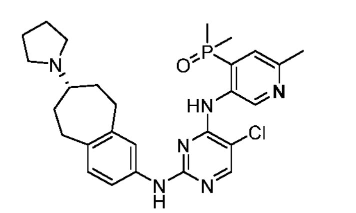
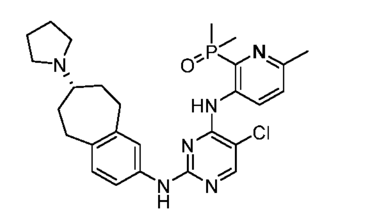
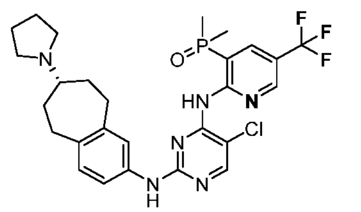
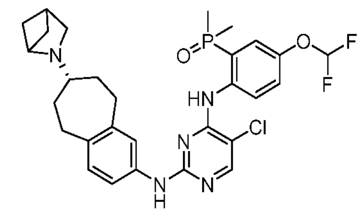
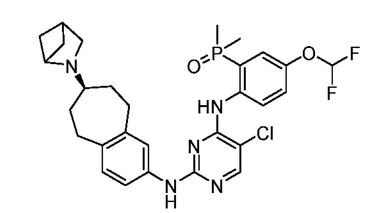
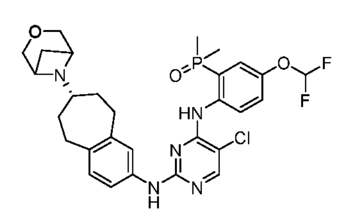
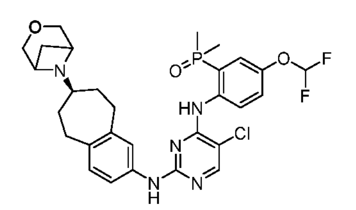
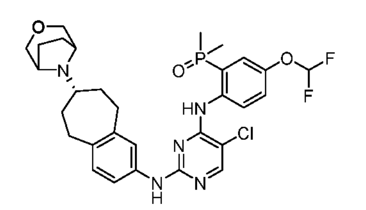
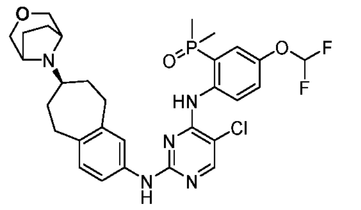

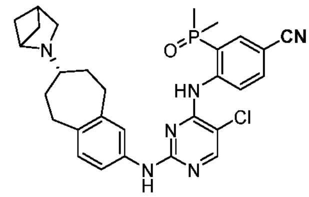
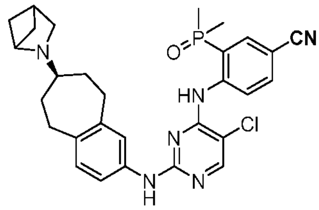
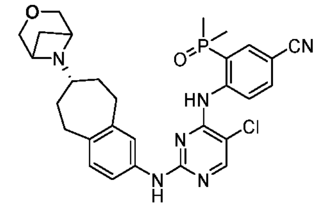
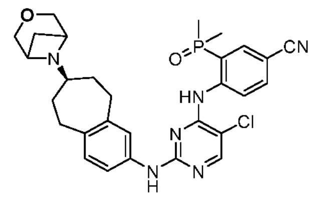
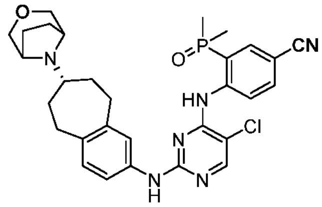
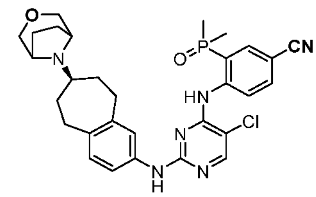
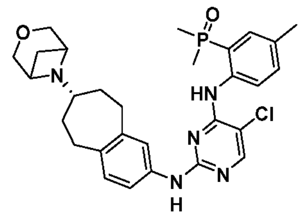
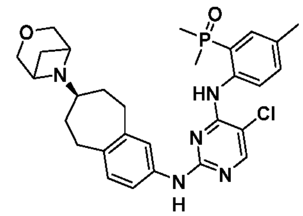
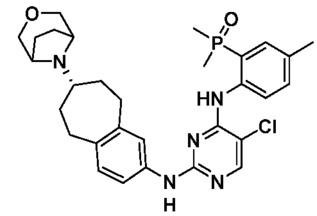
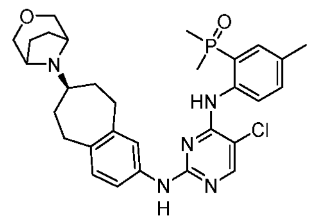
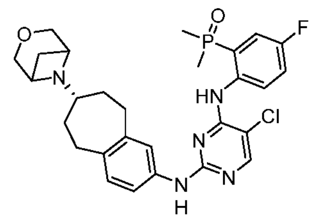
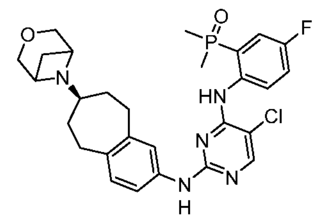
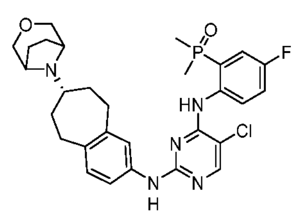
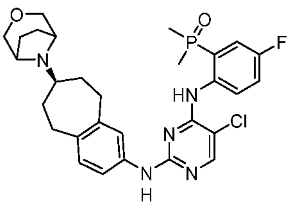
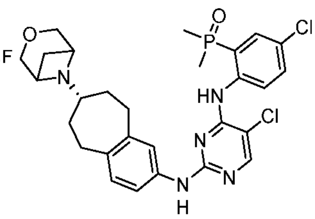
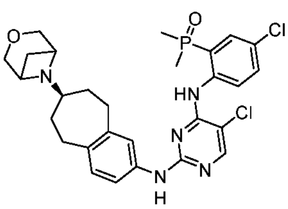
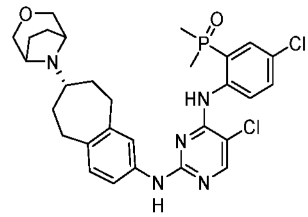
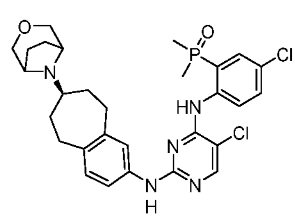
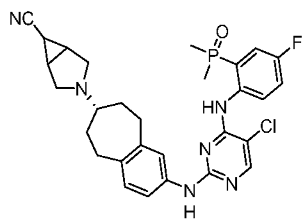
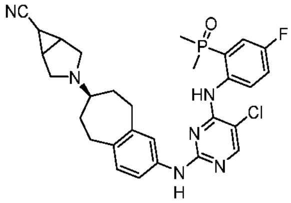
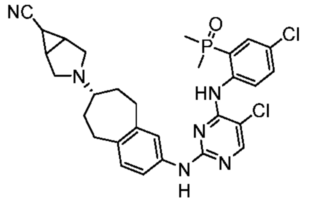
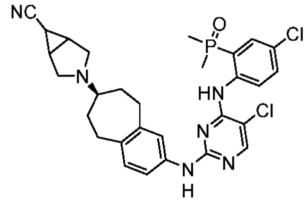

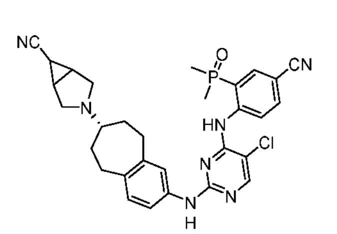
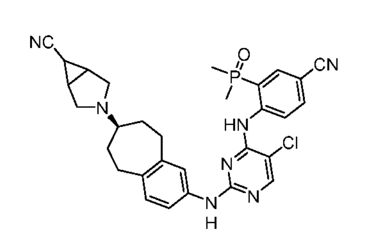
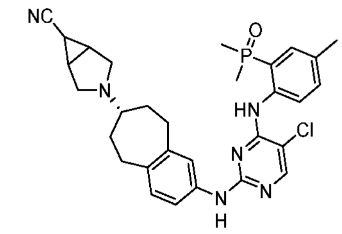
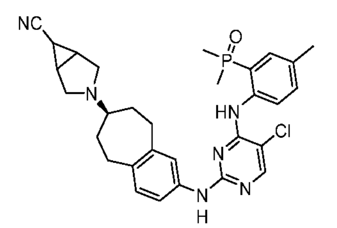
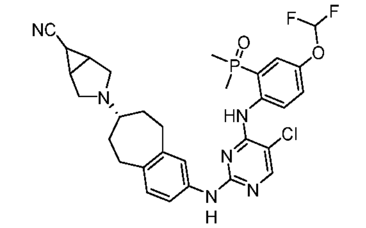
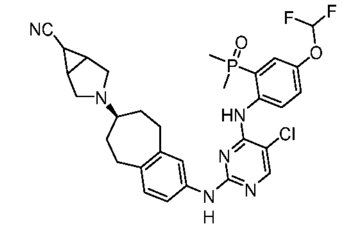
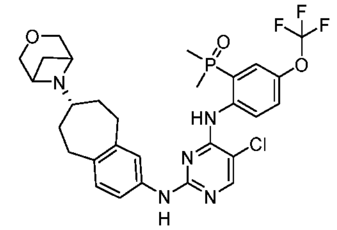
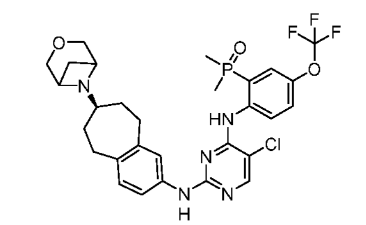
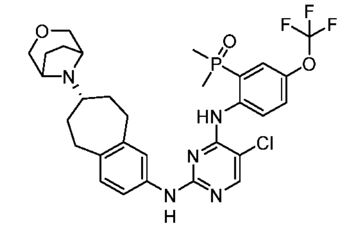
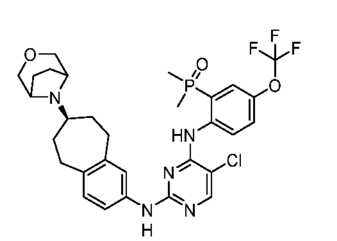
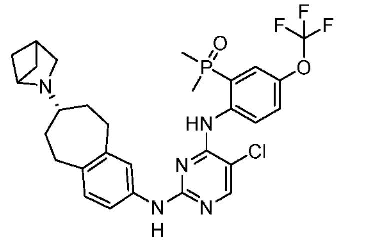
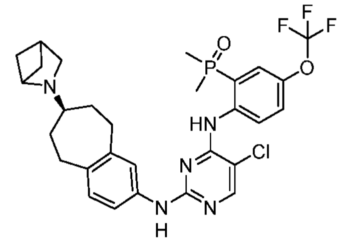
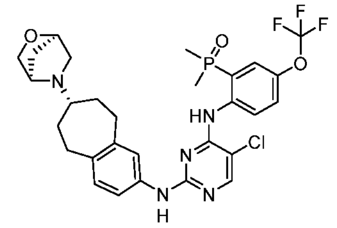
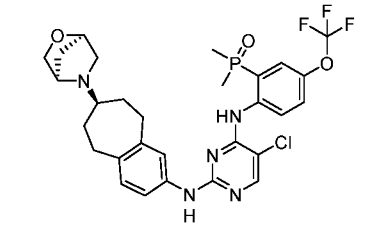
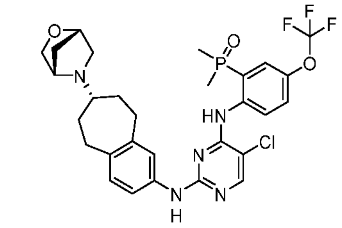
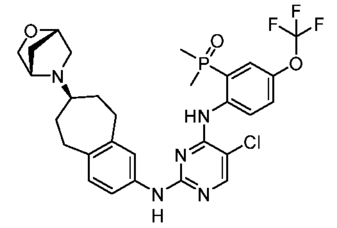
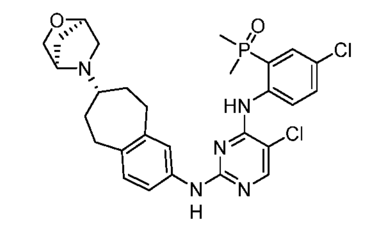
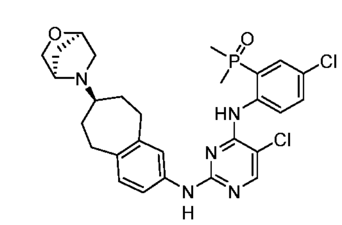

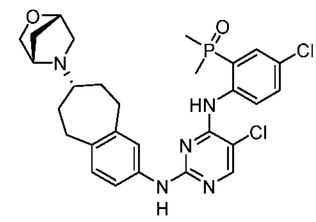
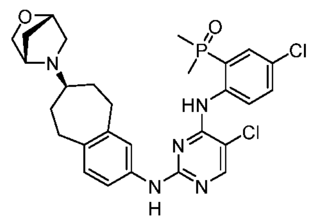
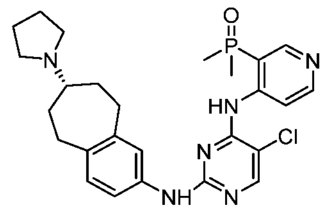

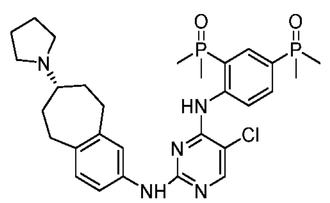
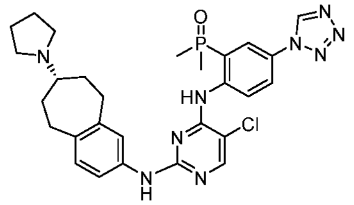
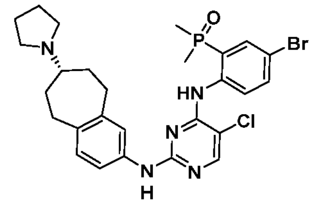

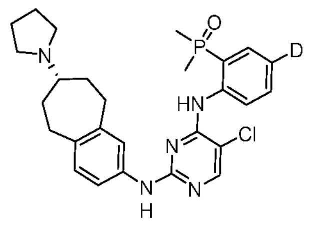
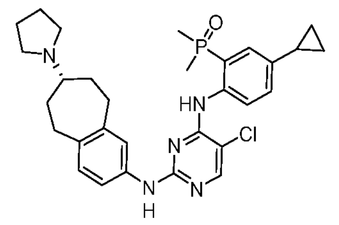
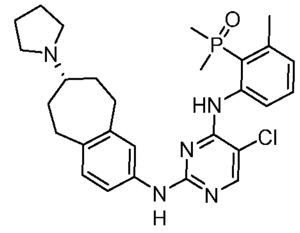

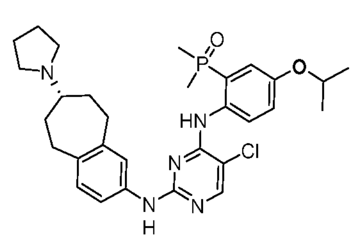
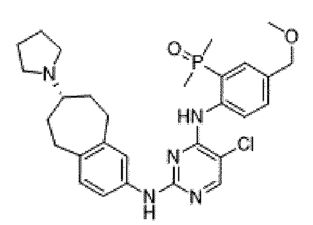
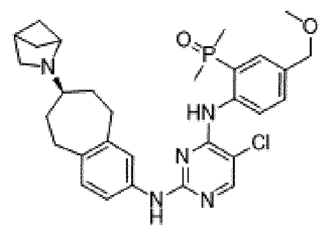

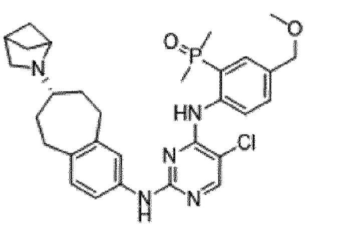
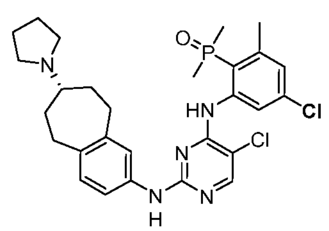
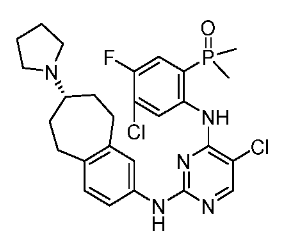

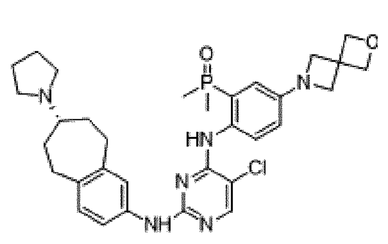
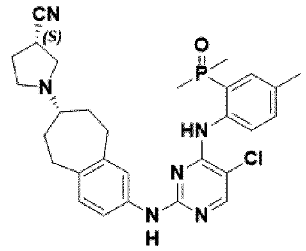
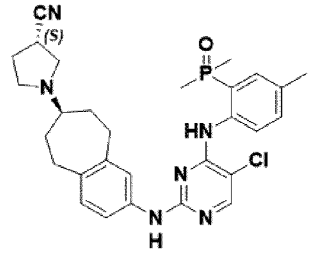

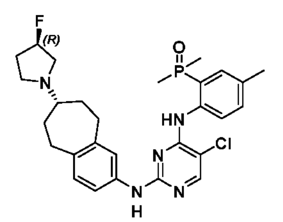
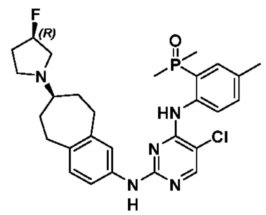
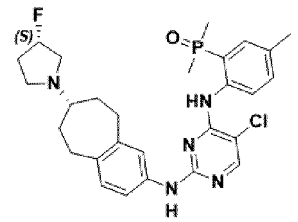
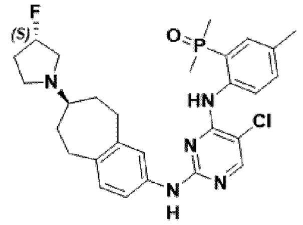
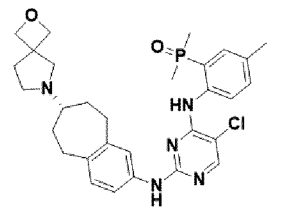
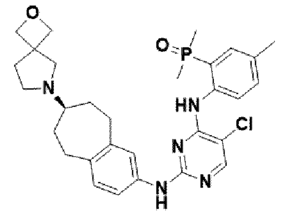
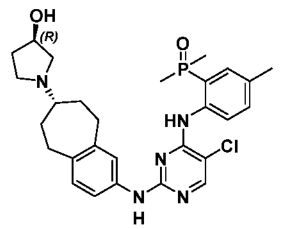
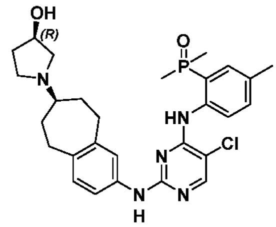
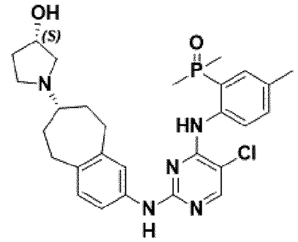
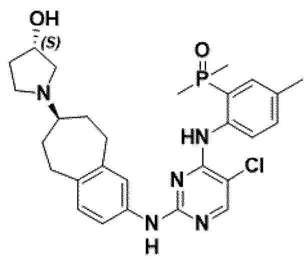
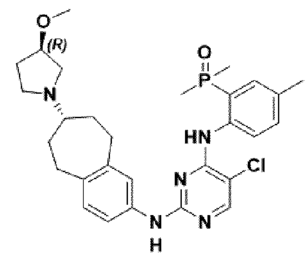
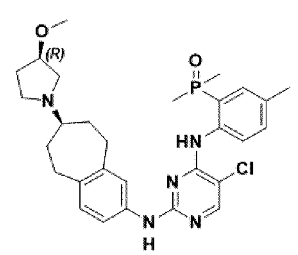
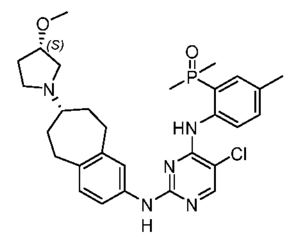
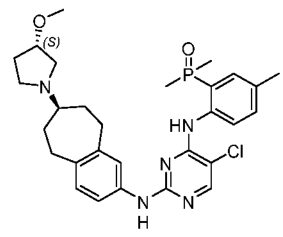
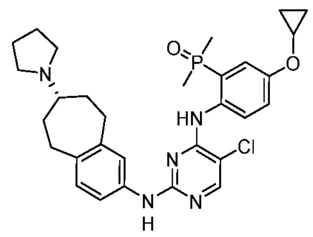
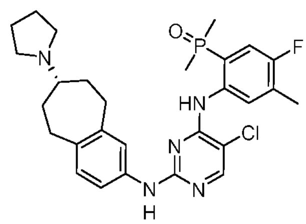
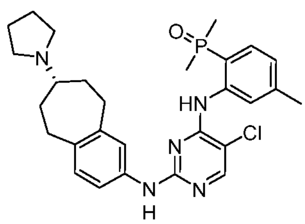
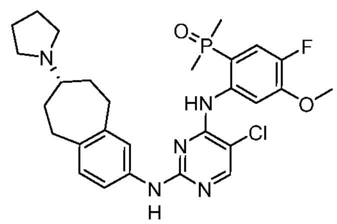

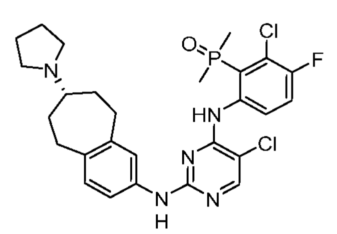

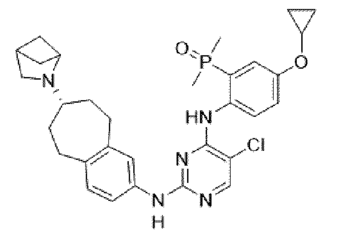

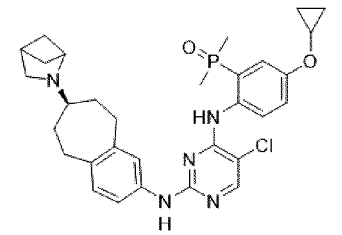

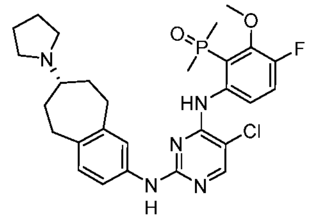

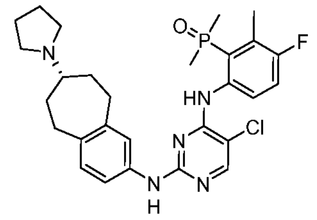

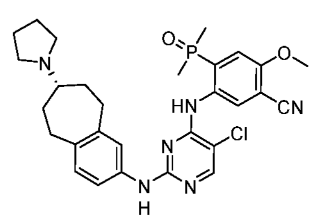

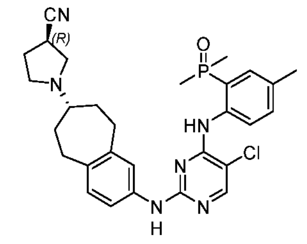

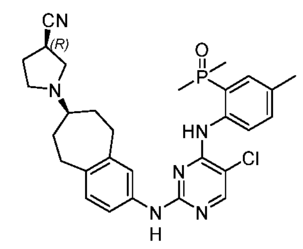

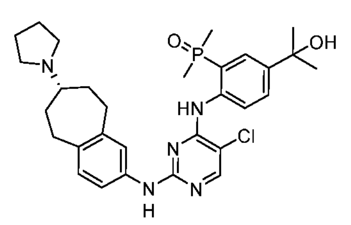

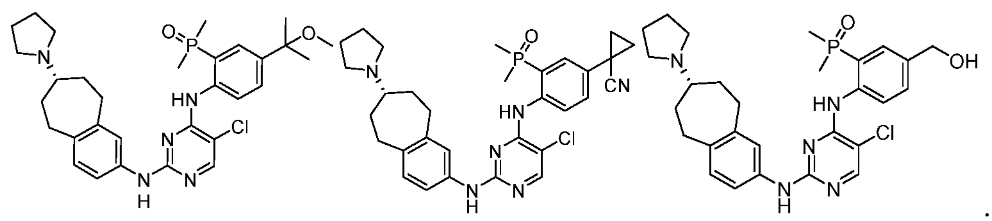

.

**10.** A method for preparing a compound of Formula I, comprising the following step: synthesis scheme 1:

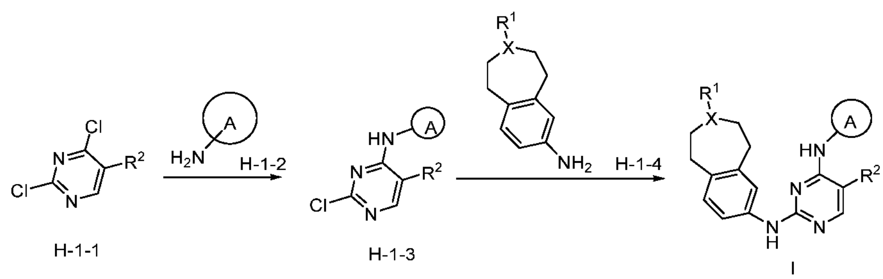


wherein the definitions of $R^1$, $R^2$, X and ring A are consistent with those defined in Formula I of general formulas; and

a compound of Formula H-1-3 is prepared from a compound of Formula H-1-1 and a compound of Formula H-1-2 in the presence of a solvent and an alkaline, and the formula of Formula I is prepared from a compound of formula H-1-3 and a compound of Formula H-1-4 in the presence of a solvent and an acid.

**11.** A method for preparing a compound of Formula 1-1, II-1, III-1 or IV-1, comprising the following step: synthesis scheme 2:

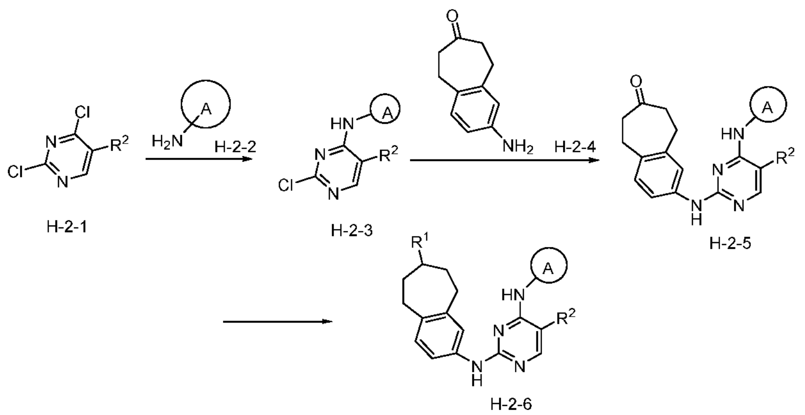


wherein the definitions of $R^2$ and ring A are consistent with those defined in Formula 1-1 of general formulas, and the definition of $R^1$ is consistent with that defined in general formula I;

a compound of Formula H-2-3 is prepared from a compound of Formula H-2-1 and a compound of Formula H-2-2 in the presence of a solvent and an alkaline, a compound of Formula H-2-5 is prepared by the reaction of a compound of Formula H-2-3 and a compound of Formula H-2-4 in the presence of a solvent and an acid, and a compound of Formula H-2-6 is obtained by reacting a compound of Formula H-2-5 with $R^1H$ or an acid addition salt thereof in the presence of a solvent and a reducing agent; and optionally, an optically pure target product is prepared by chiral resolution.

**12.** A pharmaceutical composition, comprising a therapeutically effective amount of the compound of Formula I, I-1, I-2, II, II-1, III, III-1, IV, IV-1, V, or VI or a pharmaceutically acceptable salt thereof, wherein preferably, the pharmaceutical composition further comprises one or more pharmaceutically acceptable carriers; preferably, the pharmaceutical composition is administered orally, for example, the pharmaceutical composition is in a form of tablets, capsules or a solution; and preferably, the drug is administered parenterally, for example, the pharmaceutical composition is a sterile aqueous solution, suspension or lyophilized powder.

**13.** A use of a compound of Formula I, 1-1, I-2, II, II-1, III, III-1, IV, IV-1, V, or VI or a pharmaceutically acceptable salt thereof in the preparation of a drug for preventing and/or treating diseases or disease conditions mediated by AXL protein kinase; and preferably, the diseases or disease conditions mediated by AXL protein kinase include autoimmune diseases.

**14.** A method for preventing and/or treating diseases or disease conditions mediated by AXL protein kinase, comprising: administering a compound of Formula I, I-1, I-2, II, II-1, III, III-1, IV, IV-1, V, or VI or a pharmaceutically acceptable salt thereof, or the pharmaceutical composition according to claim 12 to an individual in need; and preferably, the diseases or disease conditions mediated by the AXL protein kinase include autoimmune diseases.

| INTERNATIONAL SEARCH REPORT | International application No. PCT/CN2021/096942 |
|---|---|

**A. CLASSIFICATION OF SUBJECT MATTER**

C07D 403/00(2006.01)i; C07D 403/14(2006.01)i; C07D 405/14(2006.01)i; C07D 417/14(2006.01)i; C07D 401/14(2006.01)i; C07D 413/00(2006.01)i; A61K 31/506(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D403/-, C07D405/-, C07D417/-, C07D401/-, C07D413/-, A61K31/506, A61P35/-

Documentation searched other than minimum documentation to the extent that such documents are included in the fields searched

Electronic data base consulted during the international search (name of data base and, where practicable, search terms used)

DWPI, SIPOABS, CNABS, CNKI; STN: REGISTRY, CAPLUS, MARPAT; Axl, 受体酪氨酸激酶, RTK, UFO, ARK, Tyro7, 抑制, inhibit+, 苯并, 烯, 嘧啶, 氨, pyrimidin+, benzo+, benzocyclohepten, structural formula search according to formula I

**C. DOCUMENTS CONSIDERED TO BE RELEVANT**

| Category* | Citation of document, with indication, where appropriate, of the relevant passages | Relevant to claim No. |
|---|---|---|
| X | CN 103201280 A (RIGEL PHARMACEUTICALS, INC.) 10 July 2013 (2013-07-10) claims and description, paragraphs [0243]-[-0260], [0278]-[-0320], [0395]-[0409], [0496]-[0497], [0759] | 1-14 |
| X | CN 102356075 A (RIGEL PHARMACEUTICALS, INC.) 15 February 2012 (2012-02-15) abstract, claims, description, compounds IV -12, IV-13 in paragraphs [00131], [01861]-[1864] | 1-14 |
| X | WO 2018102366 A1 (ARIAD PHARMACEUTICALS, INC.) 07 June 2018 (2018-06-07) abstract, claims and description, embodiment 3 | 1-14 |
| A | CN 106458914 A (CHANGZHOU JIEKAI PHARMATECH CO., LTD.) 22 February 2017 (2017-02-22) entire document | 1-14 |
| A | CN 101796046 A (ASTRAZENECA AB) 04 August 2010 (2010-08-04) entire document | 1-14 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **19 August 2021** | **08 September 2021** |
| Name and mailing address of the ISA/CN<br>**China National Intellectual Property Administration (ISA/CN)**<br>**No. 6, Xitucheng Road, Jimenqiao, Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/096942**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **14**
because they relate to subject matter not required to be searched by this Authority, namely:

[1] Although claim 14 relates to a method for treatment of human or animal bodies (PCT Rule 39.1(iv)), a search is still conducted on the basis of the pharmaceutical use of the compound.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2021/096942**

| Patent document cited in search report | Publication date (day/month/year) | Patent family member(s) | Publication date (day/month/year) |
|---|---|---|---|
| CN 103201280 A | 10 July 2013 | AU 2011282742 A2 | 04 April 2013 |
| | | TW I582090 B | 11 May 2017 |
| | | US 2015259332 A1 | 17 September 2015 |
| | | US 9920041 B2 | 20 March 2018 |
| | | KR 101937495 B1 | 10 January 2019 |
| | | US 2017158683 A1 | 08 June 2017 |
| | | US 10479783 B2 | 19 November 2019 |
| | | US RE47396 E | 21 May 2019 |
| | | TW 201209053 A | 01 March 2012 |
| | | US 9067925 B2 | 30 June 2015 |
| | | US 2012028923 A1 | 02 February 2012 |
| | | CA 2804199 A1 | 02 February 2012 |
| | | US 2013090310 A1 | 11 April 2013 |
| | | EP 2598500 A1 | 05 June 2013 |
| | | RU 2015122016 A | 27 December 2016 |
| | | US 8343954 B2 | 01 January 2013 |
| | | US 9611260 B2 | 04 April 2017 |
| | | BR 112013001632 A2 | 24 May 2016 |
| | | CN 103201280 B | 10 August 2016 |
| | | RU 2672100 C2 | 12 November 2018 |
| | | KR 20130132406 A | 04 December 2013 |
| | | CA 2804199 C | 12 May 2020 |
| | | ZA 201300388 B | 25 September 2013 |
| | | US 2020115371 A1 | 16 April 2020 |
| | | WO 2012015972 A1 | 02 February 2012 |
| | | AU 2011282742 B2 | 27 August 2015 |
| | | IL 223855 A | 31 May 2016 |
| | | MX 2013000757 A | 29 April 2013 |
| | | JP 2013536179 A | 19 September 2013 |
| | | MX 347331 B | 21 April 2017 |
| | | AR 082408 A1 | 05 December 2012 |
| | | EA 201390015 A1 | 30 July 2013 |
| | | JP 2017061518 A | 30 March 2017 |
| | | US 2018162848 A1 | 14 June 2018 |
| | | AU 2011282742 A1 | 07 February 2013 |
| | | JP 6073221 B2 | 01 February 2017 |
| CN 102356075 A | 15 February 2012 | EA 022802 B1 | 31 March 2016 |
| | | IL 214208 A | 31 August 2015 |
| | | SI EP2565193 T1 | 31 July 2014 |
| | | DK 2389372 T3 | 14 December 2015 |
| | | US 8563569 B2 | 22 October 2013 |
| | | IL 214208 D0 | 31 August 2011 |
| | | US 8563716 B2 | 22 October 2013 |
| | | DK 2565193 T3 | 16 June 2014 |
| | | HR P20140454 T1 | 20 June 2014 |
| | | SI 2389372 T1 | 29 February 2016 |
| | | US 2013310364 A1 | 21 November 2013 |
| | | PT 2389372 E | 07 January 2016 |
| | | SM T201400071 B | 07 July 2014 |
| | | EP 2565193 B1 | 19 March 2014 |

Form PCT/ISA/210 (patent family annex) (January 2015)

**INTERNATIONAL SEARCH REPORT**
**Information on patent family members**

International application No.
**PCT/CN2021/096942**

| Patent document cited in search report | Publication date (day/month/year) | Patent family member(s) | Publication date (day/month/year) |
|---|---|---|---|
| | | HU E026315 T2 | 28 June 2016 |
| | | US 2020216431 A1 | 09 July 2020 |
| | | US 2014221352 A1 | 07 August 2014 |
| | | BR PI1006942 A2 | 12 April 2016 |
| | | US 2012046245 A1 | 23 February 2012 |
| | | AU 2010206683 A1 | 11 August 2011 |
| | | HR P20151325 T1 | 01 January 2016 |
| | | HK 1163076 A1 | 07 September 2012 |
| | | US 2015307515 A1 | 29 October 2015 |
| | | US 8324200 B2 | 04 December 2012 |
| | | US 2010190770 A1 | 29 July 2010 |
| | | SG 172885 A1 | 29 August 2011 |
| | | US 2012016131 A1 | 19 January 2012 |
| | | EP 2389372 B1 | 09 September 2015 |
| | | CA 2749217 A1 | 29 July 2010 |
| | | PT 2565193 E | 05 June 2014 |
| | | PL 2565193 T3 | 31 July 2014 |
| | | SI 2565193 T1 | 31 July 2014 |
| | | PE 20150621 A1 | 07 May 2015 |
| | | SM P201400078 B | 25 February 2016 |
| | | HK 1183023 A1 | 13 December 2013 |
| | | US 9566283 B2 | 14 February 2017 |
| | | EA 201190082 A1 | 28 February 2012 |
| | | EP 2565193 A1 | 06 March 2013 |
| | | ES 2555491 T3 | 04 January 2016 |
| | | WO 2010085684 A1 | 29 July 2010 |
| | | KR 101762967 B1 | 28 July 2017 |
| | | SM T201500308 B | 08 January 2016 |
| | | US 8530656 B2 | 10 September 2013 |
| | | SG 2014005318 A | 28 March 2014 |
| | | MX 2011007750 A | 29 September 2011 |
| | | ES 2473584 T3 | 07 July 2014 |
| | | US 10005767 B2 | 26 June 2018 |
| | | JP 2012515793 A | 12 July 2012 |
| | | CN 102356075 B | 10 June 2015 |
| WO 2018102366 A1 | 07 June 2018 | US 2019382379 A1 | 19 December 2019 |
| | | JP 2020503271 A | 30 January 2020 |
| | | EP 3548479 A1 | 09 October 2019 |
| CN 106458914 A | 22 February 2017 | US 2017137426 A1 | 18 May 2017 |
| | | WO 2015143692 A1 | 01 October 2015 |
| | | CN 106458914 B | 14 January 2020 |
| CN 101796046 A | 04 August 2010 | SV 2010003459 A | 30 April 2010 |
| | | EP 2183242 A2 | 12 May 2010 |
| | | ZA 201000107 A | 29 September 2010 |
| | | ZA 201000107 B | 29 September 2010 |
| | | US 7718653 B2 | 18 May 2010 |
| | | CA 2693880 A1 | 22 January 2009 |
| | | EC SP109958 A | 31 March 2010 |
| | | MX 2010000658 A | 26 March 2010 |
| | | EA 201000101 A1 | 30 August 2010 |

Form PCT/ISA/210 (patent family annex) (January 2015)

**INTERNATIONAL SEARCH REPORT**
**Information on patent family members**

International application No.
**PCT/CN2021/096942**

| Patent document cited in search report | Publication date (day/month/year) | Patent family member(s) | | | Publication date (day/month/year) |
|---|---|---|---|---|---|
| | | CO | 6260075 | A2 | 22 March 2011 |
| | | AU | 2008277446 | A1 | 22 January 2009 |
| | | KR | 20100042272 | A | 23 April 2010 |
| | | CR | 11220 | A | 20 May 2010 |
| | | BR | PI0814821 | A2 | 03 February 2015 |
| | | JP | 2010533700 | A | 28 October 2010 |
| | | US | 2009023719 | A1 | 22 January 2009 |
| | | DO | P2010000022 | A | 15 February 2010 |
| | | NI | 201000011 | A | 07 December 2010 |
| | | WO | 2009010789 | A3 | 07 May 2009 |
| | | WO | 2009010789 | A2 | 22 January 2009 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202010471712
- CN 202110003579

### Non-patent literature cited in the description


- **ROBINSON, DR et al.** *Oncogene,* 2000, vol. 19, 5548-5557
- **SEGALINY, A.I. et al.** *J. Bone Oncol,* 2015, vol. 4, 1-12
- **LINGER, RM et al.** *Ther. Targets,* 2010, vol. 14 (10), 1073-1090
- **RESCIGNO, J. et al.** *Oncogene,* 1991, vol. 6 (10), 1909-1913
- **WU YM ; ROBINSON DR ; KUNG HJ.** *Cancer Res,* 2004, vol. 64 (20), 7311-7320
- **HUNG BI et al.** *DNA Cell Biol,* 2003, vol. 22 (8), 533-540